# EUROPEAN PATENT APPLICATION

(11) **EP 4 385 539 A2**
(43) Date of publication of application: **19.06.2024**
(21) Application number: 24150063.6
(22) Date of filing: 20.02.2015
(51) Int. Cl.: A61M 5/142

(54) **SYRINGE PUMP HAVING A PRESSURE SENSOR ASSEMBLY**

(30) Priority: 21.02.2014 US 201461942986 P; 08.05.2014 US 201461990330 P
(62) Divisional of application: 19206074.7
(71) Applicant: DEKA Products Limited Partnership, Manchester, NH 03101 (US)
(72) Inventor: DESCH, Martin D, Newburyport, 01950 (US); GRAY, Larry B, Merrimack, 03054 (US); BODWELL, Jesse T, Manchester, 03109 (US); VAN DER MERWE, Dirk A., Canterbury, 03224 (US)
(74) Representative: Haseltine Lake Kempner LLP

(57) **Abstract**

There is described a pressure sensor assembly, comprising: a plunger having a sensing surface; a first pressure sensor operatively coupled to the plunger and configured to estimate a force applied to the sensing surface; and a second pressure sensor operatively coupled to the plunger and configured to estimate the force applied to the sensing surface

## Description

### BACKGROUND

### Relevant Field

The present disclosure relates to pumps. More particularly, the present disclosure relates to a system, method, and apparatus for estimating liquid delivery of a syringe pump.

### Description of Related Art

Syringe pumps are used in a variety of medical applications, such as for intravenous delivery of liquid medications, for example a patient in an intensive-care unit (ICU), for an extended length of time. Syringe pumps may be designed so that needles, tubing, or other attachments are attachable to the syringe pump. Syringe pumps typically include a plunger mounted to a shaft that pushes a liquid out of a reservoir. The reservoir may be a tube-shaped structure having a port at one end such that the plunger can push (i.e., discharge) the liquid out of the syringe pump. Syringe pumps can be coupled to an actuator that mechanically drives the plunger to control the delivery of liquid to the patient.

Syringe pumps may also be used to deliver various drugs including analgesics, antiemetics, or other fluids. The medication may be administered via an intravenous liquid line very quickly (e.g., in a bolus) or over a length of time. Syringe pumps may also be used in non-medical applications, such as in microreactors, in laboratory testing, and/or in chemical processing applications.

### SUMMARY

In accordance with one embodiment of the present disclosure, a pump for administering an agent to a patient may comprise a housing. Within said housing may be a motor, a gearbox operatively connected to said motor, a means for sensing rotation of said motor, a controller acting to control operation of said motor and monitor the quantity of said agent delivered to said patient, and a pump assembly. The pump may be configured such that the pump is interchangeable from a syringe pump or peristaltic pump respectively to a peristaltic pump or syringe pump via supplanting one pump assembly with a differing pump assembly.

In some embodiments, the pump may be field interchangeable from a syringe pump or peristaltic pump respectively to a peristaltic pump or syringe pump via supplanting one pump assembly with a differing pump assembly.

In accordance with another embodiment of the present disclosure, a syringe pump for administering an agent to a patient may comprise a housing, a lead screw, and a sliding block assembly. The sliding block assembly may comprise a cam, a cam projection fixedly coupled to the cam, and a threaded portion capable of engaging and disengaging from the lead screw. The threaded portion may be configured to be actuated between engagement and disengagement on the lead screw via rotation of the cam and cam projection.

In some embodiments, the sliding block assembly may comprise a slot with a straight expanse and an acruated expanse.

In some embodiments, rotation of the cam may cause the cam projection to move within the slot. As the cam projection moves within the straight expanse of the slot, the threaded portion may be configured to be actuated between engagement and disengagement with the lead screw.

In some embodiments, the syringe pump may further comprise a clamping means configured for clamping any of a range of plunger flange sizes.

In some embodiments, the cam projection may not enter the straight expanse of the slot until the largest of the range of plunger flange sizes has been released by the means configured for clamping any of a range of plunger flange sizes.

In some embodiments, the syringe pump may further comprise a plunger head assembly coupled to the sliding block and operative to drive a plunger of a syringe into a barrel of the syringe. A plunger tube may couple the plunger head assembly to the sliding block.

In some embodiments, the plunger tube may perform at least one or more additional functions from a list consisting of: a bushing support for at least one rotating shaft, a channel for electrical conduits to and from the plunger head assembly, and a channel for data transmission conduits to and from the plunger head assembly.

In some embodiments, the syringe pump may further comprise a barrel flange clip configured to retain a barrel flange of a syringe.

In some embodiments, the barrel flange clip may comprise a means of detecting the presence of a barrel flange. The means of detecting the presence of a barrel flange may comprise an optical sensor and a light source. The light source may be obscured in the presence of the barrel flange.

In some embodiments, the location of the cam of the sliding block assembly may be adjustable such that a user may optimize engagement of the threaded portion on the lead screw.

In some embodiments, the sliding block assembly may further include at least one bias member. The bias member may be configured to bias the threaded portion to one of an engaged position on the lead screw and a disengaged position on the lead screw.

In accordance with another aspect of the present disclosure, a syringe pump for administering an agent to a patient may comprise a housing, a lead screw, and a sliding block assembly. The sliding block assembly may comprise a threaded section configured for engaging and disengaging from the lead screw. The syringe pump may further comprise a plunger head assembly coupled to said sliding block and operative to drive a plunger of a syringe into a barrel of said syringe. The syringe pump may further comprise a clamping means configured for clamping any of a range of plunger flange sizes. The means configured for clamping any of a range of plunger flange sizes may comprise at least a first plunger flange clamp jaw and a second plunger flange clamp jaw. The first and second plunger flange clamp jaws may be configured to be actuated from a first position to a position in which at least one point of each of the first and second plunger flange clamp jaws abut an edge of the plunger flange forcing the plunger flange against the plunger head assembly and acting as an anti-siphon mechanism.

In some embodiments, the means configured for clamping any of a range of plunger flange sizes may comprise a cam, at least one cam follower, and at least one bias member. The bias member may bias said means configured for clamping any of a range of plunger flange sizes toward a first position. In some embodiments, movement of the at least one cam follower along the cam may overcome the bias member and allow the means configured for clamping any of a range of plunger flange sizes to move toward a second position.

In some embodiments, the cam, at least one cam follower, and at least one bias member may be coupled to a rotatable shaft. The cam may not be rotatable with said shaft but may be displaceable along an axial dimension of said shaft. The at least one cam follower may be fixedly coupled to the shaft and rotatable with the shaft. Rotation of the shaft may cause movement of the at least one cam follower along the cam thereby displacing the cam along the axial dimension of the shaft.

In some embodiments, the bias member may automatically return the means configured for clamping any range of plunger flange sizes to the first position in the absence of a force sufficient to overcome the bias member.

In some embodiments, the cam may comprise at least one detent, each of said detents being reached by one of the at least one cam followers when the means configured for clamping any range of plunger flange sizes has been allowed to move to the second position.

In some embodiments, the plunger head assembly may further comprise a pressure sensor for monitoring the pressure of the agent being dispensed from the syringe.

In some embodiments, the plunger flange of the syringe may be held against the pressure sensor by the means configured for clamping any range of plunger flange sizes.

In some embodiments, the syringe pump may further comprise a barrel flange clip. The barrel flange clip may be configured to retain a barrel flange of the syringe.

In some embodiments, the barrel flange clip may comprise a means of detecting the presence of a barrel flange. The means of detecting the presence of a barrel flange may comprise an optical sensor and a light source. The light source may be obscured in the presence of said barrel flange.

In accordance with another aspect of the present disclosure a syringe pump for administering an agent to a patient may comprise a housing a lead screw and a sliding block assembly. The sliding block assembly may comprise a threaded section configured for engagement and disengagement with said lead screw and movable along said lead screw. The syringe pump may further comprise a plunger head assembly coupled to said sliding block assembly and operative to drive a plunger of a syringe into a barrel of said syringe. The syringe pump may further comprise a clamping means configured for clamping any of a range of plunger flange sizes. The syringe pump may further comprise a means of monitoring the clamping means. The means of monitoring the clamping means may be capable of generating data to determine at least one characteristic of the clamped syringe.

In some embodiments, the means of monitoring the clamping means may be a potentiometer.

In some embodiments, the data generated by the means of monitoring the clamping means may be evaluated by referencing said data against a database.

In some embodiments, the data generated by the means of monitoring the clamping means may be evaluated by referencing said data against a database and data generated by at least one other sensor.

In some embodiments, the clamping means may comprise a cam, at least one cam follower, and at least one bias member. The bias member may bias said clamping means toward a first position. Movement of the at least one cam follower along the cam may overcome the bias member and allow the clamping means to move toward a second position.

In some embodiments, the cam, at least one cam follower, and at least one bias member may be coupled to a rotatable shaft. In some specific embodiments, the cam may not be rotatable with the shaft but may be displaceable along an axial dimension of said shaft. The at least one cam follower may be fixedly coupled to the shaft and rotatable with the shaft. Rotation of the shaft may cause movement of the at least one cam follower along the cam displacing the cam along the axial dimension of the shaft.

In some embodiments, the bias member may automatically return the clamping means to the first position in the absence of a force sufficient to overcome the bias member.

In some embodiments, the cam may comprise at least one detent. Each of the detents may be reached by one of the at least one cam followers when the means for clamping any range of plunger flange sizes has been allowed to move to the second position.

In some embodiments, the plunger head assembly may further comprise a pressure sensor for monitoring the pressure of the agent being dispensed from the syringe.

In some embodiments, a plunger flange of the syringe may be held against the pressure sensor by the clamping means.

In some embodiments, the barrel flange clip may comprise a means of detecting the presence of a barrel flange. The means of detecting the presence of said barrel flange may comprise an optical sensor and a light source. The light source may be obscured in the presence of said barrel flange.

In accordance with another aspect of the present disclosure, a syringe pump for administering an agent to a patient may comprise a housing, a lead screw, and a plunger head assembly operatively coupled to drive a plunger of a syringe into the barrel of a syringe with rotation of said lead screw. The syringe pump may further comprise at least one set of redundant sensors. The redundant sensors may be configured such that if part of a set of redundant sensors is compromised, the syringe pump may function in a fail operative mode for at least the duration of a therapy. One or more of the set of redundant sensors are configured to monitor the volume being infused.

In accordance with another aspect of the present disclosure, a syringe pump for administering an agent to a patient may comprise a housing and a syringe barrel holder which may be movable between a first position and a second position. The syringe barrel holder may be biased by a bias member to either the first position or the second position. The syringe pump may further comprise a syringe barrel contacting member. The barrel contacting member may be coupled to said syringe barrel holder and configured to hold the syringe in place on the housing. The syringe pump may further comprise a detector capable of sensing the position of the syringe barrel holder and generating position data based on the position of the syringe barrel holder. When a syringe is in place on said housing, the syringe barrel holder may be biased such that the syringe is held in place on said housing. The position data generated by said detector may be indicative of at least one characteristic of the syringe and evaluated to determine said characteristic.

In some embodiments the detector may be a linear potentiometer.

In some embodiments, the detector may be a magnetic linear position sensor.

In some embodiments, the syringe barrel holder may be configured to be locked in at least one of the first position and second position.

In some embodiments, the bias member may cause the syringe barrel holder to automatically adjust to the size of the syringe.

In some embodiments, position data generated by the detector may be referenced against a database to determine the at least one characteristic of the syringe.

In some embodiments, the position data generated by the detector may be referenced against a database and data from at least one other sensor to determine the at least one characteristic of the syringe.

In accordance with another aspect of the present disclosure, a method of administering an agent to a patient via a syringe pump may comprise defining one or a number of parameters for an infusion through an interface of the syringe pump. The method may further comprise referencing said parameters against a medical database and placing restrictions on further parameters to be defined through the interface of the syringe pump. One of the further parameters may be an end of infusion behavior to be executed by the syringe pump after a volume to be infused has been infused. The method may further comprise infusing said agent to said patient in accordance with the defined parameters for infusion and executing the specified end of infusion behavior.

In some embodiments, the end of infusion behavior may be selected from a list consisting of: stopping an infusion, infusing at a keep vein open rate, and continuing to infuse at the rate of the finished infusion.

In some embodiments, referencing parameters against a database and placing restrictions on further parameters may comprise referencing the agent against the database.

In accordance with one embodiment of the present disclosure, a syringe pump includes a housing, a syringe seat, and a bumper. The syringe seat is coupled to the housing. The bumper is coupled to the housing adjacent to the syringe seat. The bumper may at least partially surround a corner of the syringe seat.

In another embodiment of the present disclosure, a syringe pump includes a housing, a syringe seat, and a power supply. The syringe seat is coupled to the housing. The power supply is coupled to the housing such that the housing is configured as a heat sink for the power supply. The syringe pump may include a motor, and the motor may be coupled to the housing such that the housing is a heat sink for the motor. The housing may be die casted. The housing may comprise at least one metal and/or may be a unitary body.

In another embodiment of the present disclosure, a syringe pump includes a user interface, an antenna, and a split-ring resonator. The user interface has a front side and a backside. The antenna is disposed on the back side of the user interface. The split-ring resonator is disposed in spaced relation to the user interface and is configured to operate with the antenna.

The user interface may include a touchscreen sensor. The split-ring resonator may be disposed on a backside of the touchscreen sensor. A frame may surround the touchscreen sensor that has a gap such that the frame defines the split-ring resonator. A dielectric may be disposed within the gap.

In another embodiment of the present disclosure, a syringe pump includes a housing, a lead screw, a motor, a rotary position sensor, a sliding block assembly, a linear position sensor, and one or more processors. The lead screw is rotatable within the housing. The motor is operatively coupled to the lead screw and is configured to rotate the lead screw. The motor has an integral motor rotation sensor configured to provide a motor rotation signal. The rotary position sensor is operatively coupled to the motor or the lead screw to provide a rotation signal. The rotary position sensor may be a magnetic encoder sensor. The sliding block assembly is configured to engage with the lead screw to actuate the sliding block assembly along the lead screw in accordance with rotation of the lead screw. The linear position sensor is operatively coupled to the sliding block assembly and is configured to provide a linear position signal. The one or more processors are configured to control rotation of the motor. The one or more processors operatively receive the motor rotation signal from the integral motor rotation sensor of the motor, the rotation signal from the rotary position sensor, and the linear position signal from the linear position sensor. The one or more processors are configured to determine if a discrepancy exists among the motor rotation signal, the rotation signal, and the linear position signal. The one or more processors may be further configured to continue an infusion treatment by ignoring an inoperative one of the integral motor rotation sensor, the rotary position sensor, and a linear position sensor.

In another embodiment of the present disclosure, a syringe pump includes a housing, a lead screw, a sliding block assembly, a plunger, and first and second pivotal jaw members. The lead screw is rotatable within the housing. The sliding block assembly is configured for engaging with the lead screw to move along the lead screw in accordance with rotation of the lead screw. The plunger head assembly is coupled to the sliding block assembly and is configured to drive a plunger of a syringe into a barrel of the syringe. The first and second pivotal jaw members are each pivotally coupled to the plunger head assembly. The first and second pivotal jaw members are configured to pivot toward each other to retain a plunger flange of the syringe. The first pivotal jaw member and/or the second pivotal jaw member includes a bend.

The syringe pump may further include a dial coupled to the sliding block assembly. The dial may be operatively coupled to the first and second pivotal jaw members to pivotally actuate the first and second pivotal jaw members. The pump may include a bias member configured to bias the dial in a direction of rotation. The bias member may be configured to automatically return the first and second pivotal jaw members to a position away from each other. The bias member may be configured to automatically return the first and second pivotal jaw members to a position toward each other.

In another embodiment, a syringe pump includes a housing, a syringe seat coupled to the housing, and a retaining finger. The retaining finger is pivotally coupled to the housing and is configured to rotate toward a syringe disposed within the syringe seat to retain the syringe.

In another embodiment of the present disclosure, a method is provided for removing the effects of slack in a syringe pump having a syringe loaded on the syringe pump. The syringe has a barrel and a plunger disposed within the barrel. The method includes the acts of: receiving a target flow rate of the syringe loaded on the syringe pump;
determining a therapy actuation speed corresponding to the target flow rate; actuating the plunger of the syringe out of the barrel at a first predetermined speed until a force sensor coupled to the plunger measures a force that is less than a first predetermined force threshold; actuating the plunger of the syringe into the barrel at a second predetermined speed greater than the therapy actuation speed until the force sensor coupled to the plunger measures a force that exceeds a second predetermined threshold; and actuating the plunger of the syringe into the barrel at the therapy actuation speed. The therapy actuation speed may correspond to the target flow rate when there is no slack in the syringe pump or the syringe. The method may further include the acts of: estimating a volume discharged starting from the position of the plunger when the second predetermined threshold was exceeded; and/or stopping the syringe pump when the estimated volume discharged is equal to or exceeds a target delivery volume.

In another embodiment of the present disclosure, a method is provided for removing the effects of slack in a syringe pump having a syringe loaded on the syringe pump. The syringe has a barrel and a plunger disposed within the barrel. The method includes the acts of: receiving a target flow rate of the syringe loaded on the syringe pump;
determining a therapy actuation speed corresponding to the target flow rate; actuating the plunger of the syringe out of the barrel at a first predetermined speed until a force sensor coupled to the plunger measures a force that is less than a first predetermined force threshold or the plunger travels out of the barrel by a first predetermined distance; actuating the plunger of the syringe into the barrel at a second predetermined speed greater than the therapy actuation speed until the force sensor coupled to the plunger measures a force that exceeds a second predetermined threshold or the plunger travels into the barrel by a second predetermined distance; and actuating the plunger of the syringe into the barrel at the therapy actuation speed.

The therapy actuation speed may correspond to the target flow rate when there is no slack in the syringe pump or the syringe. The method may further include the acts of: estimating a volume discharged starting from the position of the plunger when the second predetermined threshold was exceeded; stopping the syringe pump when the estimated volume discharged is equal to or exceeds a target delivery volume; and/or using an alarm if the plunger traveled into the barrel by the second predetermined distance without the force sensor measuring a force that exceeds the second predetermined threshold.

In another embodiment of the present disclosure, a syringe pump includes a housing, a syringe seat, a lead screw, a motor, a sliding block assembly, a plunger head assembly, and one or more processors. The syringe seat is coupled to the housing and is configured to retain a syringe having a barrel and a plunger disposed within the barrel. The lead screw is rotatable within the housing. The motor is coupled to the lead screw and is configured rotate the lead screw. The sliding block assembly may be configured for engaging with the lead screw to move along the lead screw in accordance with rotation of the lead screw. The plunger head assembly is coupled to the sliding block assembly and is configured to drive a plunger of a syringe into a barrel of the syringe. The plunger head assembly has a force sensor operatively coupled to the plunger of the syringe to measure a force of the plunger head assembly on the plunger of the syringe. The one or more processors are operatively coupled to the motor and are configured to control the rotation of the motor to thereby control actuation of the plunger head assembly. The one or more processors are also operatively coupled to the force sensor to receive a measured force therefrom and are configured to: receive a target flow rate of the syringe loaded on the syringe pump; determine a therapy actuation speed corresponding to the target flow rate; command the motor to actuate the plunger of the syringe out of the barrel at a first predetermined speed until the force sensor coupled to the plunger measures a force that is less than a first predetermined force threshold; command the motor to actuate the plunger of the syringe into the barrel at a second predetermined speed greater than the therapy actuation speed until the force sensor coupled to the plunger measures a force that exceeds a second predetermined threshold; and command the motor to actuate the plunger of the syringe into the barrel at the therapy actuation speed. The therapy actuation speed may correspond to the target flow rate when there is no slack in the syringe pump or the syringe.

The one or more processors may be configured to estimate a volume discharged starting from the position of the plunger when the second predetermined threshold was exceeded.

The one or more processors may be further configured to stop the syringe pump when the estimated volume discharged is equal to or exceeds a target delivery volume.

In yet another embodiment of the present disclosure, a syringe pump includes a housing, a syringe seat, a lead screw, a motor, a sliding block assembly, a plunger head assembly, and one or more processors. The syringe seat is coupled to the housing and is configured to retain a syringe having a barrel and a plunger disposed within the barrel. The lead screw is rotatable within the housing. The motor is coupled to the lead screw and is configured rotate the lead screw. The sliding block assembly may be configured for engaging with the lead screw to move along the lead screw in accordance with rotation of the lead screw. The plunger head assembly is coupled to the sliding block assembly and is configured to drive a plunger of a syringe into a barrel of the syringe. The plunger head assembly has a force sensor operatively coupled to the plunger of the syringe to measure a force of the plunger head assembly on the plunger of the syringe. The one or more processors are operatively coupled to the motor and are configured to control the rotation of the motor to thereby control actuation of the plunger head assembly. The one or more processors are also operatively coupled to the force sensor to receive a measured force therefrom and are configured to: receive a target flow rate of the syringe loaded on the syringe pump; determine a therapy actuation speed corresponding to the target flow rate; command the motor to actuate the plunger of the syringe out of the barrel at a first predetermined speed until a force sensor coupled to the plunger measures a force that is less than a first predetermined force threshold or the plunger travels out of the barrel by a first predetermined distance; command the motor to actuate the plunger of the syringe into the barrel at a second predetermined speed greater than the therapy actuation speed until the force sensor coupled to the plunger measures a force that exceeds a second predetermined threshold or the plunger travels into the barrel by a second predetermined distance; and command the motor to actuate the plunger of the syringe into the barrel at the therapy actuation speed. The therapy actuation speed may correspond to the target flow rate when there is no slack in the syringe pump or the syringe.

The one or more processors may be further configured to estimate a volume discharged starting from the position of the plunger when the second predetermined threshold was exceeded and/or to stop the syringe pump when the estimated volume discharged is equal to or exceeds a target delivery volume
The one or more processors may be further configured to issue an alarm if the plunger traveled into the barrel by the second predetermined distance without the force sensor measuring a force that exceeds the second predetermined threshold.

The syringe pump described herein may further comprise a transceiver, and the one or more processors are configured to communicate via the transceiver with a monitoring client.

In some embodiments, the syringe pump includes a Patient-controlled analgesia ("PCA") button to deliver at least one pain medication.

Some embodiments of the present disclosure include a system for securing the syringe of a syringe pump to the side of the pump. The side loading mechanism includes a pump casing, a platform, a securing arm, and a force mechanism. The platform extends horizontally from the side of the pump casing when the pump is oriented for use. The securing arm is pivotally connected to the pump casing and to the force mechanism. The force mechanism creates a rotational force on the securing arm driving it into the platform, or a syringe placed on the platform. The force mechanism may allow the securing arm to lock in an up position, removed from the syringe on the platform. A wire structure may be attached to the end of the securing arm opposite the axis of rotation in order to engage the syringe. The securing arm may apply between one and three pounds of force on the syringe.

In some embodiments, the force mechanism includes a second arm, a roller, and an engaging plate. A first end of the second arm is connected to the first arm. The roller is attached to the second arm at the end opposite the first. The engaging plate is positioned to be engaged by the second arms and create a force on the arm that translates to the rotational force in the connected securing arm.

In certain embodiments of the present disclosure, the engaging plate is connected to a pivot point at its first end and to a spring at its second end. When the second arm engages the plate, the force of the spring and the shape of the plate persuades the arm to rotate, ultimately resulting in the rotational force of the securing arm. A section of the surface of the engaging plate engaged by the second arm may define a peak. The plate may also be sized to allow the second arm to sustain contact while rotated thirty five degrees.

In another embodiment of the present disclosure, the engaging plate is on a track allowing free movement on a plane substantially perpendicular to the surface engaged by the second arm. A spring urges the plate towards the engaged secondary arm. The shape of the plate combined with the force of the spring persuades the arm to rotate, ultimately resulting in the rotational force of the securing arm. A section of the surface of the engaging plate engaged by the second arm may define a peak. The plate may also be sized to allow the second arm to sustain contact while rotated thirty degrees.

In yet another embodiment of the present disclosure, the force mechanism includes a second arm and an engaging plate. The second arm comprises a first component connected to the securing arm, sharing its axis of rotation, and extending out substantially perpendicular to the pivot axis. A second component is attached to the first component at the end opposite the pivot and had the ability to slide towards and away from the pivot while its other movements remain uniform with the first component. A spring is connected to the first and second components urging the two apart. A roller is attached to the second component at the end opposite the pivot. The engaging plate is positioned to be engaged by the roller and compress the spring, resulting in forces that persuade the second arm and attached securing arm to rotate. A section of the surface of the engaging plate engaged by the second arm may define a peak. The plate may also be sized to allow the second arm to sustain contact while rotated five degrees.

In yet another embodiment of the present disclosure, the force mechanism includes a shaft, a first cam component, a second cam component, a spring, and a backstop. The shaft is pivitally connected to the securing arm having its longitudinal axis align with the securing arms axis of rotation. The first cam component is axially disposed around but not connected to the shaft. The first cam component is connected to and rotates with the securing arm A first end of the first cam component has a planar portion, a portion set back from the planar portion, and a portion merging the two portions with a taper. The second cam component is axially disposed around the shaft immediately next to the first cam but is not connected to the shaft. The second component has a fixed rotational orientation and the ability to slide back and forth on the shaft. The end of the second component abutting the first end of the first cam component mirrors the shape of the first component. The spring is disposed around the shaft immediately next to the second cam component on the side opposite the first component. The backstop is positioned to compress the spring resulting in the spring forcing the second component towards the first.

In some embodiments a sensor may be used to sense the angle of the securing arm. This sensor may be a Halifax sensor. The data from the sensor may be used to determine what type of syringe is being used. The system may also use the sensor data along with sensor data from a plunger driver sensor to determine what type of syringe is being used.

Certain embodiments of the present disclosure involve a method for securing the syringe of a syringe pump to the side of the pump. The method involves 1.) lifting a securing arm loaded with a downward force into a locked up position, 2.) placing a syringe onto a syringe holding ledge below the securing arm, and 3.) releasing the securing arm from the locked position to engage the syringe with the force loaded on the securing arm. In some embodiments, the downwards force loaded onto the securing arm is created by a spring. In other embodiments, a sensor tracks the positions of the arm. The sensor may be a Halifax sensor. The position of the arm may be used to indicate the syringe is properly position or to determine the type of syringe being used. Data from a plunger sensor may be used along with the position of the securing arm to determine the type of syringe being used.

Certain embodiments of the present disclosure use an apparatus for securing the syringe of a syringe pump to the side of the pump. The apparatus includes a pump casing, a platform, a securing arm, and a force mechanism. The platform projects out horizontally from the side of the pump casing when the casing is positioned for use. The rotating securing arm has a first end operatively connected to the pump casing above the ledge. The force mechanism is attached to the securing arm and produces a rotational force on the securing arm driving the end of the securing arm opposite the pivot onto the top of the ledge. The securing arm may have the ability to lock in an up position, removed from the ledge. The securing arm may also have a wire structure, configured to engage a syringe, connected at its second end. The securing arm may apply between one and three pounds of force on the syringe when in a securing position.

In some embodiments, the force mechanism may include a secondary arm, a roller, and an engaging plate. The second arm has a first end operatively attached to the secondary arm sharing its point of rotation. The roller is attached to the secondary arm at its opposing end. The engaging plate is positioned to engage the secondary arm with a force persuading the securing arm to rotate onto the top of the ledge.

In specific embodiments, one end of the engaging plate is operatively attached to the pump casing by a pivoting connector and the opposite end is attached to a spring. The spring is configured to force the engaging plate towards the engaged second arm, creating the rotational force on the connected arms. A section of the surface of the engaging plate engaged by the second arm may define a peak. The plate may also be sized to allow the second arm to sustain contact while rotated thirty degrees.

In other embodiments, the engaging plate has a free range of motion in a single direction with a spring imparting a force on the plate parallel to the range of motion. The spring urges the plate towards the engaged second arm, creating the rotational force on the arm. A section of the surface of the engaging plate engaged by the second arm may define a peak. The plate may also be sized to allow the second arm to sustain contact while rotated thirty degrees.

In another embodiment of the present disclosure, the force mechanism includes a secondary arm and an engaging plate. The secondary arm comprises a first component connected to the securing arm, sharing its axis of rotation, and extending out substantially perpendicular to the axis. A second component, connected to the first component at the end opposite the axis of rotation, having the freedom to move with respect to the first component's longitudinal axis. A spring urges the two components apart. A roller is connected to the end of the second component opposite the first component. The engaging plate is positioned to be engaged by the roller and compress the spring between the two components creating a force that urges the secondary arm to rotate. A section of the surface of the engaging plate engaged by the second arm may define a peak. The plate may also be sized to allow the second arm to sustain contact while rotated thirty five degrees.

In another embodiment of the present disclosure, the force mechanism includes a shaft, a first cam component, a second cam component, a spring, and a backstop. The shaft is connected to the securing arm at its point of rotation aligning its the longitudinal axis with the securing arm's axis of rotation. The first cam component is axially disposed around but not connected to the shaft. The first cam component is connected to and rotates with the securing arm. A first end of the component has a planar portion, a portion set back from the planar portion, and a portion merging the two portions with a taper. The second cam component is also axially disposed around the shaft and positioned immediately next to the first end of the first cam. The second component is not connected to the shaft, it is held at a fixed rotation position and able to slide up and down the shaft. The end of the second cam component abutting the first cam component mirrors the shape of the first component. The spring urges the second cam component against the first, having the ability to urge the first component and shaft to rotate depending on the orientation of the cams.

In some embodiments a sensor may be used to sense the angle of the securing arm. This sensor may be a Halifax sensor. The data from the sensor may be used to determine what type of syringe is being used. The system may also use the sensor data along with sensor data from a plunger driver sensor to determine what type of syringe is being used.

In another embodiment of the present disclosure, a method is provided for mitigating lead screw runout. This method can be applied to a syringe pump that uses a lead screw to control delivery of fluid from the syringe. The method includes: tracking the rotations of the lead screw using a rotary position sensor; tracking linear output of the lead screw using a linear position sensor; converting the rotary position data into distance output data, creating error data by comparing distance sensor data and the converted rotational data, estimating a phase and amplitude of the error data using a processor; and controlling the output of the lead screw by incorporating the estimated deviations into the assumed direct relation of rotation to distance output of the lead screw. Estimating the phase and amplitude of runout may be accomplished by cross-correlating a sine and cosine wave with the deviation data. Prior to cross-correlating the sensor data, the data may be stored as a single value for every degree of lead screw rotation and filtered through a low pass filter. Estimating the runout may include taking into account changes in the deviation amplitude when a displacement component of the lead screw nears and end of the threaded drive shaft.

The distance tracking sensor may be an optical mouse sensor. The data from the optical mouse sensor may be normalized before it is used to estimate a phase and amplitude in order to prevent sensor drift. The CIP data from the optical sensor may be normalized every ten degrees of lead screw rotation. The optical sensor may produce data in the range of 3000 CPI to 8200 CPI.

In another embodiment of the present disclosure, a system is provided for mitigating lead screw runout. The system includes a distance sensor, a rotation sensor, a processor, and a controller. The distance sensor has the ability to track linear changes in distance and is configured to track the changes of a lead screw mechanism output distance and create distance data. The rotational sensor has the ability to track rotational changes of a shaft, and is configured to track rotations of the lead screw driveshaft and create rotational data. The rotational sensor may be a Halifax sensor. The processor converts the rotational data into estimated distance output data and compares that to the distance data of the distance sensor. The processor then estimates the amplitude and phase of the difference between the distance sensor data and the estimated distance data from the rotational sensor. The amplitude and phase may be estimated by cross-correlating a sine and a cosine wave with the distance sensor data. The processor may estimate runout deviation using data from only the previous four rotations. The processor may also filter the distance data to a single value for every rotational degree. In some instances, the processor may not estimate the phase and amplitude of the runout deviation until it has received one hundred and eighty degrees of data. The controller controls the output of the lead screw using the rotational sensor to create a linear distance output and incorporating the estimated amplitude and phase of the deviations to account for lead screw runout. The controller may assume a decrease in the amplitude of runout deviation when the halfnut nears an end of the lead screw.

The distance tracking sensor may be an optical mouse sensor. The data from the optical mouse sensor may be normalized before it is used to estimate a phase and amplitude in order to prevent sensor drift. The CIP data from the optical sensor may be normalized every ten degrees of lead screw rotation. The optical sensor may produce data in the range of 3000 CPI to 8200 CPI.

In another embodiment of the present disclosure, an apparatus is provided for supplying an infusion pump with DC power. The apparatus includes a power supply, a power entry module, and an outlet adapter. The power entry module is connected to an infusion pump and is configured to receive current from the power supply and supply the pump with power. The power supply comprises an AC to DC conversion module, a AC in jack configured to receive AC current and supply the AC side of the conversion module, and a DC out jack configured to receive DC current from the conversion module and output DC current. The power supply is configured to be removable from the power entry module. The outlet adapter is in electrical communication with the AC in jack of the power supply, and is configured to plug into a wall outlet and supply power to the power source. A processor may be used to monitor power needs of the pump and adjust the output of the power source based on the pumps needs.

When attached, the power supply may be located on the top, bottom, back, or side of the infusion pump. The display of the pump may be biased towards the side of the pump in which the power supply is located when attached.

An AC in cord may be used to connect the outlet adapter and the AC in jack of the power supply. The power supply may have a spooling structure attached to it outside which is configured to have the AC in cord wrapped around it when the cord is not plugged into the wall. The power supply may also have a port configured to receive the outlet adapter once the cord has been wrapped around the spooling structure. The power supply may also incorporate a mechanism that automatically reels in the cord when commanded by a user.

A DC out cord may be used to connect the DC out jack of the power supply to the power entry module. The DC out cord may be removable from the power entry module.

The power entry module may be configured to attach to a rack, making the rack or power supply interchangeable.

In some instances, the power supply may be attached to a pole on which pumps it is supplying power to are mounted.

The power supply may also include a batter having a negative terminal in electrical communication with the DC out jack of the power supply and the positive terminal in electrical communication with the power entry module. A processor and an electric circuit may also be included. The processor and electric circuit will be configured to charge the battery when the power supply is receiving AC power and discharge the battery when no AC power is being received.

In some embodiments, the power supply will need to be removed from the pump in order to attach the pump to a poll.

In another embodiment of the present disclosure, a system is provided for providing power to an infusion pump. The system comprising a power supply and a pump. The pump includes a DC in jack (hereinafter also referred to as a DC in port). The power supply comprises an AC to DC converter, an AC in port (hereinafter also referred to as an AC in jack), and a DC out port, and is configured to supply the pump with power through the DC in jack. The power supply may have the ability to be removed from the pump.

The DC out port of the power supply may connect directly into the DC in jack of the pump, securing the power supply to the pump. The power supply may be located on the top, bottom, side, or back of the pump when attached.

A power out cord may be used to connect the DC out port of the power module with the DC in jack on the pump, putting the two in electrical communication. For instances when the power supply is connected to the pump by a cord, a holster configured to hold the power supply maybe mounted on the pump.

A power in corn may connected to the AC in port of the power supply to a wall outlet adapter, putting the two in electrical communication. The power in cord may be removable from the power supply. The power supply may include a spooling structure configured to have the power in wire wrapped around it. The power supply may also include a port configured to receive the wall outlet adapter once the cord is wound up.

A power supply may be configured to power multiple pumps. The power supply may be coupled to a pole on which a pump is mounted. The DC jack of the pump may be configured to attach the pump to a rack when the power source is not attached.

The power supply may include a battery configured to be charged by the power supply when current is flowing into the AC port, and supply power to the DC out port when no power is flowing into the AC in port. The AC port of the power supply has to receive current and convert it to the DC current before charging the battery.

In another embodiment, a syringe pump includes a body, a motor, a lead screw, a syringe seat, and a plunger head assembly. The syringe seat may be configured to slope toward an angle down. The motor is operatively coupled to the body. The lead screw is operatively coupled to the motor, and the motor is configured to actuate the lead screw. The plunger head assembly includes a dial, a plunger tube, a plunger head, and a half-nut assembly. The dial has a fully open position and a fully closed position. The dial is configured to actuate between the fully open position and the fully closed position. The plunger tube is configured to slideably engage with the body. The plunger head is operatively coupled to the plunger tube. The half-nut assembly is configured to engage the lead screw when the dial is actuated by a predetermined amount from the fully open position toward the fully closed position. The predetermined amount may be less than a halfway actuation position between the fully open position and the fully closed position.

The plunger head assembly may include two pivotable jaw members configured to grasp onto a syringe positioned within the syringe seat. The dial may be configured to actuate the pivotal jaw members into an open position.

The syringe pump may further includes a shaft operatively coupled to the dial such that the shaft and dial are configured so that actuation of the dial actuates the shaft. A cam may be coupled to the shaft. A rocker arm may be pivotally coupled to the plunger head assembly. The rocker arm may have a cam follower configured to engage the cam. One or more pivotable jaw members may be operatively coupled to the rocker arm.

The syringe pump may further includes first and second gears. The first gear is coupled to the rocker arm and the pivotable jaw member. The second gear is coupled to another pivotable jaw member. The first and second gears are configured to engage each other and to grasp onto a syringe disposed within the syringe seat. The cam and rocker arm may be configured such that addition actuation of the dial toward the closed position when the pivotable jaw members grasp onto the syringe causes the cam follower to disengage from the cam. A spring may urge the cam follower of the rocker arm toward the cam. The cam may include a detent configured to hold the cam in the detent until a predetermined amount of torque is applied to the dial to urge the dial toward the closed position. The plunger head may a shaft having a rod actuator coupled thereto. The plunger tube may include a rod and the rod is coupled to a link within the plunger head. The half-nut assembly further comprises a linear cam and the rod may be operatively coupled to the linear cam.

The half-nut assembly may further include first and second half-nut arms each having a first end and a second end. The first ends of the first and second half-nut arms are configured to engage with the leadscrew. The first and second half-nut arms may be pivotally coupled together. The second ends of the first and second half-nut arms may be configured to engage with the linear cam such that actuation of the linear cam toward the half-nut assembly causes the second ends of the first and second half-nut arms to pivotally approach each other. The first ends of the first and second half-nut arms each includes threads configured to engage the leadscrew when the second ends of the first and second half-nut arms approach each other.

In another embodiment, a syringe pump includes a body, a motor, a lead screw, a syringe seat, and a plunger head assembly. The motor is operatively coupled to the body. The lead screw is operatively coupled to the motor and is configured to actuate the lead screw. The plunger head assembly includes a dial, a plunger tube, a plunger head assembly, and a half-nut assembly. The dial has a fully open position and a fully closed position. The dial is configured to actuate between the fully open position and the fully closed position. The plunger tube is configured to slideably engage with the body. The plunger head is operatively coupled to the plunger tube. The half-nut assembly is configured to engage the lead screw when the dial is actuated by at least a predetermined amount from the fully open position toward the fully closed position. The half-nut assembly includes first and second half-nut arms pivotally coupled together and configured to engage with the lead screw.

In another embodiment, a system for securing a syringe to a syringe pump includes a pump casing, a platform, a pivotal securing arm, a force mechanism, and a display. The platform (a syringe seat) extends horizontally from a side of the casing. The pivotal securing arm is configured to engage a syringe resting on the platform. The force mechanism is connected to the arm and is configured to apply a rotational force to the arm which results in a downward force applied to the syringe. The display may be coupled to a side of the casing. The display may further include a power button, an alarm silence button, and/or a menu button. A monitoring client may be provided that is configured to at least one of receive data from the syringe pump or control the syringe pump as described herein. The monitoring client may be a tablet computer.

A method for discharging fluid from a syringe and for mitigating occlusion conditions includes actuating the plunger of a syringe into a barrel. The method monitors fluid pressure within the barrel of the syringe and determines that an occlusion exists when the fluid pressure exceeds a predetermined threshold. The method actuates the plunger out of the barrel by a predetermined amount in response to the detected occlusion and actuates the plunger of the syringe into the barrel until a measured fluid pressure within the barrel of the syringe exceeds another predetermined threshold.

In accordance with an embodiment of the present disclosure, a system for securing a syringe to a syringe pump may include having a pump casing, a platform extending horizontally from a side of the casing, a pivotal securing arm configured to engage a syringe resting on the platform, and a force mechanism, connected to the securing arm. The force mechanism may be configured to apply a rotational force to the securing arm which results in a downward force applied to the syringe.

In some embodiments of the system, the force mechanism may include a second arm having a first end connected to the securing arm and an opposite second end. In some embodiments, a roller may be attached to the second arm at the second end. An engaging plate configured to engage the roller and urge the second arm in a direction that creates the rotational force in the connected securing arm may be included.

In some embodiments, such a system may include a first end of the engaging plate connected to a pivot point and an opposite second end attached to a bias member. The bias member may be configured to create a force that urges the second arm. The bias member may be a spring.

In some embodiments, a surface of the engaging plate engaged by the second arm may define a peak. The plate may be sized to allow the second arm to sustain contact while rotated at least thirty degrees. The engaging plate may be configured to move freely in a plane substantially perpendicular to a surface engaged by the second arm. A bias member urging the engaging plate towards the second arm may be included. The engaging plate may be oriented to create a force that urges the second arm. A surface of the engaging plate engaged by the second arm may define a peak. The engaging plate may be sized to allow the second arm to sustain contact with the engaging plate while rotated substantially at least thirty degrees.

In some embodiments, the force mechanism may include a second arm connected to a securing arm. A first component having a first end connected to the securing arm and an opposite second end may be included. A second component attached to the first component at its second end may be included. The second component may be configured to move back and forth with regard to a longitudinal axis of the first component while movements in other directions are in tandem with movement of the first component. A bias member connected to the first and second components urging the two apart may be included. A roller attached to an end of the second component opposite the first component may be included. An engaging plate positioned to be engaged by the roller thereby imparting a force on the second arm that creates the rotational force in the securing arm may be included. A surface of the engaging plate engaged by the second arm may define a peak. The engaging plate may be sized to allow the second arm to sustain contact with the engaging plate while rotated substantially at least thirty degrees.

In some embodiments, the force mechanism may include a shaft attached to the securing arm wherein a longitudinal axis of the shaft is coaxial with an axis of rotation of the securing arm. A first cam component disposed around the shaft configured to rotate with the securing arm may be included. A first end of the component may have a planar portion, a portion set back from the planar portion, and a taper portion merging the two portions with a taper. A second cam component disposed around the shaft adjacent to the first end of the first cam component may be included. The component may have a fixed rotational orientation and an ability to translate back and forth on the shaft. An end of the second cam component abutting the first cam component may mirror the shape of the first cam component. A bias member may be disposed around the shaft adjacent to the second cam component on a side opposite the first cam component. A backstop positioned to bias the bias member and translate a force of the bias member to urge the second cam component towards the first may be included. The taper portions of the cams may be tapered at about a forty five degree angle with respect to the planar portion. Each cam component may have two tapered sections.

In some embodiments, the force mechanism may be configured to allow the securing arm to lock in an up position, removed from the syringe on the platform.

Some embodiments may further comprise a wire structure connected to an end of the securing arm opposite an axis of rotation. The wire structure may be configured to engage a syringe when the arm is rotated down.

In some embodiments, the securing arm may apply between about one and about three pounds of force on a syringe when in a securing position. Some embodiments may further comprise a sensor configured to track an angle of the securing arm. The sensor may be a hall effect sensor. Data from the sensor may be used to determine one or more characteristic of the syring. In some embodiments, data from the sensor, in conjunction with data from a plunger driver sensor, may be used to determine one or more characteristic of the syringe.

In accordance with an embodiment of the present disclosure, a method for securing a syringe to a syringe pump includes: Overcoming a bias force by displacing a securing arm to a first, locked position;, placing a syringe onto a syringe holding platform below the securing arm, and releasing the securing arm from the first position to thereby secure the syringe with securing arm via the bias force.

In some embodiments, the bias force may be created by a spring. Some embodiments may further include sensing the position of the securing arm. Some embodiments of the method may include alerting a user if the securing arm is not properly securing the syringe based on the position of the securing arm. Some embodiments of the method may further include determining at least one characteristic of the syringe using data gleaned from sensing the position of the securing arm. Some embodiments may further include using a processor to determining the fluid flow based on change in position of a plunger of the syringe in conjunction with the determined at least one characteristic of the syringe. Some embodiments may include using data from a plunger driving arm in conjunction with a position of the securing arm to determine at least one characteristic of the syringe. Some embodiments of the method may further include using a processor to determining the fluid flow based on change in position of a plunger in the syringe in conjunction with the determined at least one characteristic of the syringe. In some embodiments a Hall effect sensor is used to sense the position of the securing arm.

In accordance with another embodiment of the present disclosure, an apparatus for securing a syringe to a syringe pump may include a pump casing having a top, bottom, and two sides; a platform projecting out horizontally from a side of the pump casing; a rotating securing arm having a first end attached to the pump casing above the platform and an opposite second end configured to engage a top of the platform in a rotational position of the securing arm; and a force mechanism attached to the securing arm. The force mechanism may be configured to produce a rotational force on the securing arm to thereby urge the second end towards the top of the platform, some embodiments, the force mechanism may include a secondary arm having a first end operatively attached to the securing arm sharing its axis of rotation and an opposite second end. A roller attached to the secondary arm at the second end wherein the roller extends past the second end of the secondary arm may be included. An engaging plate configured to engage the roller with a force that causes the second arm to rotate in a direction that translates to the downward force of the securing arm may be included. A first end of the engaging plate may be operatively attached to the pump casing by a pivoting connector. A second end of the engaging plate may be operatively attached to a bias member. The bias member may urge the engaging plate towards the engaged second arm thereby creating a force inducing the second arm to rotate. A surface of the engaging plate which may be engaged by the second arm may define a peak. The engaging plate may be sized to allow the second arm to sustain contact with a the engaging plate while rotated substantially at least thirty degrees. The engaging plate may have a linear free range of motion on a single plane in one degree of freedom. A bias member may impart a force on the engaging plate, at least a component of the force may be in the direction of the range of motion. The bias member may urge the engaging plate towards the engaged second arm, to thereby create induce the second arm to rotate. A section of a surface of the engaging plate engaged by the second arm may define a peak. The engaging plate may be sized to allow the second arm to sustain contact with a portion of the engaging plate while the second arm is rotated substantially at least thirty degrees. In some embodiments, the force mechanism may include a secondary arm operatively attached to the securing arm such that it shares its axis of rotation. The second arm may include a first component having a first end connected to the securing arm and a second end extending from the first end and oriented substantially perpendicular to the axis of rotation. A second component having a first end connected to the second end of the first component and an opposite second end may be included. The second component may have a single degree of freedom to move, but otherwise be constrained to movement in tandem with the first component. A bias member having a first portion attached to the first component and a second portion attached to the second component may be included. The bias member may be configured to impart a biasing force biasing the first component and second component apart from one another. A roller attached to the second end of the second component may be included. The roller may extend past the second end of the second component. An engaging plate configured to be engaged by the roller to thereby compress the bias member and thereby generate the rotational force translated to the securing arm may be included.

In some embodiments, a surface of the engaging plate engaged by the second arm may define a peak. The engaging plate may be sized to allow the second arm to sustain contact with a portion of the engaging plate while the second arm is rotated substantially at least thirty degrees.

In some embodiments, the force mechanism may include a shaft attached to the securing arm such that it shares it axis of rotation and having its longitudinal axis align with the axis of rotation. A first cam component disposed around the shaft configured to rotate with the securing arm may be included. A first end of the component may have a planar portion, a portion set back from the planar portion, and a taper portion merging the two portions with a taper. A second cam component disposed around the shaft adjacent to the first end of the first cam component may be included. The component may have a fixed rotational orientation and the ability to translate back a forth on the shaft. An end of the component abutting the first cam component may mirror the shape of the first cam component. A bias member configured to urge the second cam component towards the first cam component may be included.

In some embodiments, the force mechanism may be configured to allow the securing arm to lock in an up position, in which the securing arm does not contact the platform. A wire structure connected the second end of the securing arm, configured to engage a syringe when the arm is rotated to a securing position may be included. The securing arm may apply between about 1 and about 3 pounds of force on a syringe when in a securing position. A sensor configured to sense the angle of the securing arm may be included. The sensor may be a hall effect sensor. Data from the sensor may be used to determine at least one characteristic of the syringe. In some embodiments, data from the sensor in conjunction with data from a plunger driver sensor may be used to determine one or more characteristic of the syringe.

According to an embodiment of the present disclosure, an apparatus to supply an infusion pump with DC power may include at least one power entry module connected to a housing of an infusion pump, configured to receive DC current from a power supply and supply an infusion pump with power. The module may have a port configured to receive current. The power supply may be configured to be removably attached to the power entry module creating electrical communication between the power supply and the power entry module when attached. The power supply may include an AC to DC conversion module configured to convert AC current to DC current and supply the pump with current of a constant voltage. An AC in jack configured to receive AC current and supply an AC side of the conversion module may be included. A DC out jack configured to receive DC current from the conversion module and output DC current may be included. An outlet adapter in electrical communication with the AC in jack of the power supply, configured to plug into an AC wall outlet to thereby supply the AC in jack with AC current may be included. The power supply, when attached, may be located on any one of a top, a bottom, a back, or a side of the infusion pump. A display may be disposed proximal to the location of the power supply when the power supply is attached. An AC in cord (hereinafter also referred to as a power cord) may connect the outlet adapter to the AC in jack of the power supply. The AC in cord may be removable from the power supply. A spooling structure attached to an outside of the power supply configured to have the power cord wrapped around it when the cord is not plugged in may be included. The power supply may include a port configured to receive the outlet adapter once the cord has been wrapped around the spooling structure. An enclosed reel configured to automatically reel the power cord up when commanded by a user may be included. A DC out cord to connected the DC out jack of the power supply to the power entry module, creating electrical communication between the two may be included. The DC out cord may be removable from the power entry module. The power entry module may be configured to attach to a rack, making the rack or power supply interchangeable. Connecting the power supply to the power entry module may secure the power supply to the pump. The power supply may be configured to supply multiple pumps with power. Multiple DC out cords configured to connect the DC out jack of the power supply to the power entry modules of the multiple pumps, creating electrical communication between the power supply and the pumps may be included. The power supply may be mounted on a pole on which pumps it is supplying power to are also mounted. A battery having a negative terminal operatively connected to the DC out jack of the power supply and the positive terminal operatively connected to the power entry module may be included. A processor and an electric circuit configured to charge the battery when the power supply is receiving AC current and discharge the battery when no AC current is being received may be included. In some embodiments, the power supply must be removed from the pump in order to attach the pump to a pole. A processor to monitor power needs of the pump and adjust an output of the power source based on those needs may be included. The conversion module may regulate a voltage and a current of the electricity entering the pump. In some embodiments, the pole may include a power supply and one or more attachment features for attaching an infusion pump to the pole.

In accordance with an embodiment of the present disclosure, a system for providing DC power to an infusion pump may include a pump, including a DC in jack and a power supply configured to supply the pump with power through the DC in jack. The power supply may be removable from the pump. The pump may include an AC to DC converter, an AC in adapter, a DC out adapter, and an AC outlet adapter configured to plug into an AC outlet being in communication with the AC in adapter of the power supply. The DC out adapter of the power supply may connect directly into the DC in jack of the pump, securing the power supply to the pump and creating electrical communication between the power supply and DC out adapter. The attached power supply may be located on any one of a back, a side, a top, and a bottom of the pump. The power supply may further comprise an DC out cord configured to connect the DC out adapter of the power module to the DC in jack of the pump thereby creating electrical communication between the two. The pump may include a holster configured to secure the AC to DC converter of the power supply to the pump. An AC in cord having a first end configured to connect to the AC in port of the power supply and a second end having a wall outlet adapter may be included. The AC in cord may be removable from the power supply. The power supply may further comprises a spooling mechanism for wrapping up the AC in cord. The spooling structure may be configured to have the AC in cord wrapped around it by a user. The power supply may include a port configured to receive the wall outlet adapter once the cord is wound up. A single power supply may be configured to power multiple pumps. The power supply may be capable of being coupled to the pole, the pole including at least one attachment feature for an infusion pump. The DC in jack of the pump may be configured to secure the pump to a rack and receive current from the rack when the power source is not attached. The power supply may include a battery configured to be charged by the power supply when current is flowing into the AC in port, and supply power to the DC out port when no current is flowing into the AC in port.

In accordance with an embodiment of the present disclosure a method for mitigating lead screw runout error may include tracking the rotations of a lead screw using a rotary position sensor. The method may include tracking distance output of a lead screw mechanism using a linear position sensor. The method may include converting the rotary position sensor output to a linear displacement output of the lead screw mechanism. The method may include creating error data by determining the difference between data from the linear position sensor and converted data from the rotary position sensor. The method may include estimating, based on the error data, a phase and amplitude of deviations from an assumed direct relation of rotations to distance output of the lead screw mechanism, using a processor. The method may include controlling, with a controller, the output of the lead screw mechanism. The controller may compensate for the estimated devaitions.

In some embodiments, the linear position sensor may be an optical mouse sensor. The optical mouse sensor may output data at a frequency of about 3000 CPI to about 8200 CPI. The method may further comprise normalizing the optical mouse sensor data prior to estimating a phase and amplitude to thereby mitigate sensor drift. Normalizing the data may involve recalibrating a mouse's CPI every ten degrees of rotation of the lead screw. Estimating the phase and amplitude may involve cross-correlating a sine and cosine wave with the deviation data. The method may further comprise storing the error data for a single degree of lead screw rotation into one value prior to cross-correlation. The estimating step may take into account a change in the deviation amplitude when a displacement component of the lead screw nears an end of the lead screw's threaded driveshaft. The rotary position sensor may be a hall effect sensor. The phase and amplitude of runout deviation may be estimated using data from only four previous rotations of the lead screw. The method may further comprise filtering the error data prior to estimating its phase and amplitude. The data may be filtered using a low pass filter.

In accordance with an embodiment of the present disclosure, a system for mitigating lead screw runout may include a linear position sensor configured to track a distance output of a lead screw mechanism and generate distance data. A rotary position sensor configured to track rotations of the lead screw and generate rotational data may be included. A processor may be included. The processor may be configured to convert the rotational data into converted distance output of the lead screw mechanism. The processor may be configured to create error data by determining the difference between the converted rotational data and the distance data. The processor may be configured to estimate the amplitude and phase of the error data. A controller configured to control the distance output of the lead screw mechanism may be included. The controller may compensate for the phase and amplitude of the error data.

In some embodiments, the linear position sensor may be an optical mouse sensor. The optical mouse sensor may output data at a frequency of 3000 CPI to 8200 CPI. The distance data, prior to creating the error data, may be normalized to account for drift. The data may be normalized by the processor every ten degrees of lead screw rotation. The phase and amplitude of the error data may be estimated by cross correlating a sine and a cosine wave with the data. The rotation sensor may be a hall effect sensor. The controller may assume a decrease in error data amplitude when a half nut of the lead screw mechanism nears an end of the lead screw. The phase and amplitude of the error data may be estimated using data from only the four previous rotations. Distance data may be filtered to a single value for every rotational degree of lead screw displacement. The processor may not estimate the phase and amplitude of the error data until it has received one hundred and eighty degrees of sensor data. The error data may be filtered prior to estimating its phase and amplitude. The error data may be filtered using a low pass filter.

In accordance with an embodiment of the present disclosure, a syringe pump may include a body, a motor, and a lead screw operatively coupled to the motor. The motor may be configured to actuate the lead screw. A syringe seat and a plunger head assembly may be included. The plunger head assembly may include a dial having a first position and a second position. The dial may be configured to actuate between the first position and the second position. A plunger tube configured to slideably engage with the body may be included. A plunger head may be operatively coupled to the plunger tube. A half-nut assembly configured to engage the lead screw when the dial is actuated by a predetermined amount from the first position toward the second position may be included. The predetermined amount may be less than a halfway position between the first position and the second position.

In some embodiments, the plunger head assembly may include two pivotable jaw members configured to grasp onto a plunger positioned within the syringe seat. The dial may be configured to actuate the pivotal jaw members. A shaft may be operatively coupled to the dial. The shaft and dial may be configured such that actuation of the dial actuates the shaft. A cam may be coupled to the shaft. A rocker arm pivotally coupled to the plunger head assembly may be included. The rocker arm may have a cam follower configured to engage the cam. A pivotable jaw member may be operatively coupled to the rocker arm.

In some embodiments a first gear coupled to the rocker arm and the pivotable jaw member may be included. A second gear coupled to another pivotable jaw member may be included. The first and second gears may be configured to engage each other. The pivotable jaw members may be configured to grasp onto a plunger. The cam and rocker arm may be configured such that additional actuation of the dial toward the second position when the pivotable jaw members grasp onto the plunger causes the cam follower to disengage from the cam. A bias member configured to urge cam follower of the rocker arm toward the cam may be included. The cam may include a detent configured to hold the cam in the detent until a predetermined amount of torque is applied to the dial to urge the dial toward the second position. The plunger head may include a shaft having a rod actuator coupled thereto. The plunger tube may include a rod. The rod may be coupled via a link within the plunger head. The half-nut assembly may comprise a linear cam. The rod may be operatively coupled to the linear cam. The half-nut assembly may further include first and second half-nut arms, each having a first end and a second end. The first ends of the first and second half-nut arms may be configured to engage with the leadscrew. The first and second half-nut arms may be pivotally coupled together. The second ends of the first and second half-nut arms may be configured to engage with the linear cam such that actuation of the linear cam toward the half-nut assembly causes the second ends of the first and second half-nut arms to pivotally approach each other. The first ends of the first and second half-nut arms each may include threads configured to engage the leadscrew when the second ends of the first and second half-nut arms approach each other. The syringe seat may include at least one sloped face.

According to an embodiment of the present disclosure, a syringe pump may include a body, a motor, and a lead screw operatively coupled to the motor. The motor may be configured to actuate the lead screw. A syringe seat and a plunger head assembly may be included. The plunger head assembly may include a dial having a fully open position and a fully closed position. The dial may be configured to actuate between the fully open position and the fully closed position. A plunger tube configured to slideably engage with the body may be included. A plunger head may be operatively coupled to the plunger tube. A half-nut assembly configured to engage the lead screw when the dial is actuated by a at least a predetermined amount from the fully open position toward the fully closed position may be included. The half-nut assembly may include first and second half-nut arms pivotally coupled together and configured to engage with the lead screw.

In accordance with an embodiment of the present disclosure, a system for securing a syringe to a syringe pump may include a pump casing. A platform extending horizontally from a side of the casing may be included. A pivotal securing arm configured to secure a syringe resting on the platform may be included. A force mechanism, connected to the arm, configured to apply a rotational force to the arm which results in a securing force applied to the syringe may be included. A user interface coupled to the casing may be included.

In some embodiments, the user interface may further include a power button, an alarm silence button, and a menu button.

A monitoring client may be configured to at least one of receive data from the syringe pump or control the syringe pump. The monitoring client may be a tablet computer. A monitoring client may be configured to receive data from the syringe pump.

In accordance with an embodiment of the present disclosure, a syringe pump includes a housing, a syringe seat, a plunger head, a pressure sensor, and a motor, and one or more processors. The syringe seat is operatively coupled to the housing and is configured to retain a syringe. The plunger head is configured to engage with a plunger of the syringe to actuate the plunger of the syringe. The pressure sensor is configured to coupled to the syringe to operatively estimate a fluid pressure within the syringe. The motor is operatively coupled to the plunger head to actuate the plunger head to thereby actuate the plunger of the head.

The one or more processors may be configured to cause the actuator to actuate in a first direction to thereby cause the syringe to discharge fluid. The processor(s) may monitor the pressure sensor to estimate the fluid pressure within the syringe and determine an occlusion exists when the fluid pressure exceeds a predetermined threshold. The processor(s) may cause the actuator to actuate the plunger out of the barrel by a predetermined amount, and cause the actuator to actuate the plunger of the syringe into the barrel until a measure of fluid pressure within the syringe exceeds another predetermined threshold.

In some embodiments, the predetermined amount the plunger may be actuated out of the barrel may be a function of an innerdiameter of the barrel. The another predetermined threshold may be a function of an inner diameter of the barrel.

In some embodiments, the predetermined threshold may be in a plurality of predetermined thresholds located within a lookup table. The predetermined threshold corresponds to a syringe model number as found in the lookup table.

In some embodiments, the another predetermined threshold is in a plurality of predetermined thresholds located within a lookup table. The another predetermined threshold may correspond to a syringe model number as found in the lookup table.

The predetermined amount the plunger is actuated out of the barrel is in a plurality of predetermined amounts located within a lookup table. The predetermined amount the plunger is actuated out of the barrel may correspond to a syringe model number.

In some embodiments, a force sensor coupled to the plunger may be used to monitor the fluid pressure within the barrel of the syringe. The predetermined amount may be a predetermined distance of actuation of the plunger out of the syringe and/or may be a predetermined change in volume of expansion within the barrel.

In an embodiment of the present disclosure, a pressure sensor assembly includes a plunger having a sensing surface, and first and second pressure sensors. The first pressure sensor is operatively coupled to the plunger and is configured to estimate a force applied to the sensing surface. The second pressure sensor is operatively coupled to the plunger and is configured to estimate the force applied to the sensing surface. The processor is coupled to the first and second pressure sensors.

In some embodiments, the processor estimates a magnitude of the force applied to the sensing surface and/or estimates a position on the sensing surface where the force is applied to using the first and second pressure sensors. The processor may be configured to estimate a position on the sensing surface where the force is applied thereto.

In some embodiments, the assembly includes a guide that guides the plunger such that the sensing surface moves one of away from and toward the first and second pressure sensors. A seal may be disposed over the sensing surface.

In some embodiments, a syringe pump includes the plunger head assembly configured to receive an actuatable portion of a syringe. A pressure sensor as described above may be coupled to the plunger head such that the sensing surface receives the actuatable portion of the syringe.

In some embodiments of the present disclosure, a syringe pump includes a body configured to receive an infusion tube. The embodiments may include the pressure sensor assembly described above that is coupled to the infusion tube such that the sensing surface responds to pressure within the infusion tube.

In yet another embodiment of the present disclosure, a syringe pump includes a plunger head assembly, a pressure sensor assembly, and a processor. The plunger head assembly is configured to receive an actuatable portion of a syringe. The pressure sensor assembly is coupled to the plunger head assembly and is configured to sense a force applied to the plunger head assembly. The pressure sensor assembly includes, in a specific embodiment, a plunger, and first and second pressure sensors. The plunger includes a sensing surface configured to receive the force. The first pressure sensor is coupled to the plunger and is configured to estimate the force applied to the sensing surface; Also, in a specific embodiment, a second pressure sensor is operatively coupled to the plunger and is configured to estimate the force applied to the sensing surface. The processor is coupled to the first and second pressure sensors and is configured to estimate a magnitude of the force.

The magnitude of the force is correlated with a pressure within the syringe. The pressure sensor assembly further includes a seal disposed over the sensing surface of the plunger. The seal is configured to seal the plunger head assembly from fluid ingress.

The plunger head assembly is configured to actuate the syringe and the processor is configured to determine if an occlusion exists using the first and second pressure sensors. The processor may be configured to estimate a magnitude of the force applied to the sensing surface using the first and second pressure sensors.

The processor may be configured to estimate a position on the sensing surface where the force is applied thereto. The position on the sensing surface in which the force is applied may be correlated with a syringe type, size, model #, and/or syringe manufacturer as found in an internal lookup table and/or database.

In an embodiment, a guide configured to guide the plunger such that the sensing surface moves one of away from and toward the first and second pressure sensors. The sensing surface is elongated along a first direction and is configured to receive a plurality of syringe sizes loaded into the syringe pump.

In yet another embodiment of the present disclosure, the pressure sensor assembly includes a plunger, a guide, and first and second pressure sensors. The guide is configured to guide the movement of the plunger along the third and fourth axes. The first pressure sensor is disposed adjacent to an end of the first extension opposite to the sensing surface. The first pressure sensor is configured to sense movement of the plunger within the guide. The second pressure sensor is disposed adjacent to an end of the second extension opposite to the sensing surface. The second pressure sensor is configured to sense movement of the plunger within the guide.

The plunger includes a sensing surface, and first and second extensions. The sensing surface has an elongated portion along a first axis and a width along a second axis. The first and second axes are orthogonal relative to each other. The width is about constant along a substantial portion of the elongated portion. The elongated portion of the sensing surface defines first and second ends. The first and second ends are curved. The first extension is disposed adjacent to or on the first end of the sensing surface and extends along a third axis orthogonal to the first and second axes away from sensing surface. The second extension is disposed adjacent to or on the second end of the sensing surface and extending along a fourth axis orthogonal to the first and second axes away from sensing surface. The third and fourth axes are parallel to each other.

Optionally, a seal may be at least partially disposed over at least a portion of the sensing surface and/or a brace is operatively coupled to the first and second extensions.

In another embodiment of the present disclosure, a syringe pump includes a body, a syringe seat, a syringe actuator, a memory, and one or more processors.

The syringe seat is coupled to the body. The syringe actuator is configured to actuate a syringe secured within the syringe seat. The memory is configured to store a plurality of instructions. The one or more processors, in accordance with the plurality of instructions, is configured to: prime the syringe pump in a prime phase; determine if an occlusion exists during the prime phase using a first test; stop the prime phase; initiate fluid delivery into a patient; enter into a start-up phase; determine if an occlusion exists using a second test during the start-up phase; transition from the start-up phase into a steady-state phase; and determine if an occlusion exists during the steady-state phase using a third test.

The one or more processors may be configured to start the prime phase in response to a user input. The syringe pump may include a touch screen configured such that the user input is entered into the touch screen. The touch screen is in operative communication with the one or more processors. The processor(s) and the touch screen are configured such the one or more processors receive confirmation of the user input.

The syringe pump may include a force sensor and a motor sensor. The force sensor may be configured to determine a force applied to the syringe by actuation of the syringe actuator. The motor sensor may be operatively coupled to a motor of the syringe pump (e.g., on a drive train, a leadscrew, or other device in engagement with the motor). The one or more processors may be in operative communication with the force sensor and the motor sensor. The one or more processors may be configured to use the motor position, the motor rotation speed, and the force applied to the syringe to perform at least one of the first, second, and third tests.

The first test may be that the one or more processors determine if the pressure, P, exceeds a first threshold to trigger an occlusion alarm. That is, if the first test is True (a Boolean value), then the syringe pump triggers an occlusion alarm.

The second test may be that the one or more processors determine if the pressure, P, exceeds a second threshold or an occlusion metric exceeds a third threshold multiplied by a motor speed to trigger an occlusion alarm.

The may be that the one or more processors determines a pressure, P, exceeds a fourth threshold; or an occlusion metric, OM, exceeds a fifth threshold to trigger an occlusion alarm. Optionally, the one or more processors may have the third test include a determination whether the pressure, P, minus a first, P₀, of a series of the pressure, P, exceeds a sixth threshold to trigger the occlusion alarm. The first, P₀, of a series of the pressure, P, is a first measurement of the pressure when the syringe pump enters into the steady-state phase. The occlusion metric, OM, is the pressure, P, minus an average pressure, Pavg.

The pressure, P, may be determined by the at least one processor in accordance with:
$P = \frac{{\sum }_{i = 0}^{n} \frac{F \left({Θ}_{i}\right)}{A}}{n} .$

A is the area of an internal cross-section of a barrel of a syringe. F is the force output as sensed by a force sensor configured to measure the force applied to a syringe (e.g., a force applied to a syringe plunger to actuate the syringe). *F*(Θ*ᵢ*) is the force applied to the syringe at a particular angle Θ*ᵢ* that occurs at a particular motor position as indicated by an output of a rotary sensor operatively coupled to the motor. The n indicates that the number of samples taken from i=0 to a half motor revolution.

The average pressure, Pavg, may be determined by the one or more processors in accordance with:
${P}_{avg} = \frac{{\sum }_{i = 0}^{k} {P}_{i}}{k} .$

The average pressure, Pavg, may be a series of pressure values, Pi, over the last 5 motor revolutions.

The one or more processors may be configured to transition from the start-up phase into the steady-state phase when: the occlusion metric, OM, exceeds a first value; and the occlusion metric, OM, is less than a second value.

The one or more processors may transition from the start-up phase into the stead-state phase when the occlusion metric, OM, exceeds the first value before the occlusion metric, OM, is less than the second value.

In some additional embodiments of the present disclosure, a method for operating a syringe pump, at least partially implemented by at least one processor executing a plurality of instructions configured for execution by the at least one processor, the method includes: priming the syringe pump in a prime phase; determining if an occlusion exists during the prime phase using a first test; stopping the prime phase; initiating fluid delivery into a patient; entering into a start-up phase; determining if an occlusion exists using a second test during the start-up phase; transitioning from the start-up phase into a steady-state phase; and determining if an occlusion exists during the steady-state phase using a third test.

The method may include receiving user initiation of the prime phase and/or determining a force applied to a syringe by actuation of a syringe actuator. The method may determine a motor position and determine a motor rotation speed.

The first, second and third tests may be the tests as described above and herein. The act of transitioning from the start-up phase into the steady-state phase may occur when: an occlusion metric, OM, exceeds a first value; and the occlusion metric, OM, is less than a second value.

A method for recovering from an occlusion includes the acts of: dispensing fluid into a patient using a syringe pump; monitoring the syringe pump; determining whether an occlusion associated with the syringe pump exists; stopping delivery in response to the determined occlusion; determining if a force drops below a predetermined threshold within a predetermined period of time, wherein the predetermined threshold is a predetermined percentage of the difference between a steady state force and the force at the time of the determined occlusion; reversing the syringe pump to relieve a pressure within the syringe pump is the syringe pump does not drop below the predetermined threshold within the predetermined period of time; and stopping the syringe pump when the slope drops below a predetermined value and then rises above a second predetermined value.

### BRIEF DESCRIPTION OF THE DRAWINGS

These and other aspects will become more apparent from the following detailed description of the various embodiments of the present disclosure with reference to the drawings wherein:
Fig. 1 is an illustration of an electronic patient-care system having a syringe pump in accordance with an embodiment of the present disclosure;
Figs. 2-5 show several views of a patient bedside system in accordance with an embodiment of the present disclosure;
Fig. 6 shows a close-up view of a portion of an interface of a clamp that is attachable to a pump shown in Figs. 2-5 in accordance with an embodiment of the present disclosure;
Fig. 7 shows another close-up view of another portion of the interface shown in Fig. 6 in accordance with an embodiment of the present disclosure;
Fig. 8 shows a perspective view of a pump attachable to the patient bedside system of Figs. 2-5 in accordance with an embodiment of the present disclosure;
Fig. 9 shows a perspective view of a pump shown in Figs. 2-5 in accordance with an embodiment of the present disclosure;
Figs. 10-13 show several views of a syringe pump in accordance with an embodiment of the present disclosure;
Fig. 14 shows several of the syringe pump of Figs. 10-13 mounted on a pole in accordance with an embodiment of the present disclosure;
Figs. 15-16 illustrate portions of the operation of the syringe pump of Figs. 10-13 in accordance with an embodiment of the present disclosure;
Figs. 17-18 illustrate several medical devices mounted on a pole in accordance with an embodiment of the present disclosure;
Figs. 19-22 show several views of a medical device of Figs. 17-18 in accordance with an embodiment of the present disclosure;
Fig. 23 shows several mounts mounted on a pole in accordance with an embodiment of the present disclosure;
Figs. 24-26 show several views of a mount of Fig. 23 in accordance with an embodiment of the present disclosure;
Fig. 27 shows a circuit diagram having a speaker and battery in accordance with an embodiment of the present disclosure;
Fig. 28 shows a view of an exemplary embodiment of a syringe pump in accordance with an embodiment of the present disclosure;
Fig. 29 shows a front view of an exemplary embodiment of a syringe pump in accordance with an embodiment of the present disclosure;
Fig. 30 is a view of an exemplary embodiment of the syringe pump assembly in accordance with an embodiment of the present disclosure;
Fig. 31 is another view of an exemplary embodiment of the syringe pump assembly in accordance with an embodiment of the present disclosure;
Fig. 32 is another view of an exemplary embodiment of the syringe pump assembly in accordance with an embodiment of the present disclosure;
Fig. 33 is another view of an exemplary embodiment of the syringe pump assembly in accordance with an embodiment of the present disclosure;
Fig. 34 is another view of an exemplary embodiment of the syringe pump assembly in accordance with an embodiment of the present disclosure;
Fig. 35 is a view of an exemplary embodiment of the plunger head assembly, plunger tube, and sliding block assembly of the syringe pump assembly in accordance with an embodiment of the present disclosure;
Fig. 36 is another view of an exemplary embodiment of the plunger head assembly, plunger tube, and sliding block assembly of the syringe pump assembly in accordance with an embodiment of the present disclosure;
Fig. 37 is an exploded view of an exemplary embodiment of the top of the plunger head assembly with half of the plunger head assembly removed in accordance with an embodiment of the present disclosure;
Fig. 38 is an assembled view of an exemplary embodiment of the top of the plunger head assembly with half of the plunger head assembly removed in accordance with an embodiment of the present disclosure;
Fig. 39 is a bottom view of an exemplary embodiment of the top of the plunger head assembly in accordance with an embodiment of the present disclosure;
Fig. 40 is an assembled top view of an exemplary embodiment of the bottom of the plunger head assembly and plunger tube in accordance with an embodiment of the present disclosure;
Fig. 41 is an exploded view of an exemplary embodiment of the dial shaft and related parts of the syringe pump in accordance with an embodiment of the present disclosure;
Fig. 42 is an assembled view of the exemplary embodiment of Fig. 41 in accordance with an embodiment of the present disclosure;
Fig. 43 is a partially assembled view of an exemplary embodiment of the plunger head assembly and plunger tube in accordance with an embodiment of the present disclosure;
Fig. 44 is a view of an exemplary embodiment of the plunger head assembly with the plunger head assembly housing top removed in accordance with an embodiment of the present disclosure;
Fig. 45 is a top view of the exemplary embodiment of Fig. 44 in accordance with an embodiment of the present disclosure;
Fig. 46 is a partial view of an exemplary embodiment of the plunger head assembly in which the D-shaped connector is shown in cross section in accordance with an embodiment of the present disclosure;
Fig. 47 is a view of an exemplary embodiment of the plunger head assembly, plunger tube, and sliding block assembly in which the sliding block assembly is exploded in accordance with an embodiment of the present disclosure;
Fig. 48A is an exploded view of an exemplary embodiment of the sliding block assembly in accordance with an embodiment of the present disclosure;
Fig. 48B is a view an exemplary embodiment of the lead screw, half nut, barrel cam, and drive shaft in accordance with an embodiment of the present disclosure;
Fig. 49 is a partial front view of an exemplary embodiment of the half nut and barrel cam in which the half nut is shown as transparent in accordance with an embodiment of the present disclosure;
Fig. 50 is a front view of an exemplary embodiment of the sliding block assembly in which the half nut is in an engaged position in accordance with an embodiment of the present disclosure;
Fig. 51 is a front view of an exemplary embodiment of the sliding block assembly in which the half nut is in the engaged position in accordance with an embodiment of the present disclosure;
Fig. 52 is a front view of an exemplary embodiment of the sliding block assembly in which the half nut is in the disengaged position in accordance with an embodiment of the present disclosure;
Fig. 53 is a cross sectional view of an exemplary embodiment of the sliding block assembly on the lead screw and guide rod in accordance with an embodiment of the present disclosure;
Fig. 54 is a view of an exemplary embodiment of the rear face of the syringe pump assembly in accordance with an embodiment of the present disclosure;
Fig. 55 is another view of an exemplary embodiment of the rear face of the syringe pump assembly with the gearbox in place in accordance with an embodiment of the present disclosure;
Fig. 56 is an interior view of an exemplary embodiment of the syringe pump assembly in accordance with an embodiment of the present disclosure;
Fig. 57A is another interior view of an exemplary embodiment of the syringe pump assembly with the sliding block assembly and linear position sensors in place in accordance with an embodiment of the present disclosure;
Fig. 57B is a top view of an embodiment of a magnetic linear position sensor in accordance with an embodiment of the present disclosure;
Fig. 58 is a partially assembled front view of an exemplary embodiment of the sliding block assembly, plunger tube, and plunger head assembly in accordance with an embodiment of the present disclosure;
Fig. 59A is a view of an exemplary embodiment of the syringe pump assembly in accordance with an embodiment of the present disclosure;
Figs. 59B-59J are electrical schematics of the syringe pump in accordance with an embodiment of the present disclosure;
Fig. 60 is a bottom partial view of an exemplary embodiment of the syringe pump assembly in accordance with an embodiment of the present disclosure;
Fig. 61 is a partial view of an exemplary embodiment of the syringe pump assembly in which a barrel flange of a small syringe has been clipped by the barrel flange clip in accordance with an embodiment of the present disclosure;
Fig. 62 is a partial view of an exemplary embodiment of the syringe pump assembly in which a barrel flange of a large syringe has been clipped by the barrel flange clip in accordance with an embodiment of the present disclosure;
Fig. 63 is a view of an exemplary embodiment of the syringe barrel holder in accordance with an embodiment of the present disclosure;
Fig. 64 is a partial view of an exemplary embodiment of the syringe barrel holder in accordance with an embodiment of the present disclosure;
Fig. 65 is a view of an exemplary embodiment of the syringe barrel holder in which the syringe barrel holder is locked in the fully open position in accordance with an embodiment of the present disclosure;
Fig. 66 is a view of an exemplary embodiment the syringe barrel holder linear position sensor in which the linear position sensor printed circuit board is shown as transparent in accordance with an embodiment of the present disclosure;
Fig. 67 is a view of an exemplary embodiment of a phase change detector linear position sensor in accordance with an embodiment of the present disclosure;
Fig. 68 shows a schematic of the exemplary view of a phase change detector linear position sensor in accordance with an embodiment of the present disclosure;
Fig. 69 shows a schematic of the exemplary view of a phase change detector linear position sensor in accordance with an embodiment of the present disclosure;
Fig. 70 shows a schematic of the exemplary view of a phase change detector linear position sensor in accordance with an embodiment of the present disclosure;
Fig. 71 shows a perspective view of a pump with the graphic user interface shown on the screen in accordance with an embodiment of the present disclosure;
Fig. 72 shows an example infusion programming screen of the graphic user interface in accordance with an embodiment of the present disclosure;
Fig. 73 shows an example infusion programming screen of the graphic user interface in accordance with an embodiment of the present disclosure;
Fig. 74 shows an example infusion programming screen of the graphic user interface in accordance with an embodiment of the present disclosure;
Fig. 75 shows an example infusion programming screen of the graphic user interface in accordance with an embodiment of the present disclosure;
Fig. 76 shows an example infusion programming screen of the graphic user interface in accordance with an embodiment of the present disclosure;
Fig. 77 shows an infusion rate over time graphical representation of an example infusion in accordance with an embodiment of the present disclosure;
Fig. 78 shows an infusion rate over time graphical representation of an example infusion in accordance with an embodiment of the present disclosure;
Fig. 79 shows an infusion rate over time graphical representation of an example infusion in accordance with an embodiment of the present disclosure;
Fig. 80 shows an infusion rate over time graphical representation of an example infusion in accordance with an embodiment of the present disclosure;
Fig. 81 shows an infusion rate over time graphical representation of an example infusion in accordance with an embodiment of the present disclosure;
Fig. 82 shows an example drug administration library screen of the graphic user interface in accordance with an embodiment of the present disclosure;
Fig. 83 shows a block software diagram in accordance with an embodiment of the present disclosure;
Fig. 84 shows a state diagram illustrating a method of providing a watchdog functionality in accordance with an embodiment of the present disclosure;
Figs. 85A-85F show a circuit diagram of a watchdog system that is one embodiment that implements the watchdog functionality of the state diagram of Fig. 84 in accordance with another embodiment of the present disclosure;
Fig. 86 shows another embodiment of syringe pump having a bumper in accordance with an embodiment of the present disclosure;
Fig. 87 shows an exploded view of the syringe pump of Fig. 86 in accordance with an embodiment of the present disclosure;
Fig. 88 shows a close-up view of the upper housing, the lower housing, and the power supply of the syringe pump of Fig. 86 in accordance with an embodiment of the present disclosure;
Fig. 89A shows a front view of the display of the pump of Fig. 86 in accordance with an embodiment of the present disclosure;
Fig. 89B shows a back view of the display of the pump of Fig. 86 in accordance with an embodiment of the present disclosure;
Fig. 90 shows the back of the sensor portion of the touchscreen and a frame-based split-ring resonator of for use with a near-field antenna in accordance with an embodiment of the present disclosure;
Fig. 91 shows a diagram illustrating the use of the sensors of the pump of Fig. 86 when one or more of the sensors are unavailable in accordance with an embodiment of the present disclosure;
Fig. 92 shows a side view of a syringe pump having a retaining finger to retain a syringe in accordance with an embodiment of the present disclosure;
Fig. 93 shows a close-up view of the syringe pump of Fig. 92 in accordance with an embodiment of the present disclosure;
Fig. 94 shows a circuit for storing data within an RFID tag associated with a syringe pump in accordance with an embodiment of the present disclosure;
Fig. 95 shows an equivalent circuit for impedance as seen from the RFID tag of Fig. 94 in accordance with an embodiment of the present disclosure;
Fig. 96 shows another circuit for storing data within an RFID tag associated with a syringe pump in accordance with an embodiment of the present disclosure;
Fig. 97 shows a split-ring resonator used with the circuit of Fig. 96 in accordance with an embodiment of the present disclosure;
Fig. 98 shows a flow chart diagram illustrating a method for removing the effects of slack in a syringe pump having a syringe loaded on the syringe pump in accordance with an embodiment of the present disclosure;
Fig. 99A shows a perspective view of an apparatus for side loading a syringe onto an infusion pump showing a syringe securing arm of the apparatus in a loading position in accordance with an embodiment of the present disclosure;
Fig. 99B shows another perspective view of the apparatus of Fig. 99A showing the syringe securing arm in a securing position in accordance with an embodiment of the present disclosure;
Fig. 100A shows an embodiment of a force mechanism driving a syringe securing arm, the syringe securing arm shown in a securing position, in accordance with an embodiment of the present disclosure;
Fig. 100B shows the force mechanism driving the syringe securing arm of Fig. 100A with the syringe securing arm in a loading position in accordance with an embodiment of the present disclosure;
Fig. 101A shows another embodiment of a force mechanism driving a syringe securing arm, the syringe securing arm shown in a securing position, in accordance with an embodiment of the present disclosure;
Fig. 101B shows the force mechanism driving the syringe securing arm of Fig. 101A with the syringe securing arm in a loading position in accordance with an embodiment of the present disclosure;
Fig. 102A shows another embodiment of a force mechanism driving a syringe securing arm, the syringe securing arm shown in a loading position, in accordance with an embodiment of the present disclosure;
Fig. 102B shows the force mechanism driving the syringe securing arm of Fig. 102A with the syringe securing arm in a securing position in accordance with an embodiment of the present disclosure;
Fig. 103A shows another embodiment of a force mechanism driving a syringe securing arm, the syringe securing arm shown in a loading position, in accordance with an embodiment of the present disclosure;
Fig. 103B shows the force mechanism driving the syringe securing arm of Fig. 103A with the syringe securing arm in a securing position in accordance with an embodiment of the present disclosure;
Fig. 104A shows the cam of the force mechanisms of Figs. 103A-103B when the securing arm is in the securing position in accordance with an embodiment of the present disclosure;
Fig. 104B shows the cam of the force mechanisms of Figs. 103A-103B when the securing arm is in an intermediate position in accordance with an embodiment of the present disclosure;
Fig. 104C shows the cam of the force mechanisms of Figs. 103A-103B when the securing arm is in a loading position in accordance with an embodiment of the present disclosure;
Fig. 105 shows a flow chart diagram of a method for side loading a syringe on an infusion pump in accordance with an embodiment of the present disclosure;
Fig. 106 shows an embodiment of a system for mitigating lead screw runout error in accordance with an embodiment of the present disclosure;
Fig. 107 shows a flow chart diagram of a method for mitigating lead screw runout error in accordance with an embodiment of the present disclosure;
Fig. 108 shows a side view of a pump with a modular power supply attached to the back of the pump in according with an embodiment of the present disclosure;
Fig. 109 shows a side view of a pump with an external power supply in accordance with an embodiment of the present disclosure;
Fig. 110 shows a side view of a pump with a power supply attached to the bottom of the pump in accordance with an embodiment of the present disclosure;
Fig. 111 shows a side view of a pump with a power supply attached to the top of the pump in accordance with an embodiment of the present disclosure;
Fig. 112 shows a structure for securing a power cord to power supply in accordance with an embodiment of the present disclosure;
Fig. 113 shows a system having a rack with a power supply for powering several pumps secured to the rack in accordance with an embodiment of the present disclosure;
Figs. 114A-114J show several views of a syringe pump assembly in accordance with an embodiment of the present disclosure;
Figs. 115A-115B show two views of a retaining clip of the syringe pump assembly shown in Figs. 114A-114J in accordance with an embodiment of the present disclosure;
Figs. 116A-116C show several views of the syringe pump assembly shown in Figs. 114A-114J with the syringe seat removed in accordance with an embodiment of the present disclosure;
Figs. 117A-117C show several views of the syringe seat of the syringe pump assembly shown in Figs. 114A-114J in accordance with an embodiment of the present disclosure;
Fig. 118A-118B show several views of the syringe pump assembly shown in Figs. 114A-114J with the syringe seat removed in accordance with an embodiment of the present disclosure;
Figs. 119A-119B shows several views of the syringe pump assembly shown in Figs. 114A-114J to illustrate the jaw member's action of grasping onto a flange of a plunger of a syringe in accordance with an embodiment of the present disclosure;
Fig. 120 shows the plunger head with the cover removed of the syringe pump assembly shown in Figs. 114A-114J to illustrate the mechanical effects of rotation of the dial in accordance with an embodiment of the present disclosure;
Figs. 121A-121C show several views of the plunger head with the cover removed and a circuit board removed of the syringe pump assembly shown in Figs. 114A-114J to illustrate the mechanical effects of rotation of the dial in accordance with an embodiment of the present disclosure;
Figs. 122A-122B show two views of a cam used within the plunger head assembly of the syringe pump assembly shown in Figs. 114A-114J in accordance with an embodiment of the present disclosure;
Figs. 123A-123B show two close-up views of the inner cavity of the plunger head assembly of the syringe pump assembly shown in Figs. 114A-114J in accordance with an embodiment of the present disclosure;
Fig. 124 shows the plunger head assembly of the syringe pump assembly shown in Figs. 114A-114J in accordance with an embodiment of the present disclosure;
Figs. 125A-125B show two views of the plunger head assembly of the syringe pump assembly shown in Figs. 114A-114J with the plunger tube removed in accordance with an embodiment of the present disclosure;
Figs. 126A-126I show several additional views of the syringe pump assembly of Figs. 114A-114J in accordance with an embodiment of the present disclosure;
Fig. 127 shows a perspective, side-view of the syringe pump assembly shown in Figs. 114A-114J in accordance with an embodiment of the present disclosure wherein the assembly is coupled to a display;
Fig. 128 shows a flow chart diagram of a method for discharging fluid from a syringe and for providing mitigation for an occlusion condition in accordance with an embodiment of the present disclosure;
Fig. 129 shows a syringe pump assembly in accordance with another embodiment of the present disclosure;
Fig. 130 shows a close-up view of the plunger head of the syringe pump assembly of Fig. 129 in accordance with an embodiment of the present disclosure;
Fig. 131 shows the same view of Fig. 130 with the retaining members removed to facilitate viewing of the pressure sensor assembly in accordance with an embodiment of the present disclosure;
Fig. 132 shows the back of the plunger head assembly with the back cover removed to facilitate viewing of the two pressure sensors of the pressure sensor assembly in accordance with an embodiment of the present disclosure;
Fig. 133 shows the pressure sensor assembly without the pressure sensors in accordance with an embodiment of the present disclosure;
Fig. 134 shows an exploded view of the pressure sensor assembly of Fig. 133 without the pressure sensors in accordance with an embodiment of the present disclosure;
Fig. 135 shows another exploded view of the pressure sensor assembly of Fig. 133 without the pressure sensors in accordance with an embodiment of the present disclosure;
Fig. 136 shows yet another exploded view of the pressure sensor assembly of Fig. 133 without the pressure sensors in accordance with an embodiment of the present disclosure;
Fig. 137 shows a cross-sectional view of the pressure sensor assembly without the pressure sensors of Fig. 133 in accordance with an embodiment of the present disclosure;
Fig. 138 shows another cross-sectional view of the pressure sensor assembly without the pressure sensors of Fig. 133 in accordance with an embodiment of the present disclosure;
Fig. 139 shows the cross-sectional view of the pressure sensor assembly of Fig. 138 with the pressure sensors in accordance with an embodiment of the present disclosure;
Fig. 140 shows the cross-sectional view of Fig. 139 with the pressure sensor assembly in the plunger head assembly in accordance with an embodiment of the present disclosure;
Fig. 141 shows a method for occlusion detection in accordance with an embodiment of the present disclosure;
Fig. 142 shows a method of monitoring a syringe pump's sensors for occlusion detection in accordance with an embodiment of the present disclosure;
Fig. 143 shows a methodology for performing a test used by the method illustrated in Fig. 141 in accordance with an embodiment of the present disclosure;
Fig. 144 shows another methodology for performing a test used by the method illustrated in Fig. 141 in accordance with an embodiment of the present disclosure;
Fig. 145 shows a yet another methodology for performing a test used by the method illustrated in Fig. 141 in accordance with an embodiment of the present disclosure;
Fig. 146 shows a methodology for transitioning from a start-up phase to a steady-state phase of a syringe pump within the method of Fig. 141 in accordance with an embodiment of the present disclosure;
Fig. 147 show a graphic illustration of a syringe pump transitioning from a start-up phase to a steady-state phase in accordance with an embodiment of the present disclosure;
Fig. 148 shows a flow chart diagram used to illustrate a method for recovering from an occlusion in accordance with an embodiment of the present disclosure.

### DETAILED DESCRIPTION

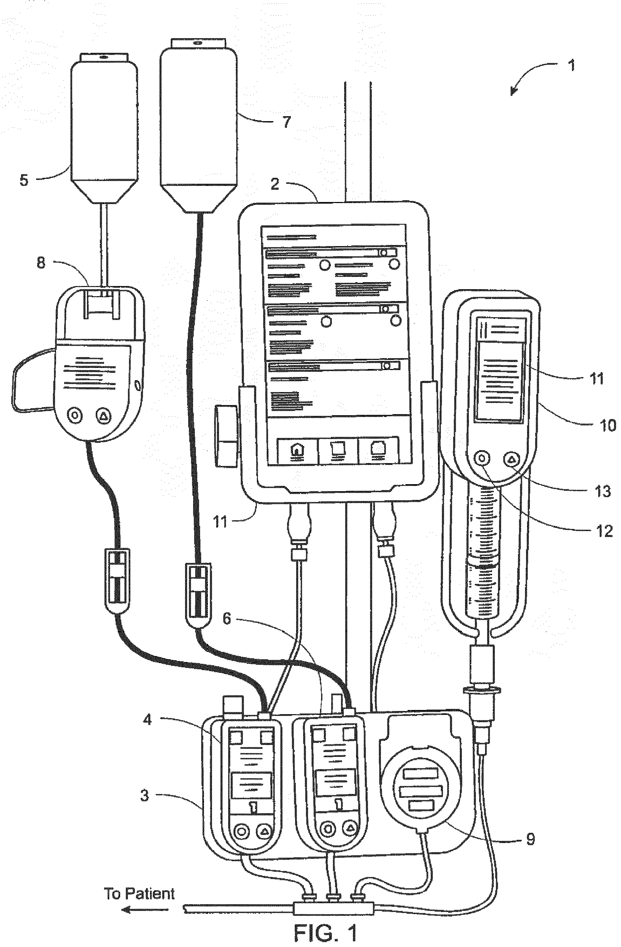

Fig. 1 shows an exemplary arrangement of a system 1 for electronic patient care in accordance with an embodiment of the present disclosure. The system 1 includes a monitoring client 2 that is linked to a number of patient-care devices via docks 3 and 11, including an infusion pump 4 connected to and delivering from a smaller bag of liquid 5, an infusion pump 6 connected to and delivering from a larger bag of liquid 7, a drip detection device 8 connected to tubing from the smaller bag 5, and a microinfusion pump 9. System 1 also includes a syringe pump 10 connected wirelessly to the monitoring client 2. In some embodiments, the monitoring client 2 may communicate with these patient-care devices in a wired fashion, as shown in Fig. 1 for the infusion pumps 4 and 6, and the microinfusion pump 9 (via docks 3 and 11). Additionally or alternatively, the monitoring client 2 may communicate wirelessly with patient-care devices, as suggested by the absence of a wired connection between the syringe pump 10 and the monitoring client 2.

In some embodiments, a wired connection between the monitoring client 2 and a patient-care device also affords an opportunity for electrical power to be supplied to the patient-care device from the monitoring client 2. In this exemplary embodiment, the monitoring client 2 may include the electronic circuitry necessary to convert the voltage to power the patient-care device from either a battery attached to the monitoring client 2 or from an Alternating Current ("AC") line voltage fed into the monitoring client 2 from a power outlet (not shown) in a patient's room. Additionally or alternatively, the dock 3 supplies power to the infusion pumps 4 and 6, and to the microinfusion pump 9, e.g., from a signal generated from an AC line voltage.

In an embodiment, the monitoring client 2 is capable of receiving information about each patient-care device with which it is linked either directly from the device itself, or via a docking station, such as, for example, the dock 3 onto which the patient-care device may be mounted. The dock 3 may be configured to receive one or more patient-care devices via a standardized connection mount, or in some cases via a connection mount individualized for the particular device. For example, infusion pumps 4 and 6 may be mounted to the dock 3 via a similar connection mount, whereas the microinfusion pump 9, for example, may be mounted to the dock 3 via a connection mount configured for the particular dimensions of the microinfusion pump's 9 housing.

The dock 3 may be configured to electronically identify the particular patient-care device being mounted on the docking station, and to transmit this identifying information to the monitoring client 2, either wirelessly or via a wired connection. Additionally or alternatively, wireless patient-care devices may transmit the identifying information wirelessly to the monitoring client 2, e.g., during a discovery protocol. Additionally, the particular patient-care device may be preprogrammed with treatment information (e.g., patient-treatment parameters such as an infusion rate for a predetermined infusion liquid) that is transmitted to the monitoring client 2. For example, the syringe pump 10 may include identity information and treatment information, such as what medication has been prescribed to the patient, what liquid is within the syringe pump's 10 reservoir, how much and how long the liquid is prescribed to be delivered to the patient, who are the authorized caregivers, etc. In some embodiments of the present disclosure, the monitoring client 2 communicates with EMR records to verify that the preprogrammed treatment information is safe for an identified patient and/or the preprogrammed treatment information matches the prescribed treatment stored in the EMR records.

In some embodiments, the drip detection device 8 may communicate with the monitoring client 2 either wirelessly or in a wired connection. If an aberrant liquid flow condition is detected (e.g., because the tubing to the patient has become occluded), a signal may be transmitted to monitoring client 2, which (1) may display the flow rate of liquid from the liquid container 5 in a user interface either locally on the monitoring client 2, or more remotely to a user interface at a nurse's station or a handheld communications device, (2) may trigger an auditory or visual alarm, and/or (3) may cause the monitoring client 2 to alter the rate of infusion of a pump 4 connected to a bag 5, by either terminating the infusion or otherwise changing the pumping rate The aberrant liquid flow condition may also cause an audible alarm (and/or vibration alarm) on the infusion pump 4 or the drip detection device 8, or cause the infusion pump 4 to modify or stop the pumping, e.g., when the aberrant liquid flow condition exceed predefined ranges of operation.

The alarms may occur simultaneously on several devices or may follow a predetermined schedule. For example, when an occlusion occurs in a line connected to the infusion pump 4, (1) the drip detection device 8 alarms using its internal speaker and an internal vibration motor, (2) thereafter, the infusion pump 4 alarms using its internal speaker and an internal vibration motor, (3) next, the monitoring client 2 alarms using its internal speaker and an internal vibration motor, and (4) finally, a remote communicator (e.g., a smart phone, blackberry-based phone, Android-based phone, iphone, etc.) alarms using its internal speaker and an internal vibration motor. In some embodiments, the syringe pump 10 may be connected to the drip detection device 8 and detect aberrant liquid flow conditions as described above.

In some embodiments, the syringe pump 10 may be programmable to allow for continued operation at a predetermined pumping rate should communications fail between the monitoring client 2 and the syringe pump 10, either because of a malfunction in the monitoring client 2, in the communications channel between the monitoring client 2 and the syringe pump 10, or in the syringe pump 10 itself. In some embodiments, this independent function option is enabled when the medication being infused is pre-designated for not being suspended or held in the event of a malfunction in other parts of the system. In some embodiments, the syringe pump 10 is programmed to operate independently in a fail safe mode and may also be configured to receive information from a drip detection device 8 directly, rather than through a monitoring client 2 (e.g., in embodiment where the drip detection device 8 is used in conjunction with the syringe pump 10); with this option, the syringe pump 10 may be programmed, in some embodiments, to stop an infusion if the drip detection device 8 detects an aberrant flow condition (such as, e.g., a freeflow condition or an air bubble present in the infusion line). In some embodiments, one or more of the pumps 4, 6, and 10 may have internal liquid flow meters and/or can operate independently as a stand-alone device. Additionally or alternatively, an internal liquid flow meter of the syringe pump 10 may be independently determined by a flow meter of the drip detection device 8 by the monitoring client 2, in embodiments where the devices 8 and 10 are used together.

The monitoring client 2 may also remotely send a prescription to a pharmacy. The prescription may be a prescription for infusing a fluid using the syringe pump 10. The pharmacy may include one or more computers connected to a network, e.g., the internet, to receive the prescription and queue the prescription within the one or more computers. The pharmacy may use the prescription to compound the drug (e.g., using an automated compounding device coupled to the one or more computers or manually by a pharmacists viewing the queue of the one or more computers), pre-fill a fluid reservoir or cartridge of a syringe pump 10, and/or program the syringe pump 10 (e.g., a treatment regime is programmed into the syringe pump 10) at the pharmacy in accordance with the prescription. The reservoir or cartridge may be automatically filled by the automated compounding device and/or the syringe pump 10 may be automatically programmed by the automated compounding device. The automated compounding device may generate a barcode, RFID tag and/or data. The information within the barcode, RFID tag, and/or data may include the treatment regime, prescription, and/or patient information. The automated compounding device may: attach the barcode to the syringe pump 10 or to the reservoir, cartridge, or disposable portion of the syringe pump 10; attach the RFID tag to the syringe pump 10 or the reservoir, cartridge, or disposable portion of the syringe pump 10; and/or program the RFID tag or memory within the syringe pump 10 or the reservoir, cartridge, or disposable portion of the syringe pump 10 with the information or data. The data or information may be sent to a database that associates the prescription with the syringe pump 10 or the reservoir, cartridge, or disposable portion of the syringe pump 10, e.g., using a serial number or other identifying information within the barcode, RFID tag, or memory.

The syringe pump 10 may have a scanner, e.g., an RFID interrogator that interrogates a reservoir, disposable portion, or cartridge of the syringe pump 10 to determine that it is the correct fluid within the fluid reservoir or it is the correct fluid reservoir, disposable portion or cartridge, the treatment programmed into the syringe pump 10 corresponds to the fluid within the fluid reservoir, disposable portion or cartridge, and/or the syringe pump 10 and reservoir, disposable portion or cartridge of the syringe pump 10 are correct for the particular patient (e.g., as determined from a patient's barcode, RFID, or other patient identification). For example, a serial number of a reservoir, disposable portion as scanned by the syringe pump 10 is compared to a serial number in electronic medical records to determine if it correctly corresponds to a patient's serial number within the electronic medical records; the syringe pump 10 may scan a RFID tag or barcode of a patient to obtain a serial number of a patient which is also compared to the patient's serial number within the electronic medical records (e.g., the serial number of a reservoir, disposable portion, or cartridge of the syringe pump 10 or a serial number stored within memory of the syringe pump 10 should be associated with the patient's serial number as scanned within the electronic medical records). The syringe pump 10 may issue an error or alarm if the serial numbers do not match, in some specific embodiments. Additionally or alternatively, the monitoring client 2 may scan the reservoir, disposable portion, cartridge, or syringe pump 10 to determine that it is the correct fluid within the fluid reservoir, it is the correct fluid reservoir, the treatment programmed into the syringe pump 10 corresponds to the fluid within the fluid reservoir or cartridge, and/or the fluid reservoir and syringe pump 10 are correct for the particular patient (e.g., as determined from a patient's barcode, RFID, or other patient identification). Additionally or alternatively, the monitoring client 2 or syringe pump 10 may interrogate an electronic medical records database and/or the pharmacy to verify the prescription or download the prescription, e.g., using a barcode serial number on the syringe pump 10, or a reservoir, cartridge, or disposable portion of the syringe pump 10.

The liquid being delivered to a patient may be monitored by the monitoring client 2 to determine if all the medications being delivered are safe for the patient. For example, the monitoring client 2 may log the medication delivered from the syringe pump 10 as communicated by the syringe pump 10 to the monitoring client 2, and the monitoring client 2 may also log the medication being delivered by the infusion pumps 4 and 6, and/or the microinfusion pump 9. The monitoring client 1 may make a determination from the logged data to determine if the aggregate amounts and types of medication being delivered are safe. For example, the monitoring client 2 may determine if the IV bag 5 is contraindicated with the medication in the syringe pump 10. Additionally or alternatively, in some embodiments, the monitoring client 2 may monitor the delivery of the liquid in the IV bag 8 and one or more boluses delivered by the syringe pump 10 to determine if the total dose exceeds a predetermined threshold, e.g., the medication in the IV bag 5 and syringe pump 10 may be the same type or class of drug, and the monitoring client 2 may determine if the drugs are safe when combined as delivered to the patient. The syringe pump 10 may also communicate with the infusion pumps 4 and 6, and/or the microinfusion pump 9 to make the same determination; In this exemplary embodiment, the syringe pump 10 may communicate with the devices directly (via wirelessly or wired communications) or through the monitoring client 2 (via wirelessly or wired communications). In some embodiments of the present disclosures, one or more communication modules (e.g., each having the capabilities to communicate via one or more protocols) may be connected to the syringe pump 10 and/or may be connected together and then connected to the syringe pump 10 to enable the syringe pump 10 to communicate via the communication modules.

The syringe pump 10 includes a touch screen interface 11 (which may be detachable), a start button 12, and a stop button 13. However, in some alternative embodiments, the button 12 is a PCA button to deliver pain medicine to a patient. The user interface 11 may be used to program treatment regimes, such as flow rates, bolus amounts, or other treatment parameters. After a treatment regime is programmed into the syringe pump 10, the syringe pump 10 may query a database (e.g., Electronic Medical Records ("EMR"), Drug Error Reduction System ("DERS"), or other database) to determine if the treatment regime is safe for the particular patient or for any patient. For example, the syringe pump 10 may query the EMR database (e.g., via a wireless link, wired link, WiFi, cell-phone network, or other communications technology) to determine if the treatment regime from the syringe pump 10 is safe based upon patient information stored (e.g., age, weight, allergies, condition, etc.) in the EMR records. Additionally or alternatively, the syringe pump 10 may query the DERS database (e.g., via a wireless link, wired link, WiFi, cell-phone network, or other communications technology) to determine if the treatment regime from the syringe pump 10 is safe based upon predetermined safety criteria in the DERS records

In some embodiments, if the treatment regime is determined to be safe, a prompt may request user confirmation of the treatment regime. After user confirmation, the user (e.g., caregiver, nurse, or other authorized person) may press the start button 12. In some embodiments, the stop button 13 may be pressed at any time to stop treatment.

In some embodiments, if the EMR and/or DERS determines that the treatment regime exceeds a first set of criteria, treatment may continue if the user confirms the treatment (e.g., with an additional warning, user passcode, and/or additional authentication or authorization, etc.); in this embodiment, the EMR or DERS may prevent the treatment from being delivered if the EMR and/or DERS determines that the treatment regime exceeds a second set of criteria, e.g., the treatment is not safe under any circumstances for any patient, for example.

### EXEMPLARY BEDSIDE ARRANGEMENT

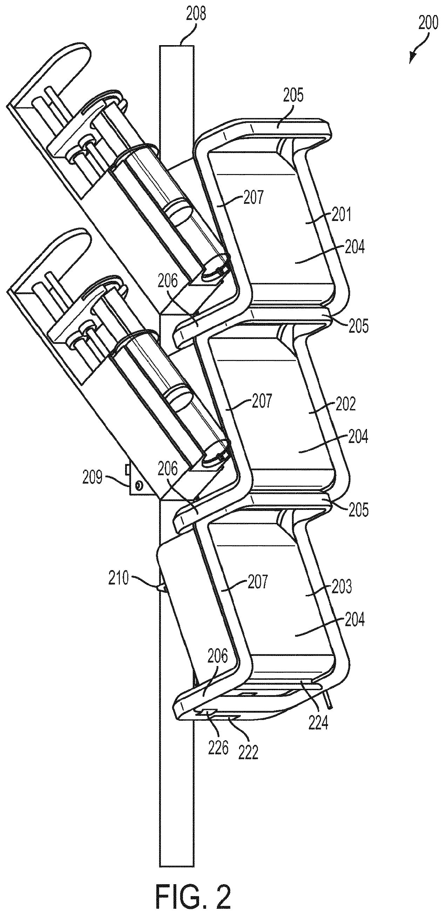

**Figs. 2-9** show various views related to a system 200. **Fig. 2** shows a system 200 that includes several pumps 201, 202, and 203. The pumps 201, 202, 203 can be coupled together to form a group of pumps that are connectable to a pole 208. The system 200 includes two syringe pumps 201, 202 and a peristaltic pump 203; however, other combinations of various medical devices may be employed.

Each of the pumps 201, 202, 203 includes a touch screen 204 which may be used to control the pumps 201, 202, 203. One of the pumps' (e.g., 201, 202, 203) touch screen 204 may also be used to coordinate operation of all of the pumps 201, 202, 203 and/or to control the other ones of the pumps 201, 202, 203.

The pumps 201, 202, and 203 are daisy chained together such that they are in electrical communication with each other. Additionally or alternatively, the pumps 201, 202, and/or 203 may share power with each other or among each other; For example, one of the pumps 201, 202, and/or 203 may include an AC/DC converter that converts AC electrical power to DC power suitable to power the other pumps.

Within the system 200, the pumps 201, 202, and 203 are stacked together using respective Z-frames 207. Each of the Z-frames 207 includes a lower portion 206 and an upper portion 205. A lower portion 206 of one Z-frame 207 (e.g., the lower portion 206 of the pump 201) can engage an upper portion 205 of another Z-frame 207 (e.g., the upper portion 205 of the Z-frame 207 of the pump 202).

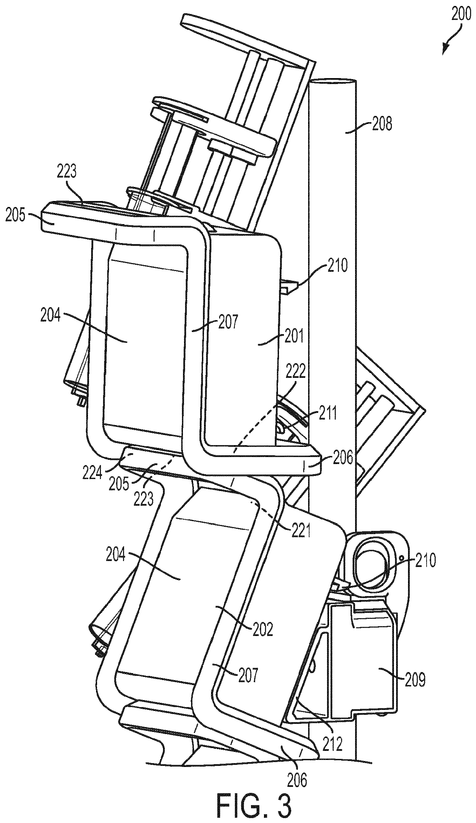

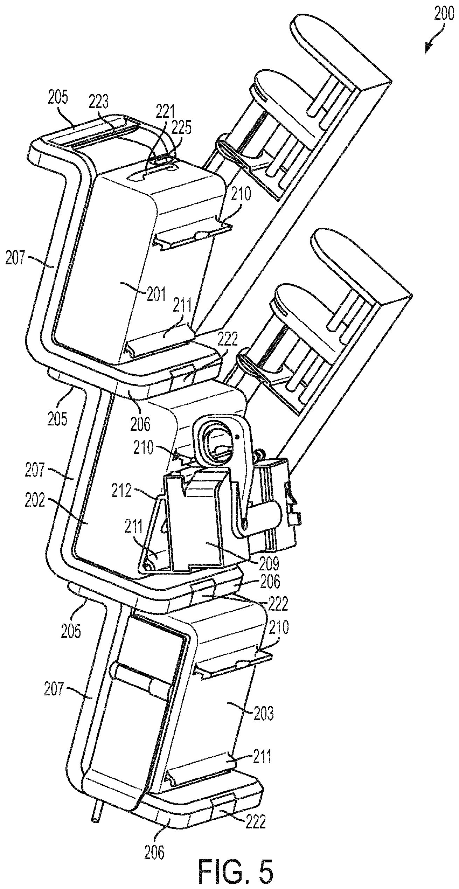

A clamp 209 may be coupled to one of the pumps 201, 202, 203 (e.g., the pump 202 as shown in **Fig. 3**). That is, the clamp 209 may be coupled to any one of the pumps 201, 202, 203. The clamp 209 is attachable to the back of any one of the pump 201, 202, 203. As is easily seen in **Fig. 5****,** each of the pumps 201, 202, 203 includes an upper attachment member 210 and a lower attachment member 211. A clamp adapter 212 facilitates the attachment of the clamp 209 to the pump 202 via a respective pump's (e.g., 201, 202, or 203) upper attachment member 210 and lower attachment member 211. In some embodiments, the clamp adapter 212 may be integral with the clamp 209.

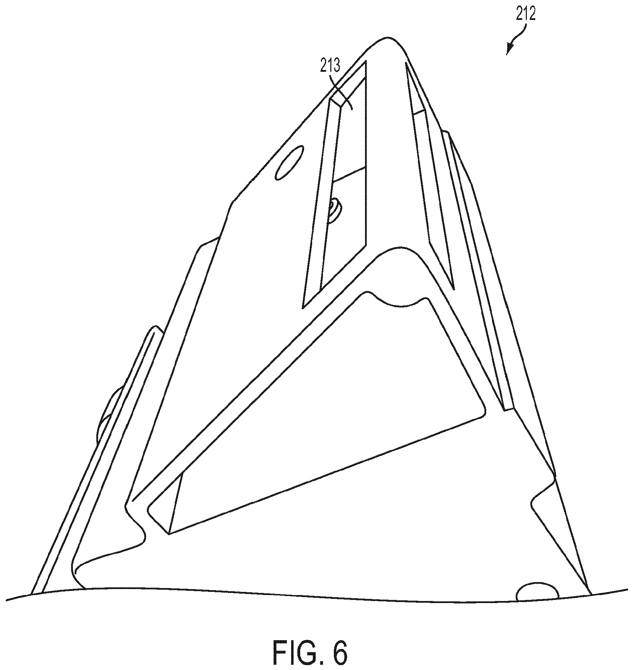

**Fig. 6** shows a close-up view of a portion of an interface of a clamp (i.e., the clamp adapter 212) that is attachable to the pump 202 (or to pumps 201 or 203) shown in **Figs. 2-5** in accordance with an embodiment of the present disclosure. The clamp adapter 212 includes a hole 213 in which a lower attachment member 211 (see **Fig. 5**) may be attached to. That is, the lower attachment member 211 is a curved hook-like protrusion that may be inserted into the hole 213 and thereafter rotated to secure the lower attachment member 211 therein.

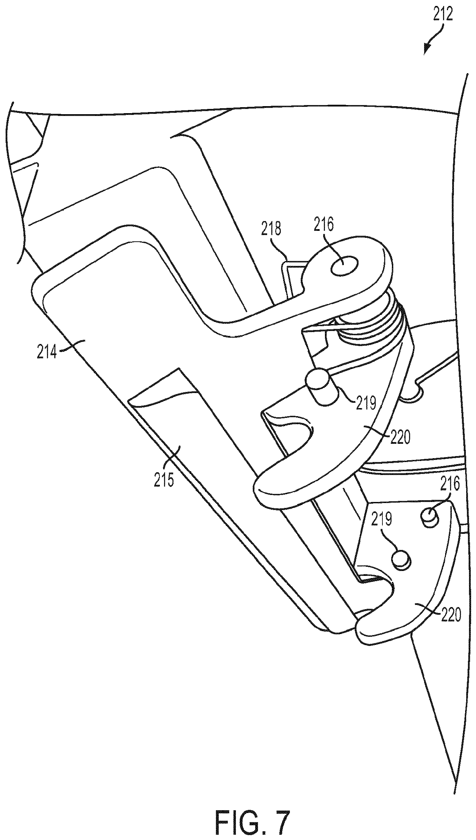

As is easily seen in **Fig. 7****,** the clamp adapter 212 also includes a latch 214. The latch 214 is pivotally mounted to the clamp adapter 212 via pivots 216. The latch 214 may be spring biased via springs 218 that are coupled to the hooks 220. Stop members 219 prevent the latch 214 from pivoting beyond a predetermined amount. After the hole 213 is inserted into the lower attachment member 211 (see **Figs. 5** and **6**), the clamp adapter 212 may be rotated to bring the latch 214 towards the upper attachment member 210 such that the latch 214 is compressed down by the upper attachment member 210 until the protrusion 215 snaps into a complementary space of the upper attachment member 210. The hooks 220 help secure the clamp adapter 212 to the pump 202.

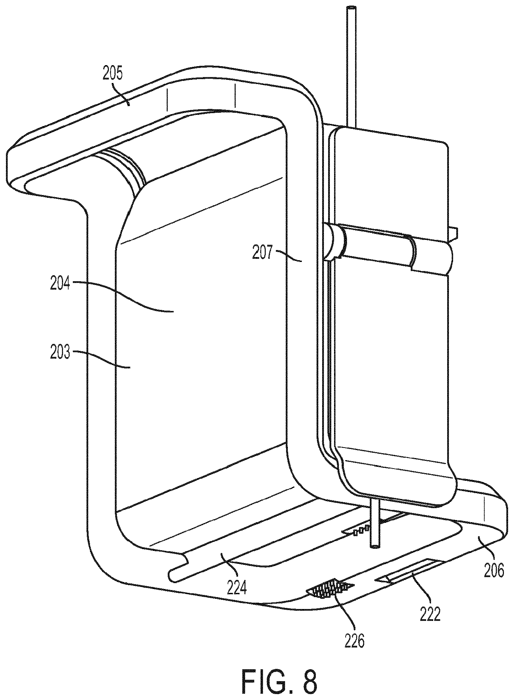

Each Z-frame 207 of the pumps 201, 202, 203 includes a recessed portion 223 (see **Fig. 5**) and a protrusion 224 (see **Fig. 8**). A protrusion 224 of the Z-frame 207 of one pump (e.g., pumps 201, 202, or 203) may engage a recessed portion 223 of another pump to enable the pump to be stacked on top of each other. Each of the pumps 201, 202, 203 includes a latch engagement member 221 that allows another one of the pumps 201, 202, 203 to be attached thereto via a latch 222 (see **Fig. 8**). The latch 222 may include a small spring loaded flange that can "snap" into the space formed under the latch engagement member 221. The latch 222 may be pivotally coupled to the lower portion 206 of the Z-frame 207.

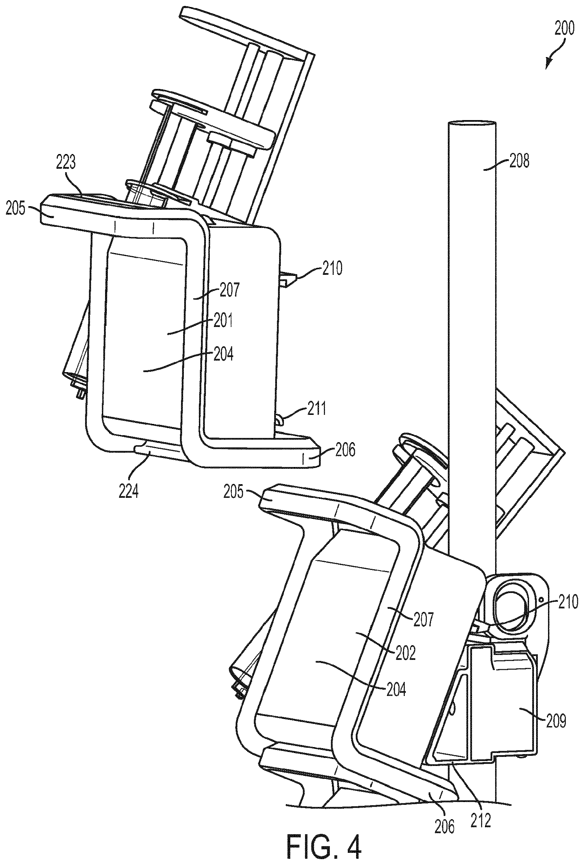

As is seen in **Fig. 3**, the latch 222 of the pump 201 may be pulled to withdraw a portion of the latch 222 out of the space under the latch engagement member 221 of the pump 202. Thereafter, the pump 201 may be rotated to pull out the protrusion 224 of the pump 201 out of the recessed portion 223 of the Z-frame 207 of the pump 202 such that the pump 201 may be removed from the stack of pumps 202, 203 (see **Fig. 4**).

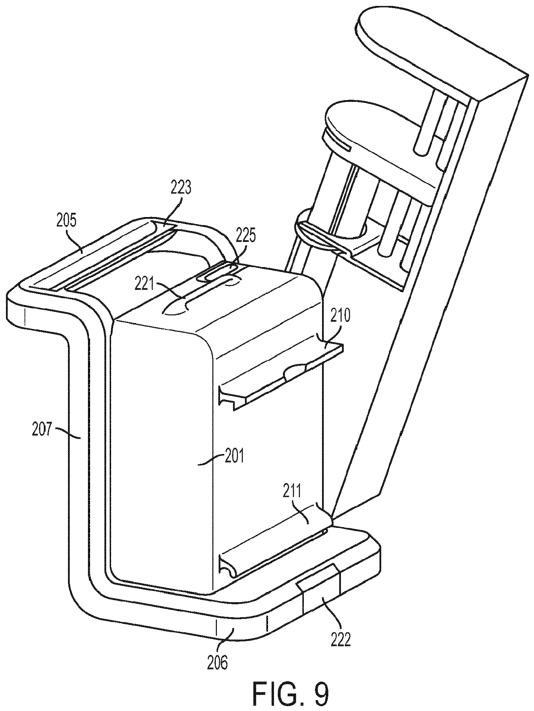

Each of the pumps 201, 202, 203 includes a top connector 225 (see **Fig. 9**) and a bottom connector 226 (see **Fig. 8**). The connectors 225 and 226 allow the stacked pumps 201, 202, and 203 to communication between each other and/or to provide power to each other. For example, if the battery of the middle pump 202 (see **Fig. 2**) fails, then the top pump 201 and/or the bottom pump 203 may provide power to the middle pump 202 as a reserve while audibly alarming.

### EXEMPLARY SYRINGE PUMP EMBODIMENT AND RELATED BEDSIDE ARRANGEMENT

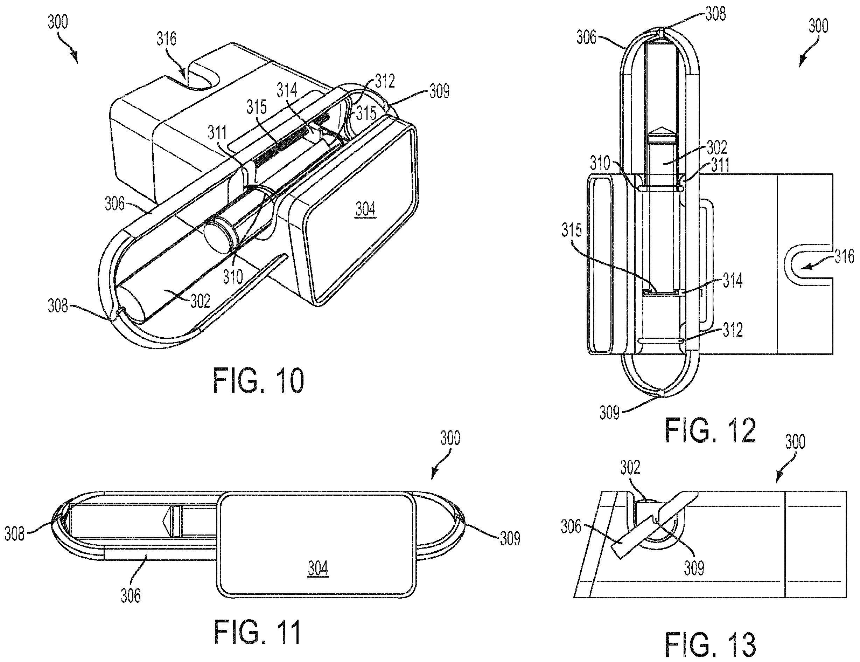

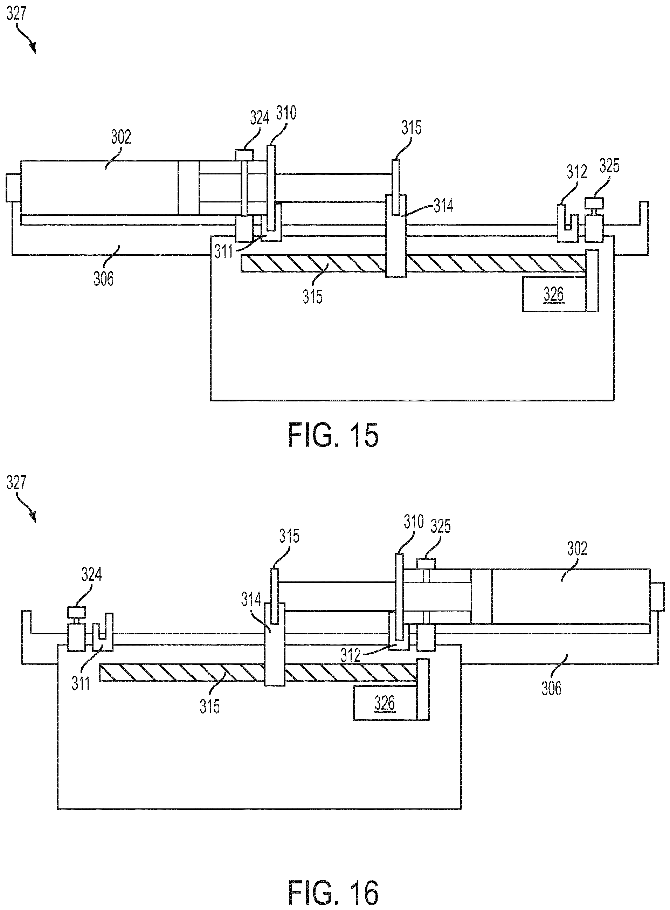

**Figs. 10-13** show several views of a syringe pump 300 in accordance with an embodiment of the present disclosure. The syringe pump 300 may have a syringe 302 loaded either facing to the left (as shown in **Figs. 10-13**) or to the right (refer to **Fig. 16****,** described below). That is, the syringe pump 300 is a bidirectional syringe pump.

The syringe 302 may be loaded into a syringe holder 306 of the syringe pump 300. The flange endpiece 31 0 of the syringe 302 may be placed in the left flange receiver 311 or in the right flange receiver 312. When the flange endpiece 310 is inserted into the left flange receiver 311, the syringe 302 faces towards the left outlet 308, which may hold a tube that is fluidly coupled to the syringe 302. An engagement member 314 may be coupled to an end fitting 315 of the syringe 302 when or after the syringe 302 is loaded into the syringe holder 306. A threaded shaft 315 that is coupled to a motor may be rotated to move the engagement member 314 in any direction to discharge fluid from the syringe 302.

The syringe 302 may also be loaded to the right (not shown in **Figs. 10-13**). The syringe holder 306 may be moved and/or adjusted such that it is moved to the right so the syringe 302 may be loaded. The syringe holder 306 may be manually moved and/or an electric motor may move the syringe holder 306 to the right. In some embodiments of the present disclosure, the syringe holder 306 extends sufficiently to the left and to the right such that no adjustment is used.

In the case where the syringe 302 is loaded facing the right, the flange endpiece 310 is loaded into the right flange receiver 312. The engagement member 314 thereafter moves to the right such that fluid may be discharged through a tube that traverses through a right outlet 309.

The pump 300 may be controlled via a touch screen 304 to set the flow rate, flow profile, and/or to otherwise monitor or control the syringe pump 300. A clamp 316 may be used to secure the syringe pump 300 to a pole (e.g., using a screw-type clamp).

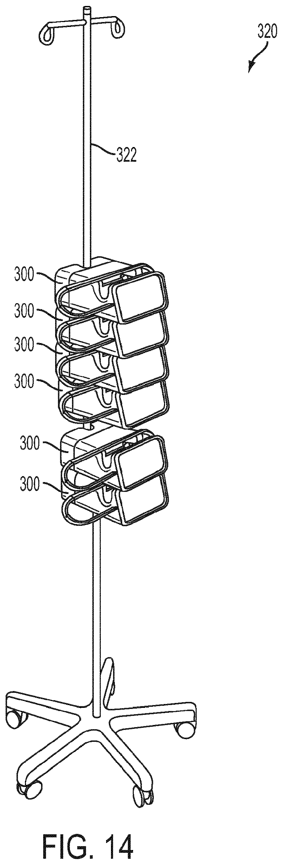

**Fig. 14** shows several of the syringe pumps 300 of **Figs. 10-13** mounted on a pole 322 in accordance with an embodiment of the present disclosure. That is, **Fig. 14** shows a system 320 that uses several syringe pumps 300 mounted on the pole 312. The pole 322 may be used in a hospital and/or in a home setting.

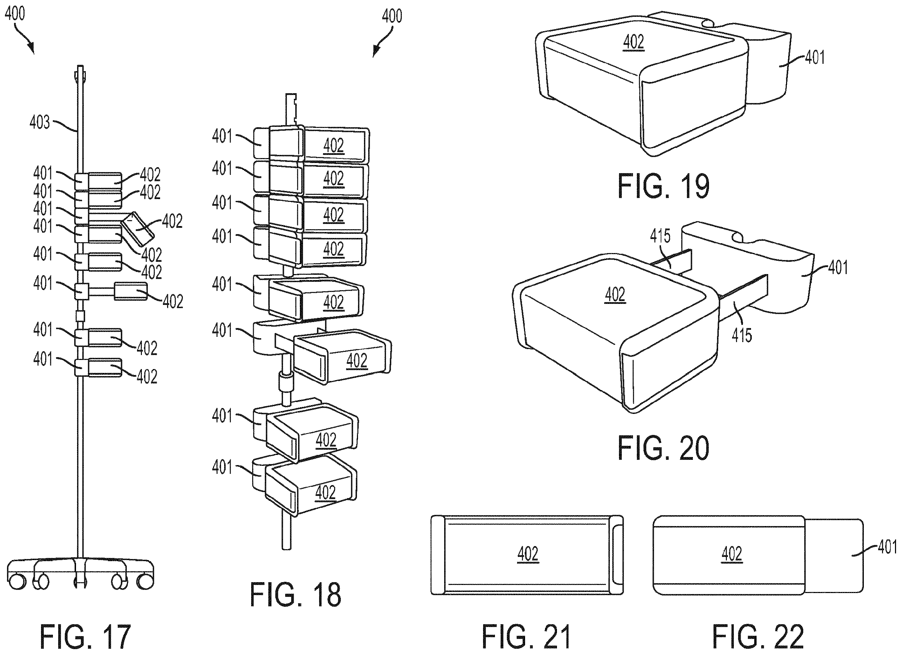

**Figs. 15-16** illustrate portions 327 of the operation of the syringe pump 300 of Figs. 21-24 in accordance with an embodiment of the present disclosure. **Fig. 15** shows the syringe 302 loaded facing the left, and **Fig. 16** shows the syringe 302 loaded to the right. As shown in **Figs. 15-16****,** a motor 326 is coupled to the threaded shaft 315 such that the motor 326 can rotate the threaded shaft 315.

A left syringe diameter sensor 324 measures the diameter of the syringe 305 to estimate the cross-sectional size of the internal space of the barrel of the syringe 302. The left syringe diameter sensor 325 may be a bar that is attached to a post such that the bar is lifted to cover the syringe 302; the post's movement out of the body of the syringe pump 300 may be measured by a linear sensor to estimate the diameter of the barrel of the syringe 302. Any linear sensor may be used including a linear potentiometer technology, an optical linear sensor technology, a hall-effect sensor technology, etc. The motor's 326 movement may thereby be correlated to fluid discharged from the syringe 302 using the estimate of the diameter of the internal space of the barrel of the syringe 302. Similarly, the right syringe diameter sensor 325 may be used to estimate the internal diameter of the barrel of the syringe 302, which may be used to estimate the fluid discharged from the syringe 302 to the right.

In some embodiments of the present disclosure, the touch screen 304 requests information from the user when the syringe 302 is loaded into the syringe pump 300 (in either the left or right configuration) and the syringe diameter sensor 324 and/or 325 is used to estimate the diameter of the internal space of the barrel of the syringe 305; The user is prompted by a touch screen 304 request for the user to enter into the touch screen 304 the manufacturer of the syringe 305. An internal database within the syringe pump 300 may be used to narrow down the range of possible model numbers associated with an estimate of the diameter of the syringe 305. When the user enters in the manufacturer of the syringe 305, the database may be used to identify a particular model number of the syringe 305 and/or a subset of possible model numbers corresponding to the estimate of the diameter of the syringe 305 and the user entered information, which in turn, may provide a more accurate internal diameter value (as stored within the database). The user may be prompted by the display on the touch screen 304 to select the syringe model from a list or enter the model of the syringe that will deliver the medication. The user may be guided through a selection process on the touchscreen 304 to identify the syringe loaded into the machine using one or more of the following aspects: syringe barrel size, plunger head size, manufacturer names, images of syringes, and model numbers. The selection process may access a database of syringes including manufacturer, model, internal diameter and image. The syringe pump 300 may use the identified syringe to set the internal diameter value for volume calculations.

### EXEMPLARY BEDSIDE ARRANGEMENTS

**Figs. 17-18** illustrate several medical devices 402 mounted on a pole 403 in accordance with an embodiment of the present disclosure. **Figs. 19-22** show several views of the medical device 402 of **Figs. 17-18****.** The medical device 402 is mounted to the pole via the clamp 401. The clamp 401 allows the medical device 402 to be pulled out and adjusted. The medical device 402 may be any medical device, such as an infusion pump, a syringe pump, a monitoring client, etc.

The medical device 402 is coupled to the pole 403 via arms 403 such that the medical device 402 may be pulled away from the pole (see **Fig. 20**) and/or pivoted on the arms 403.

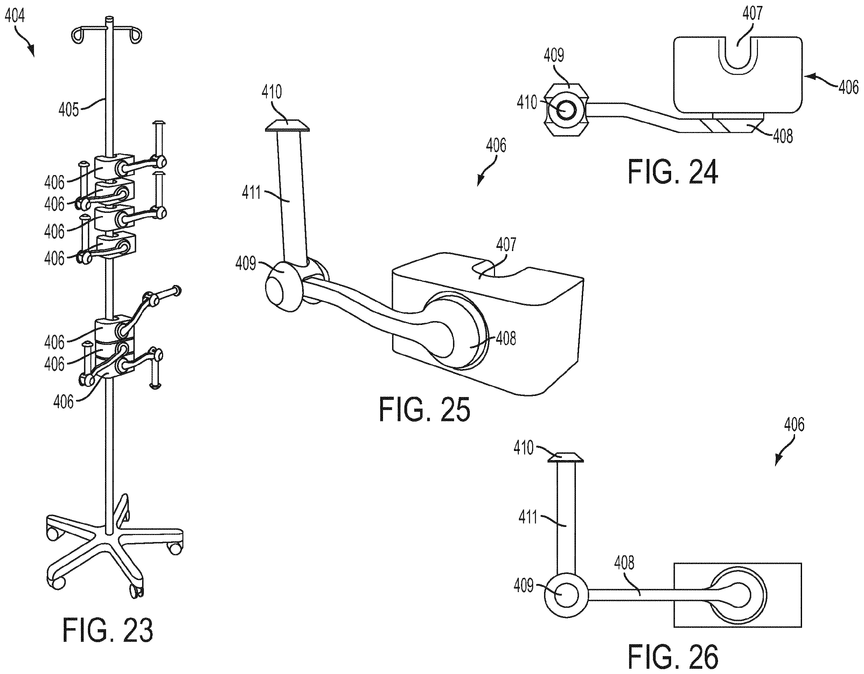

**Fig. 23** shows several mounts 406 mounted on a pole 405, and **Figs. 24-26** show several views of a mount of **Fig. 23** in accordance with an embodiment of the present disclosure. Each of the mounts 406 includes a clamp 407 (e.g., a screw-type clamp), a first arm 408 pivotally mounted to the clamp 407, and a second arm 411 pivotally mounted to the first arm 408 via a hinge 409. The end of the second arm 411 includes a coupling member 410 that can be coupled to a medical device.

### EXEMPLARY BATTERY AND SPEAKER TEST

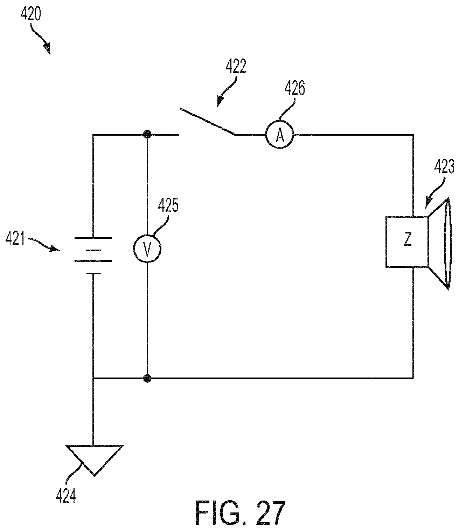

**Fig. 27** shows a circuit diagram 420 having a speaker 423 and a battery 421 in accordance with an embodiment of the present disclosure. The battery 421 may be a backup battery and/or the speaker 423 may be a backup alarm speaker. That is, the circuit 420 may be a backup alarm circuit, for example, a backup alarm circuit in a medical device, such as a syringe pump.

In some embodiments of the present disclosure, the battery 421 may be tested simultaneously with the speaker 423. When a switch 422 is in an open position, a voltmeter 425 may be used to measure the open circuit voltage of the battery 421. Thereafter, the switch 422 may be closed and the closed-circuit voltage from the battery 421 may be measured. The internal resistance of the battery 421 may be estimated by using the known impedance, Z, of the speaker423. A processor may be used to estimate the internal resistance of the battery 421 (e.g., a processor of a syringe pump). The processor may correlate the internal resistance of the battery 421 to the battery's 421 health. In some embodiments of the present disclosure, if the closed-circuit voltage of the battery 421 is not within a predetermined range (the range may be a function of the open-circuit voltage of the battery 421), the speaker 423 may be determined to have failed.

In some additional embodiments of the present disclosure, the switch 422 may be modulated such that the speaker 423 is tested simultaneously with the battery 421. A microphone may be used to determine if the speaker 423 is audibly broadcasting a signal within predetermined operating parameters (e.g., volume, frequency, spectral compositions, etc.) and/or the internal impedance of the battery 421 may be estimated to determine if it is within predetermined operating parameters (e.g., the complex impedance, for example). The microphone may be coupled to the processor. Additionally or alternatively, a test signal may be applied to the speaker 423 (e.g., by modulating the switch 422) and the speaker's 423 current waveform may be monitored by an current sensor 426 to determine the total harmonic distortion of the speaker 423 and/or the magnitude of the current; a processor may be monitored these values using the current sensor 426 to determine if a fault condition exists within the speaker 423 (e.g., the total harmonic distortion or the magnitude of the current are not within predetermined ranges).

Various sine waves, periodic waveforms, and/or signals maybe applied to the speaker 423 to measure its impedance and/or to measure the impedance of the battery 421. For example, a processor of a syringe pump disclosed herein may modulate the switch 422 and measure the voltage across the battery 421 to determine if the battery 421 and the speaker 423 has an impedance within predetermined ranges; if the estimated impedance of the battery 421 is outside a first range, the processor will determine that the battery is in a fault condition, and/or if the estimated impedance of the speaker 423 is outside a second range, the processor will determine that the speaker 423 is in a fault condition. Additionally or alternatively, if the processor cannot determine if the battery 421 or the speaker 423 has a fault condition, but has determined that at least one exists in a fault condition, the processor may issue an alert or alarm that the circuit 420 is in a fault condition. The processor may alarm or alert a user or a remote server of the fault condition. In some embodiments of the present disclosure, the syringe pump will not operate until the fault is addressed, mitigated and/or corrected.

### EXEMPLARY SYRINGE PUMP EMBODIMENT

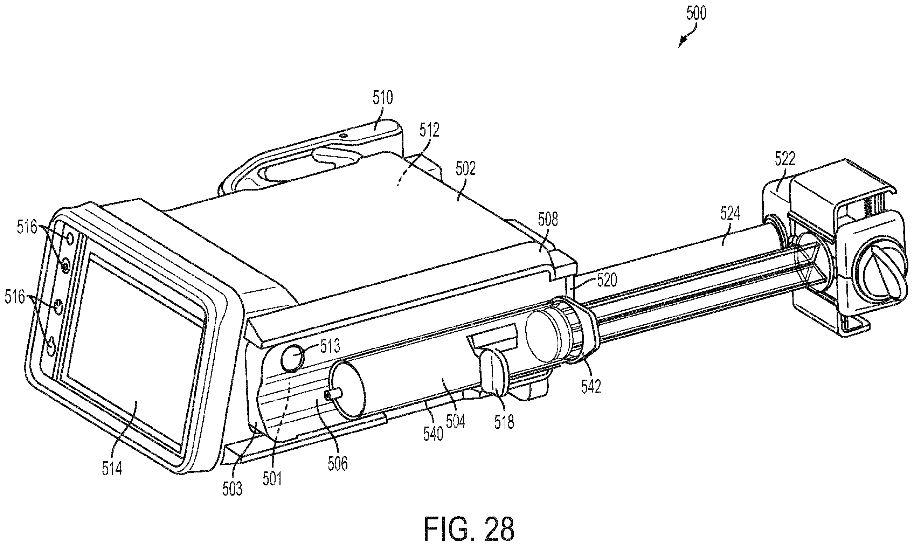

In an example embodiment, as shown in **Fig. 28****,** a syringe pump **500** is depicted. The syringe pump **500** may be used to deliver an agent, such as but not limited to, an analgesic, medicament, nutrient, chemotherapeutic agent, etc. to a patient. The syringe pump may be used to precisely delivery a quantity of an agent to a patient or deliver a precise quantity of an agent over a period of time. The syringe pump **500** may be used in any suitable application, such as though not limited to, intravenous deliver, intrathecal delivery, intra-arterial delivery, enteral delivery or feeding, etc.

The syringe pump **500** comprises a housing **502** and a syringe pump assembly **501.** In the example embodiment in **Fig. 28****,** the housing **502** is substantially a rectangular box. In alternative embodiments, the housing **502** may take any of a variety of other suitable shapes. The housing **502** may be made of any of a number of materials or combination of materials including, but not limited to, metal or plastic. The housing **502** may be extruded, injection molded, die cast, etc. In some embodiments, the housing **502** may be comprised of a number of separate parts which may be coupled together by any suitable means. In some embodiments, the housing **502** may be taken apart or comprise a removable panel to allow the syringe pump **500** to be easily serviced.

As shown in **Fig. 28****,** a syringe **504** may be seated on the syringe pump assembly **501.** The syringe **504** may be a glass, plastic, or any other type of syringe **504.** The syringe **504** may be a syringe **504** of any capacity. In some embodiments, including the embodiment in **Fig. 28****,** the syringe **504** may be seated on a syringe seat **506** comprising part of the syringe pump assembly **501.** The syringe seat **506** may comprise a contour which allows the syringe **506** to be cradled by the syringe seat **506.** The syringe seat **506** may be made of the same material as the rest of the housing **502,** a different material, or may be made of several materials. The syringe seat **506** may be coupled to the housing **502** by a mount **508** which may also serve as a spill, splash, drip, fluid, or debris guard.

In some embodiments, the syringe seat **506** may comprise part of the housing **502.** In the embodiment shown in **Fig. 28****,** the syringe seat **506** is part of a syringe pump assembly housing **503** of the syringe pump assembly **501.** In some embodiments the syringe pump assembly housing **503** may be at least partially formed as an extrusion. In such embodiments, the contours of the syringe seat **506** may be formed during extrusion.

The syringe pump assembly **501** may be inserted into the housing **502** or may be coupled thereto. In the example embodiment in **Fig. 28****,** the syringe pump assembly **501** is mostly disposed inside the housing **502.** The syringe seat **506,** syringe barrel holder **518,** barrel flange clip **520,** plunger head assembly **522,** and plunger tube **524,** each a part of the syringe pump assembly **501,** are not disposed inside the housing **502** in the exemplary embodiment shown in **Fig. 28****.** In embodiments where the syringe seat **506** is not part of the housing **502,** the mount **508** may comprise a gasket which functions as a seal to keep unwanted foreign material from entering the housing **502** and getting into portions of the syringe pump assembly **501,** which are disposed inside the housing **502.** In some embodiments, the mount **508** may overhang the syringe seat **506** and may function as a drip edge, splash guard, etc. which will shed liquid off and away from the syringe pump **500.**

In some embodiments, the syringe pump **500** may be converted into a different device such as, though not limited to, a peristaltic large volume pump. This may be accomplished by removing the syringe pump assembly **501** from the housing **502** and replacing the syringe pump assembly **501** with another desired assembly. Replacement assemblies may include for example, other infusion pumps assemblies such as a peristaltic infusion pump assembly.

In some embodiments, a clamp **510** may be coupled to the housing **502.** The clamp **510** may be any type of clamp, for example, a standard pole clamp **510** or a quick release pole clamp **510** (shown). The clamp **510** may be used to keep the syringe pump **500** at a desired location on an object such as an I.V. pole. The clamp **510** may be removably coupled to the housing **502** through a clamp mount **512.** In some embodiments, the clamp mount **512** may comprise any of a variety of fasteners such as screws, bolts, adhesive, hook and loop tape, snap fit, friction fit, magnets, etc. In some embodiments, the clamp **510** or a part of the clamp **510** may be formed as an integral part of the housing **502** during manufacture.

As shown in **Fig. 28****,** the housing **502** may also include a display **514.** The display **514** may function as a graphic user interface and allow a user to program and monitor pump operation. The display **514** may be an electronic visual display such as a, liquid crystal display, touch screen, L.E.D. display, plasma display, etc. In some embodiments, the display may be complimented by any number of data input means **516.** In the example embodiment, the data input means **516** are several user depressible buttons. The buttons may have fixed functions such as "power", "stop", "silence", "emergency stop", "start therapy", or "lock". The lock function may lock all the user inputs to avoid inadvertent commands from being issued to the syringe pump **500,** due to a touch screen display **514** being touched, buttons being depressed or touched, or any other inadvertent gesture. The data input means **516** of other embodiments may differ. In embodiments where the display **514** is a touch screen display, the data input means **516** may include a number of physically depressible buttons. The physically depressible button data input means **516** may be a back-up for the touch screen display **514** and may be used in the event that the touch screen display **514** is compromised or becomes otherwise non-functional.

In a non-limiting example embodiment, the data input means **516** may be built into the function of a touch screen display **514.** The touch screen display may detect the position of a user's finger or fingers on the screen. The touch screen may be a capacitive touch screen or any other type of touch screen. The software may display virtual buttons, slides, and other controls. The software may also detect the user's touch or the touch of a stylus to control the machine and interact with remote computers that may communicate with the syringe pump **500.** The software may also recognize multi-touch gestures which may control: the display, functioning of the syringe pump **500,** interaction of the syringe pump **500** with one or more remote computers, etc. In some embodiments, the syringe pump **500** may include sensors that detect user gestures when the user is not in contact with the display. These motion detection sensors may comprise a device that transmits invisible near-infrared light, measuring its "time of flight" after it reflects off objects. Such a measurement may allow the syringe pump **500** to detect the location of objects and the distance from the syringe pump **500** to said objects. The syringe pump **500** may thus be able to monitor and take commands via a user's limbs, hands, and fingers or movements of a user's limbs, hands, and fingers. One example of a motion detector is the PrimeSense 3D sensor made by the company PrimeSense of Israel. In some embodiments, the display **514** and data input means may be mounted onto the housing **502** during manufacture of the syringe pump **500.** The display **514** may be removed and replaced during servicing if necessary.

The syringe pump **500** may include a syringe barrel holder **518.** The syringe barrel holder **518** may securely hold the syringe barrel **540** against the syringe seat **506.** The syringe barrel holder **518** may easily be adjusted by a user to accommodate syringes **504** of various sizes. In some embodiments, the syringe barrel holder **518** may be biased so as to automatically adjust to the diameter of any size syringe **504** after the syringe barrel holder **518** is pulled out by a user. The syringe barrel holder **518** will be further elaborated upon later in the specification.

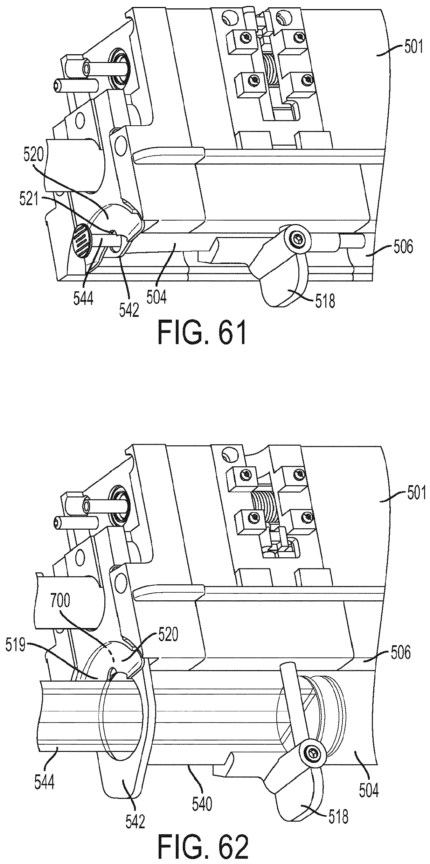

The syringe pump **500** may also include a barrel flange clip **520.** The barrel flange clip **520** in the example embodiment depicted in **Fig. 28** is disposed on an end of the syringe pump assembly housing **503** and is capable of holding the syringe barrel flange **542** in place against the end of the syringe pump assembly housing **503.** The barrel flange clip **520** is also capable of retaining any of a variety of syringe barrel flange **542** types and sizes which may be available to a user. The barrel flange clip **520** will be further elaborated upon later in the specification. For a more detailed description of the barrel flange clip **520,** see **Fig. 61** and **Fig. 62****.**

The syringe pump **500** may additionally include a plunger head assembly **522.** The plunger head assembly **522** may be attached to the syringe pump assembly **501** by a plunger tube **524.** In the example embodiment depicted in **Fig. 28****,** the plunger head assembly **522** and plunger tube **524** extend out of the housing **502** toward the right of the page.

The syringe pump **500** may also comprise a downstream pressure sensor **513** as shown in **Fig. 28****.** The downstream pressure sensor **513** may comprise part of the syringe pump assembly **501** or the housing **502.** The downstream pressure sensor **513** may take pressure measurements from a fluid line i.e. tubing extending from the syringe **504** to a patient. In some embodiments, the fluid line may include a span of tubing which is different from the rest of the tubing. For example, a span of the fluid line may be made of a deformable PVC material. Such embodiments may make fluid line pressures easier to determine.

The downstream pressure sensor **513** may comprise a cradle with a pressure sensor, such as a force sensor. In such embodiments, the fluid line may be held against the cradle and pressure sensor of the downstream pressure sensor **513** by a non-deformable or deflectable structure. The downstream pressure sensor **513** may cause the syringe pump **500** to alarm if the detected pressure falls outside of an acceptable range. The measurement of the downstream pressure sensor **513** may be referenced against a look-up table to determine the pressure in the fluid line. If an abnormal pressure reading (e.g. a high pressure generated during an occlusion event beyond a predetermined threshold) is taken, a control system of the syringe pump **500** may stop delivering fluid. In some embodiments, the syringe pump **500** may be caused to back up and relieve some of the pressure in response to the detection of pressures suggestive of an occlusion.

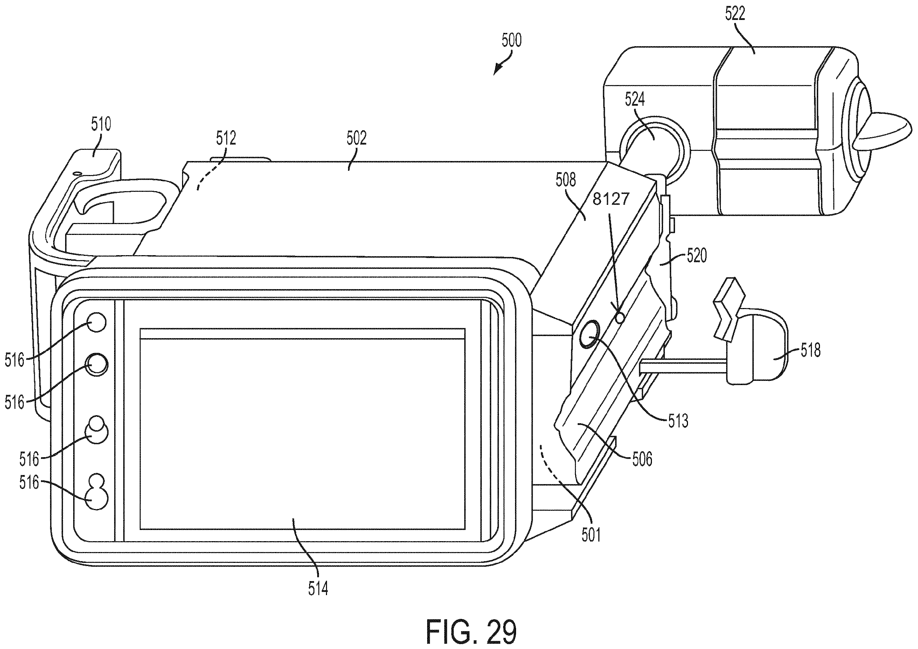

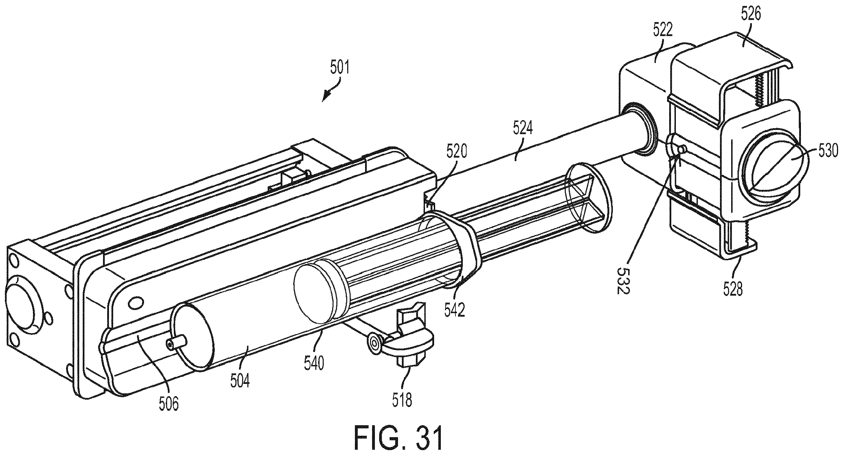

**Fig. 29** shows the syringe pump **500** from another perspective. In this view, the display **514** and data input means **516** coupled to the housing **502** face the front of the page. The clamp **510** is coupled to the housing **502** by a clamp mount **512.** The syringe pump assembly **501** is disposed mostly inside the housing **502.** The syringe seat **506,** which comprises part of the syringe pump assembly **501,** forms a substantial part of one side of the housing **502.** The mount **508** retains the syringe pump assembly **501** and helps seal the interior of the housing **502** from exposure to debris. In embodiments where the mount **508** functions as a drip edge the mount **508** may cover the syringe pump assembly **501** and help shed liquid away from the interior of the housing **502.** The syringe barrel clamp **518** extends through the syringe seat **506.** In the depicted position in **Fig. 29****,** the syringe barrel clamp **518** has been pulled away from its resting position and is biased such that it may automatically retract back toward the housing **502.** In some embodiments, the syringe barrel clamp **518** may be locked in a non-resting position, such as the position depicted in **Fig. 31****.** The barrel flange clip **520** is visible and disposed on the end of the syringe pump assembly housing **503** closest to the plunger head assembly **522.** The plunger tube **524** connects the plunger head assembly **522** to the rest of the syringe pump assembly **501** as described above. The downstream pressure sensor **513** is disposed on the syringe seat **506.**

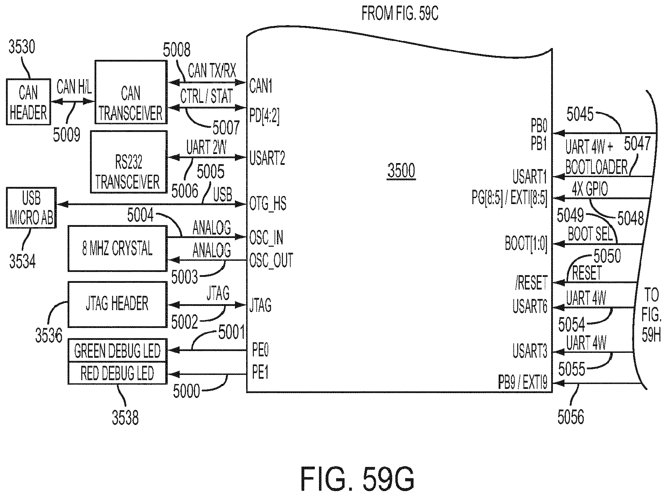

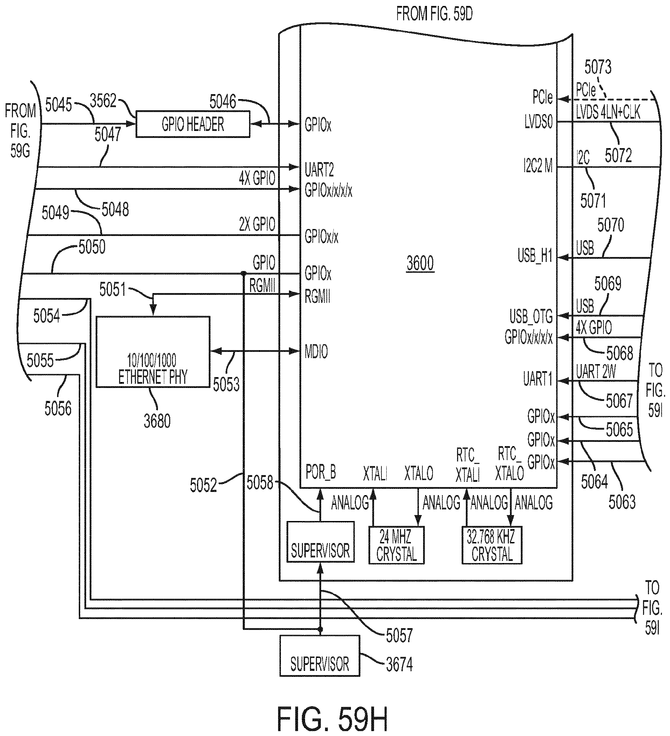

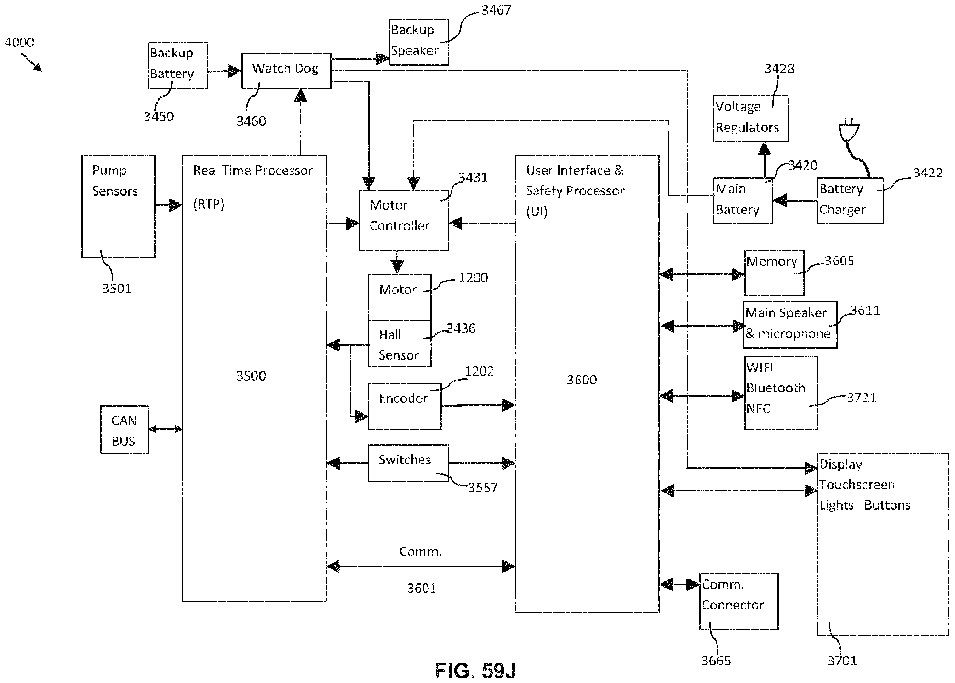

In some specific embodiments, a camera 8127 is positioned to view the syringe. The camera 8127 may be coupled to the RTP 3500 and/or to the processor 3600 of Fig. 59J to provide image data thereto. The camera 8127 may include a CCD image sensor, a CMOS image sensor, or any other type of imaging sensor. In some embodiment of the present disclosure, the camera 8127 includes an array of image sensors.

An image of the syringe loaded into the syringe seat 506 may be displayed on the display 514 as seen from the camera 8127. The processors 3500 and/or 3600 may use the images from the camera 8127 to: read QR codes on the syringe to identify the syringe, detect particulates or bubbles in the syringe, measure the location of the plunger to measure the volume delivered and thus the volume remaining, determine when the syringe state has changed, determine if the syringe is present, estimate bolus discharges, check the color of the fluid to determine if it is the correct fluid, and/or determine if syringe is missing or an improperly loaded.

By using frame differencing to detect motion and a Gaussian filter to help reduce camera's 8127 shot noise (which looks like an impurity, but smaller), the moving impurities can be detected. To locate the syringe's plunger, the fiducials on the syringe may be used, template matching (the plunger being the template) may use pattern recognition to locate the fiducials and thus the plunger.

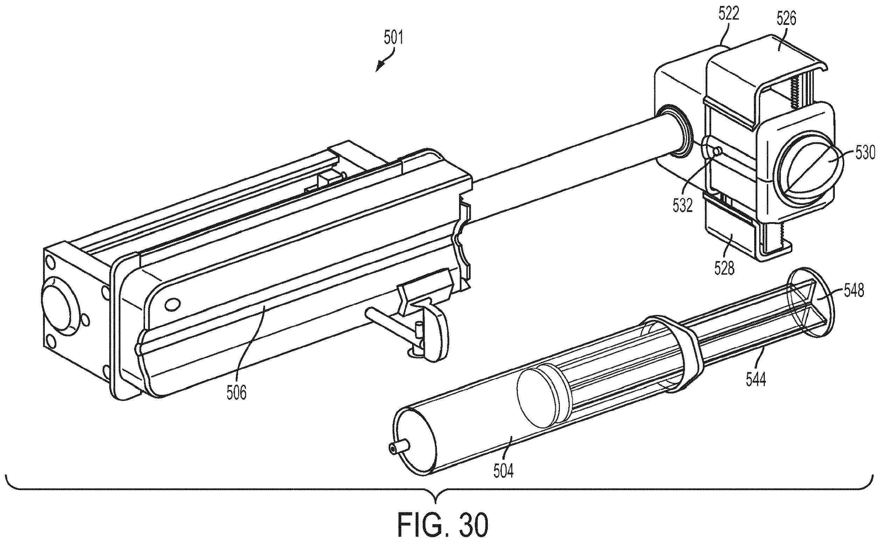

**Figs. 30-34** illustrate how a user may place a syringe **504** into the syringe pump assembly **501.** The syringe pump assembly **501** is shown by itself in **Fig. 30****.** The syringe **504** is not seated against the syringe seat **506.** As shown, the plunger head assembly **522** comprises two jaws, an upper plunger clamp jaw **526** and a lower plunger clamp jaw **528.** The upper plunger clamp jaw **526** and lower plunger clamp jaw **528** are in the open position. The upper plunger clamp jaw **526** and lower plunger clamp jaw **528** are capable of clamping and retaining the plunger flange **548** on the plunger **544** of the syringe **504.** The upper plunger clamp jaw **526** and lower plunger clamp jaw **528** may be actuated to open or closed positions via rotation of a dial **530** comprising part of the plunger head assembly **522.** The plunger head assembly **522** may also comprise a plunger pressure sensor **532.**

In **Fig. 31****,** the syringe pump assembly **501** is again shown by itself. The syringe **504** which had not been seated on the syringe seat **506** in **Fig. 30** is seated in place on the syringe seat **506** in **Fig. 31****.** The syringe barrel flange **542** is clipped in place by the barrel flange clip **520.** The syringe barrel holder **518,** has been pulled out so the syringe **504** may be placed into the syringe pump assembly **501,** but has not yet been allowed to automatically adjust to the diameter of the syringe barrel **540.** In the example embodiment shown in **Fig. 31****,** the syringe barrel holder **518** has been rotated 90° clockwise from its orientation in **Fig. 30** **to** lock it in position. Alternate embodiments may require counter-clockwise rotation, a different degree of rotation, or may not require rotation to lock the syringe barrel holder **518** in position. The plunger tube **524** and attached plunger head assembly **522** are fully extended away from the rest of the syringe pump assembly **501.** Since the dial **530** has not been rotated from the orientation shown in **Fig. 30****,** the upper plunger clamp jaw **526** and the lower plunger clamp jaw **528** are still in the open position.

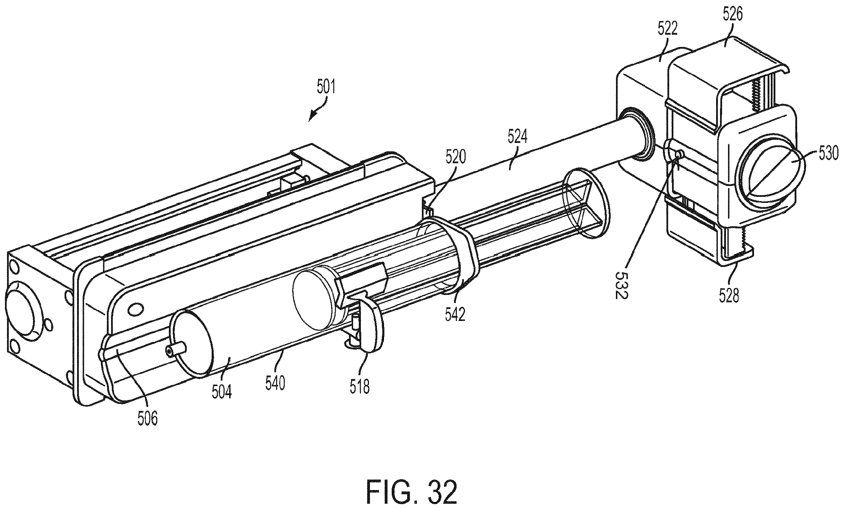

In **Fig. 32****,** the syringe pump assembly **501** is again shown by itself. The syringe **504** is seated against the syringe seat **506.** The syringe barrel holder **518** has been rotated out of the locked position and has been allowed to automatically adjust to the diameter of the syringe barrel **540.** The syringe barrel holder **518** is holding the syringe **504** in place on the syringe pump assembly **501.** The syringe **504** is additionally held in place on the syringe pump assembly **501** by the barrel flange clip **520** which retains the syringe barrel flange **542.** The plunger tube **524** and attached plunger head assembly **522** are fully extended away from the rest of the syringe pump assembly **501.** Since the dial **530** has not been rotated from the orientation shown in **Fig. 30****,** the upper plunger clamp jaw **526** and the lower plunger clamp jaw **528** are still in the open position.

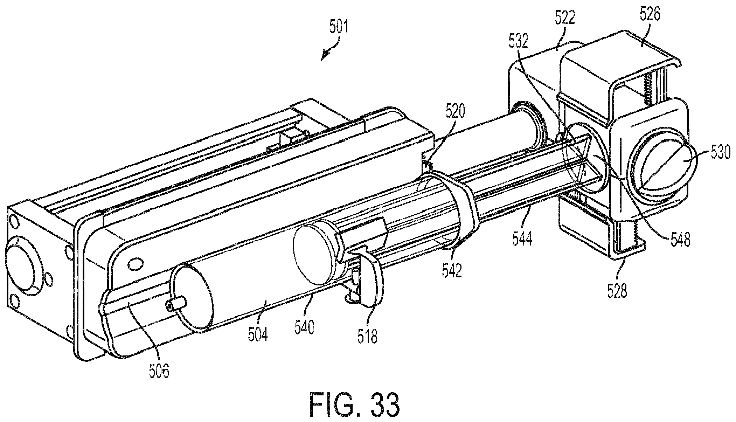

In **Fig. 33****,** the syringe pump assembly **501** is again shown by itself. The syringe **504** is seated against the syringe seat **506.** The syringe barrel holder **518** is pressing against the syringe barrel **540** and holding the syringe **504** in place on the syringe pump assembly **501.** The barrel flange clip **520** is holding the syringe barrel flange **542** and helping to the hold the syringe **504** in place on the syringe pump assembly **501.** The amount that the plunger tube **524** extends away from the rest of the syringe pump assembly **501** has been adjusted such that the plunger head assembly **522** is in contact with the plunger flange **548** on the syringe plunger **544.** Since the dial **530** has not been rotated from the orientation shown in **Fig. 30****,** the upper plunger clamp jaw **526** and the lower plunger clamp jaw **528** are still in the open position. The plunger flange **548** is in contact with the plunger pressure sensor **532.**

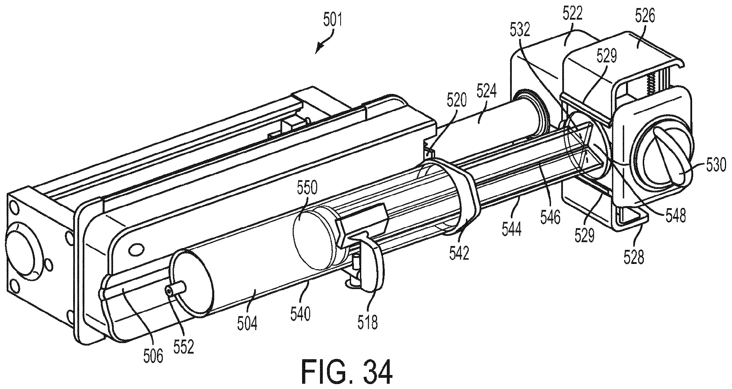

In **Fig. 34** the syringe pump assembly **501** is again shown by itself. The syringe **504** is seated against the syringe seat **506.** The syringe barrel holder **518** is pressing against the syringe barrel **540** and holding the syringe **504** in place on the syringe pump assembly **501.** The barrel flange clip **520** is clipping the syringe barrel flange **542** and helping to the hold the syringe **504** in place on the syringe pump assembly **501.** The amount that the plunger tube **524** extends away from the rest of the syringe pump assembly **501** has been adjusted such that the plunger head assembly **522** is in contact with the plunger flange **548** on the syringe plunger **544.** The dial **530** has been rotated from the orientation depicted in **Figs. 30-33****.** Consequentially, the upper plunger clamp jaw **526** and lower plunger clamp jaw **528** have moved to a closed position in which the plunger flange **548** of the syringe plunger **544** is retained by the plunger head assembly **522.** Since the upper plunger clamp jaw **526** and lower plunger clamp jaw **528** close about the horizontal centerline of the plunger head assembly **522,** the plunger flange **548** has been centered on the plunger head assembly **522.**

In the preferred embodiment, the upper plunger clamp jaw **526** and lower plunger clamp jaw **528** each comprise a fin **529** as illustrated in **Fig. 34****.** The fins **529** bow out away from the plunger head assembly **522** and toward the left of the page (relative to **Fig. 34**). The fins **529** are disposed about the upper plunger clamp jaw **526** and lower plunger clamp jaw **528** such that the fins **529** are the only part of the upper plunger clamp jaw **526** and lower plunger clamp jaw **528** to contact a plunger flange **548** when a syringe **504** is placed on the syringe pump assembly **501.** As the upper plunger clamp jaw **526** and lower plunger clamp jaw **528** are closed down on a plunger flange **548** the thickness and diameter of the plunger flange **548** determine when the upper plunger clamp jaw **526** and lower plunger clamp jaw **528** stop moving. At least some part of the fins **529** will overhang the plunger flange **548** and ensure the plunger flange **548** is retained. Since the upper plunger clamp jaw **526** and lower plunger clamp jaw **528** do not deflect, this forces the plunger flange **548** against the rest of the plunger head assembly **522.** That is, the angle of contact of the upper plunger clamp jaw **526** and lower plunger clamp jaw **528** on the plunger flange **548** results in a force with a component that pushes the plunger flange **548** against the plunger head assembly **522.** This resultant force additionally has a component which centers the plunger flange **548** on the plunger head assembly **522.** This is especially desirable because such an arrangement does not allow for any "play" of the plunger flange **548** between upper plunger clamp jaw **526** and lower plunger clamp jaw **528** and the rest of the plunger head assembly **522.** Additionally, such an arrangement is desirable because it not only securely holds the plunger flange **548** in place against the plunger head assembly **522,** but also doubles as an anti-siphon mechanism. Such an arrangement furthermore, ensures that the plunger flange **548** consistently contacts the plunger pressure sensor **532.** Any force component generated by the upper plunger clamp jaw **526** and lower plunger clamp jaw **528** which may affect readings of the plunger pressure sensor **532** may be predictable and subtracted out or otherwise compensated for.

In other embodiments, the upper plunger clamp jaw **526** and lower plunger clamp jaw **528** may not comprise fins **529.** Instead the upper plunger clamp jaw **526** and lower plunger clamp jaw **528** overhang a portion of the plunger flange **548** when in the clamped position. The upper plunger clamp jaw **526** and lower plunger clamp jaw **528** may stop moving when they abut the cruciform which comprises the plunger stem **546.** In other embodiments, the upper plunger clamp jaw **526** and lower plunger clamp jaw **528** may clamp a plunger stem **546** that need not be a cruciform. In another embodiment, the upper plunger clamp jaw 526 and lower plunger clamp jaw 528 may include a wedge, ramp, or tapered rib feature on the surfaces of the jaws that faces the pump head assembly 522. The wedge, ramp or tapered rib serve to push the plunger flange 548 toward the pump head assembly 522 until the plunger flange 548 is securely held against the pump head assembly 522.

To dispense the contents of the syringe **504,** the syringe pump **500** may actuate the plunger head assembly **522** to thereby push the plunger **544** into the syringe barrel **540.** Since the contents of the syringe **504** may not flow through or past the plunger pusher **550,** the contents of the syringe **504** are forced out of the syringe outlet **552** as the plunger **544** is advanced into the syringe barrel **540.** Any pressure generated as the plunger **544** advances into the syringe barrel **540** is transmitted to the plunger pressure sensor **532.** The plunger pressure sensor **532,** may, in some embodiments, comprise a force sensor such as a strain beam. When an occlusion occurs, fluid within the syringe barrel **540** and/or the fluid lines prevents movement of the plunger **544.** When the plunger head assembly **522** continues to advance, high forces are produced between the plunger **544** and the plunger head assembly **522.** The pressure transmitted to the plunger pressure sensor **532** may have a programmed acceptable range so that possible occlusions may be identified. If the pressure applied to the plunger pressure sensor **532** exceeds a predetermined threshold, the syringe pump **500** may alarm or issue an alert.

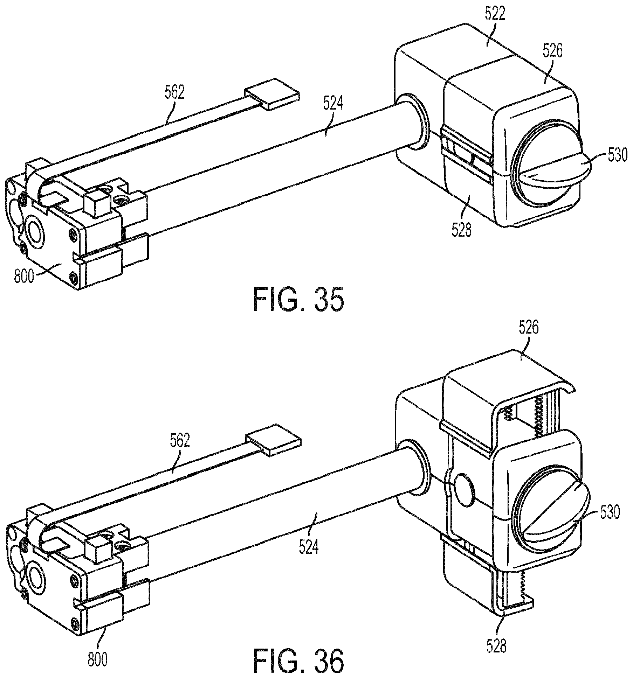

**Fig. 35** shows the plunger head assembly **522** with the upper plunger clamp jaw **526** and lower plunger clamp jaw **528** in the fully closed position. The dial **530** is oriented such that the raised part of the dial **530** is on a plane substantially parallel to the top and bottom faces of the plunger head assembly **522.** The plunger tube **524** is shown extending from the plunger head assembly **522** to the sliding block assembly **800.** One end of a flex connector **562** is attached to the sliding block assembly **800.** A position indicator mark has been placed on the dial **530** for illustrative purposes in **Fig. 35** and **Fig. 36****.**

The view shown in **Fig. 36** is similar to the view shown in **Fig. 35****.** In **Fig. 36****,** the dial **530** on the plunger head assembly **522** has been rotated approximately 135° clockwise. This rotation has in turn caused the upper plunger clamp jaw **526** and lower plunger clamp jaw **528** to separate and move to the fully open position. In alternate embodiments, the dial **530** may require more or less rotation than the approximately 135° shown in the example embodiment to transition the upper plunger clamp jaw **526** and lower plunger clamp jaw **528** from a fully open position to a fully closed position. The plunger head assembly may be capable of holding itself in this position (described later in the specification).

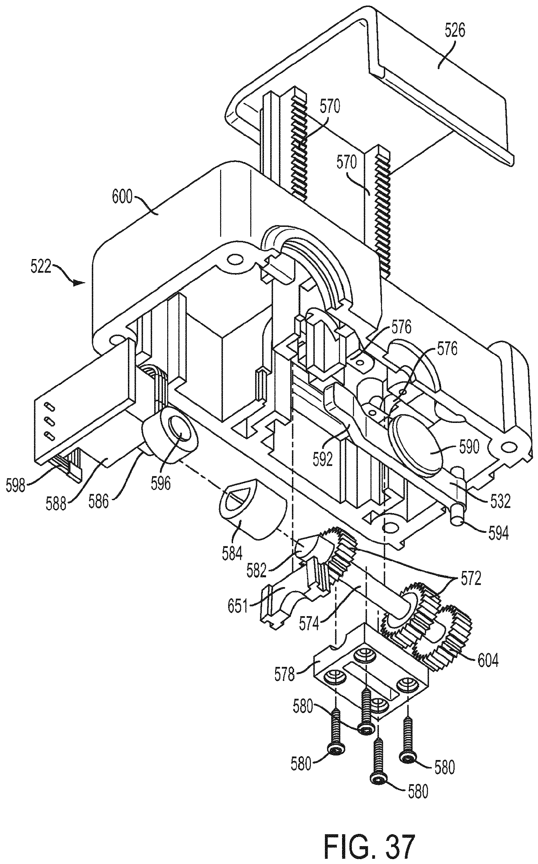

An exploded view of the top half of the plunger head assembly **522** is shown in **Fig. 37****.** As shown, the upper plunger clamp jaw **526** comprises two racks **570.** In other embodiments, there may only be one rack **570.** In some embodiments, there may be more than two racks **570.** When the plunger head assembly **522** is fully assembled, the racks **570** may interdigitate with a corresponding number of upper jaw pinion gears **572.** The upper jaw pinion gears **572** spin about the axis of an upper jaw drive shaft **574.** The upper jaw drive shaft **574** may also comprise an upper jaw drive gear **604** which will be elaborated upon later.

The plunger head assembly **522** may comprise a number of bearing surfaces for the upper jaw drive shaft **574.** In the example embodiment in **Fig. 37****,** the plunger head assembly **522** comprises two upper bearing surfaces **576** and a lower bearing surface **578** for the upper jaw drive shaft **574.** The upper bearing surfaces **576** may be coupled into the plunger head assembly housing top **600.** The upper bearing surfaces **576** may be coupled to the plunger head assembly housing top **600** by any of a variety of means including, but not limited to, screws bolts, adhesive, snap fit, friction fit, welds, a tongue in groove arrangement, pins, or may be formed as a continuous part of the plunger head assembly housing top **600** (shown). The upper bearing surfaces **576** provide a bearing surface for at least a span of the top half of the upper jaw drive shaft **574.**

The lower bearing surface **578** is coupled into the plunger head assembly housing top **600.** The lower bearing surface **578** may be coupled to the plunger head assembly housing top **600** by any suitable means such as, but not limited to, screws **580** (shown), bolts, adhesive, snap fit, friction fit, magnets, welds, a tongue in groove arrangement, etc. In some embodiments, the lower bearing surface **578** may be formed as a continuous part of the plunger head assembly housing top **600.** The lower bearing surface **578** provides a bearing surface for at least a span of the bottom half of the upper jaw drive shaft **574.**

In some embodiments, there may also be an upper dial shaft bearing surface **651** which couples into the plunger head assembly housing top **600.** The upper dial shaft bearing surface **651** may be coupled into the plunger head assembly housing top **600** by any of a variety of means including, but not limited to, screws, bolts, adhesive, snap fit, friction fit, welds, a tongue in groove arrangement (shown), pins, or may be formed as a continuous part of the plunger head assembly housing top **600.** The upper dial shaft bearing surface **651** will be further elaborated upon later.

The upper jaw drive shaft **574** may also comprise a D-shaped span **582.** The D-shaped span **582** may be located on an end of the upper jaw drive shaft **574** as shown in the example embodiment in **Fig. 37****.** The D-shaped span **582** of the upper jaw drive shaft **574** may couple into a complimentary shaped orifice in one side of a D-shaped connector **584.** The D-shaped span **582** of the upper jaw drive shaft **574** may not extend all the way through the D-shaped connector **584.** In some embodiments, the orifice may run through the entire D-shaped connector **584.** The other side of the D-shaped connector **584** may couple onto a D-shaped shaft **586** projecting out of a plunger clamp jaws position sensor **588.** Any rotation of the upper jaw drive shaft **574** may cause the D-shaped connector **584** to rotate as well. In turn, this may cause rotation of the D-shaped shaft **586** projecting from the plunger clamp jaws position sensor **588.** In some embodiments, the D-shaped span **582** of the upper jaw drive shaft **574** may extend directly into the plunger clamp jaws position sensor **588.** In such embodiments, the D-shaped connector **584** and D-shaped shaft **586** may not be needed. In some embodiments, the D-shaped span **582,** the D-shaped connector **584,** and D-shaped shaft **586** need not be D-shaped. In some embodiments they may be have a triangular shape, square shape, star shape, etc.

In some embodiments, the plunger clamp jaws position sensor **588** may comprise a potentiometer. As the D-shaped shaft **586** projecting from the plunger clamp jaws position sensor **588** rotates, the wiper of the potentiometer is slid across the resistive element of the potentiometer thus varying the resistance measured by the potentiometer. The resistance value may then be interpreted to indicate the position of the upper plunger clamp jaw **526** and lower plunger clamp jaw **528.** Alternatively, the plunger clamp jaws position sensor **588** may comprise a magnet on the end of the upper jaw drive shaft 574 and a rotary encoder such as the AS5030ATSU by Austrianmicrosytems of Austria. Alternatively, the position of the upper jaw 526 and or lower jaw 528 can be measured with a linear encoder or a linear potentiometer.

By obtaining a position from the plunger clamp jaws position sensor **588,** the syringe pump **500** may be able to determine a number of things. The position may be used to indicate whether a plunger flange **548** has been clamped by the plunger head assembly **522.** The position may indicate whether a plunger flange has been correctly clamped by the plunger head assembly **522.** This may be accomplished by referencing the determined position against a position or a range of positions which may be acceptable for a specific syringe **504.** The information about the specific syringe **504** being used may be input by a user or may be gathered by one or more other sensors comprising other parts of the syringe pump **500.**

Since the position measured by the plunger clamp jaws position sensor **588** depends on the diameter and thickness of a clamped plunger flange **548,** the positional information may also be used to determine information about the specific syringe **504** being used (for example, its type, brand, volume, etc.). This may be accomplished by referencing the measured position against a database of positions which would be expected for different syringes **504.** In embodiments where there are a number of sensors gathering information about the syringe **504,** the positional information generated by the plunger clamp jaws position sensor **588** may be checked against data from other sensors to make a more informed decision on which specific syringe **504** is being utilized. If the position measured by the plunger clamp jaws position sensor **588** does not correlate with data gathered by other sensors, the syringe pump **500** may alarm.

As shown in **Fig. 37****,** the plunger head assembly housing top **600** may also house the plunger pressure sensor **532** mentioned earlier. The plunger pressure sensor **532** may comprise a plunger pressure sensor push plate **590.** The plunger pressure sensor push plate **590** may be a nub, a disc, or any other suitable shape. The plunger pressure sensor push plate **590** may be flat or rounded. The plunger pressure sensor push plate **590** may extend out of the plunger head assembly **522** such that it may physically contact a plunger flange **548** clamped against the plunger head assembly **522.** The plunger pressure sensor push plate **590** may directly transmit any force applied to it to a plunger pressure sensor input surface **596.** In some embodiments, the plunger pressure sensor push plate **590** may be attached to a plunger pressure sensor lever **592.** The plunger pressure sensor lever **592** may be pivotally coupled to a plunger pressure sensor pivot **594.** The plunger pressure sensor pivot **594** may be disposed at any point along the length of the plunger pressure sensor lever **594.** In the example embodiment in **Fig. 37****,** any force applied to the plunger pressure sensor push plate **590** is transmitted through the plunger pressure sensor lever **592** to the plunger pressure sensor input surface **596.** In some specific embodiments, the plunger pressure sensor lever 592 and plunger pressure sensor pivot 594 may serve to constrain the motion of the plunger pressure plate 590 to a plane perpendicular to the plunger flange 548 and minimize resistance to free movement of the plunger pressure plate 590. Although the location of the plunger pressure sensor pivot **594** in relation to the plunger pressure sensor push plate **590** does not multiply the force exerted against the plunger pressure sensor input surface **596** in **Fig. 37****,** other embodiments may use different arrangements to create a mechanical advantage.

The force measurement which is read via the plunger pressure sensor **532** may be interpreted to determine the hydraulic pressure of the fluid being dispensed. This may contribute to safety of operation because the sensed fluid pressure may be useful in identifying possible occlusions so that they may be corrected. The pressure may be monitored such that if the pressure exceeds a predefined value, the syringe pump **500** may alarm. The pressure measurement from the plunger pressure sensor **532** may be checked against the pressure measurement from the downstream pressure sensor **513** (see **Fig. 28**) in embodiments including both a plunger pressure sensor **532** and a downstream pressure sensor 513. This may help to ensure greater accuracy. If the pressure measurements do not correlate, an alarm may be generated. Additionally, since the sensors are redundant, if one of the plunger pressure sensor **532** or downstream pressure sensor **513** fails during a therapy, the syringe pump **500** may function on only one of the sensors in a fail operative mode.

As shown in **Fig. 37****,** a number of electrical conduits **598** run to and from the both the plunger pressure sensor **532** and the plunger clamp jaws position sensor **588.** The conduits **598** provide power to the plunger pressure sensor **532** and plunger clamp jaws position sensor **588.** The electrical conduits **598** also comprise the data communication pathways to and from the plunger pressure sensor **532** and the plunger clamp jaws position sensor **588.**

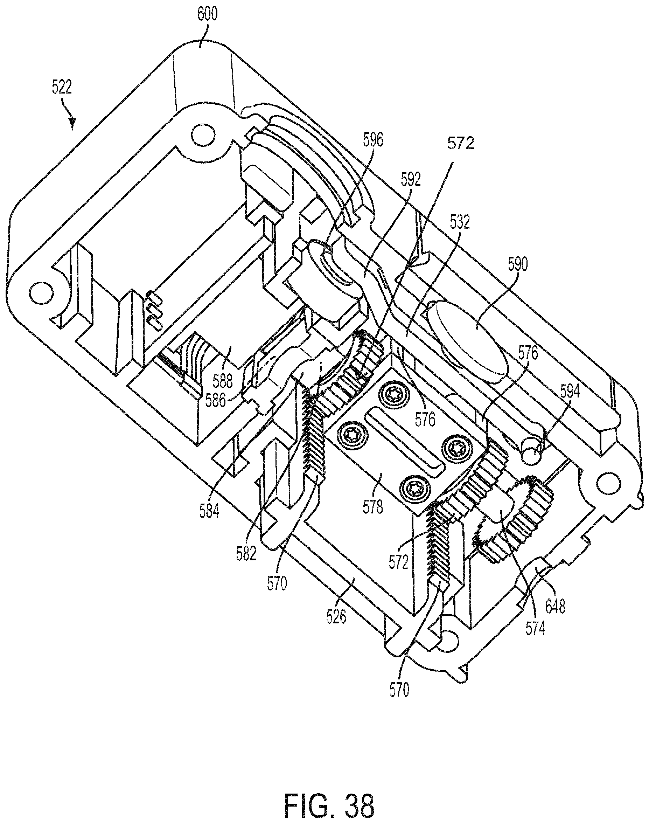

**Fig. 38** shows an assembled view of the top half of the plunger head assembly **522.** In **Fig. 38****,** the upper plunger clamp jaw **526** is in a closed position. The two racks **570** on the upper plunger clamp jaw **526** are engaged with the two pinion gears **572** on the upper jaw drive shaft **574** such that any rotation of the upper jaw drive shaft **574** translates into linear displacement of the upper plunger clamp jaw **526.** The upper jaw drive shaft **574** is surrounded by the upper bearing surfaces **576** and the lower bearing surface **578.**

The D-shaped span **582** of the upper jaw drive shaft **574** and the D-shaped shaft **586** of the plunger clamp jaws position sensor **588** are coupled together by the D-shaped connector **584.** Any rotation of the upper jaw drive shaft **574** will cause rotation of the D-shaped span **582,** D-shaped connector **584,** and D-shaped shaft **586.** As mentioned above this rotation may cause the wiper to slide across the resistive element of the plunger clamp jaws position sensor **588** in embodiments where the plunger clamp jaws position sensor **588** comprises a potentiometer.

The plunger pressure sensor **532** is also shown in **Fig. 38****.** The plunger pressure sensor push plate **590** may extend out of the plunger head assembly **522** such that it may physically contact a plunger flange **548** (see **Fig. 30**) clamped against the plunger head assembly **522.** The plunger pressure sensor push plate **590** may directly transmit any force applied to it to a plunger pressure sensor input surface **596.** In some embodiments, including the one shown in **Fig. 38****,** the plunger pressure sensor push plate **590** may be attached to a plunger pressure sensor lever **592.** The plunger pressure sensor lever **592** may be pivotally coupled to a plunger pressure sensor pivot **594.** The plunger pressure sensor pivot **594** may be disposed at any point along the length of the plunger pressure sensor lever **592.** In the example embodiment in **Fig. 38****,** any force applied to the plunger pressure sensor push plate **590** is transmitted through the plunger pressure sensor lever **592** to the plunger pressure sensor input surface **596.** Although the location of the plunger pressure sensor pivot **594** in relation to the plunger pressure sensor push plate **590** does not multiply the force exerted against the plunger pressure sensor input surface **596** in **Fig. 38****,** other embodiments may use different arrangements to create a mechanical advantage.

The plunger head assembly housing top **600** also includes the top half of a dial shaft passage **648** for a dial shaft **650** (not shown) which will be explained later in the specification. In the example embodiment shown in **Fig. 38****,** the dial shaft passage **648** passes through the right face of the plunger head assembly housing top **600.**

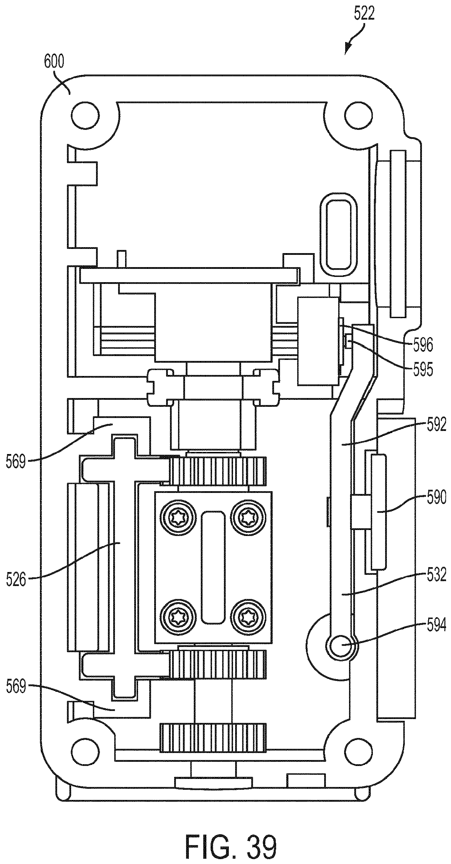

**Fig. 39** shows another assembled view of the top half of the plunger head assembly **522.** As shown in **Fig. 39** the plunger head assembly housing top **600** may comprise upper jaw guides **569.** The upper jaw guides **569** are sized and disposed such that they form a track-way in which the upper plunger clamp jaw **526** may move along. In the example embodiment, the upper jaw guides **569** are formed as a continuous part of the plunger head assembly housing top **600** and span the entire height of the side wall of the plunger head assembly housing top **600.** In other embodiments, the upper jaw guides **569** may only span a part of the height of the side wall of plunger head assembly housing top **600.**

As shown in **Fig. 39****,** the plunger pressure sensor **532** may comprise a plunger pressure sensor force concentrator **595.** In embodiments where the plunger pressure sensor push plate **590** transmits force directly to the plunger pressure sensor input surface **596,** the plunger pressure sensor force concentrator **595** may help to concentrate the force applied to the plunger pressure sensor push plate **590** while exerting it against the plunger pressure sensor input surface **596.** In embodiments where the plunger pressure sensor **532** comprises a plunger pressure sensor lever **592** on a plunger pressure sensor pivot **594,** the plunger pressure sensor force concentrator **595** may be on the end and face of the plunger pressure sensor lever **592** which presses against the plunger pressure sensor input surface **596.** This may help to concentrate the force exerted against the plunger pressure sensor input surface **596** which may increase accuracy. It may also help to concentrate the force at the center of the plunger pressure sensor input surface **596,** making measurements more consistent and accurate.

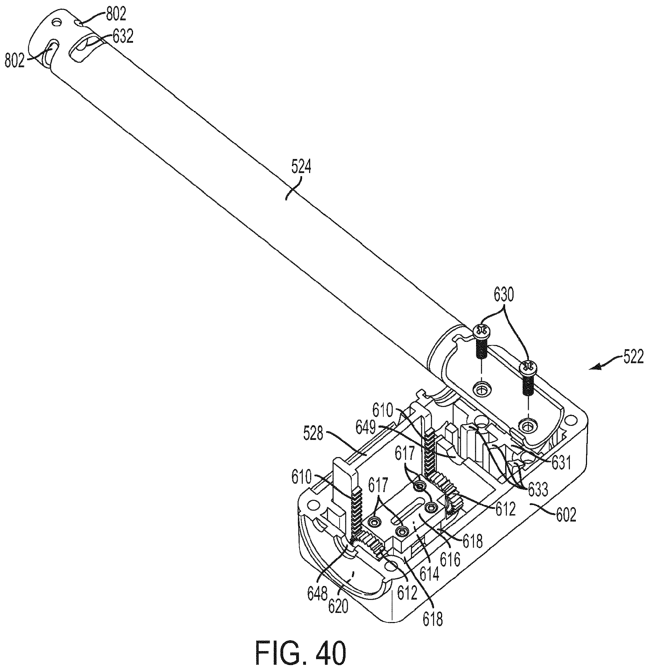

The bottom half of the plunger head assembly **522** and the plunger tube **524** are shown in **Fig. 40****.** As shown, the lower plunger clamp jaw **528** comprises two lower plunger clamp jaw racks **610.** In other embodiments, there may only be one lower plunger clamp jaw rack **610.** In some embodiments, there may be more than two lower plunger clamp jaw racks **610.** Each lower plunger clamp jaw rack **610** interdigitates with a lower plunger clamp jaw pinion gear **612.** The lower plunger clamp jaw pinion gears **612** are capable of rotating about the axis of a lower clamp jaw drive shaft **614.** A lower jaw drive gear **620** is also disposed on the lower clamp jaw drive shaft **614.** The lower jaw drive gear **620** will be elaborated upon later.

Similar to the upper half of the plunger head assembly **522** the lower half of the plunger head assembly **522** may comprise a number of bearing surfaces for the lower jaw drive shaft **614.** In the example embodiment in **Fig. 40****,** the plunger head assembly **522** comprises one upper bearing surface **616** and two lower bearing surfaces **618** for the lower jaw drive shaft **614.** The upper bearing surface **616** is coupled into the plunger head assembly housing bottom **602.** The upper bearing surface **616** may be coupled to the plunger head assembly housing bottom **602** by any of a variety of means including, but not limited to, screws **617** (shown), bolts, adhesive, snap fit, friction fit, welds, a tongue in groove arrangement, pins, or may be formed as a continuous part of the plunger head assembly housing bottom **602.** The upper bearing surface **616** provide a bearing surface for at least a span of the top half of the lower jaw drive shaft **614.**

The lower bearing surfaces **618** are coupled into the plunger head assembly housing bottom **602.** The lower bearing surfaces **618** may be coupled to the plunger head assembly housing bottom **602** by any suitable means such as, but not limited to, screws, bolts, adhesive, snap fit, friction fit, magnets, welds, a tongue in groove arrangement, pin (shown), etc. In some embodiments, the lower bearing surfaces **618** may be formed as a continuous part of the plunger head assembly housing bottom **602.** The lower bearing surfaces **618** provide a bearing surface for at least a span of the bottom half of the lower jaw drive shaft **614.**

In some embodiments, there may also be a lower dial shaft bearing surface **649** which is coupled to the plunger head assembly housing bottom **602.** The lower dial shaft bearing surface **649** may be coupled into the plunger head assembly housing bottom **602** by any of a variety of means including, but not limited to, screws, bolts, adhesive, snap fit, friction fit, welds, a tongue in groove arrangement, pins, or may be formed as a continuous part of the plunger head assembly housing bottom **602** as shown. The lower half of the dial shaft passage **648** mentioned above is cut through the right face of the plunger head assembly housing bottom **602** The lower dial shaft bearing surface **649** and dial shaft passage **648** will be further elaborated upon later.

As shown in **Fig. 40****,** the plunger tube **524** may be coupled into the bottom half of the plunger head assembly **522.** In the example embodiment shown in **Fig. 40****,** the plunger tube **524** is coupled by two screws **630** onto a plunger tube cradle **631.** In other embodiments, the number or type of fastener/coupling method may be different. For example, the plunger tube **524** may be coupled to the plunger tube cradle **631** by any other suitable means such as, but not limited to, bolts, adhesive, snap fit, friction fit, magnets, welds, a tongue in groove arrangement, pin, etc. The plunger tube cradle **631** may comprise arcuated ribs **633** which are arced such that they are flush with the outside surface of the plunger tube **524** and support the plunger tube **524.** In some embodiments, a portion of the arc of the plunger tube **524** may be eliminated on the span of the plunger tube **524** which is coupled inside of the plunger head assembly **522** when the syringe pump **500** is fully assembled. In the embodiment shown in **Fig. 40****,** about a 180° segment, or the upper half of the plunger tube **524** has been eliminated. The end of the plunger tube **524** opposite the end of the plunger tube **524** coupled to the plunger tube cradle **631** may comprise a number of plunger tube cutouts **802** which will be explained later. There may also be a conduit opening **632** near the plunger tube cutouts **802.**

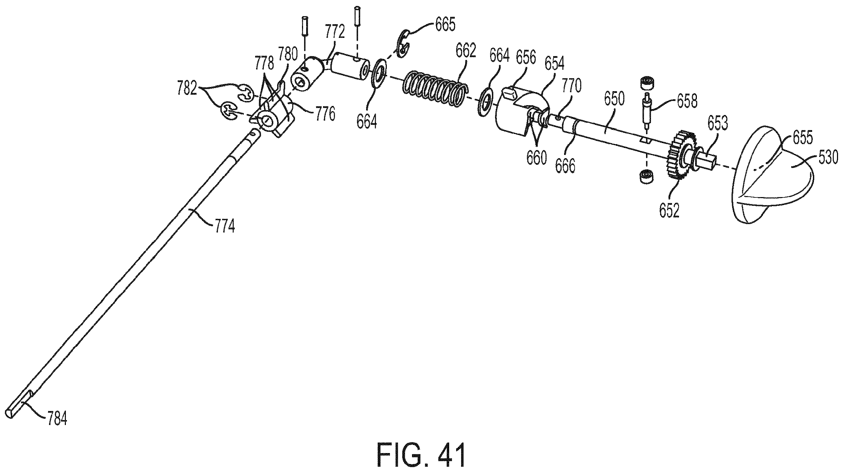

In **Fig. 41****,** the dial **530** of the plunger head assembly **522** is shown exploded away from a dial shaft **650** to which it couples onto when assembled. As shown, the dial shaft **650** comprises a square shaped end **653.** The square shaped end **653** of the dial shaft **650** fits into a square shaped orifice **655** in the dial **530** such that as the dial **530** is rotated, the dial shaft **650** is caused to rotate as well. In other embodiments, the square shaped end **653** of the dial shaft **650** and square shaped orifice **655** on the dial **530** need not necessarily be square shaped, but rather D-shaped, hexagonal, or any other suitable shape.

A dial shaft gear **652** may be disposed about the dial shaft **650.** As the dial shaft **650** is rotated, the dial shaft gear **652** may be caused to rotate about the axis of the dial shaft **650.** A dial shaft cam **654** may be slidably coupled to the dial shaft **650** such that the dial shaft cam **654** is capable of sliding along the axial direction of the dial shaft **650** and the dial shaft 650 freely rotates inside the dial shaft cam 654. The dial shaft cam **654** may comprise one or more dial shaft cam ears **656.** The dial shaft cam ears **656** may also be referred to as dial shaft cam guides since they perform a guiding function. In the example embodiment, the dial shaft cam **654** comprises two dial shaft cam ears **656.** In the example embodiment, the cam surface of the dial shaft cam **654** is substantially a section of a double helix. At the end of cam surface of the dial shaft cam **654** there may be one or more dial shaft cam detents **660.** The end of the dial shaft cam **654** opposite the cam surface may be substantially flat.

A dial shaft cam follower **658** may be coupled into the dial shaft **650** such that it rotates with the dial shaft **650.** In the example embodiment shown in **Fig. 41** the dial shaft cam follower **658** runs through the dial shaft **650** such that at least a portion of the dial shaft cam follower **658** projects from the dial shaft **650** on each side of the dial shaft **650.** This effectively creates two dial shaft cam followers **658** which are offset 180° from each other. Each end of the dial shaft cam follower **658** follows one helix of the double helix shaped cam surface of the dial shaft cam **654.**

A bias member may also be placed on the dial shaft **650.** In the example embodiment, a dial shaft compression spring **662** is placed on the dial shaft **650.** The dial shaft compression spring **662** may have a coil diameter sized to fit concentrically around the dial shaft **650.** In the example embodiment depicted in **Fig. 41****,** the dial shaft compression spring **662** is retained on each end by dial shaft washers **664.** A dial shaft retaining ring **665** may fit in an annular groove **666** recessed into the dial shaft **650.**

In **Fig. 41****,** the end of the dial shaft **650** opposite the square shaped end **653** features a peg-like projection **770.** The peg-like projection **770** may couple into a joint of a double universal joint **772.** The peg-like projection **770** may couple into the double universal joint **772** by any suitable means such as, but not limited to, screws, bolts, adhesive, snap fit, friction fit, magnets, welds, a tongue in groove arrangement, pin (shown), etc. The other joint of the double universal joint **772** may also couple onto a driven shaft **774.** The other joint of the double universal joint **772** may be coupled onto the driven shaft **774** by any suitable means such as, but not limited to, screws, bolts, adhesive, snap fit, friction fit, magnets, welds, a tongue in groove arrangement, pin (shown), etc. The dial shaft **650** and the driven shaft **774** may be oriented approximately perpendicular to each other.

In some embodiments, a driven shaft bushing **776** may be included on the driven shaft **774.** In the example embodiment shown in **Fig. 41** the driven shaft bushing **776** is a sleeve bushing. The inner surface of the driven shaft bushing **776** comprises the bearing surface for the driven shaft **774.** The outer surface of the driven shaft bushing **776** may comprise a number of driven shaft bushing projections **778** which extend outwardly from the outer surface of the driven shaft bushing **776.** In the example embodiment in **Fig. 41****,** the driven shaft bushing projections **778** are spaced approximately 120° apart from each other along the arc of the outer surface of the driven shaft bushing **776.** In the example embodiment shown in **Fig. 41****,** the driven shaft bushing projection **778** which projects toward the top of the page comprises a nub **780** which extends from the top edge of the driven shaft bushing projection **778** toward the top of the page. The driven shaft bushing **776** is held in place on the drive shaft **774** by driven shaft retaining rings **782.** One of the driven shaft retaining rings **782** may be clipped into place on the driven shaft **774** on each side of the driven shaft bushing **776.** The end of the driven shaft **774** not coupled into the double universal joint **772** may comprise a driven shaft D-shaped segment **784.**

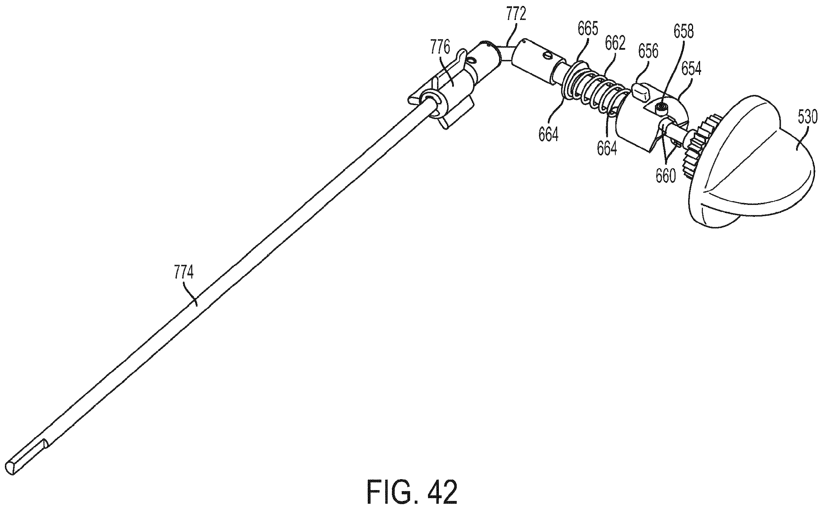

When assembled, as shown in **Fig. 42****,** the dial shaft compression spring **662** biases the dial shaft cam **654** against the dial shaft cam follower **658** such that the ends of the dial shaft cam follower **658** are at the bottom of the cam surface of the dial shaft cam **654.** One dial shaft washer **664** abuts the dial shaft retaining ring **665** and the other dial shaft washer **664** abuts the flat side of the dial shaft cam **654.** Preferably, the distance between the dial shaft washers **664** is at no point greater than or equal to the resting length of the dial shaft compression spring **662.** This ensures that there is no "slop" and that the dial shaft cam **654** is always biased against the ends of the dial shaft cam follower **658.**

As shown, the double universal joint **772** connects dial shaft **650** to the driven shaft **774** when assembled. The driven shaft bushing **776** is clipped into place on the driven shaft **774** by driven shaft retaining rings **782** (see **Fig. 41****).** In the embodiment depicted in **Fig. 42** the dial shaft **650** functions as the drive shaft for the driven shaft **774.** Any rotation of the dial shaft **650** generated through rotation of the dial **530** will be transmitted via the double universal joint **772** to the driven shaft **774.**

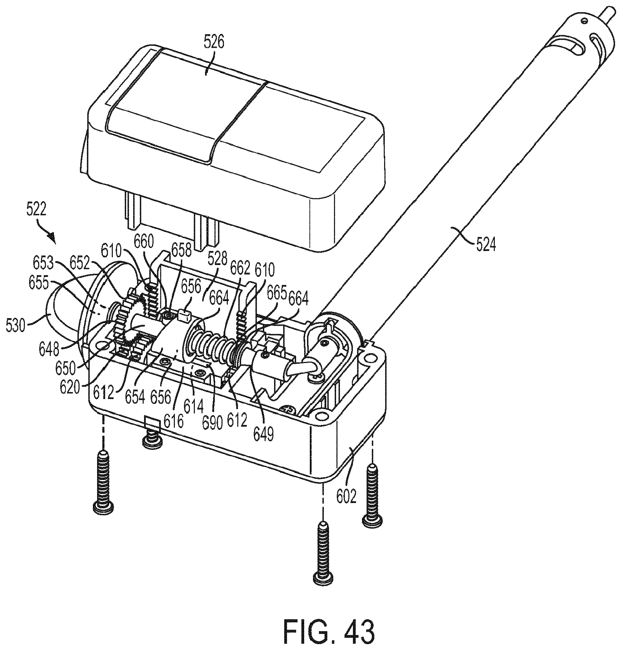

**Fig. 43** shows the whole plunger head assembly **522** with the plunger tube **524** coupled in place. The top half of the plunger head assembly **522** is exploded away from the bottom half of the plunger head assembly **522.** The bottom half of the dial shaft **650** is sitting in the lower dial shaft bearing **649** on the plunger head assembly housing bottom **602.** Another span of the bottom half of the dial shaft **650** is seated on the portion of the dial shaft passage **648** located on the plunger head assembly housing bottom **602.** As shown, the dial shaft passage **648** functions as a second bearing surface for the dial shaft **650.** The square shaped end **653** of the dial shaft **650** extends beyond the dial shaft passage **648** and couples into the square shaped orifice **655** on the dial **530.**

As shown in **Fig. 43****,** the dial shaft gear **652** on the dial shaft **650** interdigitates with the lower jaw drive gear **620.** As the dial **530** is rotated, the dial shaft **650** and dial shaft gear **652** also rotate. Rotation is transmitted through the dial shaft gear **652** to the lower jaw drive gear **620.** Rotation of the lower jaw drive gear **620** rotates the lower clamp jaw drive shaft **614** and the lower clamp jaw pinion gears **612** on the lower clamp jaw drive shaft **614.** Since the lower clamp jaw pinion gears **612** interdigitate with the lower plunger clamp jaw racks **610,** any rotation of the lower clamp jaw pinion gears **612** is translated into linear displacement of the lower plunger clamp jaw **528.** Thus, in the shown embodiment, rotating the dial **530** is the means by which a user may actuate the lower plunger clamp jaw **528** to an open or clamped position.

In the embodiment shown in **Fig. 43****,** rotation of the dial **530** also causes a linear displacement of the dial shaft cam **654** away from the dial **530** and in the axial direction of the dial shaft **650.** As shown in the example embodiment, the upper bearing surface **616** for the lower clamp jaw drive shaft **614** comprises a dial shaft cam ear slit **690** which functions as a track for a dial shaft cam ear **656.** One of the dial shaft cam ears **656** projects into the dial shaft cam ear slit **690.** This ensures that the dial shaft cam **654** may not rotate with the dial **530** and dial shaft **650** because rotation of the dial shaft cam ear **656** is blocked by the rest of the upper bearing surface **616** for the lower clamp jaw drive shaft **614.**

The dial shaft cam ear slit **690** does, however, allow the dial shaft cam **654** to displace linearly along the axial direction of the dial shaft **650.** As the dial **530** and dial shaft **650** are rotated, the dial shaft cam follower **658** also rotates. The dial shaft cam follower's **658** location on the dial shaft **650** is fixed such that the dial shaft cam follower **658** is incapable of linear displacement. As the ends of the dial shaft cam follower **658** ride up the cam surface of the dial shaft cam **654,** the dial shaft cam **654** is forced to displace toward the right face of the plunger head assembly housing bottom **602** (relative to **Fig. 43**). The dial shaft cam ears **656** also slide in this direction within the dial shaft cam ear slit **690.** This causes the dial shaft compression spring **662** to compress between the dial shaft washer **664** abutting the dial shaft cam **654** and the dial shaft washer **664** abutting the dial shaft retaining ring **665.** The restoring force of the dial shaft compression spring **662** serves to bias the dial **530,** and all parts actuated by the dial **530** to their original positions prior to any dial **530** rotation. If the dial **530** is released, the dial **530** and all parts actuated by the dial **530** will be caused to automatically return to their original orientations prior to any dial **530** rotation due to the expansion of the compressed dial shaft compression spring **662.** In the example embodiment, the original position prior to any dial **530** rotation, is the position depicted in **Fig. 35** where the upper plunger clamp jaw **526** and lower plunger clamp jaw **528** are fully closed.

In some embodiments, including the embodiment shown in **Fig. 43****,** the dial shaft cam **654** may comprise a dial shaft cam detent **660** along the cam surface of the dial shaft cam **654.** The dial shaft cam detent **660** may allow a user to "park" the dial shaft cam follower **658** at a desired point along the cam surface of the dial shaft cam **654.** In the example embodiment, the dial shaft cam detent **660** may be reached by the dial shaft cam follower **658** when the dial **530** has been fully rotated. When the dial shaft cam follower **658** is in the dial shaft cam detent **660,** the dial shaft compression spring **662** may not automatically return the dial **530** and all parts actuated by the dial **530** to their orientation prior to any rotation of the dial **530.** A user may need to rotate the dial **530** such that the dial shaft cam follower **658** moves out of the dial shaft cam detent **660** before the restoring force of the compressed dial shaft compression spring **662** may be allowed to expand the dial shaft compression spring **662** to a less compressed state.

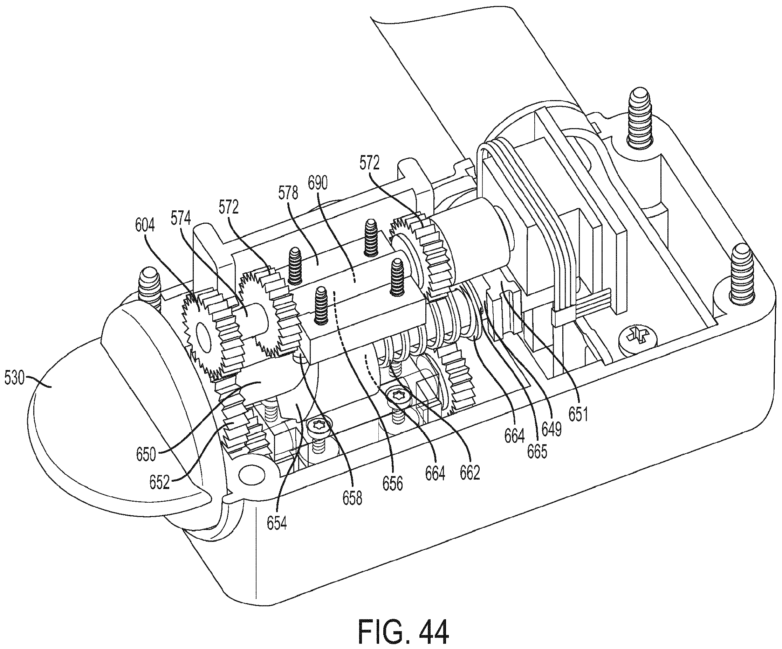

**Fig. 44** shows a similar view to the view illustrated in **Fig. 43****.** In **Fig. 44****,** the plunger head assembly housing top **600** and some parts comprising the top half of the plunger head assembly **522** are not visible. Among the parts that are visible are the upper dial shaft bearing **651,** upper clamp jaw drive shaft **574,** the upper clamp jaw pinion gears **572,** and the upper jaw drive gear **604.** As shown in **Fig. 44****,** when assembled the dial shaft **650** is sandwiched between the upper dial shaft bearing **651** and lower dial shaft bearing **649,** the dial shaft gear **652** on the dial shaft **650** interdigitates with the upper jaw drive gear **604.** As the dial **530** is rotated, the dial shaft **650** and dial shaft gear **652** also rotate. Rotation is transmitted through the dial shaft gear **652** to the upper jaw drive gear **604.** Rotation of the upper jaw drive gear **604** rotates the upper clamp jaw drive shaft **574** and the upper clamp jaw pinion gears **572** on the upper clamp jaw drive shaft **574.**

Referring back to **Fig. 38****,** the upper clamp jaw pinion gears **572** interdigitate with the upper plunger clamp jaw racks **570.** Any rotation of the upper clamp jaw pinion gears **572** is translated into linear displacement of the upper plunger clamp jaw **526.** Thus rotation of the dial **530** is the means by which a user may actuate the upper plunger clamp jaw **526** (not shown in **Fig. 44**) to an open or clamped position.

The lower bearing surface **578** for the upper jaw drive shaft **574** is also visible in **Fig. 44****.** The lower bearing surface **578** for the upper jaw drive shaft **574** may comprise a second dial shaft cam ear slit **690** in embodiments where the dial shaft cam **654** comprises more than one dial shaft cam ear **656.** The second dial shaft cam ear slits **690** may functions as a track for a dial shaft cam ear **656.** One of the dial shaft cam ears **656** projects into the second dial shaft cam ear slit **690.** This ensures that the dial shaft cam **654** may not rotate with the dial **530** and dial shaft **650** because rotation of the dial shaft cam ear **656** is blocked by the rest of the lower bearing surface **578** for the upper clamp jaw drive shaft **574.**

The second dial shaft cam ear slit **690** does, however, allow the dial shaft cam **654** to displace linearly along the axial direction of the dial shaft **650.** As the dial **530** and dial shaft **650** are rotated, the dial shaft cam follower **658** also rotates. The dial shaft cam follower's **658** location on the dial shaft **650** is fixed such that the dial shaft cam follower **658** is incapable of linear displacement. As the ends of the dial shaft cam follower **658** ride up the cam surface of the dial shaft cam **654,** the dial shaft cam **654** is forced to displace toward the right face of the plunger head assembly housing bottom 602 (relative to **Fig. 44**). A dial shaft cam ear **656** also slides in this direction within the second dial shaft cam ear slit **690.** This causes the dial shaft compression spring **662** to compress between the dial shaft washer **664** abutting dial shaft cam **654** and the dial shaft washer **664** abutting the dial shaft retaining ring **665.** The dial shaft compression spring **662,** dial **530,** and all parts actuated by the dial **530** may then behave per the above description.

In some embodiments, the upper jaw drive gear **604** (best shown in **Fig. 37**) and lower jaw drive gear **620** (best shown in **Fig. 43**) may be substantially identical gears. Additionally, the upper jaw pinion gears **572** (best shown in **Fig.** 37) and lower clamp jaw pinion gears **612** (best shown in **Fig. 40**) may be substantially identical gears. In such embodiments, the upper plunger clamp jaw **526** and lower plunger clamp jaw **528** (see **Figs. 30-34**) will experience an equal amount of linear displacement per degree of rotation of the dial **530.** Since the point of interdigitation of the upper jaw drive gear **604** on dial shaft gear **652** is opposite the point of interdigitation of the lower jaw drive gear **620** on the dial shaft gear **652,** the upper plunger clamp jaw **526** and lower plunger clamp jaw **528** will linearly displace in opposite directions.

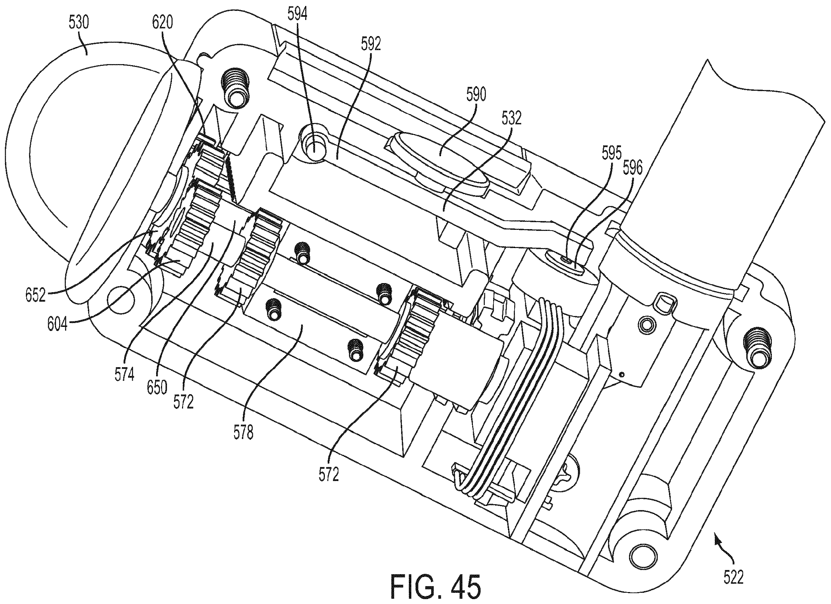

**Fig. 45** shows a view similar to the view shown in **Fig. 44****.** **Fig. 45** depicts an assembled view of the plunger head assembly **522** from a slightly different perspective. As shown in **Fig. 45****,** the dial **530** is coupled to the dial shaft **650.** The dial shaft gear **652** is in an interdigitating relationship with both the upper jaw drive gear **604** and the lower jaw drive gear **620.** The upper jaw drive gear **604** is disposed on the upper jaw drive shaft **574** along with two upper jaw pinion gears **572.** The upper jaw pinion gears **572** may be spaced apart by the lower bearing surface **578** for the upper jaw drive shaft **574** as shown in **Fig. 45****.**

The plunger pressure sensor **532** in the embodiment depicted in **Fig. 45** comprises a plunger pressure sensor push plate **590** which extends out of the plunger head assembly **522** such that it may physically contact a plunger flange **548** (as shown in **Fig. 34**) clamped against the plunger head assembly **522.** The plunger pressure sensor push plate **590** is attached to a plunger pressure sensor lever **592.** The plunger pressure sensor lever **592** is pivotally coupled to a plunger pressure sensor pivot **594.** The plunger pressure sensor pivot **594** is disposed at the left end of the plunger pressure sensor lever **594** (relative to **Fig. 45**). In the example embodiment in **Fig. 45****,** any force applied to the plunger pressure sensor push plate **590** is transmitted through the plunger pressure sensor lever **594** to the plunger pressure sensor input surface **596.** Although the location of the plunger pressure sensor pivot **594** in relation to the plunger pressure sensor push plate **590** does not multiply the force exerted against the plunger pressure sensor input surface **596** in **Fig. 45****,** other embodiments may use different arrangements to create a mechanical advantage. The plunger pressure sensor **532** in **Fig. 45** also comprises a plunger pressure sensor force concentrator **595** which is a small projection extending from the plunger pressure sensor lever **592** to the plunger pressure sensor input surface **596.** The plunger pressure sensor force concentrator **595** concentrates force exerted against the plunger pressure sensor input surface **596** to help promote a more accurate pressure reading.

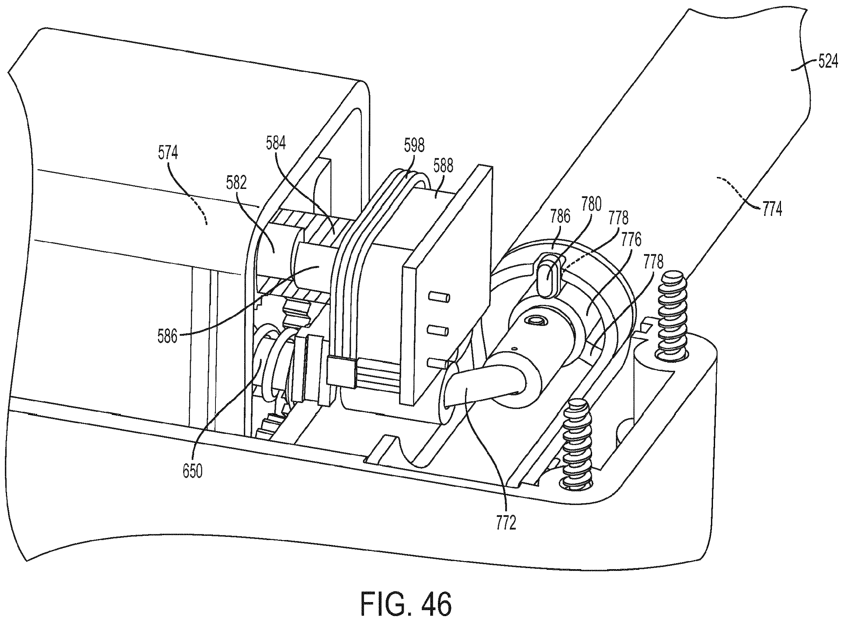

**Fig. 46** shows a close up of how the upper jaw drive shaft **574** is connected to the D-shaped shaft **586** projecting from the plunger clamp jaws position sensor **588.** In the embodiment depicted in **Fig. 46****,** the upper jaw drive shaft **574** comprises a D-shaped span **582.** The D-shaped span **582** of the upper jaw drive shaft **574** projects into a complimentary shaped orifice in the D-shaped connector **584.** The D-shaped connector **584** in **Fig. 46** is shown in cross-section. A D-shaped shaft **586** projecting out of the plunger clamp jaws position sensor **588** also projects into the D-shaped connector **584.** Any rotation of the upper jaw drive shaft **574** may cause the D-shaped connector **584** to rotate as well. In turn, this may cause rotation of the D-shaped shaft **586** projecting from the plunger clamp jaws position sensor **588.** As mentioned above this rotation may cause the wiper to slide across the resistive element of the plunger clamp jaws position sensor **588** in embodiments where the plunger clamp jaws position sensor **588** comprises a potentiometer.

**Fig. 46** also shows the dial shaft **650** connected to the double universal joint **772.** As shown in the example embodiment in **Fig. 46****,** the driven shaft **774** is also coupled to the double universal joint projects down the interior of the hollow plunger tube **524.** The nub **780** on the driven shaft bushing projection **778** of the driven shaft bushing **776** is seated in a plunger tube notch **786** recessed into the edge of the plunger tube **524** to lock the nub **780** within the plunger tube notch **786.** Seating the nub **780** in the plunger tube notch **786** restricts the driven shaft bushing **776** from rotation because the nub **780** may not rotate through the sides of the plunger tube notch **786.** Each of the driven shaft bushing projection **778** abuts the interior surface of the plunger tube **524** which keeps the driven shaft bushing **776** centered in the plunger tube **524.**

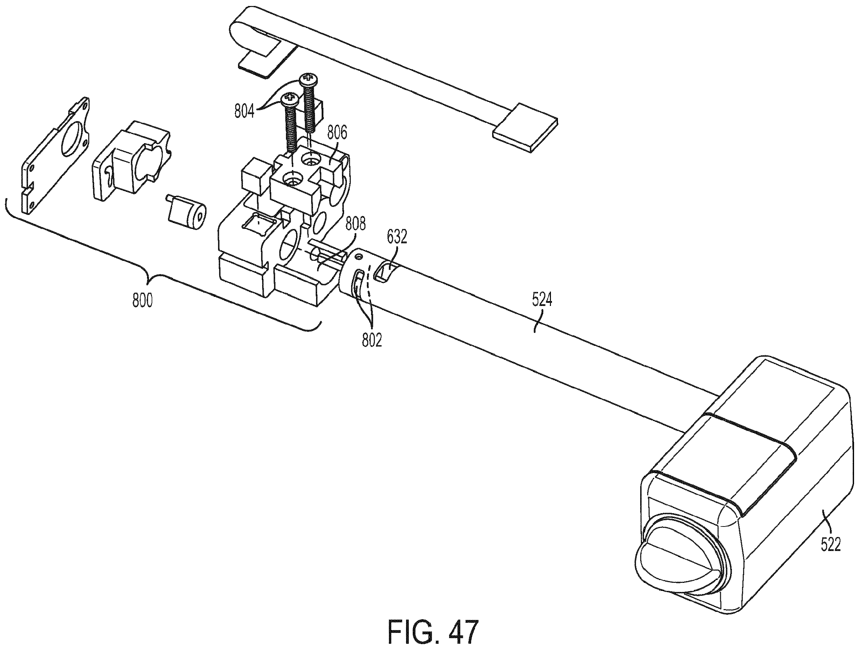

The plunger tube **524** may also serve as a channel for the electrical conduits **598** to and from the plunger clamp jaws position sensor **588** and the plunger pressure sensor **532.** Since the plunger tube **524** is sealed to liquid when the syringe pump is fully assembled, the plunger tube **524** protects the electrical conduits **598** from exposure to liquid. The electrical conduits **598** exit the plunger tube **524** through the conduit opening **632** of the plunger tube **524** shown in **Fig. 47****.**

**Fig. 47** depicts an exploded view of a sliding block assembly **800.** As shown, the plunger tube **524** which extends from the plunger head assembly **522** comprises two plunger tube cutouts **802.** The plunger tube cutouts **802** are cut into the front and back sides of the plunger tube **524.** In **Fig. 47****,** only the front plunger tube cutout **802** is visible. The plunger tube cutouts **802** allow the plunger tube to be non-rotationally coupled to the sliding block assembly **800.** In the example embodiment, two plunger tube coupling screws **804** run through a plunger tube bracket **806,** down the plunger tube cutouts **802** and into a plunger tube support **808.** The plunger tube **524,** is thus tightly sandwiched between the plunger tube bracket **806** and the plunger tube support **808.** Any rotation of the plunger tube **524** is obstructed by plunger tube coupling screws **804** which abut the top and bottom edges of the plunger tube cutouts **802.** Similarly, any axial displacement of the plunger tube **524** is obstructed by the plunger tube coupling screws **804** which abut the sides of the plunger tube cutouts **802.** In other embodiments, the plunger tube **524** may be coupled to the sliding block assembly **800** by any other suitable means such as, but not limited to, bolts, adhesive, snap fit, friction fit, magnets, welds, a tongue in groove arrangement, pin, etc.

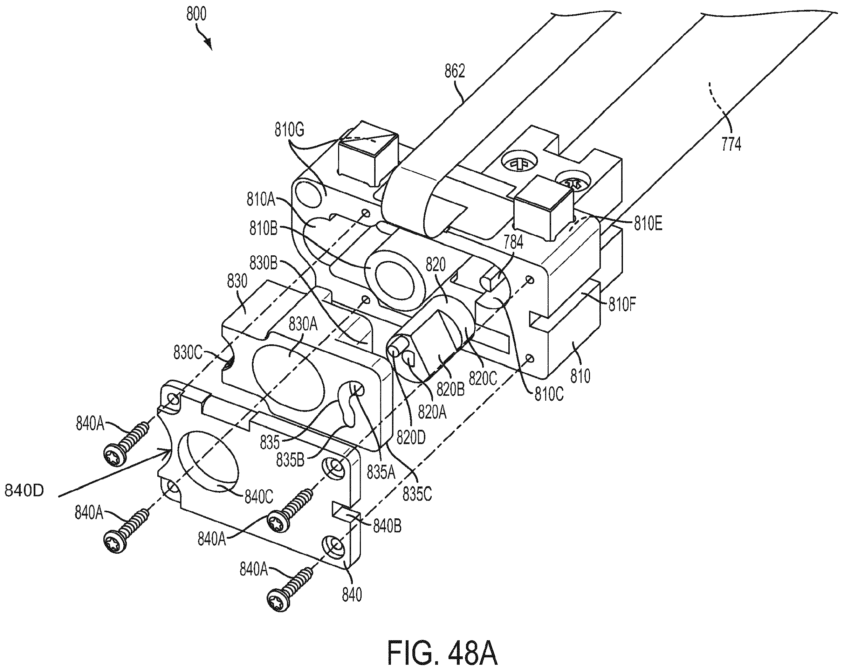

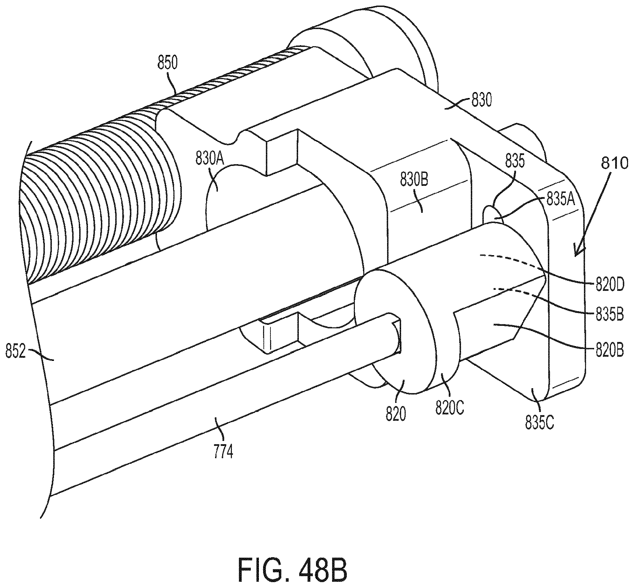

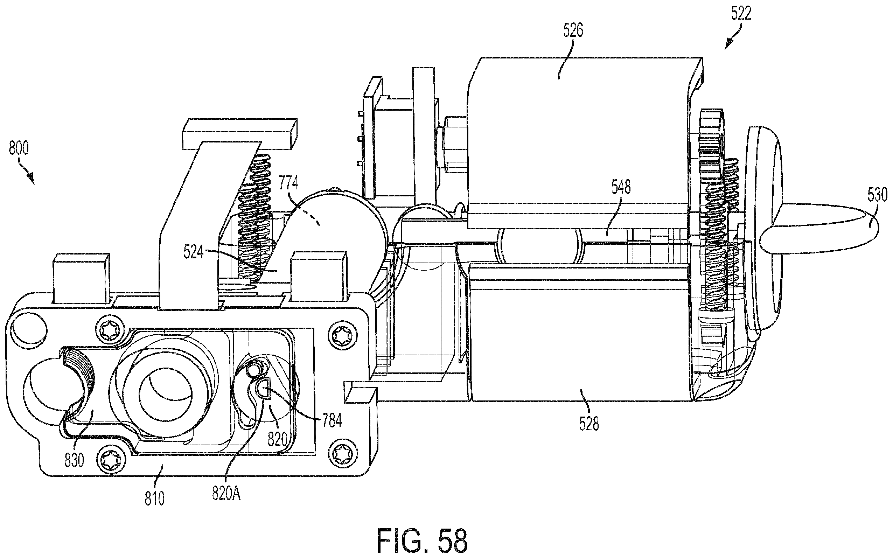

A closer exploded view of the sliding block assembly **800** is shown in **Fig. 48A****.** The sliding block assembly **800** comprises a number of parts. The sliding block assembly **800** comprises a half nut housing **810,** a barrel cam **820,** a half nut **830,** and a half nut cover plate **840.** The half nut housing **810** may be manufactured from any suitable strong material will not significantly deform under the applied loads such as, metal, nylon, glass-filled plastics, molded plastic, a polyoxymethylene plastic such as Delrin, etc. The half-nut **830** is preferably fabricated from bearing metals such as brass, bronze etc that interact well with stainless steel surfaces typical of lead screws. The barrel-cam **820** is preferably fabricated from a hard metal such as stainless to form a good bearing pair with the half nut **830.** The half nut housing **810** comprises a lead screw void **810A.** The lead screw void **810A** allows the lead screw **850** (not shown, see **Fig. 48B****)** to pass through the half nut housing **810.** The lead screw void **810A** has a diameter larger than the lead screw **850** which ensures that the lead screw **850** passes uninhibited through the lead screw void **810A** irrespective of the point on the lead screw **850** at which sliding block assembly **800** is located. The sliding block assembly 800 includes a ribbon cable 562 to receive power from and for communications with the circuit board 1150 (refer to Fig. 58A).

The half nut housing **810** may also comprise a guide rod bushing **810B.** The guide rod bushing **810B** in the example embodiment depicted in **Fig. 48A** is formed as continuous piece of the half nut housing. The guide rod **852** (not shown, see **Fig. 48B**) extends through the guide rod bushing **810B** in the half nut housing **810** with the interior surface of the guide rod bushing **810B** serving as a bearing surface for the guide rod **852.** In some embodiments, the guide rod bushing **810B** may not be formed as a continuous part of the half nut housing **810** but rather coupled to the half nut housing **810** in any number of suitable ways. The guide rod bushing **810B** may be made from a lubricious material such as bronze, brass, PTFE, delrin etc, which provides a low friction surface to mate with a hard surface of a guide rod **852** (Fig. 48B).

The half nut housing **810** may also comprise a barrel cam void **810C.** The barrel cam void **810C** may be sized such that it has a diameter slightly larger than the diameter of the barrel cam **820.** When the sliding block assembly **800** is fully assembled, the barrel cam **820** may fit into the barrel cam void **810C** on the half nut housing **810.** In some embodiments, the barrel cam void **810C** may extend all the way through the half nut housing **810.** In the example embodiment shown in **Fig. 48A****,** the barrel cam void **810C** does not extend all the way through the half nut housing **810.** The barrel cam void **810C** may function as a bushing for the barrel cam **820** when the sliding block assembly **800** is fully assembled. The barrel cam void 810C and barrel cam 820 may be manufactured with a clearance fit. In one example the diametrical clearance between the barrel cam void 810C and the barrel cam 820 is 0.001 to 0.005 inches.

In some embodiments, including the embodiment depicted in **Fig. 48A****,** the half nut housing **810** may include a half nut void **810D.** The half nut void **810D,** may be recessed into the half nut housing **810** such that the half nut **830** may fit in the half nut void **810D** when the sliding block assembly **800** is fully assembled. In some embodiments, the lead screw void **810A,** barrel cam void **810C,** and half nut void **810D** may all be part of a single void recessed into the half nut housing **810.**

The half nut housing **810** may comprise a driven shaft aperture **810E.** The driven shaft aperture **810E** extends through the half nut housing **810** and into the barrel cam void **810C.** In **Fig. 48A** the driven shaft D-shaped segment or shaft collar **784** is shown protruding into the barrel cam void **810C** through the driven shaft aperture **810E.**

The half nut housing **810** may additionally comprise a half nut housing groove **810F.** In the example embodiment in **Fig. 48A****,** the half nut housing groove **810F** is recessed into the half nut housing **810.** The half nut housing groove **810F** is recessed along the entire side of the half nut housing **810.** The half nut housing groove **810F** extends in a direction parallel to the direction of elongation of the plunger tube **524,** lead screw **850,** and guide rod **852** (shown, e.g., in Fig. **48B**).

In some embodiments, the half nut housing **810** may comprise at least one limit switch **810G** (not shown). In the example embodiment depicted in **Fig. 48A****,** the half nut housing **810** may comprise two limit switches **810G** (not shown). One limit switch **810G** is located on the front of the half nut housing **810** and the other limit switch **810G** is located on the back of the half nut housing **810.** The limit switch(es) **810G** may be used to limit the range of movement of the sliding block assembly along the lead screw **850** (**Fig. 48B**). The limit switches **810G** will be further elaborated upon later.

As previously mentioned, the barrel cam **820** fits into the barrel cam void **810C** in the half nut housing **810** when the sliding block assembly **800** is fully assembled. As shown, the barrel cam **820** comprises a D-shaped orifice **820A** which extends through the entire barrel cam **820** along the axial direction of the barrel cam **820.** The D-shaped orifice **820A** is sized and shaped to allow the barrel cam **820** to be coupled onto the driven shaft D-shaped segment **784.** When the D-shaped orifice **820A** of the barrel cam **820** is coupled onto the driven shaft D-shaped segment **784** any rotation of the driven shaft **774** and driven shaft D-shaped segment **784** causes the barrel cam **820** to rotate as well. The barrel cam 820 may be joined to the driven shaft 774 in any of the standard methods including but not limited to set screws, pins, adhesive, friction fit, welds, etc.

As shown in **Fig. 48A** the barrel cam **820** is generally a truncated cylinder, and comprises a barrel cam flat **820B** which is cut into the barrel cam **820** along a chord of the front facing base of the cylinder of the barrel cam **820.** The barrel cam flat 820B may be cut such that some distance from the barrel cam center-line so that the full diameter of the barrel cam 820 remains. The remaining material of barrel cam 820 on the far side of the centerline relative to the half-nut 830B bearing surface provides a bearing surface to transfer forces from the half-nut 820 to the barrel cam void 820C along the entire length of the barrel cam 820.

The barrel cam flat **820B** may not extend along the entire barrel cam **820** leaving some of the cylinder of the barrel cam **820** to have an unadulterated, classic cylindrical shape. This is desirable because the classic cylindrically shaped portion of the barrel cam **820** may act as a journal within the barrel cam void **810C** which may act as a bushing. In the example embodiment depicted in **Fig. 48A****,** the barrel cam flat **820B** extends along the barrel cam **820** until a barrel cam shoulder **820C** begins. The barrel cam shoulder **820C** may extend perpendicularly from the surface of the barrel cam flat **820B.** In the example embodiment in **Fig. 48A****,** the expanse of the barrel cam **820** with the unadulterated, classic cylindrical shape is the barrel cam shoulder **820C.**

As shown, the barrel cam **820** may also comprise a barrel cam pin **820D.** The barrel cam pin **820D** in the example embodiment in **Fig. 48A** projects perpendicularly from the front facing base of the cylinder of the barrel cam **820.** The barrel cam pin **820D** projects from the front facing base of the barrel cam **820** near the chord from which the barrel cam flat **820B** has been extended into the cylinder of the barrel cam **820.**

The sliding block assembly **800** may also comprise a half nut **830** as mentioned above. In the example embodiment in **Fig. 48A****,** the half nut **830** comprises a half nut slot **835.** The half nut slot **835** is sized such that it may act as a track-way for the barrel cam pin **820D.** The half nut slot **835** comprises an arcuate section **835A** and an end section **835B** which is not curved or arced. The half nut slot **835** may be cut into a half nut slot plate **835C** which extends perpendicularly from a half nut cam follower surface **830B.** The half nut cam follower surface **830B** and the half nut slot **835** will be further elaborated on in the following paragraphs.

The half nut **830** may comprise a guide rod bushing void **830A.** The guide rod bushing void **830A** of the half nut **830** allows the guide rod bushing **810B** to pass through the half nut **830.** In the example embodiment shown in **Fig. 48A****,** the guide rod bushing void **830A** is substantially larger than the diameter of the guide rod bushing **810B.** Additionally, the guide rod bushing void **830A** in the half nut **830** may have an elliptical shape or stadium shape. Such a shape allows the guide rod bushing **810B** to fit comfortably within the guide rod bushing void **830A** when the half nut **830** is engaged, disengaged, or in transition between either position.

The half nut **830** may also comprise a span of half nut threads **830C.** The half nut threads **830C** are capable of engaging the threads of the lead screw **850** (not shown, see **Fig. 48B**). In the example embodiment shown in **Fig. 48A****,** the half nut threads **830C** are V-shaped threads. V-shaped threads may be desirable because such a shape may help to self align the half nut threads **830C** on the lead screw **850.**

As mentioned above, the sliding block assembly **800** may also comprise a sliding block cover plate **840.** The sliding-block, cover plate **840** may be coupled onto the half nut housing **810** such that the barrel cam **820** and half nut **830** are kept in place within the sliding block assembly **800** when the sliding block assembly **800** is fully assembled. In the example embodiment shown in **Fig. 48A** the sliding block cover plate **840** may be coupled onto the half nut housing **810** by sliding block cover plate screws **840A** as shown, or by any suitable means such as, but not limited to, bolts, adhesive, snap fit, friction fit, magnets, welds, a tongue in groove arrangement, pin, etc. The sliding block cover plate **840** may comprise a cover plate groove **840B** to assist in guiding the half nut housing **810.** The cover plate groove **840B** may be recessed into the sliding block cover plate **840.** In the example embodiment shown in **Fig. 48A** the cover plate groove **840B** is recessed along an entire side edge of the sliding block cover plate **840.** The cover plate groove **840B** may sized and disposed such that it lines up with the half nut housing groove **810F** on the half nut housing **810.**

The sliding block cover plate **840** may comprise a guide rod bushing aperture **840C.** The guide rod bushing aperture **840C** is sized and disposed such that the guide rod bushing **810B** may project through the guide rod bushing aperture **840C.** The guide rod bushing aperture **840C** may have a diameter substantially equal to, or slightly larger than, the outer diameter of the guide rod bushing **810B.**

The edge of the sliding block cover plate **840** opposite the cover plate groove **840B,** may comprise a lead screw trough **840D.** The lead screw trough **840D** may be an arced section recessed into the edge of the sliding block cover plate **840.** The lead screw trough **840D,** in conjunction with the lead screw void **810A** of the half nut housing **810** allows the sliding block assembly 800 to be placed on the lead screw **850.**

In operation, the sliding block assembly **800** may be caused to move along the axial direction of the lead screw **850** and guide rod **852** as a result of lead screw **850** rotation. The sliding block assembly **800** may also be moved along the axial direction of the lead screw **850** and guide rod **852** by a user. For a user to move the sliding block assembly **800** along the axial direction of the lead screw **850** the user may need to adjust the location of the plunger head assembly **522** relative to the rest of the syringe pump assembly **501** as shown and described in relation to **Figs. 32-33****.** This may only be done by a user when the half nut **830** is not engaged with the lead screw **850**

**Fig. 48B** shows the half nut **830** in an engaged position on the lead screw **850.** The half nut housing **810,** and half nut cover plate **840** visible in **Fig. 48A** have been removed in **Fig. 48B****.** When the half nut **830** is in engagement with the lead screw **850,** the half nut threads **830C** may operatively be engaged with the threads of the lead screw **850.** Any rotation of the lead screw **850** may cause the half nut **830** to move in the axial direction of the lead screw **850.**

To move the half nut **830** between an engaged and disengaged position on the lead screw **850,** the barrel cam **820** must be rotated. As the barrel cam **820** is rotated, the barrel cam pin **820D** may move along the half nut slot **835** in the half nut slot plate **835C.** In the example embodiment shown in **Fig. 48B****,** when the barrel cam pin **820D** is located in the arcuate section **835A** of the half nut slot **835,** the half nut **830** is engaged with the lead screw 850. The arcuate section **835A** of the half nut slot **835** may be shaped such that any movement of the barrel cam pin **820D** within the arcuate section **835A** of the half nut slot **835** does not result in any movement of the half nut **830.**

When the barrel cam **820** is rotated such that the barrel cam pin **820D** enters the straight, end section **835B** of the half nut slot **835,** further rotation of the barrel cam **820** may cause the half nut **830** to disengage from the lead screw **850.** The straight nature of the end section **835B** ensures that the further rotation of the barrel cam **820** causes the barrel cam pin **820D** to pull the half nut **830** away from the lead screw **850** until the barrel cam pin **820D** reaches the end of the end section **835B.** Rotation of the barrel cam **820** in the opposite direction will cause the barrel cam pin **820D** to push the half nut **830** back into engagement with the lead screw **850.**

In the example embodiment in **Fig. 48B****,** when the barrel cam **820** has disengaged the half nut **830** from the lead screw **850,** the half nut cam follower surface **830B** rests in the void created by the barrel cam flat **820B.** When the half nut **830** is disengaged, the distance between the half nut threads **830C** and their point of full engagement on the lead screw **850** is less than or equal to the length of the sagitta of the cylindrical segment removed from the barrel cam **820** to create the barrel cam flat **820B.** As the barrel cam **820** is rotated to engage the half nut **830** with the lead screw **850,** , the pin **820D** in the straight, end section 835B moves the half-nut toward the lead screw **850** until the halfnut 830 is at least partial engaged with the lead screw 850. As the pin 820D exits the end section 835B, the untruncated arc of barrel cam **820** rotates onto the half nut cam follower surface **830B** of the half nut **830.** The untruncated arc of the barrel may push the half nut **830** into full engagement with the lead screw **850** and supplements the action of the barrel cam pin **820D** in the half nut slot **835.**

Referring back to the example embodiment shown in **Fig. 48A****,** the driven shaft **774** to which the barrel cam **820** is coupled may not deflect when the barrel cam **820** has engaged, disengaged, or is transitioning the half nut **830** from an engaged or disengaged position on the lead screw **850.** As shown, the barrel cam void **810C** in the half nut housing **810** supports the barrel cam **820** when the sliding block assembly **800** is fully assembled. Consequently, any force promoting deflection of the driven shaft **774** is checked by the barrel cam **820** abutting the sides of the barrel cam void **810C.** This ensures that the half nut threads **830C** may not skip on the threads of the lead screw **850** under high axial loads. It also creates minimal drag as the sliding block assembly **800** travels along the lead screw **850** with rotation of the lead screw **850.**

In some embodiments, the fit of the half nut **830** and the barrel cam **820** may be adjustable. In such embodiments, a portion of the barrel cam housing 810 that defines the barrel cam void **810C** may have an adjustable position relative to the guide rod that can be adjusted for example by rotation of a set screw or other adjustment means. This may also allow a user to adjust the barrel cam **820** to an optimal or near optimal position. Alternatively, inserts may be added to the barrel cam void 810C or the barrel cam 820 may be replaced with different sized barrel cam 820 to position the half-nut 830D/barrel cam 820 interface at the optimal location. In such a position, the barrel cam **820** may engage the half nut threads **830C** on the lead screw **850** such that there is zero or minimal backlash without loading the half nut threads **830C** against the lead screw **850** and creating excessive drag.

In alternate embodiments, the barrel cam pin **820D** may be optional. In some alternate embodiments, the barrel cam pin **820D** may be replaced by one or more bias members. The bias members may bias the half nut **830** to the disengaged position. In such embodiments, rotation of the barrel cam **820** may cause the half nut **830** engage or disengage with the lead screw **850.** When the barrel cam flat **820B** is not contacting the half nut cam follower surface **830B** the one or more bias members may be overcome and the half nut threads **830C** may be engaged with the threads of the lead screw **850.** As the barrel cam flat **820B** rotates onto the half nut cam follower surface **830B,** the bias member(s) may act as a spring return which automatically biases the half nut **830** out of engagement with the lead screw **850** and against the barrel cam flat **820B.** The barrel cam 820 may include a transitional cam surface between the barrel cam flat 820 B and the untruncated arc of barrel cam 820 to facilitate displacing the half nut 830 toward the lead screw 850. Use of the barrel cam pin **820D** may be desirable because such an arrangement requires less torque to engage or disengage the half nut **830** than embodiments which may employ one or more bias members as a substitute. Some embodiments may use both the barrel cam pin **820D** and one or more bias members to effect engagement or disengagement of the half nut **830.**

In some embodiments, the bias member may bias the half nut **830** towards the engaged position, in which case, the barrel cam pin **820** may be configured to lift the half nut threads **830C** off the lead screw **850.**

In another alternative embodiment, the barrel cam **820** may not comprise a barrel cam pin **820D** and the half nut **830** may not comprise a half nut slot **835.** In such embodiments, the barrel cam flat **820B** may comprise a magnet and the half nut cam follower surface **830B** may also comprise a magnet. Instead of using the barrel cam pin **820D** to pull the half nut **830** away from the lead screw **850,** the magnet on the half nut cam follower surface **830B** may be attracted to the magnet on the barrel cam flat **820B** and be pulled off the lead screw **850** toward the barrel cam flat **820B** when the barrel cam **820** has been rotated the appropriate amount. In some embodiments, the barrel cam **820** may be a simple two pole magnet. In such embodiments, the barrel cam **820** may be disposed such that it may repel or attract a magnet on the half nut cam follower surface **830B.** When like poles of the magnets face each other, the half nut is forced into engagement with the lead screw **850.** By rotating the driven shaft **774** and therefore the magnetic barrel cam **820,** opposite poles may be made to face each other. In turn, this may cause the half nut **830** to disengage from the lead screw **850** as it is attracted to the magnetic barrel cam **820.**

In some embodiments, a magnet may be configured to bias the half nut **830** towards the engaged position, in which case, the barrel cam pin **820** may be configured to lift the half nut threads **830C** off of the lead screw **850.**

The guide rod **852** is also visible in **Fig. 48B****.** In **Fig. 48B** the guide rod **852** extends in an axial direction parallel to that of the lead screw **850.** The guide rod passes through the guide rod bushing void **830A** in the half nut **830.** In the example embodiment, the guide rod **852** is made of a hard and durable material. For example, in some embodiments, the guide rod **852** may be made of a material such as stainless steel. In other embodiments, the guide rod **852** may be chromium plated.

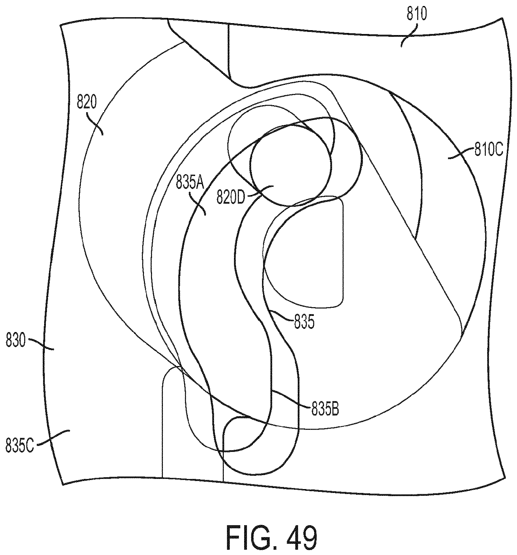

**Fig. 49** shows a close up view of the half nut slot plate **835C.** The half nut slot plate **835C** is transparent in the **Fig. 49****.** The half nut slot **835** is shown in the half nut slot plate **835C.** As described above, the half nut slot **835** comprises an arcuate section **835A** and a straight, end section **835B.** The barrel cam **820** is shown behind the transparent half nut slot plate **835C.** As shown, the barrel cam pin **820D** is located in the arcuate section **835A** of the half nut slot **835.** As mentioned above, when the barrel cam pin **820D** is in the arcuate section **835A** of the half nut slot **835** the half nut **830** is engaged with the lead screw **850** as shown in **Fig. 48B****.** The barrel cam **820** is disposed in the barrel cam void **810C** in the half nut housing **810.** The barrel cam void **810C** acts as a bushing for the barrel cam **820** and supports the barrel cam **820.**

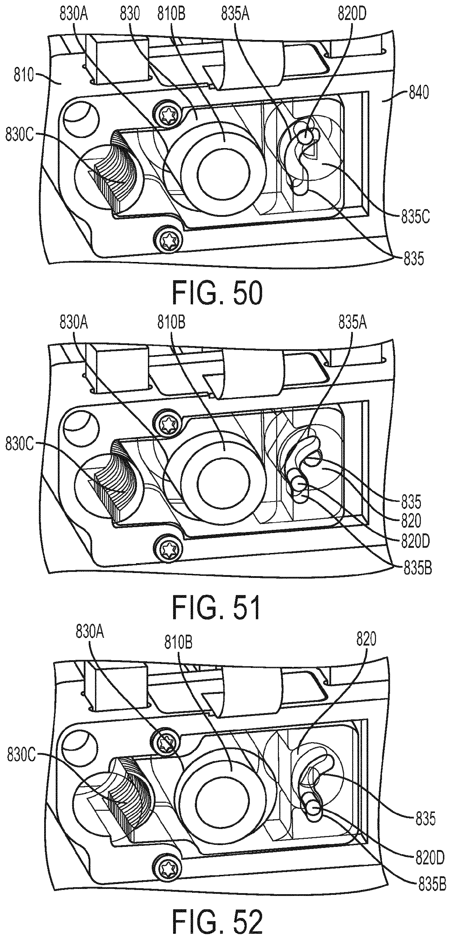

**Figs. 50-52** show sliding block assembly **800** with the half nut cover plate **840** and half nut **830** shown as transparent. In **Figs. 50-52****,** the half nut **830** transitions from an engaged position (**Fig. 50**) to a disengaged position (**Fig. 52**). As shown in **Fig. 50** the half nut **830** is in the engaged position. The barrel cam pin **820D** is located in arcuate section **835A** of the half nut slot **835.** The half nut threads **830C** are at the far left extent (relative to **Figs. 50-52**) of their range of movement. The guide rod bushing **810B** of the half nut housing **810** projects through the guide rod bushing void **830A** of the half nut **830.** As shown, the guide rod bushing **810B** is located at the far right end of the guide rod bushing void **830A.** In the example embodiment shown in **Figs. 50-52** the guide rod bushing void **830A** in the half nut **830** is roughly stadium shaped.

The barrel cam **820** has been rotated such that the barrel cam pin **820D** is about to cross from the arcuate section **835A** of the half nut slot **835** and into the end section **835B** of the half nut slot **835** in **Fig. 51****.** As shown, the half nut threads **830C** have not moved from the engaged position and are still at the far left extent (relative to **Figs. 50-****52**) of their range of movement. Similarly, the half nut 830 may not have moved relative to the guide rod bushing **810B** from the position depicted and described in relation to **Fig. 50****.**

In **Fig. 52** the barrel cam **820** has been rotated such that the barrel cam pin **820D** has moved into the straight, end section **835B** of the half nut slot **835.** As described above, further rotation of the barrel cam **820** once the barrel cam pin **820D** enters the end section **835B** of the half nut slot **835** causes the half nut **830** to disengage. As shown, the half nut **830,** and consequentially the half nut threads **830C,** have moved from the far left extent (relative to **Figs. 50-52**) of their range of movement and toward the right of the page. The half nut **830** has moved in relation to the guide rod bushing **810B,** such that the guide rod bushing **810B** is now near the far left end of the guide rod bushing void **830A.**

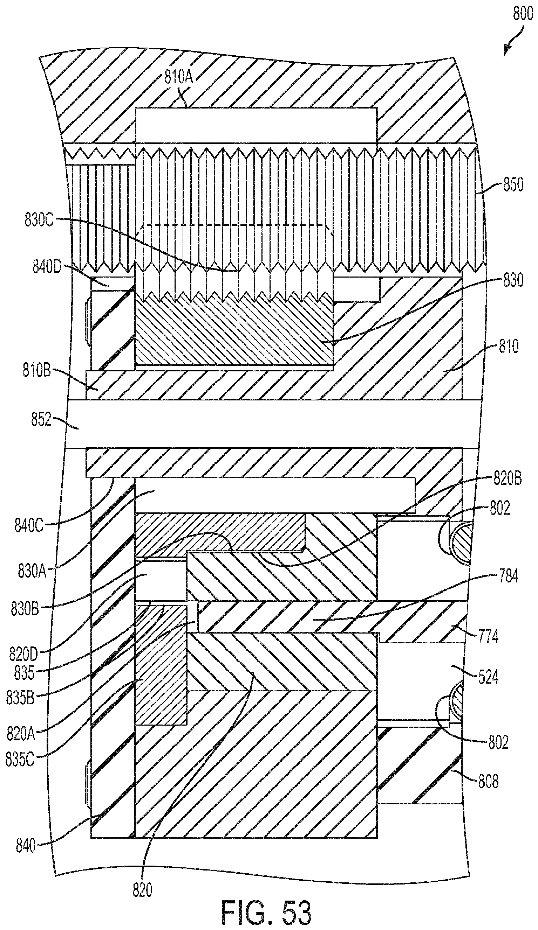

**Fig. 53** shows a cross section of most of the components comprising an embodiment of the sliding block assembly **800.** The sliding block assembly **800** is depicted fully assembled in **Fig. 53****.** The lead screw **850** and guide rod **852** are not depicted in cross section in **Fig. 53****.** As shown, the lead screw **850** extends through the lead screw void **810A** in the half nut housing **810** and over the lead screw trough **840D** in the half nut cover plate **840.** The guide rod extends through the guide rod bushing **810B.** The guide rod bushing **810B** extends through both the guide rod bushing void **830A** in the half nut **830** and the guide rod bushing aperture **840C** in the half nut cover plate **840.**

In the example embodiment shown in **Fig. 53****,** the half nut **830** is in the disengaged position. The half nut threads **830C** are not operatively interdigitated with the threads of the lead screw **850.** The guide rod bushing **810B** is near the top of the guide rod bushing void **830A** in the half nut **830.** The half nut cam follower surface **830B** is near or is abbuting (depending on the embodiment) the barrel cam flat **820B** on the barrel cam **820.** Additionally, the barrel cam pin **820D** is at the end of the straight, end section **835B** of the half nut slot **835** which is cut into the half nut slot plate **835C.**

**Fig. 53** also shows the D-shaped orifice **820A** of the barrel cam **820** coupled onto the driven shaft D-shaped segment **784** of the driven shaft **774.** The plunger tube **524** through which the driven shaft **774** is disposed can be seen coupled onto the sliding block assembly **800** by means of screws running through the plunger tube cutouts **802** and into the plunger tube support **808.**

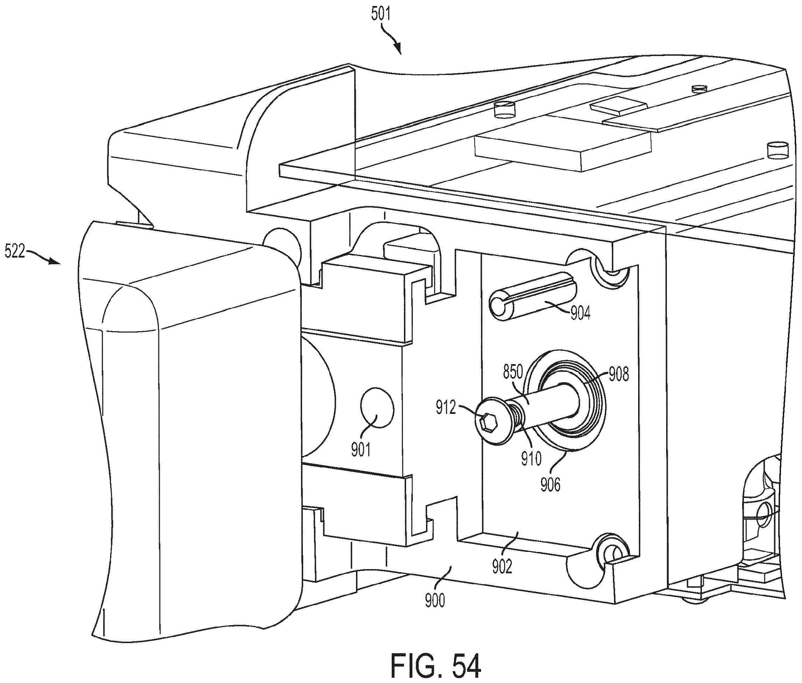

**Fig. 54** shows a view of a portion of an embodiment of the syringe pump assembly **501.** At the left side of **Fig. 54****,** a section of the plunger head assembly **522** is visible. As shown in **Fig. 54****,** the rear face **900** of the syringe pump assembly **501** may comprise a rear face guide rod hole **901.** The rear face guide rod hole **901** may run through the entire rear face **900** of the syringe pump assembly **501** at an angle perpendicular to the rear face **900** of the syringe pump assembly **501.** As shown, the guide rod hole **901** may be substantially cylindrical.

The rear face **900** of the syringe pump assembly **501** may comprise a gearbox depression **902.** As shown, the gearbox depression **902** is recessed into the rear face **900** of the syringe pump assembly **501.** In the example embodiment, the gearbox depression **902** is a roughly rectangular shaped depression. In other embodiments, the gearbox depression **902** may have alternative shapes.

As shown in **Fig. 54****,** an anti-rotation pin **904** projects out of the gearbox depression **902.** The anti-rotation pin **904** in the example embodiment shown in **Fig. 54** is cylindrical. In alternate embodiments, the anti-rotation pin **904** may take any other suitable shape. As shown in **Fig. 54****,** the gearbox depression **902** in the rear face **900** of the syringe pump assembly **501** may also comprise a lead screw void **906.** The lead screw void **906** may be cut all the way through the rear face **900** of the syringe pump assembly **501** and allow at least a portion of the lead screw **850** to project beyond of the rear face **900** of the syringe pump assembly **501.** As shown in the example embodiment, the section of the lead screw **850** which projects beyond the rear face **900** of the syringe pump assembly **501** is not threaded.

In the example embodiment shown in **Fig. 54****,** the section of the lead screw **850** that is visible is smaller in diameter than the lead screw void **906.** This is desirable because it may allow a rear face lead screw bearing **908** to be placed in the lead screw void **906** to provide a bearing surface for the lead screw **850.** In the example embodiment in **Fig. 54** a lead screw bearing is disposed in the lead screw void **906** to provide a bearing surface for the lead screw **850.**

As shown, the end of the of the section of the lead screw **850** which projects out of the rear face **900** may comprise a threaded bore **910.** In the example embodiment shown in **Fig. 54****,** a gearbox attachment fastener **912** is coupled into the threaded bore **910** on the end of the lead screw **850.** In the example embodiment, the gearbox attachment fastener **912** is a screw with a hex socket head. In other embodiments, any other suitable fastener, or fastener head may be used.

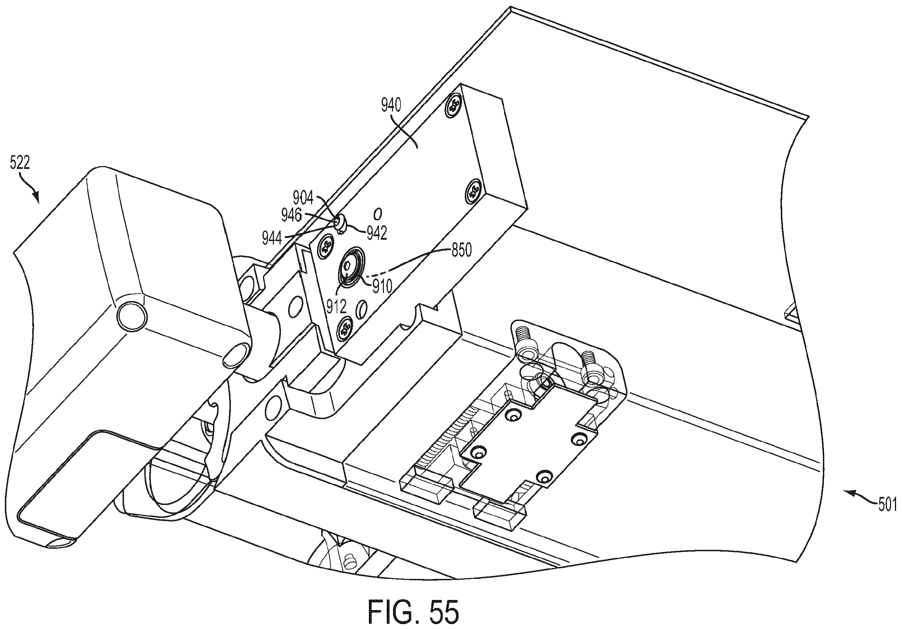

In **Fig. 55****,** another view of a portion of an embodiment of the syringe pump assembly **501** is shown. At the left side of **Fig. 55****,** part of the plunger head assembly **522** is also visible. The gearbox **940** is shown in place in the gearbox depression **902** on the rear face **900** of the syringe pump assembly **501.** As shown, the anti-rotation pin **904** may project through an anti-rotation pin hole **942** in the gearbox **940.** The anti-rotation pin **904** ensures that the gearbox **940** causes rotation of the lead screw **850** and that the gearbox **940** may not rotate around the axis of the lead screw **850.** As shown, the anti-rotation pin **942** does not help to hold the gearbox **940** against the rear face **900** of the syringe pump assembly **501.** In alternate embodiments, the anti-rotation pin **904** may have a threaded anti-rotation pin bore **944** (not shown) similar to that of the end of the lead screw **850** described in above in relation to **Fig. 54****.** An anti-rotation pin gearbox fastener **946** may be threaded into the thread anti-rotation pin bore **944** to help hold the gearbox **940** against the back face **900** of the syringe pump assembly **501.** The gearbox **940** may be friction locked onto the lead screw **850** to ensure that rotation of the gears in the gearbox **940** is transmitted to the lead screw **850** with zero or minimal backlash.

In embodiments where the syringe pump assembly **501** may be removed from the housing **502** (see **Fig. 28**) and replaced with another assembly such as a peristaltic large volume pump assembly, the gearbox **940** may be compatible with a replacement assembly.

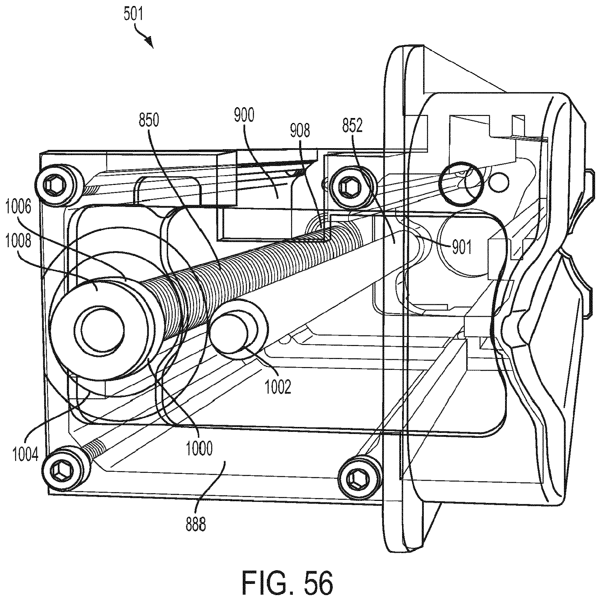

**Fig. 56** shows an embodiment of the interior of the syringe pump assembly **501.** As shown, the front face **888** of the syringe pump assembly **501** is shown as transparent. As shown, the guide rod **852** projects perpendicularly from the interior of the rear face **900** of the syringe pump assembly **501** and toward the front of the page. The lead screw **850** may similarly project into the interior of the syringe pump assembly **501** through the rear face lead screw bearing **908** at an angle perpendicular to the interior of the rear face **900** of the syringe pump assembly **501**. The guide rod **852** and lead screw **850** may run parallel to each other. In the example embodiment in **Fig. 56****,** the lead screw **850** is offset toward the left of the page from the guide rod **852.**

As shown, one end of the guide rod **852** is seated in the rear face guide rod hole **901.** The other end of the guide rod **852** is seated in the front face **888** of the syringe pump assembly **501.** In the example embodiment depicted in **Fig. 56****,** the end of the guide rod **852** facing the front of the page is smaller in diameter than the rest of the guide rod **852.** This section of the guide rod **852** may be placed in a guide rod hole **1002** in the front face **888** of the syringe pump assembly **501** when the syringe pump assembly **501** is fully assembled. The guide rod hole **1002** may extend through the entire front face **888** of the syringe pump assembly **501** at an angle substantially perpendicular to the front face **888.** The smaller diameter section of the guide rod 852 may have a diameter slightly though not substantially smaller than the diameter of the guide rod hole **1002** such that the guide rod **852** may fit snuggly in the guide rod hole **1002** when the syringe pump assembly **501** is assembled. The end of the guide rod **852** may be flush with the plane of the front face **888** of the syringe pump assembly **501.** Though both the guide rod hole **1002** and the section of the guide rod **852** seated in the guide rod hole **1002** are cylindrical in the example embodiment shown in **Fig. 56**, their shape may differ in alternate embodiments.

The lead screw **850** is seated in a lead screw depression **1000** in the front face **888** of the syringe pump assembly **501.** In the example embodiment shown in **Fig. 56**, the depth of the lead screw depression **1000** is substantially the thickness of the front face **888** of the syringe pump assembly **501.** In embodiments where the depth of the lead screw depression **1000** is substantially the depth of the front face **888,** a circular plateau **1004** may be raised off the front face **888** of the syringe pump assembly **501** to accommodate the depth of the lead screw depression **1000.** The center of the circular plateau **1004** may be concentric with the center of a cylindrical lead screw depression **1000** as shown in **Fig. 56****.** In some embodiments, the edges of the circular plateau **1004** may extend perpendicularly from the front face **888** of the syringe pump assembly **501** to the raised circular plateau. In the example embodiment illustrated in **Fig. 56**, the edges of the circular plateau **1004** curve up from the front face **888** of the syringe pump assembly **501** to the circular plateau **1004.**

As shown, the lead screw depression **1000** may house a front face lead screw bearing **1006** which surrounds the end of the lead screw **850** and provides a bearing surface for the lead screw **850.** In some embodiments, such as the embodiment depicted in **Fig. 56**, a Belleville washer **1008** may be seated against the bottom of the lead screw depression **1000.** The Belleville washer **1008** may ensure that there is no "play" of the lead screw **850** when the lead screw **850** is seated in the lead screw depression **1000.**

In some embodiments, the Belleville washer **1008** may be replaced by non-compliant end cap which loads the front face lead screw bearing **1006** against the lead screw **850.** In such embodiments, the end cap may be threaded on its out diameter. The lead screw depression **1000** may feature complimentary threads to which the end cap may screw into. Again the end cap may also ensure that there is no "play" of the lead screw **850** when the lead screw **850** is seated in the lead screw depression **1000.**

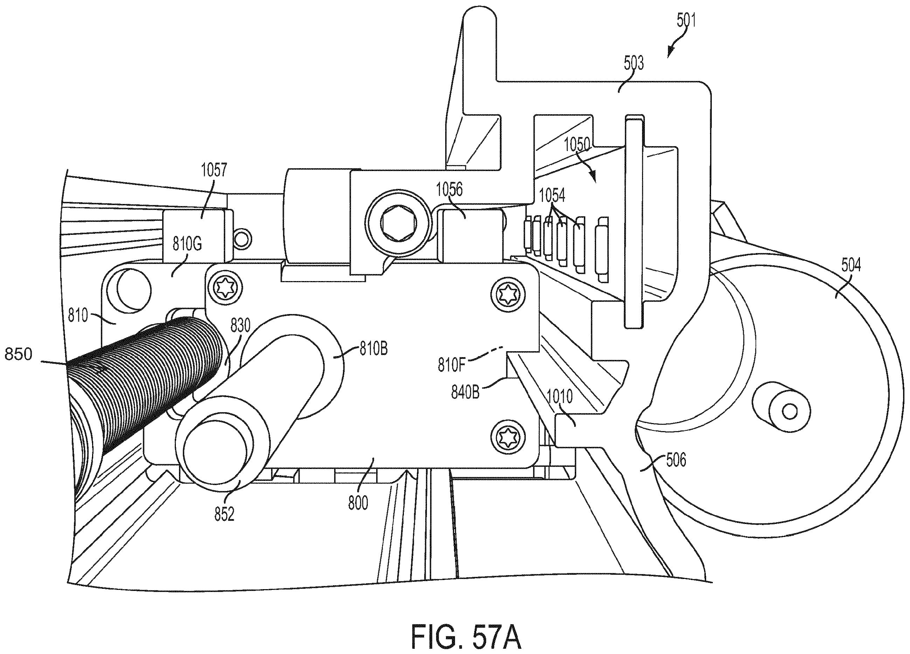

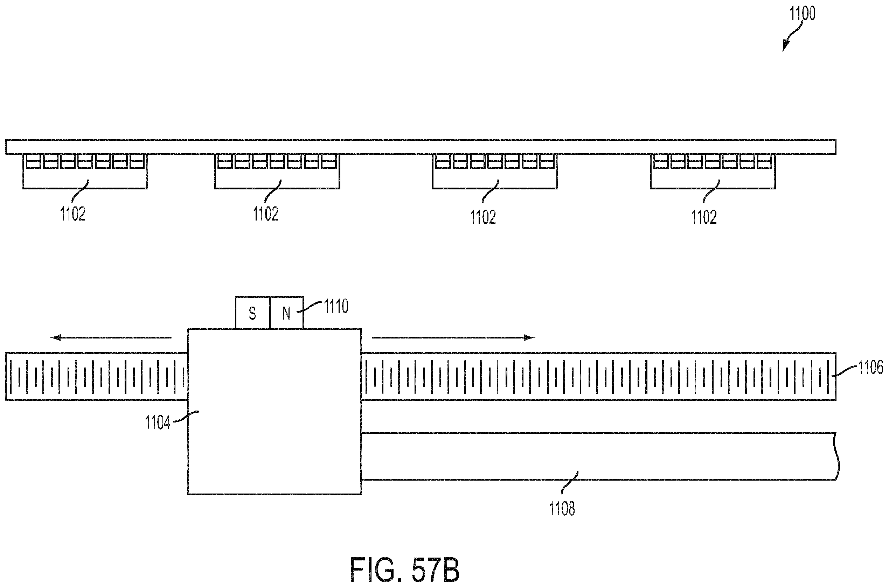

**Fig. 57** shows a view of the interior of the syringe pump assembly **501.** The front face **888** which is shown as transparent in **Fig. 56**, is not present in **Fig. 57A****.** As shown, the sliding block assembly **800** described above is in place within the syringe pump assembly **501.** The guide rod **852** extends through the guide rod bushing **810B** in the half nut housing **810.** The when the half nut **830** is disengaged from the lead screw **850**, the sliding block assembly **800** may be free to slide about the axial direction of the guide rod **852.**

Movement of the sliding block assembly **800** is also guided by a syringe pump assembly guide rail **1010.** In the example embodiment shown in **Fig. 57**, the syringe pump assembly guide rail **1010** extends from the interior face of the syringe seat **506.** The syringe pump assembly guide rail **1010** is shaped such that the half nut housing groove **810F** and cover plate groove **840B** on the sliding block assembly **800** may fit on the syringe pump assembly guide rail **1010** and slide along the syringe pump assembly guide rail **1010.** The syringe pump assembly guide rail **1010** also ensures that the sliding block assembly **800** may not rotate within the syringe pump assembly **501.** The syringe pump assembly guide rail **1010** may be formed as part of the extrusion in embodiments where the syringe pump assembly housing **503** is formed by extrusion.

As shown in **Fig. 57**, when half nut **830** of the sliding block assembly **800** is engaged with the lead screw **850,** the lead screw **850** may cause linear movement of the sliding block assembly **800** along the axial direction of the lead screw **850.** To cause linear movement of the sliding block assembly **800**, the lead screw **850** must be rotated. In the example embodiment in **Fig. 57**, the rotational motion of the lead screw **850** causes the half nut **830** and consequently the sliding block assembly **800** to move along the lead screw **850** due to the pitch of the threads of the lead screw **850.** The amount of linear movement per 360° rotation of the lead screw **850** may vary depending on the pitch of the threads of the lead screw **850** which may differ in various embodiments.

As mentioned above, the half nut housing **810** of the sliding block assembly **800** may comprise one or more limit switches **810G.** In the example embodiment in **Fig. 57**, a limit switch **810G** is not shown, although it is indicated that a limit switch **810G** may be located on the front of the half nut housing **810.** In other embodiments, there may be multiple limit switches **810G** which may be disposed about other portions of the sliding block assembly **800.** In embodiments where a limit switch may be disposed on the front of the half nut housing **810**, the limit switch **810G** may prevent the sliding block assembly **800** from being driven into the front face **888** (shown in **Fig. 56**) of the syringe pump assembly **501.**

In embodiments comprising a limit switch **810G**, the limit switch **810G** may be a micro switch, although hall sensors and magnets, optical sensors, etc. could also be used. In embodiments where the limit switch **810G** comprises a micro switch, the micro switch may be actuated when the sliding block assembly **800** nears a predefined location along the lead screw **850.** In some embodiments, when the limit switch **810G** is in the actuated position, the lead screw **850** may not be further rotated to advance the sliding block assembly **800** in the direction of the predefined location.

As shown in **Fig. 57**, the syringe pump assembly **501** may additionally comprise a sliding block linear position sensor **1050** to determine the sliding block assembly's **800** location on the lead screw **850.** In some embodiments, the sliding block linear position sensor **1050** may be used to determine the amount of contents left in a syringe **504** which may be in place on the syringe pump assembly **501.** In such embodiments, the sliding block linear position sensor **1050** may be used to determine a quantified volume of syringe **504** contents or may be used as a "gas gauge" which generates a more general syringe **504** contents volume reading.

In some embodiments, the sliding block linear position sensor **1050** may comprise a linear potentiometer. In such embodiments, the wiper of the sliding block linear position sensor **1050** may be disposed such that it slides across the resistive element of the potentiometer with movement of the sliding block assembly **800** along the lead screw **850.** The resistance measured by the sliding block linear position sensor **1050** may be used to determine the location of the sliding block assembly **800** along the lead screw **850.**

In some embodiments, including the example embodiment shown in **Fig. 57**, the sliding block linear position sensor **1050** may comprise an array of sliding block magnetic linear position sensors **1054.** The sliding block magnetic linear position sensors **1054** may be any suitable magnetic linear position sensor. An example of a suitable magnetic linear position sensor is the "AS5410 Absolute Linear 3D Hall Encoder" available from Austriamicrosystems of Austria. As shown, the sliding block assembly **800** may include a sliding block assembly magnet **1056** which is mounted a suitable distance away from the sliding block magnetic linear position sensors **1054** and may be used in conjunction with the array of sliding block magnetic linear position sensors **1054** in order to determine the location of the sliding block assembly **800** on the lead screw **850.** In some embodiments, the location of the sliding block magnetic linear position sensors **1054** may differ. As shown, the sliding block **800** includes a second magnet **1057** disposed such that it may interact with the sliding block magnetic linear position sensors **1054** when they are placed in an alternate location.

**Fig. 57B** shows an example of a possible linear position sensor **1100** arrangement to estimate the position of a sliding block assembly 800. In the example linear position sensor **1100** arrangement, the linear position sensor **1100** comprises an array of magnetic linear position sensors **1102** such as the "AS5410 Absolute Linear 3D Hall Encoder" available from Austriamicrosystems of Austria mentioned above. A position changing block **1104** (e.g., the sliding block assembly 800) is depicted at a position along a position changing block lead screw **1106.** A position changing block arm **1108** projects off the page as indicated by the broken line defining its rightmost edge. An object attached to the position changing block arm **1108** may be caused to move with the position changing block **1104** as the position changing block **1104** moves along the lead screw **1106.** The position changing block 1104 in Fig. 57B may be considered the sliding block assembly 800 in Fig. 57A.

In the example linear position sensor **1100** arrangement shown in **Fig. 57B****,** the position changing block **1104** comprises a position changing block magnet **1110.** As shown, the position changing block magnet is located on the face of the position changing block closest to the array of magnetic linear position sensors **1102.** The position changing block magnet **1110** is a dipole magnet. The north pole of the position changing block magnet **1110** is oriented to face toward the right of the page while the south pole faces the left of the page. As the position changing block **1104** moves along the position changing block lead screw **1106**, the position changing block magnet **1110** also moves. This movement may be measured by the array of magnetic linear position sensors **1102** and analyzed to determine an absolute location of the position changing block **1104** along the position changing block lead screw **1106.** In some embodiments, the array of magnetic linear position sensors **1102** may be used to determine differential movements of the position changing block **1104.**

As shown in **Fig. 58** an embodiment of the sliding block assembly **800** is shown assembled with the half nut cover plate **840** (see **Fig. 48**) removed. The half nut **830** is depicted in the engaged position and is shown as transparent so that the half nut housing **810** and the barrel cam **820** may be seen behind it. The driven shaft D-shaped segment **784** of the driven shaft **774** is shown in the D-shaped orifice **820A** of the barrel cam **820.** The driven shaft **774** extends through the plunger tube **524** which couples the sliding block assembly **800** and plunger head assembly **522** together.

Referring back to **Fig. 42**, the driven shaft **774** couples into a double universal joint **772.** The double universal joint **772** translates any rotational motion from the dial **530** which rotates the dial shaft **650** to rotational motion of the driven shaft **774.** Rotational motion of the driven shaft **774** in turn causes rotation of the barrel cam **820.** Rotation of the barrel cam **820** engages or disengages the half nut **830** as described above.

As also detailed above, rotation of the dial **530** causes linear displacement of the upper plunger clamp jaw **526** and lower plunger clamp jaw **528.** The dial **530** is thus multi-functional. When rotated, the dial **530** both engages or disengages the half nut **830** and opens or closes the upper plunger clamp jaw **526** and lower plunger clamp jaw **528.** It should be noted that the arcuate section **835A** of the half nut slot **835** is shaped such that the half nut **830** does not begin to disengage until the largest plunger flange **548** (not shown) which can be accepted by the upper plunger clamp jaw **526** and lower plunger clamp jaw **528** has been released by the upper plunger clamp jaw **526** and lower plunger clamp jaw **528.** When the plunger flange **548** (not shown) has been released and the half nut **830** has disengaged, the dial shaft cam follower **658** on the dial shaft **650** may sit in the dial shaft cam detents **660** of the dial shaft cam **654** as described in relation to **Fig. 43****.** As put forth in the detailed description of **Fig. 43****,** this would allow a user to "park" the dial **530** in the fully rotated position where the half nut **830** is disengaged and the upper plunger clamp jaw **526** and lower plunger clamp jaw **528** are in the open position. In the example embodiment depicted in **Fig. 58**, when the dial **530** is in the "parked" position, a user may remove their hand from the dial **530** and easily adjust the plunger head assembly **522** so that a syringe **504** (not shown) may be inserted onto the syringe pump assembly **501** (see **Figs. 30-****34** for example illustrations and discussion of syringe **504** placement onto the syringe pump assembly **501**).

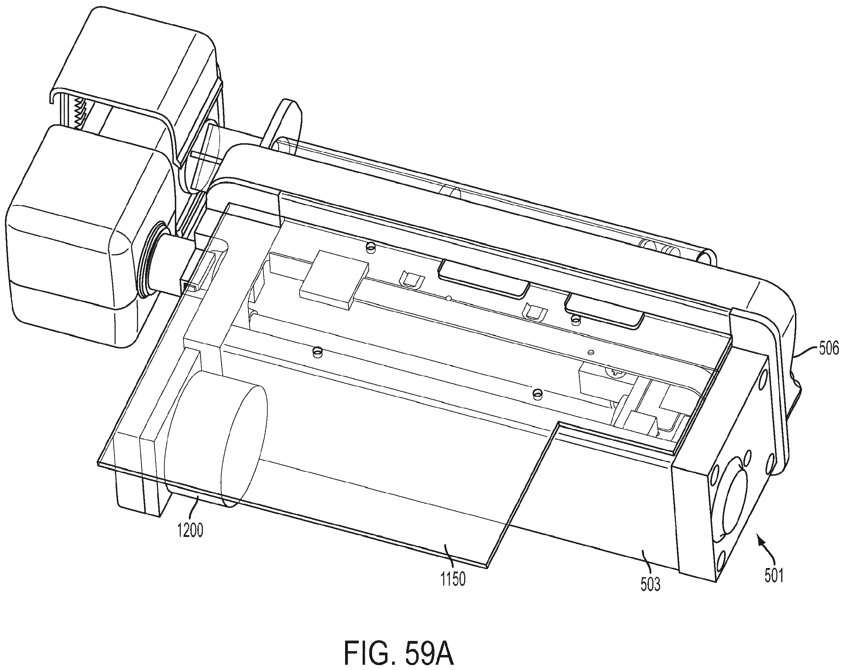

**Fig. 59A** shows an embodiment of the syringe pump assembly **501.** As shown, the syringe pump assembly **501** is fully assembled. A syringe **504** is seated on the syringe seat **506** of the syringe pump assembly housing **503.** The gearbox **940** is shown in place on the syringe pump assembly **501.** The motor **1200** which drives the gearbox **940** is also shown coupled to the gearbox **940.** A main printed circuit board (PCB) **1150** is shown transparently on the syringe pump assembly **501.** The main PCB **1150** is coupled to the top of the syringe pump assembly housing **503.** In the example embodiment, the flex connector **562** extending from the sliding block assembly **800** is connected to the main PCB **1150.** The electrical system comprising the main PCB will be described in **Figs. 59A-59J****.**

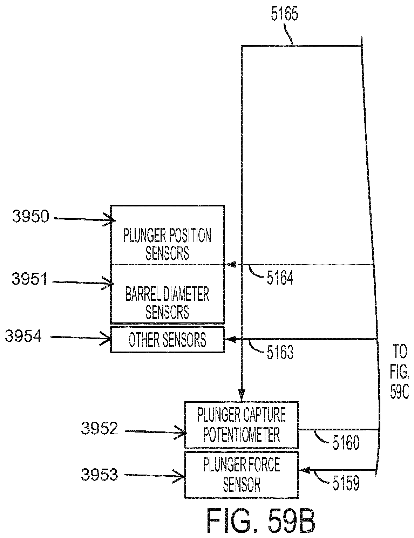

The electrical system **4000** of the syringe pump **500** (see **Fig. 28**) is described in a block schematic in **Figs. 59B-59J****.** The electrical system **4000** controls the operation of the syringe pump **500** based on inputs from the user interface **3700** and sensors **3501.** The electrical system **4000** includes a power system comprised of a rechargeable main battery **3420** and battery charger **3422** that plugs into the AC mains. The electrical system **4000** is architected to provide safe operation with redundant safety checks, and allow the syringe pump **500** to operate in fail operative modes for some errors and fail safe for the rest.

The high level architecture of multiple processors is shown in the last block diagram detailing the electrical system **4000**, **Fig. 59J****.** In one example the electrical system **4000** is comprised of two main processors, a real time processor **3500** and a User Interface/Safety Processor **3600.** The electrical system **4000** may also comprise a watchdog circuit **3460**, motor control elements **3431**, sensors **3501**, and input/output elements. One main processor referred to as the Real Time Processor (hereafter RTP) **3500** may control the speed and position of the motor **1200** that rotates the lead screw **850** (see **Fig. 48B**). The RTP **3500** may control the motor **1200** based on input from the sensors **3501** and commands from the User Interface & Safety Processor (hereafter UIP) **3600.** The UIP **3600** may manage telecommunications, manage the user interface **3701,** and provide safety checks on the RTP **3500.** The UIP **3600** may estimate the volume pumped based on the output of a motor encoder **1202** and may signal an alarm or alert when the estimated volume differs by more than a specified amount from a desired volume or the volume reported by the RTP **3500.** The watch dog circuit **3460** monitors the functioning of the RTP **3500.** If the RTP **3500** fails to clear the watch dog circuit **3460** on schedule, the watch dog **3460** may disable the motor controller **3431**, sound an alarm and turn on one or a number of failure lights at the user interface **3701.** The RTP **3500** uses the sensor inputs to control the motor **1200** position and speed in a closed-loop controller (further described below). The telecommunications may include a WIFI driver and antenna to communicate with a central computer or accessories, a Bluetooth driver and antenna to communicate with accessories, tablets, cell-phones etc. and a Near Field Communication (NFC) driver and antenna for RFID tasks and a Bluetooth. In **Fig. 59J** these components are collectively referred to with the reference number **3721.** The user interface **3701** may include a display **514** (see **Fig. 28****).** In some embodiments, the display **514** may be a touch screen. In some embodiments the user interface **3701** may comprise one or more buttons or data input means **516** (see **Fig. 28****)** via which a user may communicate with the syringe pump **500.**

The detailed electrical connections and components of the electrical system **4000** are shown in **Figs. 59B-****59I.** **Figs. 59B-59I** also depict a number of line traces **5000-5169** running to and from various components. A number of sensors of the syringe pump **500** are shown in **Fig. 59B****.** As shown, plunger position sensors **3950,** a barrel diameter sensor **3951,** a plunger capture potentiometer sensor **3952,** a plunger force sensor **3953,** and other sensors **3954** are shown. The plunger position sensors **3950** may be any of the plunger position sensors described herein. The barrel diameter sensor **3951** may be the syringe barrel holder linear position sensors **1540** to be described herein. The plunger capture potentiometer sensor **3952** may not necessarily be a potentiometer sensor in all embodiments. In some embodiments, the plunger capture potentiometer sensor **3952** may be the plunger clamp jaws position sensor **588** described herein. The plunger force sensor **3953** may be the plunger pressure sensor **532** described herein. The plunger capture potentiometer 3952 may be a switch to detect a syringe 504 loaded into the syringe seat **506.** The above sensors may communicate signals respective of and indicative of what they are sensing to the RTP **3500** or another component.

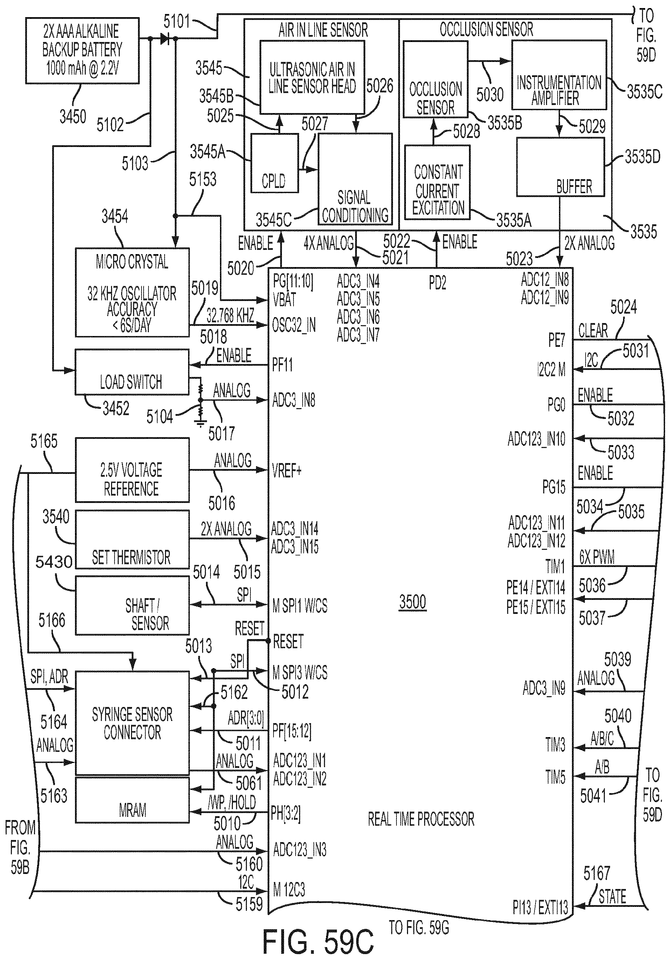

As shown in **Fig. 59C**, a thermistor **3540** may provide a signal to the RTP **3500** indicative of the temperature of the infusate in an infusion line. Alternatively the thermistor **3540** may measure a temperature in the syringe pump 500 or the temperature of the circuit **4000.** In different embodiments, suitable replacement components may be used in place of the specific parts listed in the **Figs. 59B-59I****.** In some embodiments, the electrical system **4000** may comprise additional components. In some embodiments the electrical system **4000** may comprises fewer components than the number of components shown in **Figs. 59B-59J****.**

Two sensors which may be located downstream of the syringe pump **500** are shown in **Fig. 59C****.** One sensor is an air-in-line sensor **3545.** The other is an occlusion sensor **3535.** Both are connected to the RTP **3500.** These sensors are optional. The air-in-line sensor **3545** may detect the presence of air in the section of an infusion line in near the air-in-line sensor **3545.** In an example embodiment, the air-in-line sensor **3545** may comprise an ultrasonic sensor **3545B**, a logic unit **3545A** and a signal conditioning unit **3545C.** In some embodiments, the syringe pump **500** may not comprise an air-in-line sensor **3545.**

The occlusion sensor **3535** may measure the internal pressure of an infusate in an infusion line. In some embodiments, the occlusion sensor **3535** may be the downstream pressure sensor **513** described herein. In an example embodiment, the occlusion sensor **3535** may comprise a force sensor **3535B**, an amplifier **3535A**, a signal amplifier **3535C** and a buffer **3535D.** The buffer **3535D** may protect the RTP **3500** from over-voltages due to high forces generated from pressures applied to the force sensor **3535B.** In alternative embodiments, the occlusion sensor **3535** may differ.

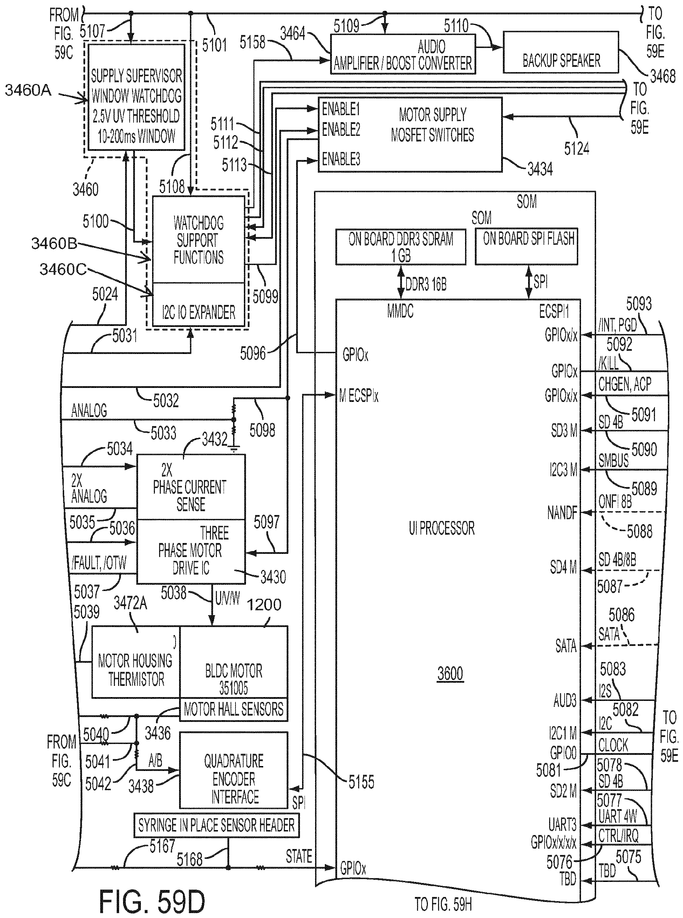

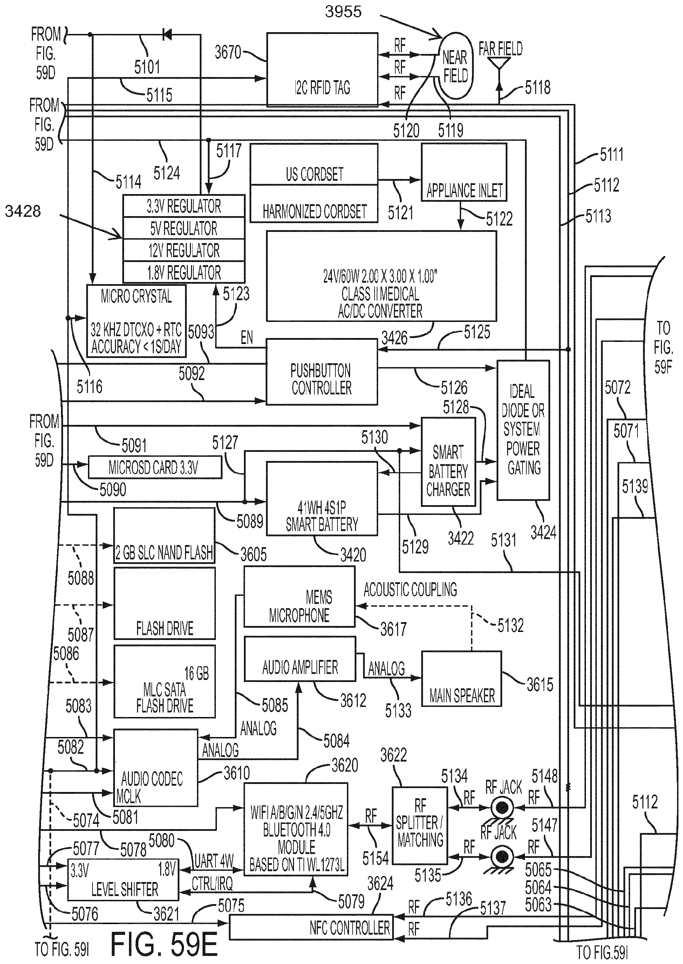

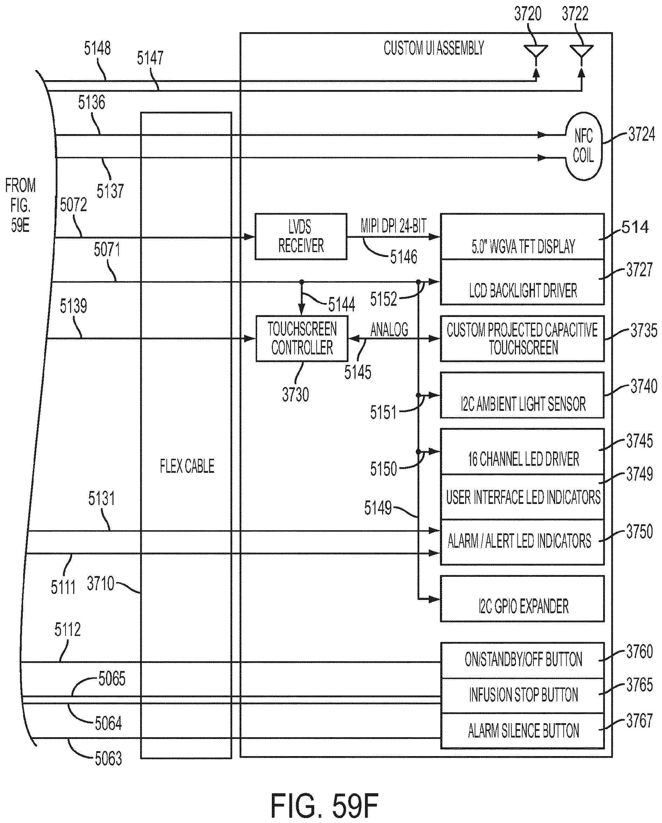

The watch dog circuit **3460** is shown in **Figs. 59D****.** The watch dog circuit **3460** may enabled by an I2C command from the RTP **3500.** The watch dog circuit **3460** may signal an error and disable the motor controller **3430** (e.g., via chip 3434) if it does not receive a signal from the RTP **3500** at a specified frequency. The watch dog circuit **3460** may signal the user via an audible alarm. The audible alarm may be issued via an amplifier **3464** and/or backup speaker **3468.** The watch dog circuit **3460** may signal the user with visual alarm LEDs **3750** (shown in Fig. **59F**) if an abnormal condition is detected. In one embodiment, the RTP **3500** must "clear" the watchdog **3460** between 10 ms and 200 ms after the watch dog circuit's **3460** last clear. In some embodiments, the watch dog circuit **3460** is comprised of a window watchdog **3460A**, a logic circuit **3460B** (which may include one or more flip-flop switches) and an IO expander **3460C** that communicates with the RTP **3500** over an I2C bus. A backup battery **3450** (see **Fig. 59C****)** may provide power to the watch dog circuit **3460** and backup speaker system (which may comprise an audio amplifier **3464**, and a backup speaker **3468**) in case the main battery **3420** (see **Fig. 59E**) fails. The backup battery **3450** may provide power to the RTP **3500** and UIP **3600** to maintain the internal timekeeping, which may be especially desirable when the main battery **3420** is changed. The RTP **3500** may also monitor the voltage of the backup battery **3450** with a switch such as the "FAIRCHILD FPF1005 LOAD SWITCH" **3452** shown in **Fig. 59C****.**

The RTP **3500** directly controls the speed and position of the motor **1200.** The motor **1200** may be any of a number of types of motors **1200** including a brushed DC motor, a stepper motor, or a brushless DC motor. In the embodiment illustrated in **Figs. 59B-59J**, the syringe pump **500** is driven by a brushless direct current (BLDC) servo motor **1200.** In one example embodiment, the RTP **3500** receives signals from the hall-sensors **3436** of a brushless DC motor **1200** and does the calculations to commutate power to the winding of the motor **1200** to achieve a desired speed or position. The commutation signals may be sent to the motor controller **3430** which selectively connects the windings to the motor power supply **3434.** The motor **1200** may be monitored for damaging or dangerous operation via current sensors **3432** and a temperature sensor **1200A.**

The signals from the hall sensors **3436** may be supplied to both the RTP **3500** and to an encoder **1202.** In one embodiment, three hall signals are generated. Any two of the three hall signals may be sent to the encoder **1202.** The encoder **1202** may use these signals to provide a position signal to the UIP **3600.** The UIP **3600** estimates the total volume of fluid dispensed by the syringe pump **500** from the position signal of the encoder **1202.** In some specific embodiments, each syringe pump **500** may be calibrated during assembly to establish the nominal volume / stroke that may be stored in memory. The UIP **3600** estimated volume may then be compared at regular intervals to the volume which would be expected for a commanded therapy. In some embodiments, the interval between comparisons may be shorter for different infusates, for example short half-life infusates. The therapy may specify, among other parameters, a flow rate, duration, and a total volume to be infused (VTBI). In any case, the expected volume based on the programmed therapy at a given time during that therapy may be calculated and compared to the volume estimated by the UIP **3600.** The UIP **3600** may signal an alert or alarm if the difference between UIP **3600** estimated volume and the expected volume for therapy is outside of a predefined threshold. The UIP **3600** may signal an alarm if the difference between UIP **3600** estimated volume and the expected volume for the therapy is outside another predefined threshold.

The UIP **3600** may also compare the estimated volume to the volume reported by the RTP **3500.** The UIP **3600** may signal an alert if the difference between UIP **3600** estimated volume and the RTP **3500** reported volume is outside a predefined threshold. The UIP **3600** may signal an alarm if the difference between UIP **3600** estimated volume and the RTP **3500** reported volume is outside a second threshold.

In some embodiments, the UIP **3600** may compare the RTP **3500** reported volume to the expected volume for the therapy and signal an alert if the two values differ by more than a predefined threshold. The UIP **3600** may signal an alarm if the difference between the RTP **3500** reported volume and the expected volume for the therapy differ by more than another predefined threshold. The values of the alert and alarm thresholds may be different for comparisons between different sets of volumes. The thresholds may be stored memory. The thresholds may vary depending on a number of different parameters, such as, but not limited to, medication, medication concentration, clinical usage, patient, therapy type, or location. The thresholds may be predefined in a DERS (Drug Error Reduction System) database and downloaded from the device gateway server.

Optionally, in some embodiments, a rotary encoder 5430 may be used to estimate the rotation of the motor threaded screw 1200. The motor sensor 5430 may be formed by a magnet on the motor's 1200 shaft with a Hall Effect sensor nearby to estimate the position of the threaded shaft.

An RFID tag **3670** (see **Fig. 59E****)** may be connected by an I2C bus to the UIP **3600** and to a near field antenna **3955.** The RFID tag **3670** may be used by med-techs or other users or personnel to acquire or store information when the syringe pump **500** is in an unpowered state. The UIP **3600** may store service logs, error codes, etc. in the RFID tag **3670.** The service logs, error codes, etc. may be accessible by an RFID reader. A med-tech, for example, could inspect unpowered syringe pumps **500** in storage or evaluate non-functioning syringe pumps **500** by using an RFID reader to interrogate the RFID tag **3670.** In another example, a med-tech or other personnel may perform service on the syringe pump **500** and store any related service information in the RFID tag **3670.** The UIP **3600** may then cull the latest service information from the RFID tag **3670** and store it in memory **3605** (see **Fig. 59E**).

The main battery **3420** may supply all the power to the syringe pump **500.** The main battery **3420** may be connected via a system power gating element **3424** to the motor power supply **3434.** All of the sensors and processors described herein may be powered by one of the several voltage regulators **3428** (see **Fig. 59E****).** The main battery **3420** may be charged from AC power via a battery charger **3422** and an AC/DC converter **3426.** The UIP **3600** be connected to one or more memory chips **3605.**

The UIP **3600** controls the main audio system which comprises a main speaker **3615** and the audio-chips **3610** (audio codec), **3612** (audio amplifier) (see **Fig. 59E****).** The main audio system may be capable of producing a range of sounds indicating, for example, alerts and alarms. The audio system may also provide confirmatory sounds to facilitate and improve user interaction with the display **514** and/or data input means **516** (see **Fig. 28****).** The main audio system may include a microphone **3617** which may be used to confirm the operation of the main speaker **3615** as well as the backup speaker **3468.** The main audio system may produce one or more tones, modulation sequences and/or patterns of sound and the audio codec chip **3610** may compare the signal received from the microphone **3617** to the signal sent to the main speaker **3615.** The use of one or more tones and comparison of signals may allow the system to confirm main speaker **3615** function independently of any ambient noise. Alternatively the UIP **3600** or the audio codec **3610** may confirm that the microphone **3617** produces a signal at the same time a signal is sent to the speaker amplifier **3612.**

The UIP **3600** may provide a range of different wireless signals for different uses. The UIP **3600** may communicate with the hospital wireless network via a dual band WiFi using chips **3621, 3620,** and **3622** and antennas **3720** and **3722.** The spatially diverse dual antenna may be desirable because in may be capable of overcoming dead spots within a room due to multiple paths and cancellation. A hospital device gateway may communicate DERS, CQI (Continuous Quality Improvement), prescriptions, patient data, etc. to the syringe pump 500 via the WiFi system.

The Bluetooth system using, the same chips **3621**, **3620** and **3622** (see **Fig. 59E**) and antennas **3720** and **3722** (see **Fig. 59F****),** may provide a convenient method to connect auxiliaries to the syringe pump 500 that may include pulse-oximeters, blood pressure readers, bar-code readers, tablets, phones, etc. The Bluetooth may include version 4.0 to allow low power auxiliaries which may communicate with the syringe pump 500 periodically such as, for example, a continuous glucose meter that sends an update once a minute.

The NFC system may be comprised of an NFC controller **3624** (see **Fig.59E****)** and an antenna **3724** (see **Fig. 59F****).** The NFC controller **3624** may also be referred to as an RFID reader. The NFC system may be used to read RFID chips identifying drugs or other inventory information. The RFID chips may also be used to identify patients and caregivers. The NFC controller **3624** may also interact with a similar RFID reader on, for example, a phone or tablet computer to input information including prescriptions, bar-code information, patient, care-giver identities, etc. The NFC controller **3624** may also provide information to phone or tablet computers such as the syringe pump **500** history or service conditions. The RFID antennas **3720** and **3722** and/or NFC antenna **3724** may preferably be located around or near the display **514** screen, so all interaction with the syringe pump **500** occurs on or near the display **514** whether reading an RFID chip or interacting with a touch screen display **514** or other data input means **516** near the display.

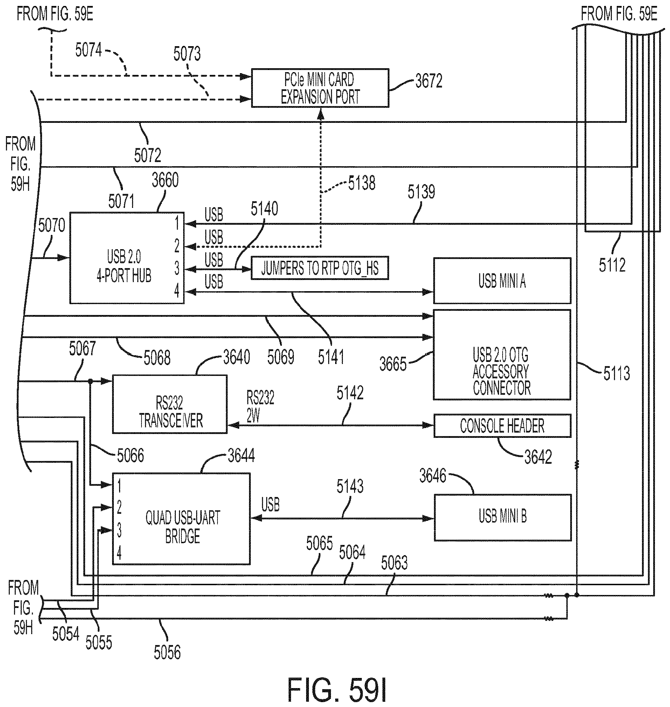

The UIP **3600** may include a medical grade connector **3665** (see **Fig. 59I**) so that other medical devices may plug into the syringe pump **500** and provide additional capabilities. The connector **3665** may implement a USB interface.

The display **514** may include the RFID antennas **3720**, **3722**, the NFC antenna **3724**, the display **514**, the touch screen **3735**, an LCD backlight driver **3727**, a light sensor **3740**, a 16 channel LED driver **3745**, LED indicator lights **3747** and **3749**, and three buttons **3760**, **3765**, **3767.** The buttons may collectively be referred to herein as data input means **516.** The display **514** may include a backlight **3727** and an ambient light sensor **3740** to allow the display **514** brightness to automatically respond and/or adjust to ambient light. The first button **3760** may be the "Power" button, while another button **3765** may be an infusion stop button. These buttons **3760**, **3765** may not provide direct control of the syringe pump **500**, but rather provide a signal to the UIP **3600** to either initiate or terminate infusion. The third button **3767** may silence an alarm or alert at the main speaker **3615** and at the backup speaker **3468.** Silencing the alarm or alert will not clear the fault, but may end the audible alarm or alert. The electrical system **4000** described above, or an alternative embodiment of the electrical system **4000** described above may be used with the syringe pump **500** described herein.

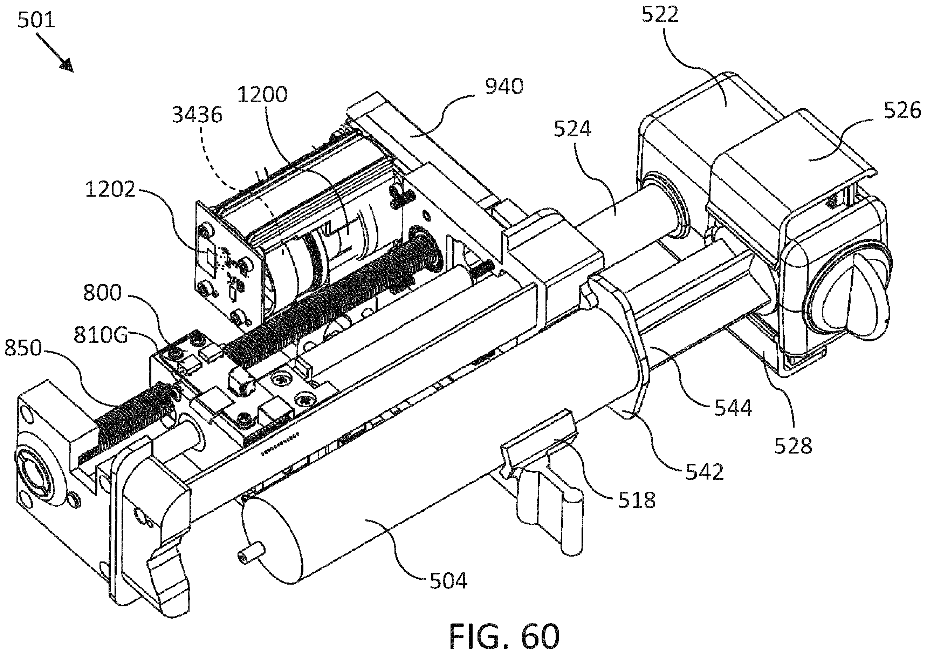

**Fig. 60** shows an exemplary embodiment of the syringe pump assembly **501.** In **Fig. 60** the syringe pump assembly housing **503** which is shown in **Fig. 59A** has been removed. As shown, a syringe **504** is in place on the syringe pump assembly **501** and is being held by the syringe barrel holder **518.** The sliding block assembly **800** is located approximately in the middle of the axial length of the lead screw **850.** Since the plunger tube **524** connects the sliding block assembly **800** to the plunger head assembly **522**, the plunger head assembly **522** is at location where it has caused the syringe plunger **544** to dispense about half of the content of the syringe **504.**

As shown, a motor **1200** is operatively coupled to the gearbox **940** in **Fig. 60****.** Rotation of the motor **1200** is transmitted through the gearbox **940** to drive the rotation of the lead screw **850.** As described above, since the upper plunger clamp jaw **526** and lower plunger clamp jaw **528** are closed on the plunger flange **548**, the half nut **830** is engaged with the lead screw **850.** Consequently, in the embodiment depicted in **Fig. 60** as the motor **1200** causes the lead screw **850** to rotate, the sliding block assembly **800** will travel along the axial length of the lead screw **850.** As motor **1200** rotates the lead screw **850** such that the sliding block assembly **800** moves toward the left of the page (relative to **Fig. 60**), the sliding block assembly's **800** movement will additionally cause the plunger tube **524** and plunger head assembly **522** to displace toward the left of the page. As the plunger head assembly **522** displaces toward the left of the page, the syringe plunger **544** is advanced into the syringe barrel **540** of the syringe **504** and the contents of the syringe are dispensed.

The motor **1200** may be any suitable motor **1200.** As shown in **Fig. 59A** a small profile pancake motor **1200** may be used to drive the rotation of the lead screw **850.** The embodiment shown in **Fig. 60** does not use a pancake motor **1200.** The motor **1200** shown in **Fig. 60** is an alternative motor that also has hall sensors **3436** to inform commutation of the motor **1200.** As shown in **Fig. 60**, the motor **1200** may comprise a magnet on the rotor that is detected by a rotary encoder **1202.** The rotary encoder **1202** may be any of a variety of suitable rotary encoders **1202** such as the AS5055 by Austrianmicrosystems of Austria. In some embodiments, the rotary encoder **1202** may be a magnetic. The rotary encoder **1202** may be used to monitor rotation of the lead screw **850.** Information from the rotary encoder **1202** may be used to determine when a given amount of the contents of the syringe **504** has been dispensed. Additionally, the rotary encoder **1202** may be used to determine the location of the sliding block assembly **800** on the lead screw **850.**

To ensure that the rotary encoder **1202** is functioning properly, a self test may be preformed. The motor **1200** may be powered to move the sliding block assembly **800** back and forth along a distance of the lead screw **850.** Measurements from the rotary encoder **1202** may be confirmed against the measurements of the sliding block assembly linear position sensor **1050.** The same self test may also be used to confirm the hall sensors **3436** of the brushless motor **1200** are functioning properly.

As previously indicated, the syringe pump **500** includes a number of sensor redundancies. This allows the syringe pump **500** to function in a fail operative mode if deemed appropriate. In the event that the rotary encoder **1202** fails, the hall sensors **3436** of the brushless motor **1200** may be used in a fail operative mode to measure the dispensation of syringe **504** contents via the rotation of the motor 1200 and provide a feed-back signal for the motor controller. Alternatively the location of the sliding block assembly **800** along the lead screw **850** may be used in a fail operative mode to measure the dispensation of syringe **504** contents via position of the sliding block assembly 800 and provide a feed-back signal for the controller. Alternatively the sliding block assembly linear position sensor **1050**, may be used to monitor the dispensation of syringe **504** contents via position of the sliding block assembly 800 on the lead screw and to provide a feed-back signal for the controller. In some embodiments, the motor hall sensors **3436** or the linear sliding block assembly linear position sensor **1050** may be used to monitor the position of the sliding block assembly **800** on the lead screw **850** to avoid driving the sliding block assembly **800** against the pump frame.

In the event of a failure of the rotary encoder **1202**, the syringe pump **500** may finish a therapy if a therapy is in progress and disallow a user from commencing another therapy until the syringe pump **500** has been serviced. In the event of a failure of the rotary encoder **1202** the syringe pump **500** may alarm. In some embodiments, if the rotary encoder **1202** fails and the motor **1200** is being used to deliver at a low flow rate, the syringe pump **500** may not finish the therapy. If such a failure occurs, the syringe pump **500** may alarm and the syringe pump **500** may finish a therapy if a therapy is in progress and disallow a user from commencing another therapy until the syringe pump **500** has been serviced. The controller of the syringe pump **500** may base its decision to continue a therapy based on the risk level of the infusate being delivered to a patient. If the risk of non-delivery to a user is higher than the risk of delivering with reduced accuracy, the syringe pump **500** will deliver in a fail operative mode.

**Fig. 61** shows a small volume syringe **504** in place on the syringe pump assembly **501.** Only a small portion of the syringe pump assembly **501** is visible in **Fig. 61****.** As shown, the syringe **504** is held in place against the syringe seat **506** by the syringe barrel clamp **518.** The syringe barrel flange **542** is clipped in place against the syringe pump assembly **501** by the barrel flange clip **520.** The barrel flange clip **520** is slightly offset from the rest of the syringe pump assembly **501** such that there is small gap between the syringe pump assembly **501** and the barrel flange clip **520.** When a user places the syringe **504** on the syringe seat **506**, the user may also place the syringe barrel flange **542** into the small gap between the syringe pump assembly **501** and the barrel flange clip **520.**

As shown in **Fig. 61**, the outward edge of the barrel flange clip **520** bows out toward the left of the page. This helps to guide the syringe barrel flange **542** into the gap between the barrel flange clip **520** and the syringe pump assembly **501.** The barrel flange clip **520** may also include one or a number of cutouts **521.** In the example embodiment in **Fig. 61**, the cutouts **521** of the barrel flange clip comprise two valleys. The first valley is recessed into the center span of the outward edge of the barrel flange clip **520.** The second valley, which is recessed into the lowest span of the first valley, is considerably smaller and shallower. In other embodiments, the cutouts **521** may be different in shape, size, etc. The plunger **544** of the small syringe **504** in **Fig. 61** is located entirely within the cutouts **521** in the barrel flange clip **520.** Without the cutouts **521** in the barrel flange clip **520**, the plunger **544** of the syringe **504** would contact the outward edge of the barrel flange clip **520** and obstruct user placement of the syringe barrel flange **542** into the gap between the barrel flange clip **520** and the syringe pump assembly **501.**

**Fig. 62** shows a large volume syringe **504** in place on the syringe pump assembly **501.** Only a small portion of the syringe pump assembly **501** is visible in **Fig. 62****.** As shown, the syringe **504** is held in place against the syringe seat **506** by the syringe barrel clamp **518.** The syringe barrel flange **542** is clipped in place against the syringe pump assembly **501** by the barrel flange clip **520.** The barrel flange clip **520** is slightly offset from the rest of the syringe pump assembly **501** such that there is small gap between the syringe pump assembly **501** and the barrel flange clip **520.** When a user places the syringe **504** on the syringe seat **506,** the user may also place the syringe barrel flange **542** into the small gap between the syringe pump assembly **501** and the barrel flange clip **520.**

As shown in **Fig. 62**, the barrel flange clip **520** may also include a roughly semi-circular depression **519** which thins the barrel flange clip **520.** The roughly semi-circular depression **519** may be included to accommodate the plunger flange **548** (not shown) of a syringe **504.** In embodiments where the barrel flange clip **520** includes the roughly semi-circular depression **519**, the plunger **544** may be advanced a distance equal to the depth of the semi-circular depression **519** further into the syringe barrel **540.** This is desirable because it allows more of the contents of the syringe **504** to be administered to a patient.

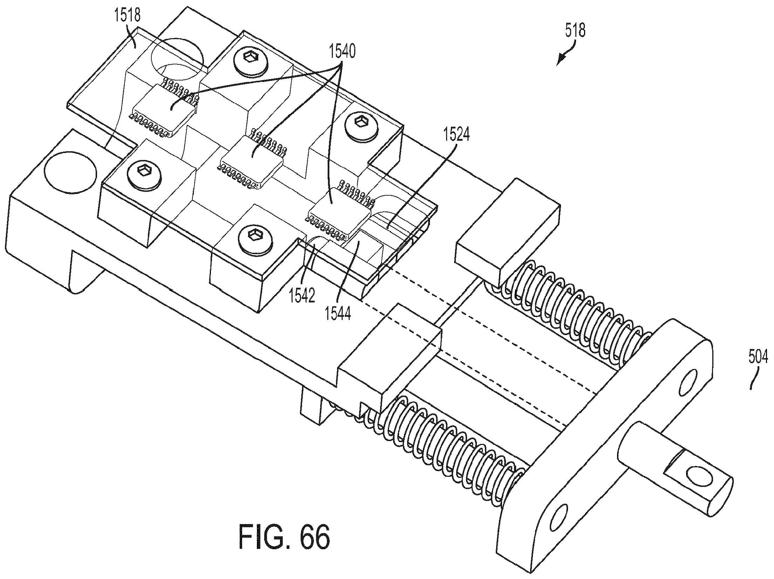

As shown in **Fig. 62**, the barrel flange clip **520** may include a barrel flange sensor **700.** The barrel flange sensor **700** may be comprised of any number of suitable sensors. In some embodiments, the barrel flange sensor **700** may function in a binary (yes/no) manner to indicate whether a syringe barrel flange **542** is clipped by the barrel flange clip **520.** In some embodiments, the barrel flange sensor **700** may comprise a micro switch which is actuated as the syringe barrel flange **524** is placed in the gap between the syringe pump assembly **501** and the barrel flange clip **520.** In other embodiments, the barrel flange sensor **700** may comprise a photosensor. Insertion of the syringe barrel flange **542** into the gap between the syringe pump assembly and the barrel flange clip **520** may block a light source for the barrel flange sensor **700** in embodiments where the barrel flange sensor **700** comprises a photosensor. In such embodiments, the barrel flange sensor **700** may indicate a syringe barrel flange **542** is clipped in place when the light source is blocked. In other embodiments, the barrel flange sensor **700** may be comprised of a different sensor than those described above. The barrel flange sensor **700** may be caused generate an alarm in the event that other sensors, such as the plunger clamp jaws position sensor **588** (mentioned above) or the syringe barrel holder linear position sensor **1540** (see **Fig. 66**), detect a syringe **504** in place of the syringe pump assembly **501** when the barrel flange sensor **700** does not detect a syringe **504** in place and an initiation of a therapy is attempted.

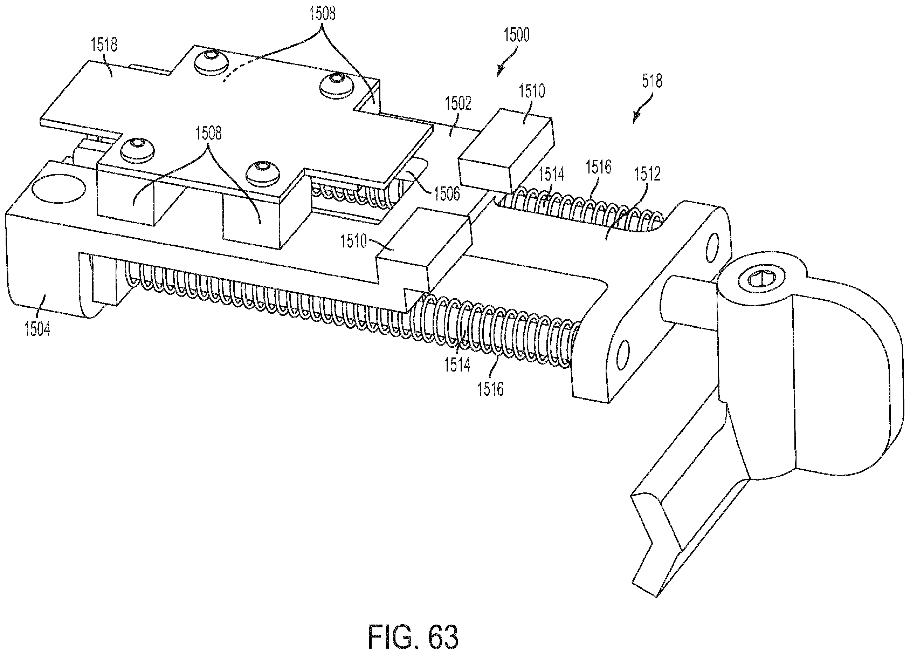

**Fig. 63** shows an embodiment of part of the syringe barrel holder **518.** As shown in **Fig. 63**, the syringe barrel holder **518** comprises a syringe barrel holder housing **1500.** In the example embodiment, the syringe barrel holder housing **1500** has a planate base plate **1502.** The planate base plate **1502** comprises a syringe barrel holder housing member **1504** at its left end (relative to **Fig. 63**). The syringe barrel holder housing member **1504** projects off the bottom of the syringe barrel holder housing **1500** at an angle substantially perpendicular to the plane of the planate base plate **1502.** The syringe barrel holder housing member **1504** may extend substantially perpendicularly from the entire length of the left end of the planate base plate **1502.** In some embodiments, the syringe barrel holder housing member **1504** may take the form of a rectangular prism. In the example embodiment shown in **Fig. 63**, the syringe barrel holder housing member **1504** has a form close to a rectangular prism, but the bottom edges of the syringe barrel holder housing member **1504** have been rounded off.

As shown in **Fig. 63**, the planate base plate **1502** may have a base plate slot **1506** cut into it. The base plate slot **1506** may be cut into the planate base plate **1502** from the left edge (relative to Fig. 63) of the planate base plate **1502.** The base plate slot **1506** may extend into the planate base plate **1502** at an angle substantially perpendicular to the left edge of the planate base plate **1502.** The base plate slot does not extend all the way across the planate base plate **1502** and stops short of the right edge.

On the flanks of the base plate slot **1506**, one or more syringe barrel holder housing posts **1508** may be disposed. In the example embodiment shown in **Fig. 63**, four syringe barrel holder housing posts **1508** flank the base plate slot **1506.** The four syringe barrel holder housing posts **1508** are divided up such that there are two syringe barrel holder housing posts **1508** on each flank of the base plate slot **1506.** The syringe barrel holder housing posts **1508** extend substantially perpendicularly from the top face of the planate base plate **1502** toward the top of the page. The syringe barrel holder housing posts **1508** in the example embodiment shown in **Fig. 63** have the form of rectangular prisms. In alternate embodiment, the syringe barrel housing posts **1508** may be cylindrical or have any other suitable shape.

The planate base plate **1502** may also comprise one or more syringe barrel holder housing bodies **1510.** In the example embodiment shown in **Fig. 63**, there are two syringe barrel holder housing bodies **1510.** The syringe barrel holder housing bodies **1510** projects perpendicularly from the top of the planate base plate **1502** toward the top of the page. The syringe barrel holder housing bodies **1510** have the form of rectangular prisms. As shown, the syringe barrel holder housing bodies **1510** may overhang the right edge of the planate base plate **1502.** The syringe barrel holder housing bodies **1510** may comprise one side which is flush with the front edge or back edge (relative to **Fig. 63**) of the planate base plate **1502.**

In some embodiments, the syringe barrel holder housing **1500** may comprise a "T" shaped member **1512.** In the example embodiment shown in **Fig. 63**, the stem portion of the "T" shaped member extends toward the right of the page from the right edge of the planate base plate **1502.** The "T" shaped member **1512** may extend on a plane substantially parallel to the plane of the planate base plate **1502.** In the example embodiment, the "T" shaped member **1512** projects from roughly the center of the right edge of the planate base plate **1502.** The cross portion of the "T" shaped member **1512** is roughly parallel with the right edge of the planate base plate **1502.** The cross portion of the "T" shaped member **1512** overhangs the stem equally on both sides of the stem.

As shown in **Fig. 63**, syringe barrel holder guide rails **1514** may extend substantially perpendicularly from the right face of the syringe barrel holder housing member **1504** and into the left faces of the overhanging cross portions of the "T" shaped member **1512.** The syringe barrel holder guide rails **1514** may extend substantially parallel to each other. In the example embodiment shown in **Fig. 63**, a coil spring **1516** surrounds each syringe barrel holder guide rail **1514.** One end of each coil spring **1516** may abut the left face of the cross portion of the "T" shaped member **1512.** In the example embodiment, the coil springs **1516** are compression springs. In alternate embodiments, other bias members or bias member arrangements may be utilized.

As shown in the embodiment in **Fig. 63**, a syringe barrel holder printed circuit board (PCB) **1518** may be held in place on the syringe barrel holder housing posts **1508.** The syringe barrel holder PCB may be coupled in place on the syringe barrel holder housing posts **1508** by any suitable means. In the example embodiment shown in **Fig. 63**, the syringe barrel holder PCB is coupled to the syringe barrel holder housing posts **1508** by screws.

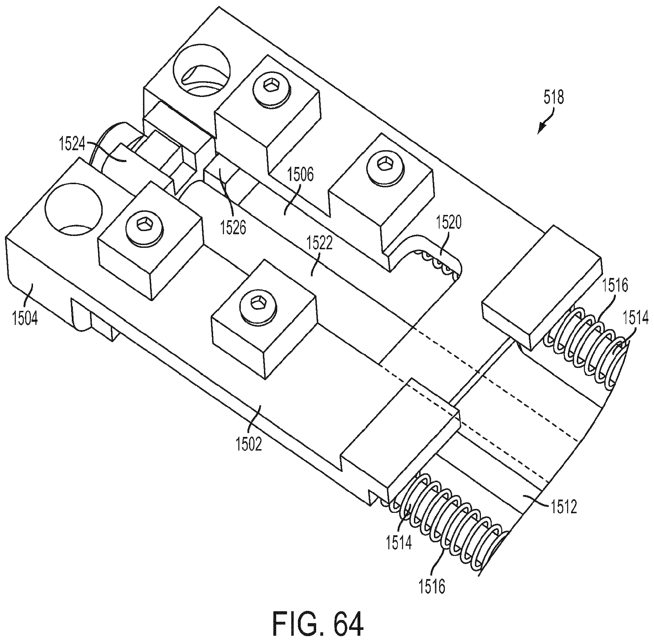

**Fig. 64** shows an embodiment of part of the syringe barrel holder **518.** In the embodiment shown in **Fig. 64**, the syringe barrel holder PCB **1518** shown in **Fig. 63** has been removed. As shown in **Fig. 64** the base plate slot **1506** may extend down into the syringe barrel holder housing member **1504.** The base plate slot **1508** may comprise a base plate notch catch **1520.** In embodiments where the base plate slot **1508** comprises a base plate notch catch **1520** the base plate notch catch **1520** may be a void in the planate base plate **1502** of the syringe barrel holder housing **1500.** In the example embodiment, the void of the base plate notch catch **1520** extends out from the right end section of the base plate slot **1508** at an angle substantially perpendicular to the side of the base plate slot **1508.**

The syringe barrel holder **518** may also comprise a syringe barrel holder arm rod **1522.** In the example embodiment shown in **Fig. 64**, the syringe barrel holder arm rod **1522** extends through an appropriately sized bore in the approximate center of the "T" shaped member **1512** (only the stem of the "T" shaped member **1512** is visible in **Fig. 64**). The syringe barrel holder arm rod **1522** may be movably coupled to the syringe barrel holder **518.** In embodiments where the syringe barrel holder arm rod **1522** is movably coupled to the syringe barrel holder **518**, the syringe barrel holder arm rod **1522** may move along a direction parallel to the edges of the stem of the "T" shaped member **1512.** In the example embodiment in **Fig. 64**, the syringe barrel holder arm rod **1522** is able to slide along the bore in the "T" shaped member **1512** and uses the bore in the "T" shaped member **1512** as a linear motion bearing. In the example embodiment, the syringe barrel holder arm rod **1522** is longer than the length of the stem of the "T" shaped member **1512.**

As shown in **Fig. 64**, one end of the syringe barrel holder arm rod **1522** may comprise a collar which may be a "U" shaped member **1524.** The "U" shaped member **1524** may be fixedly coupled to the syringe barrel holder arm rod **1522.** In the example embodiment, the bottom span of the "U" shaped member **1524** is thicker than the uprights of the "U" shaped member **1524.** The thick bottom span of the "U" shaped member **1524** comprises a hole which allows the "U" shaped member **1524** to be coupled onto the syringe barrel holder arm rod **1522** when the syringe barrel holder **518** is assembled. In the example embodiment, the uprights of the "U" shaped member **1524** extend up through the base plate slot **1506** and are substantially flush with the plane of the top face of the planate base plate **1502.** The uprights of the "U" shaped member **1524** may constrain the syringe barrel holder arm rod **1522** from rotation since any rotation is blocked by the uprights of the "U" shaped member **1524** abutting the edges of the base plate slot **1506.**

In the example embodiment shown in **Fig. 64**, the syringe barrel holder **518** comprises a bias bar **1526.** The bias bar **1526** in the example embodiment, is roughly rectangular in shape. The bias bar **1526** may comprise two holes which allow the bias bar **1526** to be placed on the syringe barrel holder guide rails **1514.** The bias bar **1526** may be capable of guided movement along the axial direction of the syringe barrel holder guide rails **1514.** In the example embodiment, the end of the coil springs **1516** on the syringe barrel holder guide rails **1514** not abutting the cross portion of the "T" shaped member **1512** abuts the front face of the bias bar **1526.** In the example embodiment shown in **Fig. 64** the maximum distance between the face of the bias bar **1526** which one end of the coil springs **1516** abut and the face of the "T" shaped member **1512** which the other end of the coil springs **1516** abut is shorter than the uncompressed length of the coil springs **1516.** This ensures that the bias bar **1526** will always be biased toward the position shown in **Fig. 64****.**

As shown in **Fig. 64**, the bias bar **1526** may comprise a cutout which allows the bias bar **1526** to fit around at least part of the syringe barrel holder arm rod **1522.** The "U" shaped member **1524** may abut the face of the bias bar **1526** opposite the side which the coil springs **1516** abut. In such embodiments, the action of the coil springs **1516** biasing the bias bar **1526** toward the position depicted in **Fig. 64**, additionally biases the syringe barrel holder arm rod **1522** to the position depicted in **Fig. 64****.**

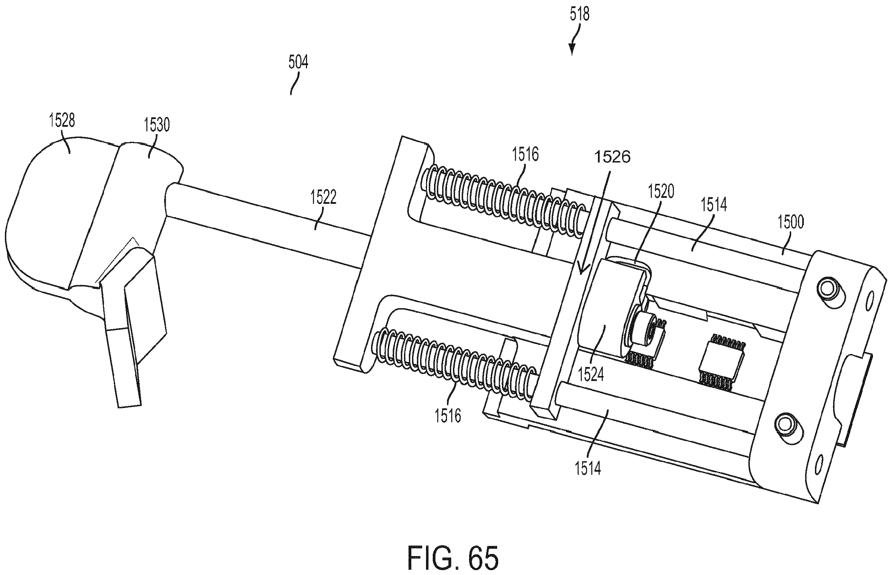

In the example embodiment in **Fig. 65**, the syringe barrel holder **518** is shown in the fully open position. To move the syringe barrel holder **518** to the open fully open position, a user may grasp the syringe barrel holder grip **1528.** In the example embodiment shown in **Fig. 65****,** the syringe barrel holder grip **1528** is a projection which extends from the barrel contacting structure **1530** of the syringe barrel holder **518** which is fixedly coupled to the syringe barrel holder arm rod **1522.** After grasping the syringe barrel holder grip **1528**, a user may pull the syringe barrel holder arm rod **1522** away from the syringe barrel holder housing **1500.** This action causes the "U" shaped member **1524** which is fixedly attached to the syringe barrel holder arm rod **1522** to move as well. Since the "U" shaped member **1524** may not pass through the bias bar **1526**, the bias bar **1526** moves with the "U" shaped member **1524** and syringe barrel holder arm rod **1522.** As the bias bar **1526** moves along the syringe barrel holder guide rails **1514**, the coil springs become compressed such that if a user releases the syringe barrel holder grip **1528**, the restoring force of the coil springs will automatically return the bias bar **1526**, "U" shaped member **1524**, and syringe barrel holder arm rod **1522** to the positions shown in **Fig. 64****.**

To hold the syringe barrel holder **518** in the fully open position against the bias of the coil springs **1516**, the syringe barrel holder **518** may be locked in the open position. As shown, the syringe barrel holder **518** may be locked in the open position by rotating the syringe barrel holder arm rod **1522** and all parts fixedly coupled to the syringe barrel holder arm rod **1522.** In **Fig. 65**, the syringe barrel holder arm rod **1522** has been rotated substantially 90° such that the bottom span of the "U" shaped member **1524** is disposed within the base plate notch catch **1520.** When the "U" shaped member is rotated into the base plate notch catch **1520**, the restoring force of the coil springs **1516** is not capable of returning the syringe barrel holder **518** to the position shown in **Fig. 64** because travel of the "U" shaped member **1524** is blocked by the base plate notch catch **1520.**

After rotating the syringe barrel holder arm rod **1522** such that the syringe barrel holder **518** is locked in the open position, a user may release the syringe barrel holder grip **1528** to grasp a syringe **504** (not shown) and put it in place. As mentioned above, the syringe barrel holder **518** will remain in the fully open position. A user may then rotate the syringe barrel holder arm rod **1522** 90° back to its original, unlocked position and allow the syringe barrel holder **518** to hold the syringe **504** in place.

Referring back to **Fig. 31** the syringe barrel holder **518** is shown fully open and rotated into the locked position. In the fully open position, the syringe barrel contacting structure **1530** and syringe barrel holder grip **1528** are at their furthest possible distance from the syringe seat **506** of the syringe pump assembly **501.** In some embodiments, this distance may be substantially larger than the diameter of the largest syringe **504** which may be accepted by the syringe pump **500.** In **Fig. 31**, a syringe **504** has been put in place against the syringe seat **506** while the syringe barrel holder **518** has be locked in the open position. In **Fig. 32**, the syringe barrel holder has been rotated out of the locked position and has been allowed to automatically adjust to the size of the syringe barrel **540.** As mentioned in the discussion of **Fig. 65**, this automatic adjustment is a result of the restoring force of the coil springs **1516** automatically pushing the bias bar **1526**, "U" shaped member **1524**, and the syringe barrel holder arm rod **1522** toward the position depicted in **Fig. 64****.**

In **Fig. 66**, an example embodiment of the syringe barrel holder **518** is shown. In the embodiment depicted in **Fig. 66** the syringe barrel holder PCB **1518** is shown as transparent. The syringe barrel holder PCB **1518** may comprise one or a number of syringe barrel holder linear position sensors **1540.** In the example embodiment, there are three syringe barrel holder linear position sensors **1540.** The syringe barrel holder linear position sensors **1518** may be used to determine the size of the syringe **504** (not shown) which the syringe barrel holder **518** is holding in place.

In some embodiments, there may only be a single syringe barrel holder linear position sensor **1540.** In such embodiments, the syringe barrel holder linear position sensor **1540** may be a linear potentiometer. In embodiments where the syringe barrel holder linear position sensor **1540** is a linear potentiometer, the syringe barrel holder linear position sensor **1540** may comprise a barrel sizing wiper **1542** which may slide across the resistive element of the potentiometer with movement of the syringe barrel holder arm rod **1522.** When a syringe **504** (not shown) is held by the syringe barrel holder **518**, the size of the syringe **504** (not shown) will determine the position of the barrel sizing wiper **1542** along the linear potentiometer type syringe barrel holder linear position sensor **1540.** Since the location of the wiper **1542** will vary the resistance measured by the linear position sensor **1540**, the resistance measured may be used to establish information (size, volume, brand, etc.) about the syringe **504** (not shown) being used. In some embodiments, the resistance measurement may be referenced with a database or resistance measurements which would be expected from different syringes **504** to determine information about the syringe **504.** The resistance measurement may additionally be used to determine whether a syringe **504** is properly held by the syringe barrel holder **518.** For example, if the resistance measurement indicates that the syringe barrel holder **518** is in the fully open position (as it is in **Fig. 66**), an alarm may be generated and a therapy may not be initiated.

In some embodiments, including the example embodiment shown in **Fig. 66**, the syringe barrel holder linear position sensors **1540** may be magnetic linear position sensors. Any suitable magnetic linear position sensor may be used for the syringe barrel holder linear position sensor **1540.** The syringe barrel holder linear position sensors **1540** may be the same type of sensors as the sliding block assembly linear position sensors **1050.** An example of a suitable magnetic linear position sensor is the "AS5410 Absolute Linear 3D Hall Encoder" available from Austriamicrosystems of Austria. The syringe barrel holder linear position sensors **1540** gather their positional data from a syringe barrel holder magnet **1544** placed at a suitable distance from the syringe barrel holder linear position sensors **1540.** In the example embodiment shown in **Fig. 66**, the syringe barrel holder magnet **1544** rests on the bottom span of the "U" shaped member **1524** between the two uprights of the "U" shaped member **1524.** The absolute location of the syringe barrel holder magnet may be measured by the syringe barrel holder linear position sensors **1540.** Since the measured absolute location of the syringe barrel holder magnet **1544** may vary depending on the syringe **504** (not shown) being held by the syringe barrel holder **518**, the absolute location of the syringe barrel holder magnet **1544** can be used to determine specific information (for example, size, volume, brand, etc.) about the syringe **504** (not shown) being held. In some embodiments, the absolute location of the syringe barrel holder magnet **1544** may be referenced with a database to determine information about the syringe **504** being utilized. In such embodiments, the database may be a database of absolute locations which would be expected with different syringes **504.** The absolute position measurement may also be used to determine whether a syringe **504** is correctly held in place by the syringe barrel holder **518.** For example, if the absolute position measurement indicates that the syringe barrel holder **518** is in the fully open position (as it is in **Fig. 66**), an alarm may be generated and a therapy may not be initiated.

In some embodiments, the data gathered by the syringe barrel holder linear position sensor **1540** may be compared to data gathered by other sensors to make a more informed decision on the specific syringe **504** being used. For example, in embodiments where a plunger clamp jaws position sensor **588** may make a determination on the type of syringe **504** being used (see discussion of **Fig. 37**) the data from the plunger clamp jaws position sensor **588** and linear position sensor **1540** may be compared. If the data gathered by the syringe barrel holder linear position sensor **1540** does not correlate with data gathered by other sensors, an alarm may be generated.

In some embodiments, data from the plunger clamp jaws position sensor **588** may be first referenced against a syringe **504** database to narrow down acceptable syringe barrel **540** measurements. In some embodiments, data from the syringe barrel holder linear position sensor may be referenced against a syringe **504** database to set a range of acceptable plunger flange **548** measurements.

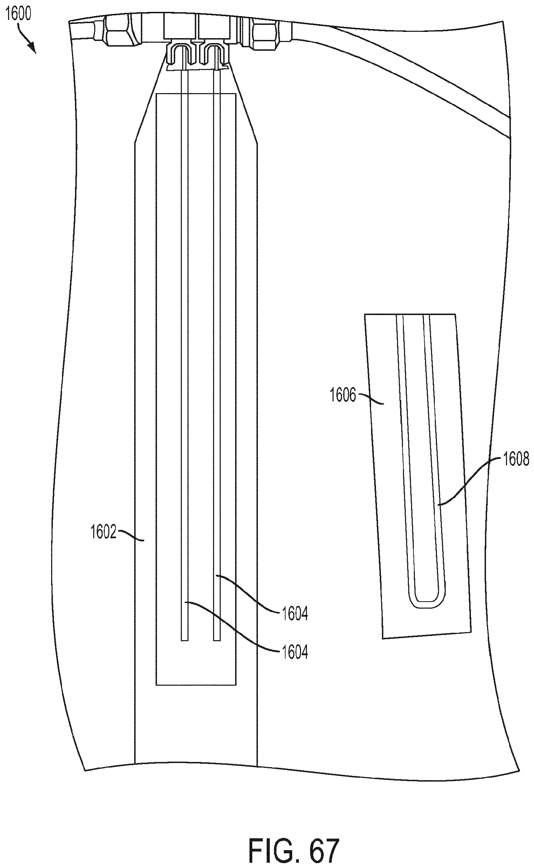

**Fig. 67** shows a basic example of part of an alternative linear position sensor. The part of the alternative linear position sensor in **Fig. 67** is a line stretcher **1600.** In the example embodiment, the line stretcher **1600** comprises a stationary portion and a moving portion. The stationary portion comprises an FR-4 PCB substrate **1602.** On the substrate **1602** there are two microstrips **1604.** As shown, the microstrips **1604** extend parallel to each other. The microstrips **1604** act as transmission lines for a signal at a known frequency. The microstrips **1604** do not allow the signal to propagate into the ambient environment. The width of the microstrips **1604** is chosen so that it is suitable for the desired impedance. In an example embodiment, the desired impedance is 50 Ω.

The moving portion in the example embodiment comprises a moving portion FR-4 PCB substrate **1606.** As shown, the moving portion FR-4 PCB substrate comprises a moving portion microstrip **1608.** The moving portion microstrip **1608** may be substantially "U" shaped. The uprights of the "U" shaped moving portion microstrip **1608** extend parallel to each other and are spaced such that when the line stretcher **1600** is assembled they may contact the two microstips **1604** on the stationary portion. The moveable portion microstrips **1608** have a width chosen so that it is suitable for desired amount of impedance (50 Ω in the example embodiment). The bottom span of the "U" shaped movable portion microstrip **1608** connects the two uprights of the "U" shaped movable portion microstrip **1608** and is substantially perpendicular to the two uprights. When fully assembled, the bottom span of the "U" shaped movable portion microstrip **1604** forms a bridge between the two microstrips **1604** on the stationary portion of the line stretcher **1600.** Any signal sent through one of the microstrips **1604** on the stationary portion may cross via the moving portion microstrip **1608** to the other microstrip **1604** on the stationary portion. By sliding the moving portion along the direction of extension of the stationary portion microstrips **1604** the signal must travel a greater or shorter distance before crossing from one stationary portion microstrip **1604** to the other. By manipulating the amount of travel of the signal, a user may predictably create a phase change of the signal. To reduce wear on the metal microstrips **1604** and **1608** a thin sheet of insulation **1609** may be placed between the microstrips **1604** and **1608**, creating a capacitive coupling.

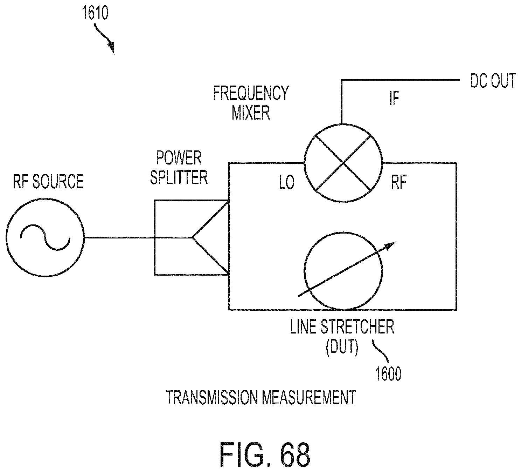

**Fig. 68** shows an example of the line stretcher **1600** being incorporated into a phase change detector **1610.** As shown, the phase change detector **1610** comprises a signal source shown as "RF SOURCE" in the example shown in **Fig. 68****.** The source signal in the example shown in **Fig. 68** travels from the "RF SOURCE" to a "POWER SPLITTER". The "POWER SPLITTER" splits the signal, keeping the two output signals in a constant phase relationship with one another. One of the signals travels directly to a "FREQUENCY MIXER". The other signal is delayed before it is allowed to reach the "FREQUENCY MIXER". In **Fig. 68**, the signal is delayed by the line stretcher **1600** (see **Fig. 67**). Delaying the signal causes the delayed signal to be predictably out of phase with the non-delayed signal which travels directly to the "FREQUENCY MIXER". The delayed signal travels from line stretcher **1600** to the "FREQUENCY MIXER". In the example embodiment shown in **Fig. 68** the "FREQUENCY MIXER" is a double balanced frequency mixer. As is well known in the art, two identical frequency, constant-amplitude signals sent to a mixer will result in a DC output which is proportional to the phase difference between the two signals.

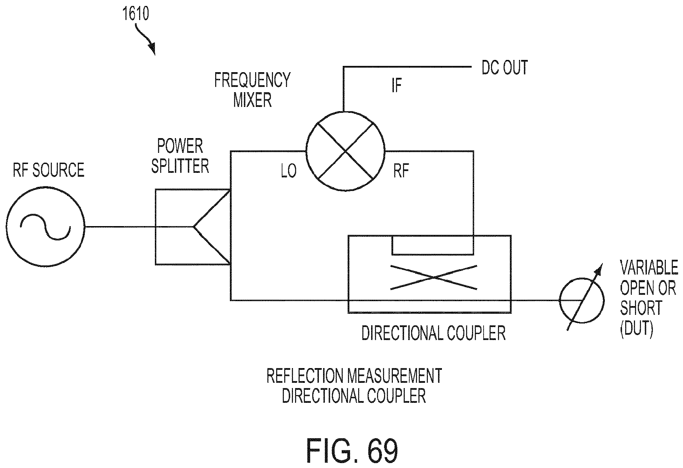

**Fig. 69** depicts a slightly different embodiment of the phase change detector **1610.** In **Fig. 69** the delay means is not a line stretcher **1600** such as the one described in **Fig. 67****.** The delay means is a variable open or short. As the object whose linear position is to be measured linearly displaces, the short or open's location on a transmission line may be caused to move proportionally. As shown, the signal travels through a "DIRECTIONAL COUPLER" which may be any suitable directional coupler. As one of the two signals the signal enters the "DIRECTIONAL COUPLER" from the "POWER SPLITTER" the signal is sent out of another port of the "DIRECTIONAL COUPLER to an open or short. The open or short causes the signal to reflect back to the port from which it traveled to reach the open or short. The signal reflected back into the port is then directed by the "DIRECTIONAL COUPLER" to travel into the "FREQUENCY MIXER". The delay of the signal caused by the distance traveled to and from the point of reflection causes a phase shift in the signal. The amount of phase shift of the signal is dependent on the distance from the port from which the signal exits the "DIRECTIONAL COUPLER" to the open or short. This distance may be caused to change in consequence to movement of the object whose linear position is to be measured. The second signal output of the "POWER SPLITTER" travels directly to the "FREQUENCY MIXER". As is well known in the art, two identical frequency, constant-amplitude signals sent to a mixer will result in a DC output which is proportional to the phase difference between the two signals.

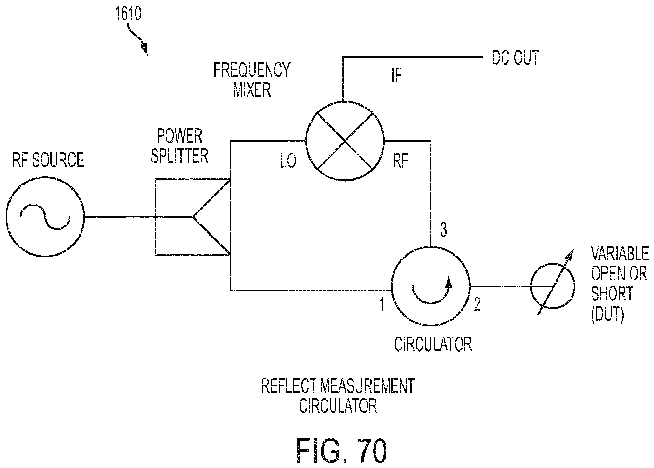

As shown in **Fig. 70**, the "DIRECTIONAL COUPLER" may be replaced with another piece of equipment such as a circulator. The phase change detector **1610** in **Fig. 70** functions very similarly to the phase change detector **1610** in **Fig. 69****.** One signal from the power splitter travels directly to the "FREQUENCY MIXER". The other signal is delayed. The delay is caused in the same manner as described above. Instead of using a "DIRECTIONAL COUPLER", however, a "CIRCULATOR" may be used to direct the signal. As the signal enters the "CIRCULATOR" at port 1 the signal is circulated to port 2. The signal travels from port 2 to the short or open and is reflected back into port 2. The reflected, phase shifted signal entering port 2 of the "CIRCULATOR" is circulated to port 3. The signal exits port 3 and travels to the "FREQUENCY MIXER" As is well known in the art, two identical frequency, constant-amplitude signals sent to a mixer will result in a DC output which is proportional to the phase difference between the two signals. Since the phase difference is dependent on the distance of the short or open from port 2 of the "CIRCULATOR" and the distances varies in proportion to the location of the object whose linear location is to be found the DC output of the mixer may be used to determine the objects location.

In some embodiments, the phase change detector **1610** may be used to substitute for the syringe barrel holder linear position sensors **1540** (see **Fig. 66**) or the sliding block magnetic linear position sensors **1054** (see **Fig. 57A**). In some embodiments, only one of the syringe barrel holder linear position sensors **1540** or the sliding block magnetic linear position sensors **1054** may be substituted for with the phase change detector **1610.** In some embodiments, a phase change detector **1610** may be used in conjunction with one or both the syringe barrel holder linear position sensors **1540** or the sliding block magnetic linear position sensors **1054** and function as a cross check or backup.

In embodiments where the sliding block assembly linear position sensor **1054** (see **Fig. 57A**) is substituted for with a phase change detector **1610**, the phase change detector **1610** may be used to detect the position of the sliding block assembly **800** along the lead screw **850** (see **Fig. 57A**)**.** If the phase shift detector **1610** uses a line stretcher **1600** (see **Fig. 67**) the moveable portion of the line stretcher **1600** may be caused to move along the stationary portion of the line stretcher **1600** with movement of the sliding block assembly **800** along the lead screw **850.** In turn this would cause the degree of phase change to reflect the position of the sliding block assembly **800** on the lead screw **850.** Consequently, the DC output voltage of the mixer (see **Fig. 68**) may be used to determine the position of the sliding block assembly **800.** The positional data generated by the phase change detector **1610** may be used in the same manner as described above in relation to the prior discussion of sliding block assembly **800** linear position sensing.

In embodiments where the phase change detector **1610** uses a variable short or open (see **Fig. 69** and **Fig. 70****),** movement of the sliding block assembly **800** along the lead screw **850** may cause the short or open to change its location along the transmission line. In turn this would cause the degree of phase change to specify the position of the sliding block assembly **800** along the lead screw **850.** Consequently, the DC output voltage of the mixer (see **Fig. 69** and **Fig. 70**) may be used to determine the position of the sliding block assembly **800.**

In embodiments where the syringe barrel holder linear position sensors **1540** (see **Fig. 66**) is substituted for by the phase change detector **1610**, the phase change detector **1610** may be used to may be used to determine the size of the syringe **504** (see **Fig. 28**)**.** If the phase change detector **1610** uses a line stretcher **1600** (see **Fig. 67**) the moveable portion of the line stretcher **1600** may be caused to move along the stationary portion of the line stretcher **1600** with movement of the syringe barrel holder arm rod **1522.** In turn this would cause the degree of phase change to reflect the position of the syringe barrel holder arm rod **1522.** Since the position of the syringe barrel holder arm rod **1522** is dependent upon various characteristics of the syringe **504**, the DC output voltage of the mixer (see **Fig. 68**) may be used to determine the position of the of the syringe barrel holder arm rod **1522** and therefore a number of characteristics of the syringe **504.**

In embodiments where the phase change detector **1610** uses a variable short or open (see **Fig. 69** and **Fig. 70****),** movement of the syringe barrel holder arm rod **1522** may cause the short or open to change its location along a transmission line. In turn this would cause the degree of phase change to specify the position of the syringe barrel holder arm rod **1522.** Since the position of the syringe barrel holder arm rod **1522** is dependent upon various characteristics of the syringe **504**, the DC output voltage of the mixer (see **Fig. 69** and **Fig. 70**) may be used to determine the position of the syringe barrel holder arm rod **1522** and therefore a number of characteristics of the syringe **504.** The positional data generated by the phase change detector **1610** may be used in the same manner as described above in relation to the prior discussion of syringe barrel holder linear position sensing.

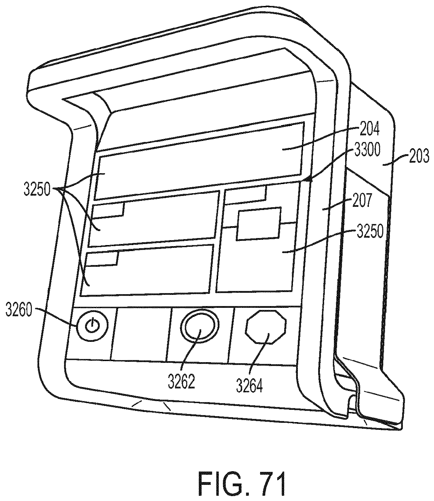

An example embodiment of the graphic user interface (hereafter GUI) **3300** is shown in **Fig. 71****.** The GUI **3300** enables a user to modify the way that an agent may be infused by the syringe pump **500** by customizing various programming options. Though the following discussion mostly details the use of the GUI **3300** with the syringe pump **500**, it should be appreciated that the GUI **3300** may be used with other pumps, including the other pumps mentioned in this specification. For example, the GUI **3300** may be used with the pump **201, 202,** or **203** (as shown in **Fig. 71**) detailed in the discussion of **Figs. 2-9****.** For purposes of example, the GUI **3300** detailed as follows uses a screen **3204** which is a touch screen display **514** (see **Fig. 28****)** as a means of interaction with a user. In other embodiments, the means of interaction with a user may be different. For instance, alternate embodiments may comprise user depressible buttons or rotatable dials, audible commands, etc. In other embodiments, the screen **3204** may be any electronic visual display such as a, liquid crystal display, L.E.D. display, plasma display, etc.

As detailed in the preceding paragraph, the GUI **3300** is displayed on the display **514** of the syringe pump 500. Each syringe pump **500** may have its own individual screen **3204.** In arrangements where there are multiple syringe pumps **500** or a syringe pump **500** and one or more other pumps, the GUI **3300** may be used to control multiple pumps. Only the master pump may require a screen **3204.** As shown in **Fig. 71**, the pump **203** is seated in a Z-frame **3207.** As shown, the GUI **3300** may display a number of interface fields **3250.** The interface fields **3250** may display various information about the pump **203**, infusion status, and/or the medication, etc. In some embodiments, the interface fields **3250** on the GUI **3300** may be touched, tapped, etc. to navigate to different menus, expand an interface field **3250,** input data, and the like. The interface fields **3250** displayed on the GUI **3300** may change from menu to menu.

The GUI **3300** may also have a number of virtual buttons. In the non-limiting example embodiment in **Fig. 71** the display has a virtual power button **3260**, a virtual start button **3262**, and a virtual stop button **3264.** The virtual power button **3260** may turn the syringe pump **500** on or off. The virtual start button **3262** may start an infusion. The virtual stop button **3264** may pause or stop an infusion. The virtual buttons may be activated by a user's touch, tap, double tap, or the like. Different menus of the GUI **3300** may comprise other virtual buttons. The virtual buttons may be skeuomorphic to make their functions more immediately understandable or recognizable. For example, the virtual stop button **3264** may resemble a stop sign as shown in **Fig. 71****.** In alternate embodiments, the names, shapes, functions, number, etc. of the virtual buttons may differ.

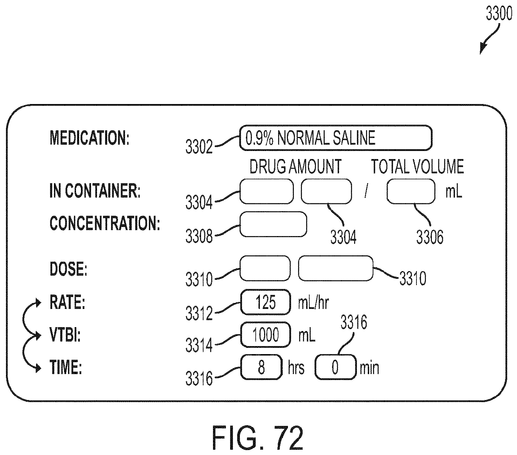

As shown in the example embodiment in **Fig. 72****,** the interface fields **3250** of the GUI **3300** (see **Fig. 71**) may display a number of different programming parameter input fields. For the GUI **3300** to display the parameter input fields, a user may be required to navigate through one or a number of menus. Additionally, it may be necessary for the user to enter a password before the user may manipulate any of the parameter input fields.

In **Fig. 72**, a medication parameter input field **3302**, in container drug amount parameter input field **3304**, total volume in container parameter input field **3306**, concentration parameter input field **3308**, dose parameter input field 3310, volume flow rate (hereafter abbreviated as rate) parameter input field 3312, volume to be infused (hereafter VTBI) parameter input field **3314**, and time parameter input field **3316** are displayed. The parameters, number of parameters, names of the parameters, etc. may differ in alternate embodiments. In the example embodiment, the parameter input fields are graphically displayed boxes which are substantially rectangular with rounded corners. In other embodiments, the shape and size of the parameter input fields may differ.

In the example embodiment, the GUI **3300** is designed to be intuitive and flexible. A user may choose to populate a combination of parameter input fields which are simplest or most convenient for the user. In some embodiments, the parameter input fields left vacant by the user may be calculated automatically and displayed by the GUI **3300** as long as the vacant fields do not operate independently of populated parameter input fields and enough information can be gleaned from the populated fields to calculate the vacant field or fields. Throughout **Figs. 72-76**, fields dependent upon on another are tied together by curved double-tipped arrows.

The medication parameter input field **3302** may be the parameter input field in which a user sets the type of infusate agent to be infused. In the example embodiment, the medication parameter input field **3302** has been populated and the infusate agent has been defined as "0.9% NORMAL SALINE". As shown, after the specific infusate has been set, the GUI **3300** may populate the medication parameter input field **3302** by displaying the name of the specific infusate in the medication parameter input field **3302.**

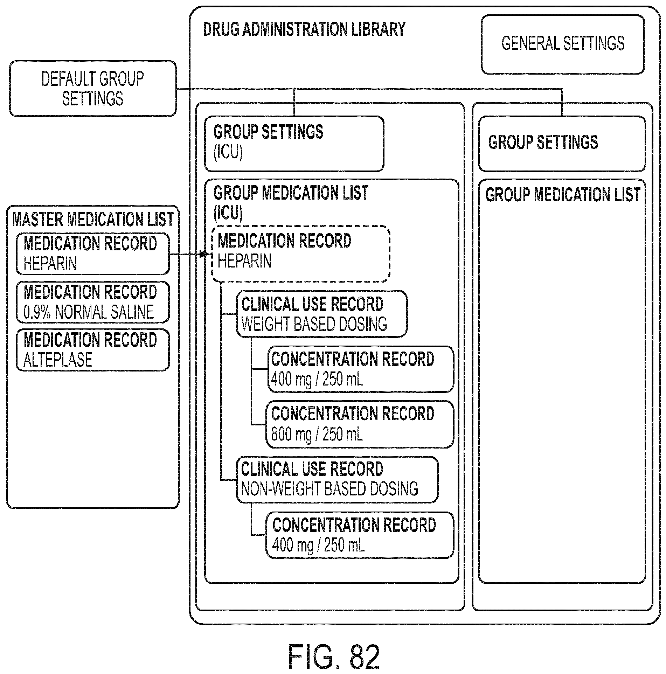

To set the specific infusate agent to be infused, a user may touch the medication parameter input field **3302** on the GUI **3300.** In some embodiments, this may cull up a list of different possible infusates. The user may browse through the list until the desired infusate is located. In other embodiments, touching the in medication parameter input field **3302** may cull up a virtual keyboard. The user may then type the correct infusate on the virtual keyboard. In some embodiments, the user may only need to type only a few letters of the infusate on the virtual keyboard before the GUI **3300** displays a number of suggestions. For example, after typing "NORE" the GUI **3300** may suggest "NOREPINEPHRINE". After locating the correct infusate, the user may be required to perform an action such as, but not limited to, tapping, double tapping, or touching and dragging the infusate. After the required action has been completed by the user, the infusate may be displayed by the GUI 3300 in the medication parameter input field **3302.** For another detailed description of another example means of infusate selection see **Fig. 82****.**

In the example embodiment in **Fig. 72**, the parameter input fields have been arranged by a user to perform a volume based infusion (for instance mL, mL/hr, etc.). Consequentially, the in container drug amount parameter input field **3304** and total volume in container parameter input field **3306** have been left unpopulated. The concentration parameter input field **3308** and dose parameter input field **3310** have also been left unpopulated. In some embodiments, the in container drug amount parameter input field **3304**, total volume in container parameter input field **3306**, concentration parameter input field **3308**, and dose parameter input field **3310** may be locked, grayed out, or not displayed on the GUI **3300** when such an infusion has been selected. The in container drug amount parameter input field **3304**, total volume in container parameter input field **3306**, concentration parameter input field **3308**, and dose parameter input field **3310** will be further elaborated upon in subsequent paragraphs.

When the GUI **3300** is being used to program a volume base infusion, the rate parameter input field **3312**, VTBI parameter input field **3314**, and time parameter input field **3316** do not operate independent of one another. A user may only be required to define any two of the rate parameter input field **3312**, VTBI parameter input field **3314**, and time parameter input field **3316.** The two parameters defined by a user may be the most convenient parameters for a user to set. The parameter left vacant by the user may be calculated automatically and displayed by the GUI **3300.** For instance, if a user populates the rate parameter input field **3312** with a value of 125 mL/hr (as shown), and populates the VTBI parameter input field **3314** with a value of 1000mL (as shown) the time parameter input field **3316** value may be calculated by dividing the value in the VTBI parameter input field **3314** by the value in the rate parameter input field **3312.** In the example embodiment shown in **Fig. 72**, the quotient of the above calculation, 8hrs and 0 min, is correctly populated by the GUI **3300** into the time parameter input field **3316.**

For a user to populate the rate parameter input field **3312**, VTBI parameter input field **3314**, and time parameter input field **3316** the user may touch or tap the desired parameter input field on the GUI **3300.** In some embodiments, this may cull up a number pad with a range or number, such as 0-9 displayed as individual selectable virtual buttons. A user may be required to input the parameter by individually tapping, double tapping, touching and dragging, etc. the desired numbers. Once the desired value has been input by a user, a user may be required to tap, double tap, etc. a virtual "confirm", "enter", etc. button to populate the field. For another detailed description of another example way of defining numerical values see **Fig. 82****.**

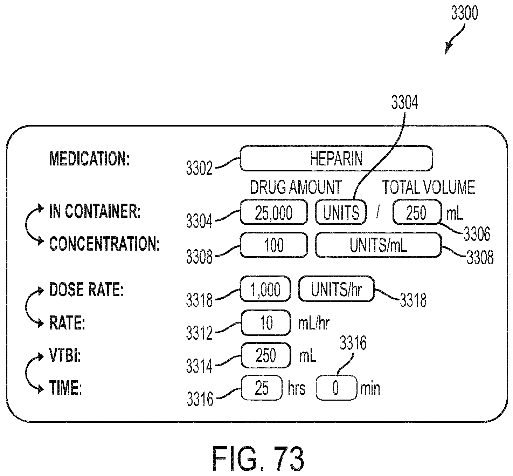

**Fig. 73** shows a scenario in which the infusion parameters being programmed are not those of a volume based infusion. In **Fig. 73**, the infusion profile is that of a continuous volume/time dose rate. In the example embodiment shown in **Fig. 73****,** all of the parameter input fields have been populated. As shown, the medication parameter input field **3302** on the GUI **3300** has been populated with "HEPARIN" as the defined infusate. As shown, the in container drug amount parameter input field **3304**, total volume in container input field **3306**, and concentration parameter input field **3308** are populated in **Fig. 73****.** Additionally, since a volume/time infusion is being programmed the dose parameter input field **3310** shown in **Fig. 72** has been replaced with a dose rate parameter input field **3318.**

The in container drug amount parameter input field **3304** is a two part field in the example embodiment shown in **Fig. 73****.** In the example embodiment in **Fig. 73** the left field of the in container drug amount parameter input field **3304** is a field which may be populated with a numeric value. The numeric value may defined by the user in the same manner as a user may define values in the rate parameter input field **3312**, VTBI parameter input field **3314**, and time parameter input field **3316.** In the example embodiment shown in **Fig. 73**, the numeric value displayed by the GUI **3300** in the in left field of the in container drug amount parameter input field **3304** is "25,000".

The parameter defined by the right field of the in container drug amount parameter input field **3304** is the unit of measure. To define the right of the in container drug amount parameter input field **3304**, a user may touch the in container drug amount parameter input field **3304** on the GUI **3300.** In some embodiments, this may cull up a list of acceptable possible units of measure. In such embodiments, the desired unit of measure may be defined by a user in the same manner as a user may define the correct infusate. In other embodiments, touching the in container drug amount parameter input field **3304** may cull up a virtual keyboard. The user may then type the correct unit of measure on the virtual keyboard. In some embodiments the user may be required to tap, double tap, etc. a virtual "confirm", "enter", etc. button to populate the left field of the in container drug amount parameter input field **3304.**

The total volume in container parameter input field **3306** may be populated by a numeric value which defines the total volume of a container. In some embodiments, the GUI **3300** may automatically populate the total volume in container parameter input field **3306** based on data generated by one or more sensors. In other embodiments, the total volume in container parameter input field **3306** may be manually input by a user. The numeric value may defined by the user in the same manner as a user may define values in the rate parameter input field **3312**, VTBI parameter input field **3314**, and time parameter input field **3316.** In the example embodiment shown in **Fig. 73** the total volume in container parameter input field **3306** has been populated with the value "250" mL. The total volume in container parameter input field **3306** may be restricted to a unit of measure such as mL as shown.

The concentration parameter input field **3308** is a two part field similar to the in container drug amount parameter input field **3304.** In the example embodiment in **Fig. 73** the left field of the concentration parameter input field **3308** is a field which may be populated with a numeric value. The numeric value may defined by the user in the same manner as a user may define values in the rate parameter input field **3312**, VTBI parameter input field **3314**, and time parameter input field **3316.** In the example embodiment shown in **Fig. 73**, the numeric value displayed by the GUI 3300 in the in left field of the concentration parameter input field **3308** is "100".

The parameter defined by the right field of the concentration parameter input field **3308** is a unit of measure/volume. To define the right field of the concentration parameter input field **3308**, a user may touch the concentration parameter input field **3308** on the GUI **3300.** In some embodiments, this may cull up a list of acceptable possible units of measure. In such embodiments, the desired unit of measure may be defined by a user in the same manner as a user may define the correct infusate. In other embodiments, touching the concentration parameter input field **3308** may cull up a virtual keyboard. The user may then type the correct unit of measure on the virtual keyboard. In some embodiments the user may be required to tap, double tap, etc. a virtual "confirm", "enter", etc. button to store the selection and move on to a list of acceptable volume measurements. The desired volume measurement may be defined by a user in the same manner as a user may define the correct infusate. In the example embodiment shown in **Fig. 73** the right field of the concentration parameter input field **3308** is populated with the unit of measure/volume "UNITS/mL".

The in container drug amount parameter input field **3304**, total volume in container input field **3306**, and concentration parameter input field **3308** are not independent of one another. As such, a user may only be required to define any two of the in container drug amount parameter input field **3304**, total volume in container input field **3306**, and concentration parameter input field **3308.** For instance, if a user were to populate the concentration parameter input field **3308** and the total volume in container parameter input field **3306**, the in container drug amount parameter input field may be automatically calculated and populated on the GUI **3300.**

Since the GUI **3300** in **Fig. 73** is being programmed for a continuous volume/time dose, the dose rate parameter input field **3318** has been populated. The user may define the rate at which the infusate is infused by populating the dose rate parameter input field **3318.** In the example embodiment in **Fig. 73**, the dose rate parameter input field **3318** is a two part field similar to the in container drug amount parameter input field **3304** and concentration parameter input field **3308** described above. A numeric value may defined in the left field of the dose rate parameter input field **3318** by the user in the same manner as a user may define values in the rate parameter input field **3312.** In the example embodiment in **Fig. 73**, the left field of the dose rate parameter input field **3318** has been populated with the value "1000".

The right field of the dose rate parameter input field **3318** may define a unit of measure/time. To define the right field of the dose rate parameter input field **3318**, a user may touch the dose rate parameter input field **3318** on the GUI **3300.** In some embodiments, this may cull up a list of acceptable possible units of measure. In such embodiments, the desired unit of measure may be defined by a user in the same manner as a user may define the correct infusate. In other embodiments, touching the dose rate parameter input field **3304** may cull up a virtual keyboard. The user may then type the correct unit of measure on the virtual keyboard. In some embodiments the user may be required to tap, double tap, etc. a virtual "confirm", "enter", etc. button to store the selection and move on to a list of acceptable time measurements. The desired time measurement may be defined by a user in the same manner as a user may define the correct infusate. In the example embodiment shown in **Fig. 73** the right field of the dose rate parameter input field **3318** is populated with the unit of measure/time "UNITS/hr".

In the example embodiment, the dose rate parameter input field **3318** and the rate parameter input field **3312** are not independent of one another. After a user populates the dose rate parameter input field **3318** or the rate parameter input field **3312**, the parameter input field left vacant by the user may be calculated automatically and displayed by the GUI **3300** as long as the concentration parameter input field **3308** has been defined. In the example embodiment shown in **Fig. 73**, the rate parameter input field **3312** has been populated with an infusate flow rate of "10 mL/hr". The dose rate parameter input field **3318** has been populated with "1000" "UNITS/hr".

In the example embodiment shown in **Fig. 73** the VTBI parameter input field **3314** and time parameter input field **3316** have also been populated. The VTBI parameter input field **3314** and time parameter input field **3316** may be populated by a user in the same manner described in relation to **Fig. 72****.** When the GUI **3300** is being programmed to a continuous volume/time dose rate infusion, the VTBI parameter input field **3314** and the time parameter input field **3316** are dependent on one another. A user may only need to populate one of the VTBI parameter input field **3314** or the time parameter input field **3316.** The field left vacant by the user may be calculated automatically and displayed on the GUI **3300.**

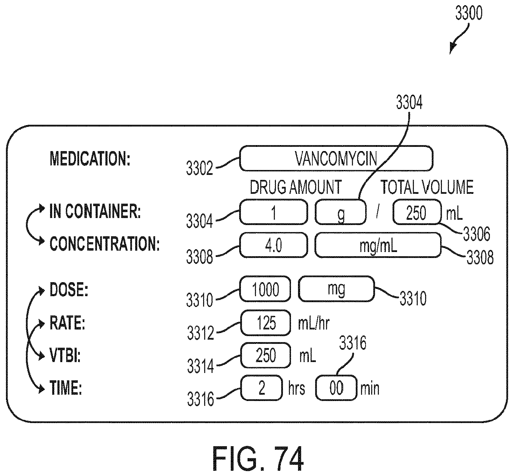

**Fig. 74** shows a scenario in which the infusion parameters being programmed are those of a drug amount based infusion herein referred to as an intermittent infusion. In the example embodiment shown in **Fig. 74**, all of the parameter input fields have been populated. As shown, the medication parameter input field **3302** on the GUI **3300** has been populated with the antibiotic "VANCOMYCIN" as the defined infusate.

As shown, the in container drug amount parameter input field **3304**, total volume in container input field **3306**, and concentration parameter input field **3308** are laid out the same as in **Fig. 73****.** In the example embodiment in **Fig. 74**, the left field of the in container drug amount parameter input field **3304** has been populated with "1". The right field of the in container drug amount parameter input field **3304** has been populated with "g". Thus the total amount of Vancomycin in the container has been defined as one gram. The total volume in container parameter input field **3306** has been populated with "250" ml. The left field of the concentration parameter input field **3308** has been populated with "4.0". The right field of the concentration parameter input field has been populated with "mg/mL".

As mentioned in relation to other possible types of infusions which a user may be capable of programming through the GUI **3300**, the in container drug amount parameter input field **3304**, total volume in container input field **3306**, and concentration parameter input field **3308** are dependent upon each other. As above, this is indicated by the curved double arrows connecting the parameter input field names. By populating any two of these parameters, the third parameter may be automatically calculated and displayed on the correct parameter input field on the GUI **3300.**

In the example embodiment in **Fig. 74**, the dose parameter input field **3310** has been populated. As shown, the dose parameter input field **3310** comprises a right and left field. A numeric value may defined in the right field of the dose parameter input field **3310** by the user in the same manner as a user may define values for other parameter input fields which define numeric values. In the example embodiment in **Fig. 74**, the left field of the dose parameter input field **3310** has been populated with the value "1000".

The right field of the dose parameter input field **3310** may define a unit of mass measurement. To define the right field of the dose parameter input field **3310**, a user may touch the dose parameter input field **3310** on the GUI **3300.** In some embodiments, this may cull up a list of acceptable possible units of measure. In such embodiments, the desired unit of measure may be defined by a user in the same manner as a user may define the correct infusate. In other embodiments, touching the dose parameter input field **3310** may cull up a virtual keyboard. The user may then type the correct unit of measure on the virtual keyboard. In some embodiments the user may be required to tap, double tap, slide, etc. a virtual "confirm", "enter", etc. button to store the selection and move on to a list of acceptable mass measurements. The desired mass measurement may be defined by a user in the same manner as a user may define the correct infusate. In the example embodiment shown in **Fig. 74** the right field of the dose parameter input field **3310** is populated with the unit of measurement "mg".

As shown, the rate parameter input field **3312**, VTBI parameter input field **3314**, and the time parameter input field **3316** have been populated. As shown, the rate parameter input field **3312** has been populated with "125" mL/hr. The VTBI parameter input field **3314** has been defined as "250" mL. The time parameter input field **3316** has been defined as "2" hrs "00" min.

The user may not need to individually define each of the dose parameter input field **3310**, rate parameter input field **3312**, VTBI parameter input field **3314,** and the time parameter input field **3316.** As indicated by the curved double arrows, the dose parameter input field **3310** and the VTBI parameter input field **3314** are dependent upon each other. Input of one value may allow the other value to be automatically calculated and displayed by the GUI 3300. The rate parameter input field **3312** and the time parameter input field **3316** are also dependent upon each other. The user may need to only define one value and then allow the non-defined value to be automatically calculated and displayed on the GUI **3300.** In some embodiments, the rate parameter input field **3312**, VTBI parameter input field **3314**, and the time parameter input field **3316** may be locked on the GUI **3300** until the in container drug amount parameter input field **3304**, total volume in container parameter input field **3306** and concentration parameter input field **3308** have been defined. These fields may be locked because automatic calculation of the rate parameter input field **3312**, VTBI parameter input field **3314**, and the time parameter input field **3316** is dependent upon values in the in container drug amount parameter input field **3304**, total volume in container parameter input field **3306** and concentration parameter input field **3308.**

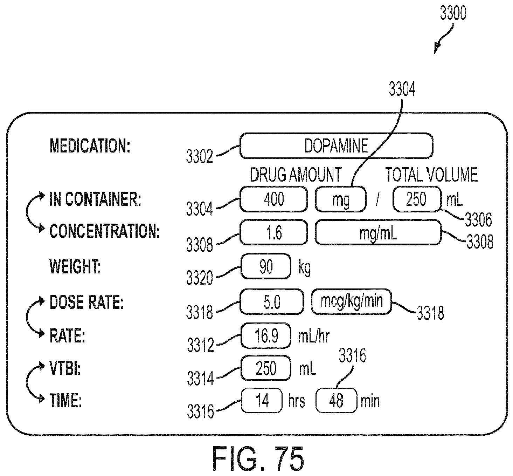

In scenarios where an infusate may require a body weight based dosage, a weight parameter input field **3320** may also be displayed on the GUI **3300.** The example GUI **3300** shown on **Fig. 75** has been arranged such that a user may program a body weight based dosage. The parameter input fields may be defined by a user as detailed in the above discussion. In the example embodiment, the infusate in the medication parameter input field **3302** has been defined as "DOPAMINE". The left field of the in container drug amount parameter input field **3304** has been defined as "400". The right field of the in container drug amount parameter input field **3304** has been defined as "mg". The total volume in container parameter input field **3306** has been defined as "250" ml. The left field of the concentration parameter input field **3308** has been defined as "1.6". The right field of the concentration parameter input field **3308** has been defined as "mg/mL". The weight parameter input field **3320** has been defined as "90" kg. The left field of the dose rater parameter input field **3318** has been defined as "5.0". The right field of the dose rate parameter input field **3318** has been defined as "mcg/kg/min". The rate parameter input field **3312** has been defined as "16.9" mL/hr. The VTBI parameter input field **3314** has been defined as "250"mL. The time parameter input field **3316** has been defined as "14" hrs "48" min.

To define the weight parameter input field **3320**, a user may may touch or tap the weight parameter input field **3320** on the GUI **3300.** In some embodiments, this may cull up a number pad with a range of numbers, such as 0-9 displayed as individual selectable virtual buttons. A user may be required to input the parameter by individually tapping, double tapping, touching and dragging, etc. the desired numbers. Once the desired value has been input by a user, a user may be required to tap, double tap, etc. a virtual "confirm", "enter", etc. button to populate the field.

As indicated by the curved double arrows, some parameter input fields displayed on the GUI **3300** may be dependent upon each other. As in previous examples, the in container drug amount parameter input field **3304**, total volume in container parameter input field **3306**, and concentration parameter input field **3308** may be dependent upon each other. In **Fig. 75**, the weight parameter input field **3320**, dose rater parameter input field **3318**, rate parameter input field **3312**, VTBI parameter input field **3314**, and the time parameter input field **3316** are all dependent upon each other. When enough information has been defined by the user in these parameter input fields, the parameter input fields not populated by the user may be automatically calculated and displayed on the GUI **3300.**

In some embodiments, a user may be required to define a specific parameter input field even if enough information has been defined to automatically calculate the field. This may improve safety of use by presenting more opportunities for user input errors to be caught. If a value entered by a user is not compatible with already defined values, the GUI **3300** may display an alert or alarm message soliciting the user to double check values that the user has entered.

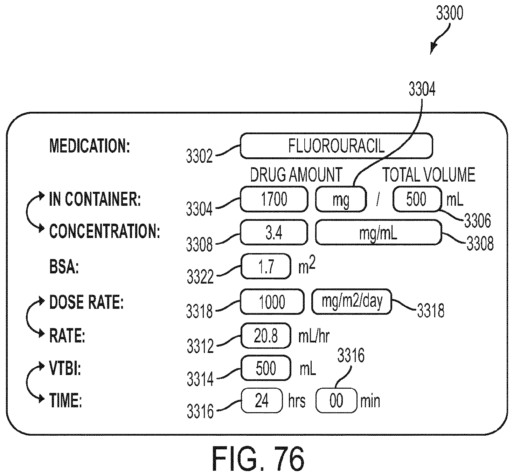

In some scenarios the delivery of infusate may be informed by the body surface area (BSA) of a patient. In **Fig. 76**, the GUI **3300** has been set up for a body surface area based infusion. As shown, a BSA parameter input field **3322** may be displayed on the GUI **3300.** The parameter input fields may be defined by a user as detailed in the above discussion. In the example embodiment, the infusate in the medication parameter input field **3302** has been defined as "FLUOROURACIL". The left field of the in container drug amount parameter input field **3304** has been defined as "1700". The right field of the in container drug amount parameter input field **3304** has been defined as "mg". The total volume in container parameter input field **3306** has been defined as "500" ml. The left field of the concentration parameter input field **3308** has been defined as "3.4". The right field of the concentration parameter input field **3308** has been defined as "mg/mL". The BSA parameter input field **3320** has been defined as "1.7" m². The left field of the dose rate parameter input field **3318** has been defined as "1000". The right field of the dose rate parameter input field **3318** has been defined as "mg/m2/day". The rate parameter input field **3312** has been defined as "20.8" mL/hr. The VTBI parameter input field **3314** has been defined as "500"mL. The time parameter input field **3316** has been defined as "24" hrs "00" min. The dependent parameter input fields are the same as in Fig. 75 with the exception that the BSA parameter input field **3322** has taken the place of the weight parameter input field **3320.**

To populate the BSA parameter input field **3322**, the user may touch or tap the BSA parameter input field **3322** on the GUI **3300.** In some embodiments, this may cull up a number pad with a range of numbers, such as 0-9 displayed as individual selectable virtual buttons. In some embodiments, the number pad and any of the number pads detailed above may also feature symbols such as a decimal point. A user may be required to input the parameter by individually tapping, double tapping, touching and dragging, etc. the desired numbers. Once the desired value has been input by a user, a user may be required to tap, double tap, etc. a virtual "confirm", "enter", etc. button to populate the field.

In some embodiments, a patient's BSA may be automatically calculated and displayed on the GUI **3300.** In such embodiments, the GUI **3300** may query the user for information about the patient when a user touches, taps, etc. the BSA parameter input field **3322.** For example, the user may be asked to define a patient's height and body weight. After the user defines these values they may be run through a suitable formula to find the patient's BSA. The calculated BSA may then be used to populate the BSA parameter input field **3322** on the GUI **3300.**

In operation, the values displayed in the parameter input fields may change throughout the course of a programmed infusion to reflect the current state of the infusion. For example, as the infusate is infused to a patient, the values displayed by the GUI **3300** in the in container drug amount parameter input field **3304** and total volume in container parameter input field **3306** may decline to reflect the volume of the remaining contents of the container. Additionally, the values in the VTBI parameter input field **3314** and time parameter input field **3316** may also decline as infusate is infused to the patient.

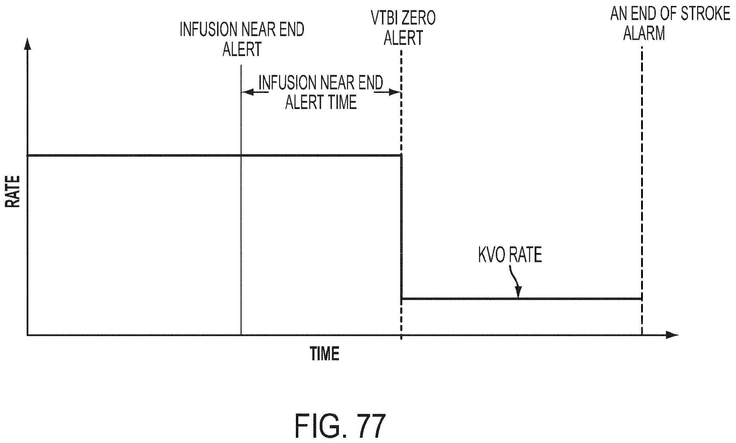

**Fig. 77** is an example rate over time graph detailing one behavioral configuration of a syringe pump **500** (see **Fig. 28****)** over the course of an infusion. Though the following discussion mostly details behavioral configurations of a syringe pump **500**, it should be appreciated that the graphs shown in **Fig. 77-81** may also detail the behavioral configurations of other pumps, including the other pumps mentioned in this specification. The graph in **Fig. 77** details an example behavioral configuration of the syringe pump **500** where the infusion is a continuous infusion (an infusion with a dose rate). As shown, the graph in **Fig. 77** begins at the initiation of infusion. As shown, the infusion is administered at a constant rate for a period of time. As the infusion progresses, the amount of infusate remaining is depleted.

When the amount of infusate remaining reaches a pre-determined threshold, an "INFUSION NEAR END ALERT" may be triggered. The point at which "INFUSION NEAR END ALERT" is issued may be configured by the user. The "INFUSION NEAR END ALERT" may also be configured to be triggered sooner on short-half life drugs. The "INFUSION NEAR END ALERT" may be in the form of a message on the GUI 3300 and may be accompanied by flashing lights, and audible noises such as a series of beeps. The "INFUSION NEAR END ALERT" allows time for the care giver and pharmacy to prepare materials to continue the infusion if necessary. As shown, the infusion rate may not change over the "INFUSION NEAR END ALERT TIME".

When the syringe pump **500** (see **Fig. 28****)** has infused the VTBI to a patient a "VTBI ZERO ALERT" may be triggered. The "VTBI ZERO ALERT" may be in the form of a message on the GUI **3300** and may be accompanied by flashing lights and audible noises such as beeps. As shown, the "VTBI ZERO ALERT" causes the pump to switch to a keep-vein-open (hereafter KVO) rate until a new infusate container may be put in place. The KVO rate is a low infusion rate (for example 5-25mL/hr). The rate is set to keep the infusion site patent until a new infusion may be started. The KVO rate may be configurable by the group (elaborated upon later) or medication and can be modified on the syringe pump **500.** The KVO rate is not allowed to exceed the continuous infusion rate. When the KVO rate can no longer be sustained and the syringe has reached the end of its stoke, an "END OF STROKE ALARM" may be triggered. When the "END OF STROKE ALARM" is triggered, all infusion may stop. The "END OF STROKE ALARM" may be in the form of a message on the GUI **3300** and may be accompanied by flashing lights and audible noises such as beeps.

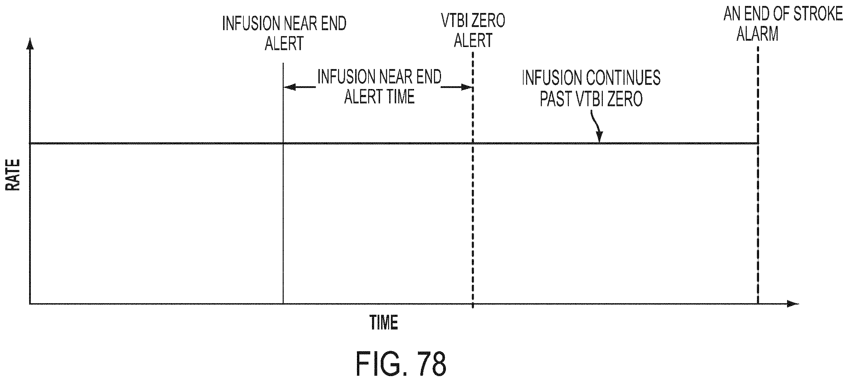

**Fig. 78** shows another example rate over time graph detailing one behavioral configuration of a syringe pump **500** (see **Fig. 28****)** over the course of an infusion. The graph in **Fig. 78** details an example behavioral configuration of a syringe pump **500** where the infusion is a continuous infusion (an infusion with a dose rate). The alerts in the graph shown in **Fig. 78** are the same as the alerts shown in the graph in **Fig. 77****.** The conditions which propagate the alerts are also the same. The rate, however, remains constant throughout the entire graph until the "END OF STROKE ALERT" is triggered and the infusion is stopped. By continuing infusion at a constant rate, it is ensured that the blood plasma concentration of the drug remains at therapeutically effective levels. Configuring the pump to continue infusion at a constant rate may be especially desirable in situations where the infusate is a drug with a short half-life. In some embodiments, the end of infusion behavior of the syringe pump **500** may be restricted depending on the defined infusate. For example, when the defined infusate is a short half-life drug the end of infusion behavior of the syringe pump **500** may be limited only to continuing to infuse at the rate of the finished infusion.

The syringe pump **500** (see **Fig. 28****)** may also be used to deliver a primary or secondary intermittent infusion. During an intermittent infusion, an amount of a drug (dose) is administered to a patient as opposed to a continuous infusion where the drug is given at a specified dose rate (amount/time). An intermittent infusion is also delivered over a defined period of time, however, the time period and dose are independent of one another. The previously described **Fig. 73** shows a setup of the GUI **3300** for a continuous infusion. The previously described **Fig. 74** shows a setup of the GUI **3300** for an intermittent infusion.

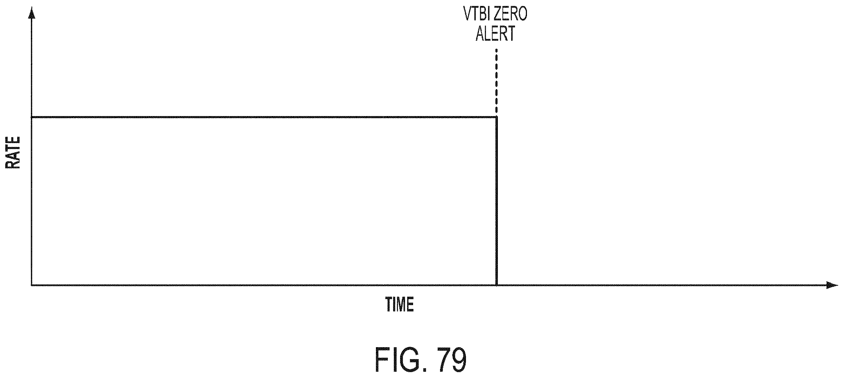

**Fig. 79** is an example rate over time graph detailing the one behavioral configuration of a syringe pump **500** (see **Fig. 28****)** over the course of an intermittent infusion. As shown, the intermittent infusion is given at a constant rate until all infusate programmed for the intermittent infusion has been depleted. In the example behavioral configuration, the syringe pump **500** has been programmed to issue a "VTBI ZERO ALERT" and stop the infusion when all the infusate has been dispensed. In this configuration, the user may be required to manually clear the alert before another infusion may be started or resumed.

Depending on the group (further elaborated upon later) or the medication, it may be desirable to configure the syringe pump **500** to behave differently at the end of an intermittent infusion. Other configurations may cause a syringe pump **500** (see **Fig. 28****)** to behave differently. For example, in scenarios where the intermittent infusion is a secondary infusion, the pump **201, 202, 203** (see **Fig. 2**) may be configured to automatically switch back to the primary infusion after issuing a notification that the secondary intermittent infusion has been completed. In alternate configurations, the a syringe pump **500** may be configured issue a "VTBI ZERO ALERT" and drop the infusion rate to a KVO rate after completing the intermittent infusion. In such configurations, the user may be required to manually clear the alert before a primary infusion is resumed.

A bolus may also be delivered as a primary intermittent infusion when it may be necessary or desirable to achieve a higher blood plasma drug concentration or manifest a more immediate therapeutic effect. In such cases, the bolus may be delivered by a pump **201**, **202**, **203** (see **Fig. 2**) executing the primary infusion. The bolus may be delivered from the same container which the primary infusion is being delivered from. A bolus may be performed at any point during an infusion providing there is enough infusate to deliver the bolus. Any volume delivered via a bolus to a patient is included in the value displayed by the VTBI parameter input field **3314** of the primary infusion.

Depending on the infusate, a user may be forbidden from performing a bolus. The dosage of a bolus may be pre-set depending on the specific infusate or infusate concentration being used. Additionally, the period of time over which the bolus occurs may be pre-defined depending on the infusate being used. After performing a bolus, the bolus function may be locked for a pre-defined period of time. In some embodiments, a user may be capable of adjusting these pre-sets by adjusting various setting on the GUI **3300.** In some situations, such as those where the drug being infused has a long half-life (vancomycin, teicoplanin, etc.), a bolus may be given as a loading dose to more quickly reach a therapeutically effective blood plasma drug concentration.

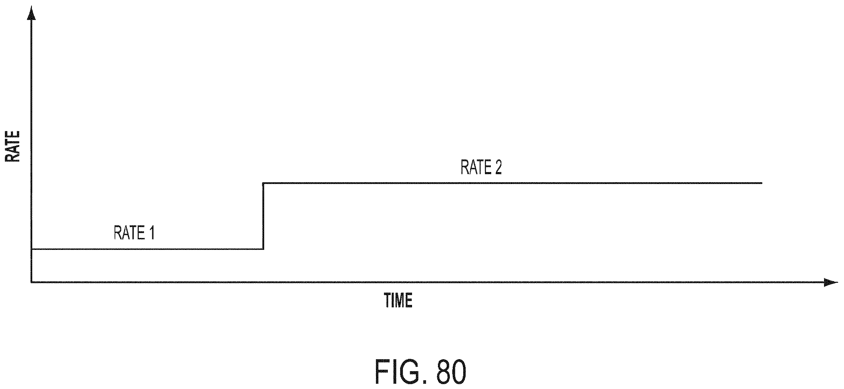

**Fig. 80** shows another rate over time graph in which the flow rate of the infusate has been titrated to "ramp" the patient up on the infusate. Titration is often used with drugs which register a fast therapeutic effect, but have a short half life (such as norepinephrine). When titrating, the user may adjust the delivery rate of the infusate until the desired therapeutic effect is manifested. Every adjustment may be checked against a series of limits defined for the specific infusate being administered to the patient. If an infusion is changed by more than a pre-defined percentage, an alert may be issued. In the exemplary graph shown in **Fig. 80**, the rate has been up-titrated once. If necessary, the rate may be up-titrated more than one time. Additionally, in cases where titration is being used to "wean" a patient off of a drug, the rate may be down-titrated any suitable number of times.

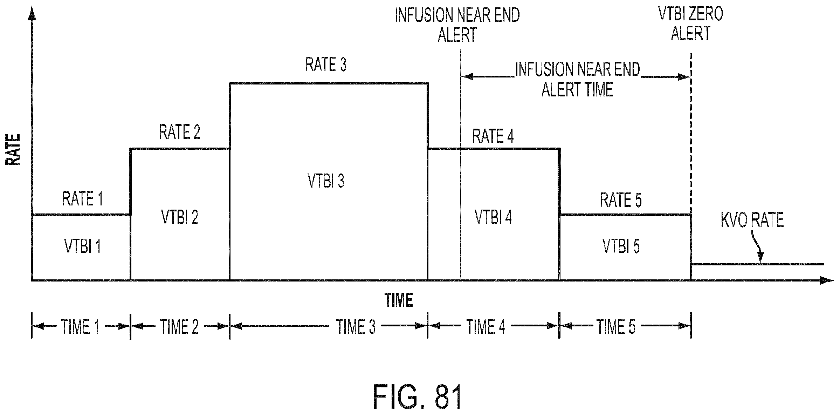

**Fig. 81** is another rate over time graph in which the infusion has been configured as a multi-step infusion. A multi-step infusion may be programmed in a number of different steps. Each step may be defined by a VTBI, time, and a dose rate. Multi-step infusions may be useful for certain types of infusates such as those used for parenteral nutrition applications. In the example graph shown in **Fig. 81**, the infusion has been configured as a five step infusion. The first step infuses a "VTBI 1" for a length of time, "Time 1", at a constant rate, "Rate 1". When the time interval for the first step has elapsed, the pump moves on to the second step of the multi-step infusion. The second step infuses a "VTBI 2" for a length of time, "Time 2", at a constant rate, "Rate 2". As shown, "Rate 2" is higher than "Rate 1". When the time interval for the second step has elapsed, the pump moves on to the third step of the multi-step infusion. The third step infuses a "VTBI 3" for a length of time, "Time 3", at a constant rate, "Rate 3". As shown "Rate 3" is the highest rate of any steps in the multi-step infusion. "Time 3" is also the longest duration of any step of the multi-step infusion. When the time interval for the third step has elapsed, the pump move on to the fourth step of the multi-step infusion. The fourth step infuses a "VTBI 4" for a length of time, "Time 4", at a constant rate, "Rate 4". As shown, "Rate 4" has been down-titrated from "Rate 3". "Rate 4" is approximately the same as "Rate 2". When the time interval for the fourth step of the multi-step infusion has elapsed, the pump move on to the fifth step. The fifth step infuses a "VTBI 5" for a length of time, "Time 5", at a constant rate, "Rate 5". As shown, "Rate 5" has been down-titrated from "Rate 4" and is approximately the same as "Rate 1".

The "INFUSION NEAR END ALERT" is triggered during the fourth step of the example infusion shown in **Fig. 81****.** At the end of the fifth and final step of the multi-step infusion, the "VTBI ZERO ALERT" is triggered. In the example configuration shown in the graph in **Fig. 81**, the rate is dropped to a KVO rate after the multi-step infusion has been concluded and the "VTBI ZERO ALERT" has been issued. Other configurations may differ.

Each rate change in a multi-step infusion may be handled in a variety of different ways. In some configurations, the syringe pump **500** (see **Fig. 2**) may display a notification and automatically adjust the rate to move on to the next step. In other configurations, the syringe pump **500** may issue an alert before changing the rate and wait for confirmation from the user before adjusting the rate and moving on to the next step. In such configurations, the pump **500** may stop the infusion or drop to a KVO rate until user confirmation has been received.

In some embodiments, the user may be capable of pre-programming infusions. The user may pre-program an infusion to automatically being after a fixed interval of time has elapsed (e.g. 2 hours). The infusion may also be programmed to automatically being at a specific time of day (e.g. 12:30 pm). In some embodiments, the user may be capable of programming the syringe pump **500** (see **Fig. 28****)** to alert the user with a callback function when it is time to being the pre-programmed infusion. The user may need to confirm the start of the pre-programmed infusion. The callback function may be a series of audible beeps, flashing lights, or the like.

In arrangements where there is more than one pump **201, 202, 203** (see **Fig. 2**), the user may be able to program a relay infusion. The relay infusion may be programmed such that after a first pump **201, 202, 203** has completed its infusion, a second pump **201, 202, 203** may automatically being a second infusion and so on. The user may also program a relay infusion such that the user is alerted via the callback function before the relay occurs. In such a programmed arrangement, the relay infusion may not being until confirmation from a user has been received. A pump **201, 202, 203** may continue at a KVO rate until user confirmation has been received.

**Fig. 82** shows an example block diagram of a "Drug Administration Library" data structure. The data structure may be stored in any file format or in any database (e.g., an SQL database). In the upper right hand corner there is a box which is substantially rectangular, though its edges are rounded. The box is associated with the name "General Settings". The "General Settings" may include settings which would be common to all devices in a facility such as, site name (e.g. XZY Hospital), language, common passwords, and the like.

In **Fig. 82**, the "Drug Administration Library" has two boxes which are associated with the names "Group Settings (ICU)" and "Group Settings". These boxes form the headings for their own columns. These boxes may be used to define a group in within a facility (e.g. pediatric intensive care unit, emergency room, sub-acute care, etc.) in which the device is stationed. Groups may also be areas outside a parent facility, for example, a patient's home or an inter-hospital transport such as an ambulance. Each group may be used to set specific settings for various groups within a facility (weight, titration limits, etc.). These groups may alternatively be defined in other manners. For example, the groups may be defined by user training level. The group may be defined by a prior designated individual or any of a number of prior designated individuals and changed if the associated patient or device is moved from one specific group within a facility to another.

In the example embodiment, the left column is "Group Settings (ICU)" which indicates that the syringe pump **500** (see **Fig. 28****)** is stationed in the intensive care unit of the facility. The right column is "Group Settings" and has not been further defined. In some embodiments, this column may be used to designate a sub group, for example operator training level. As indicated by lines extending to the box off to the left of the block diagram from the "Group settings (ICU)" and "Group Settings" columns, the settings for these groups may include a preset number of default settings.

The group settings may include limits on patient weight, limits on patient BSA, air alarm sensitivity, occlusion sensitivity, default KVO rates, VTBI limits, etc. The group settings may also include parameters such as whether or not a review of a programmed infusion is necessary for high risk infusates, whether the user must identify themselves before initiating an infusion, whether the user must enter a text comment after a limit has been overridden, etc. A user may also define the defaults for various attributes like screen brightness, or speaker volume. In some embodiments, a user may be capable of programming the screen to automatically adjust screen brightness in relation to one or more conditions such as but not limited to time of day.

As also shown to the left of the block diagram in **Fig. 82**, each facility may have a "Master Medication List" defining all of the infusates which may be used in the facility. The "Master Medication List" may comprise a number of medications which a qualified individual may update or maintain. In the example embodiment, the "Master Medication List" only has three medications: Heparin, 0.9% Normal Saline, and Alteplase. Each group within a facility may have its own list of medications used in the group. In the example embodiment, the "Group Medication List (ICU)" only includes a single medication, Heparin.

As shown, each medication may be associated with one or a number of clinical uses. In **Fig. 82** the "Clinical Use Records" are defined for each medication in a group medication list and appear as an expanded sub-heading for each infusate. The clinical uses may be used to tailor limits and pre-defined settings for each clinical use of the infusate. For Heparin, weight based dosing and non-weight based dosing are shown in **Fig. 82** as possible clinical uses. In some embodiments, there may be a "Clinical Use Record" setting requiring the user to review or re-enter a patient's weight (or BSA) before beginning an infusion.

Clinical uses may also be defined for the different medical uses of each infusate (e.g. stroke, heart attack, etc.) instead of or in addition to the infusate's dose mode. The clinical use may also be used to define whether the infusate is given as a primary continuous infusion, primary intermittent infusion, secondary infusion, etc. They may also be use to provide appropriate limits on the dose, rate, VTBI, time duration, etc. Clinical uses may also provide titration change limits, the availability of boluses, the availability of loading doses, and many other infusion specific parameters. In some embodiments, it may be necessary to provide at least one clinical use for each infusate in the group medication list.

Each clinical use may additionally comprise another expanded sub-heading in which the concentration may also be defined. In some cases, there may be more than one possible concentration of an infusate. In the example embodiment in **Fig. 82**, the weight base dosing clinical use has a 400mg/250mL concentration and an 800 mg/250mL concentration. The non-weight based dosing clinical use only has one concentration, 400mg/mL. The concentrations may also be used to define an acceptable range for instances where the user may customize the concentration of the infusate. The concentration setting may include information on the drug concentration (as shown), the diluents volume, or other related information.

In some embodiments, the user may navigate to the "Drug Administration Library" to populate some of the parameter input fields shown in **Figs. 72-76****.** The user may also navigate to the "Drug Administration Library" to choose from the clinical uses for each infusate what type of infusion the syringe pump **500** (see **Fig. 28****)** will administer. For example, if a user were to select weight based Heparin dosing on **Fig. 82**, the GUI **3300** might display the infusion programming screen shown on **Fig. 75** with "Heparin" populated into the medication parameter input field **3302.** Selecting a clinical use of a drug may also prompt a user to select a drug concentration. This concentration may then be used to populate the concentration parameter input field **3308** (see **Figs. 72-76**). In some embodiments, the "Drug Administration Library" may be updated and maintained external to the syringe pump **500** and communicated to the syringe pump **500** via any suitable means. In such embodiments, the "Drug Administration Library" may not be changeable on the syringe pump **500** but may only place limits and/or constraints on programming options for a user populating the parameter input fields shown in **Fig. 72-76****.**

As mentioned above, by choosing a medication and clinical use from the group medication list, a user may also be setting limits on other parameter input fields for infusion programming screens. For example, by defining a medication in the "Drug Administration Library" a user may also be defining limits for the dose parameter input field **3310,** dose rate parameter input field **3318**, rate parameter input field **3312**, VTBI parameter input field **3314**, time parameter input field **3316**, etc. These limits may be pre-defined for each clinical use of an infusate prior to the programming of an infusion by a user. In some embodiments, limits may have both a soft limit and a hard limit with the hard limit being the ceiling for the soft limit. In some embodiments, the group settings may include limits for all of the medications available to the group. In such cases, clinical use limits may be defined to further tailor the group limits for each clinical usage of a particular medication.

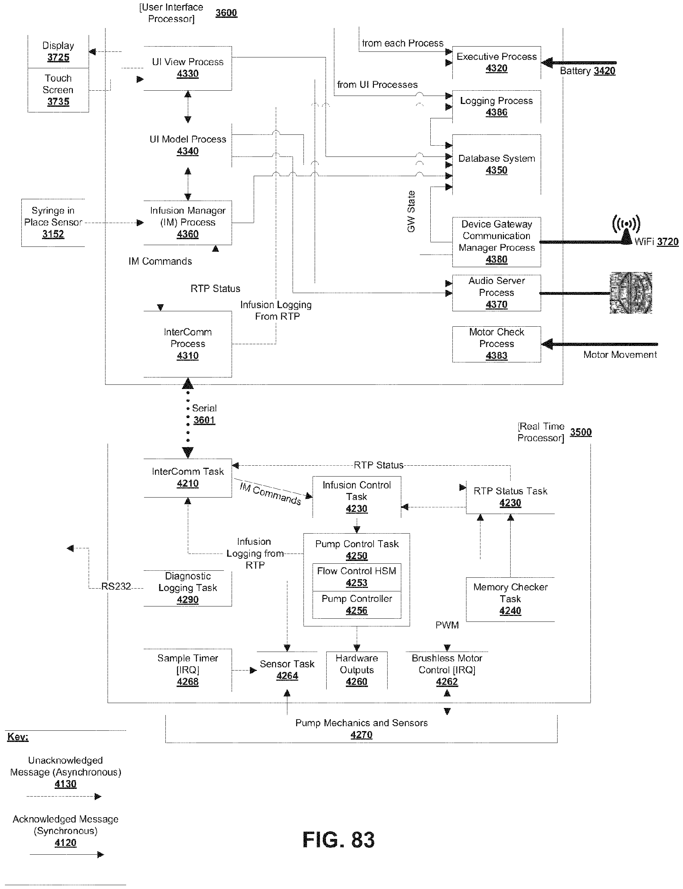

The software architecture of the syringe pump **500** is shown schematically in **FIG 83****.** The software architecture divides the software into cooperating subsystems that interact to carry out the required pumping action. The software is equally applicable to all the embodiments described herein. It is also possible to apply the software to other pumps not described herein. Each subsystem may be composed of one or more execution streams controlled by the underlying operating system. Useful terms used in the art include operating system, subsystem, process, thread and task.

Asynchronous messages **4130** are used to `push' information to the destination task or process. The sender process or task does not get confirmation of message delivery. Data delivered in this manner is typically repetitive in nature. If messages are expected on a consistent schedule, the receiver process or task can detect a failure if a message does not arrive on time.

Synchronous messages **4120** may be used to send a command to a task or process, or to request ('pull') information from a process or task. After sending the command (or request), the originating task or process suspends execution while awaiting a response. The response may contain the requested information, or may acknowledge the receipt of the sent message. If a response is not received in a timely manner, the sending process or task may time out. In such an event, the sending process or task may resume execution and/or may signal a error condition.

An operating system (OS) is a collection of software that manages computer hardware resources and provides common services for computer programs. The operating system may act as an intermediary between programs and the computer hardware. Although some application code may be executed directly by the hardware, the application code may frequently make a system call to an OS function or be interrupted by it.

The RTP **3500** may run on a Real Time Operating System (RTOS) that has been certified to a safety level for medical devices. An RTOS is a multitasking operating system that aims at executing real-time applications. Real-time operating systems often use specialized scheduling algorithms so that they can achieve a deterministic nature of behavior. The UIP **3600** may run on a Linux operating system. The Linux operating system is a Unix-like computer operating system.

A subsystem is a collection of software (and perhaps hardware) assigned a specific set of (related) system functionality or functionalities. A subsystem has clearly defined responsibilities and a clearly defined interface to other subsystems. A subsystem is an architectural division of the software that uses one or more processes, threads or tasks.

A process is an independent executable running on a Linux operating system which runs in its own virtual address space. The memory management hardware on the CPU is used to enforce the integrity and isolation of this memory, by write protecting code-space, and disallowing data access outside of the process' memory region. Processes can only pass data to other processes using inter-process communication facilities.

In Linux, a thread is a separately scheduled, concurrent path of program execution. On Linux, a thread is always associated with a process (which must have at least one thread and can have multiple threads). Threads share the same memory space as its 'parent' process. Data can be directly shared among all of the threads belonging to a process but care must be taken to properly synchronize access to shared items. Each thread has an assigned execution priority.

A Task on an RTOS (Real Time Operating System) is a separately scheduled, concurrent path of program execution, analogous to a Linux 'thread'. All tasks share the same memory address space which consists of the entire CPU memory map. When using an RTOS that provides memory protection, each task's effective memory map is restricted by the Memory Protection Unit (MPU) hardware to the common code space and the task's private data and stack space.

The processes on the UIP **3600**, communicate via IPC calls as shown by the one-way arrows in **FIG 83****.** Each solid-lined arrow represents a synchronous message **4120** call and response, and dotted-line arrows are asynchronous messages **4130.** The tasks on the RTP **3500** similarly communicate with each other. The RTP **3500** and UIP **3600** may be bridged by an asynchronous serial line **3601**, with one of an InterComm Process **4110** or InterComm Task **4210** on each side. The InterComm Process **4110** presents the same communications API (Application Programming Interface) on both sides of the bridge, so all processes and tasks can use the same method calls to interact.

The Executive Process **4320** may invoked by the Linux system startup scripts after all of the operating system services have started. The Executive Process **4320** then starts the various executable files that comprise the software on the UIP 3600. If any of the software components should exit or fail unexpectedly, the Executive Process 4320 may be notified, and may generate the appropriate alarm.

While the system is running, the Executive Process **4320** may act as a software 'watchdog' for various system components. After registering with the Executive Process **4320**, a process is required to 'check in' or send a signal periodically to the Executive Process **4320.** Failure to 'check in' at the required interval may be detected by the Executive Process **4320.** Upon detection of a failed subsystem, the Executive Process **4320** may take remedial action of either: do nothing, declaring an alarm, or restarting the failed process. The remedial action taken is predetermined by a table entry compiled into the Executive Process **4320.** The 'check-in' interval may vary from process to process. The amount of variance between 'check-in' times for different processes may be based in part on the importance of the process. The check-in interval may also vary during syringe pump **500** operation to optimize the pump controller response by minimizing computer processes. In one example embodiment, during syringe **504** loading, the pump controller may check-in less frequently than during active pumping.

In response to the required check-in message, the Executive Process **4320** may return various system status items to processes that checked-in. The system status items may be the status of one or more components on the syringe pump **500** and/or errors. The System Status items may include: battery status, WiFi connection status, device gateway connection status, device status (Idle, Infusion Running, Diagnostic Mode, Error, Etc.), technical error indications, and engineering log levels.

A thread running in the Executive Process **4320** may be used to read the state of the battery **3420** from an internal monitor chip in the battery **3420.** This may be done at a relatively infrequent interval such as every 10 seconds.

The UI View **4330** implements the graphical user interface (GUI **3300** see **Fig. 71**), rendering the display graphics on the display **514**, and responding to inputs on the touch screen in embodiments comprising a touch screen or to inputs communicated via other data input means **516.** The UI View **4330** design is stateless. The graphic being displayed may be commanded by the UI Model Process **4340**, along with any variable data to be displayed. The commanded graphic may be refreshed periodically regardless of data changes.

The style and appearance of user input dialogs (Virtual keyboard, drop down selection list, check box etc.) may be specified by the screen design, and implemented entirely by the UI View **4330.** User input may be collected by the UI View **4330**, and sent to the UI Model **4340** for interpretation. The UI View **4330** may provide for multi-region, multi-lingual support with facilities for the following list including but not limited to: virtual keyboards, unicode strings, loadable fonts, right to left entry, translation facility (loadable translation files), and configurable numbers and date formats.

The UI Model **4340** implements the screen flows, and so controls the user experience. The US Model **4340** interacts with the Ul View **4330**, specifying the screen to display, and supplies any transient values to be displayed on the screen. Here screen refers the image displayed on the physical display **514** and the defined interactive areas or user dialogs i.e. buttons, sliders, keypads etc, on the touch screen **3735.** The UI Model **4340** interprets any user inputs sent from the UI View **4330**, and may either update the values on the current screen, command a new screen, or pass the request to the appropriate system service (i.e. 'start pumping' is passed to the RTP **3500**).

When selecting a medication to infuse from the Drug Administration Library, the UI Model 4340 interacts with the Drug Administration Library stored in the local data base which is part of the Database System **4350.** The user's selections setup the run time configurations for programming and administering the desired medication.

While the operator is entering an infusion program, The UI Model **4340** may relay the user's input values to the Infusion Manager **4360** for validation and interpretation. Therapeutic decisions may not be made by the UI Model **4340.** The treatment values may be passed from the Infusion Manager **4360** to the UI Model **4340** to the UI View **4330** to be displayed for the user.

The UI Model **4340** may continuously monitor the device status gathered from the Infusion Manager **4360** (current infusion progress, alerts, etc.) for possible display by the UI View **4330.** Alerts/Alarms and other changes in system state may provoke a screen change by the UI Model **4340.**

The Infusion Manager Process (IM) **4360** may validate and controls the infusion delivered by the syringe pump **500.** To start an infusion, the user may interact with the UI View/Model **4330/4340** to select a specific medication and clinical use. This specification selects one specific Drug Administration Library (DAL) entry for use. The IM **4360** loads this DAL entry from the database **4350,** for use in validating and running the infusion.

Once a Drug Administration Library entry is selected, the IM **4340** may pass the dose mode, limits for all user enterable parameters, and the default values (if set) up to the UI Model **4340.** Using this data, the UI Model **4340** may guide the user in entering the infusion program.

As each parameter is entered by the user, the value may sent from the UI View/Model **4330/4340** to the IM **4360** for verification. The IM **4360** echoes the parameters back to the UI View/Model **4330/4340**, along with an indication of the parameter's conformance to the DAL limits. This allows the UI View/Model **4330/4340** to notify the user of any values that are out of bounds.

When a complete set of valid parameters has been entered, the IM **4360** also may return a valid infusion indicator, allowing the UI View/Model **4330/4340** to present a 'Start' control to the user.

The IM **4360** may simultaneously make the infusion/pump status available to the UI View/Model **4330/4340** upon request. If the UI View/Model **4330/4340** is displaying a 'status' screen, it may request this data to populate it. The data may be a composite of the infusion state, and the pump state.

When requested to run the (valid) infusion, the IM **4360** may pass the Infusion Worksheet' containing user specified data and the 'Infusion Template' containing the read-only limits from the DAL as a CRC'd binary block to the Infusion Control Task **4220** running on the RTP **3500.** The Infusion Control Task **4220** on the RTP **3500** takes the same user inputs, conversions and DERS inputs and recalculates the Infusion Worksheet. The Infusion Control Task **4220** calculated results may be stored in a second CRC'd binary block and compared to the first binary block from the UIP **3600.** The infusion calculations performed on the UIP **3600** may be recalculated and double checked on the RTP **3500** before the infusion is run.

Coefficients to convert the input values (ie. □l, grams, %, etc.) to a standard unit such as ml may be stored in the UIP **3600** memory or database system **4350.** The coefficients may be stored in a lookup table or at specific memory locations. The lookup table may contain 10's of conversion values. In order to reduce the chance that flipping a single bit will resulting in the wrong conversion factor being used, the addresses for the conversion values may be distributed among the values from zero to 4294967296 or 2³². The addresses may be selected so that the binary form of one address is never just one bit different from a second address.

While an infusion is running, the IM **4360** may monitor its progress, sequences, pauses, restarts, secondary infusions, boluses, and KVO (keep vein open) scenarios as needed. Any user alerts requested during the infusion (Infusion near complete, KVO callback, Secondary complete callback, etc) may be tracked and triggered by the IM **4360.**

Processes on the UIP **3600** may communicate with each other via a proprietary messaging scheme based on a message queue library that is available with Linux. The system provides for both acknowledged (synchronous message **4120)** and unacknowledged (asynchronous message **4130)** message passing.

Messages destined for the Real-time Processor (RTP) **3500** may be passed to the InterComm Process **4310** which forwards the messages to the RTP **3500** over a serial link **3601.** A similar InterComm Task **4210** on the RTP **3500** may relay the message to its intended destination via the RTP **3500** messaging system.

The messaging scheme used on this serial link **3601** may provide for error detection and retransmission of flawed messages. This may be needed to allow the system to be less susceptible to electrical disturbances that may occasionally 'garble' inter-processor communications.

To maintain a consistent interface across all tasks, the message payloads used with the messaging system may be data classes derived from a common baseclass (MessageBase). This class adds both data identity (message type) and data integrity (CRC) to messages.

The Audio Server Process **4370** may be used to render sounds on the system. All user feedback sounds (key press beeps) and alarm or alert tones may be produced by playing pre-recorded sound files. The sound system may also be used to play music or speech if desired.

Sound requests may be symbolic (such as "Play High Priority Alarm Sound"), with the actual sound file selection built into the Audio Server process **4370.** The ability to switch to an alternative soundscape may be provided. This ability may be used to customize the sounds for regional or linguistic differences.

The Device Gateway Communication Manager Process (DGCM) **4380** may manage communications with the Device Gateway Server over a Wi-Fi network **3620**, **3622**,**3720.** The DGCM **4380** may be started and monitored by the Executive Process **4320.** If the DGCM **4380** exits unexpectedly, it may be restarted by the Executive Process **4320** but if the failures are persistent the system may continue to function without the gateway running.

It may be the function of the DGCM **4380** to establish and maintain the Wi-Fi connection and to then establish a connection to the Device Gateway. All interactions between the DGCM **4380** and the Device Gateway use a system such as the system described in the cross referenced nonprovisional application for **System, Method, and Apparatus for Electronic Patient Care** (Attorney Docket No. J85).

If the connection to the gateway is unavailable or becomes unavailable, the DGCM **4380** may discontinue any transfers in progress, and attempt to reconnect the link. Transfers may be resumed when the link is reestablished. Network and Gateway operational states are reported periodically to the Executive Process **4320.** The Executive Process **4320** distributes this information for display to the user.

The DGCM **4380** may function as an autonomous subsystem, polling the Device Gateway Server for updates, and downloading newer items when available. In addition the DGCM **4380** may monitor the logging tables in the database, uploading new log events as soon as they are available. Events that are successfully uploaded may be flagged as such in the database. After a reconnection to the Device Gateway Server, the DGCM **4380** may 'catch up' with the log uploads, sending all items that were entered during the communications disruption. Firmware and Drug Administration Library updates received from the Gateway may be staged in the UIP's **3600** file system for subsequent installation. Infusion programs, clinical advisories, patient identification and other data items destined for the device may be staged in the database.

The DGCM **4380** may report connection status and date/time updates to the Executive Process **4320.** There may not be other direct connections between the DGCM **4380** and any of the other operational software. Such a design decouples the operational software from the potentially transient availability of the Device Gateway and Wi-Fi network.

The Motor Check **4383** software may read a hardware counter or encoder **1202** (**FIG 60**) that reports motor **1200** rotation. The software in this module may independently estimate the motor's **1200** movements, and compare them to the expected motion based on the user inputs for rate of infusion. This may be an independent check for proper motor control. However, the primary motor **1200** control software may executed on the RTP **3500.**

Event information may be written to a log via the Logging Process **4386** during normal operation. These events may consist of internal machine status and measurements, as well as therapy history events. Due to the volume and frequency of event log data, these logging operations may be buffered in a FIFO queue while waiting to be written to the database.

A SQL database (PostgreSQL) may be used to store the Drug Administration Library, Local Machine Settings, Infusion History and Machine Log data. Stored procedures executed by the database server may be used to insulate the application from the internal database structures.

The database system **4350** may be used as a buffer for log data destined for the Device Gateway server, as well as a staging area for infusion settings and warnings sent to the pump from the Gateway.

Upon requesting the start of an infusion, the DAL entry and all user selected parameters may be sent to the Infusion Control Task **4220.** All of the DAL validations and a recalculation of the infusion rate and volume based upon the requested dose may be performed. The result may be checked against the results calculated by the IM **4360** on the UIP **3600.** These results may be required to match to continue.

When running an infusion, the Infusion Control Task **4220** may control the delivery of each infusion 'segment'; i.e. one part of an infusion consisting of a volume and a rate. Examples of segments are: a primary infusion, KVO, bolus, remainder of primary after bolus, primary after titration, etc. The infusion segments are sequenced by the IM Process **4360** on the UIP **3600.**

The Pump Control Task **4250** may incorporate the controllers that drive the pumping mechanism. The desired pumping rate and amount (VTBI) may be specified in commands sent from the Infusion Control Task **4220.**

The Pump Control **4250** may receive periodic sensor readings from the Sensor Task **4264.** The new sensor readings may be used to determine the motor speed and position, and to calculate the desired command to send to the Brushless Motor Control IRQ **4262.** The receipt of the sensor message may trigger a recalculation of the controller output.

While pumping fluid, the Pump Control Task **4250** may perform at least one of the following tasks: controlling pumping speed, measuring volume delivered, measuring air detected (over a rolling time window), measuring fluid pressure or other indications of occlusions, and detecting upstream occlusions.

Relevant measurements may be reported to the RTP Status Task **4230** periodically. The Pump Control **4250** may execute one infusion segment at a time, stopping when the commanded delivery volume has been reached. The Sensor Task **4264** may read and aggregate the sensor data used for the dynamic control of the pumping system.

The sensor task **4264** may be scheduled to run at a consistent 1 kHz rate (every 1.0 ms) via a dedicated counter/timer. After all of the relevant sensors are read, the data may be passed to the Pump Control Task **4250** via an asynchronous message **4120.** The periodic receipt of this message may be used as the master time base to synchronize the syringe pump's 500 control loops.

The RTP Status Task **4230** may be the central repository for both the state and the status of the various tasks running on the RTP **3500.** The RTP Status Task **4230** may distribute this information to both the IM **4360** running on the UIP **3600**, as well as to tasks on the RTP **3500** itself.

The RTP Status Task **4230** may also be charged with fluid accounting for the ongoing infusion. Pump starts and stops, as well as pumping progress may be reported to RTP Status **4230** by the Pump Control Task **4256.** The RTP Status Task **4230** may account for at least one of the following: total volume infused, primary volume delivered, primary VTBI (counted down), volume delivered and VTBI of a bolus while the bolus is in progress, and volume delivered and VTBI of a secondary infusion while the secondary infusion is in progress.

All alerts or alarms originating on the RTP **3500** may be funneled through the RTP Status Task **4230**, and subsequently passed up to the UIP **3600.**

While the unit is in operation, the program flash, and RAM memory may be continually tested by the Memory Checker Task **4240.** This test may be non-destructive. This test may be scheduled so that the entire memory space on the RTP **3500** is tested every few hours. Additional periodic checks may be scheduled under this task if needed.

Tasks running on the RTP **3500** may be required to communicate with each other as well as to tasks that are executing on the UIP **3600.**

The RTP **3500** messaging system may use a unified global addressing scheme to allow messages to be passed to any task in the system. Local messages may be passed in memory utilizing the facilities of the RTOS' message passing, with off-chip messages routed over the asynchronous serial link **3601** by the InterComm Task **4210.**

The InterComm Task **4210** may manage the RTP **3500** side of the serial link **3601** between the two processors. The InterComm Task **4210** is the RTP **3500** equivalent of the InterComm Process **4310** on the UIP **3600.** Messages received from the UIP **3600** may be relayed to their destination on the RTP **3500.** Outbound messages may be forwarded to InterComm Process **4310** on the UIP **3600.**

All messages between the RTP **3500** and the UIP **3600** may be checked for data corruption using an error-detecting code (32 bit CRC). Messages sent over the serial link **3601** may be re-sent if corruption is detected. This provides a communications system that is reasonably tolerant to ESD. Corrupted messages within the processor between processes may be handled as a hard system failure. All of the message payloads used with the messaging system may be data classes derived from a common baseclass (MessageBase) to assure consistency across all possible message destinations.

Brushless Motor Control IRQ **4262** may not run as a task; it may be implemented as a strict foreground (interrupt context) process. Interrupts are generated from the commutator or hall sensors **3436**, and the commutation algorithm may be run entirely in the interrupt service routine.

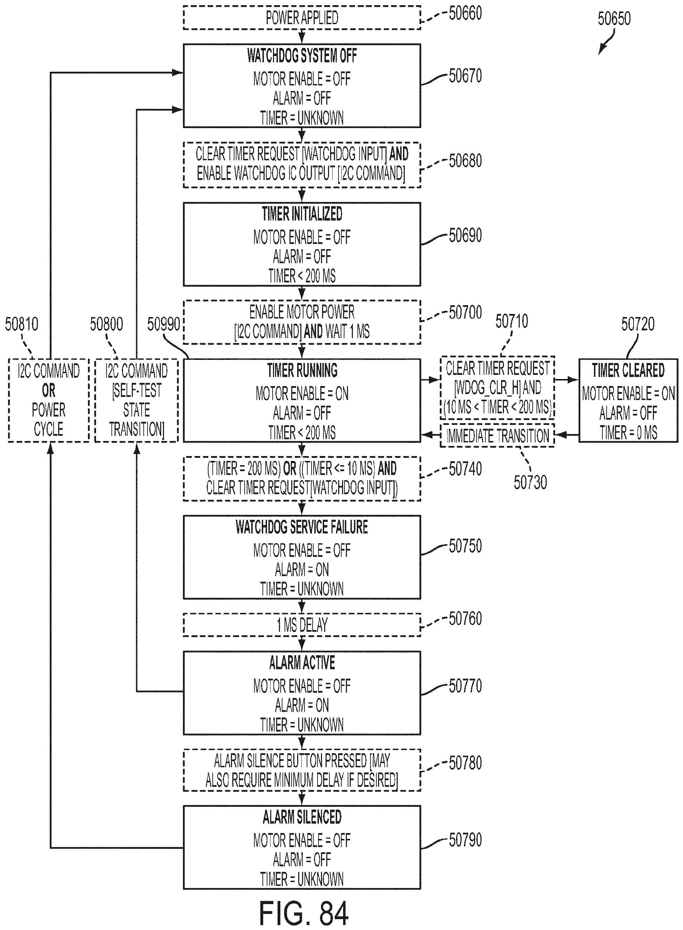

Fig. 84 shows a state diagram illustrating a method 50650 of providing a watchdog functionality in accordance with an embodiment of the present disclosure. The method 50650 is shown as a state diagram and includes states, 50670, 50690, 50990, 50720, 50750, 50770 and 50790, and transition conditions 50660, 50680, 50700, 50710, 50730, 50740, 50760, 50780, 50800, and 50810.

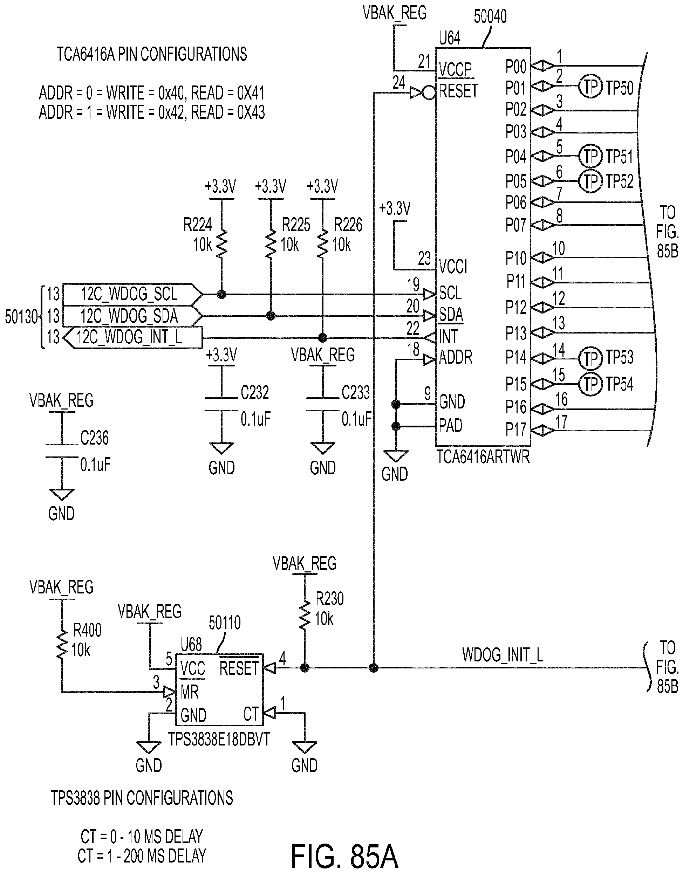

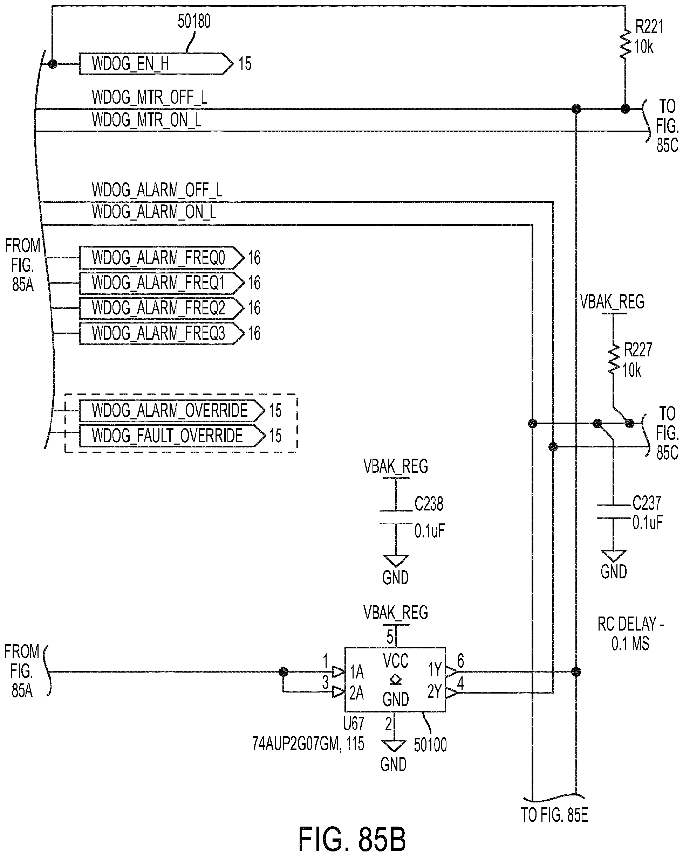

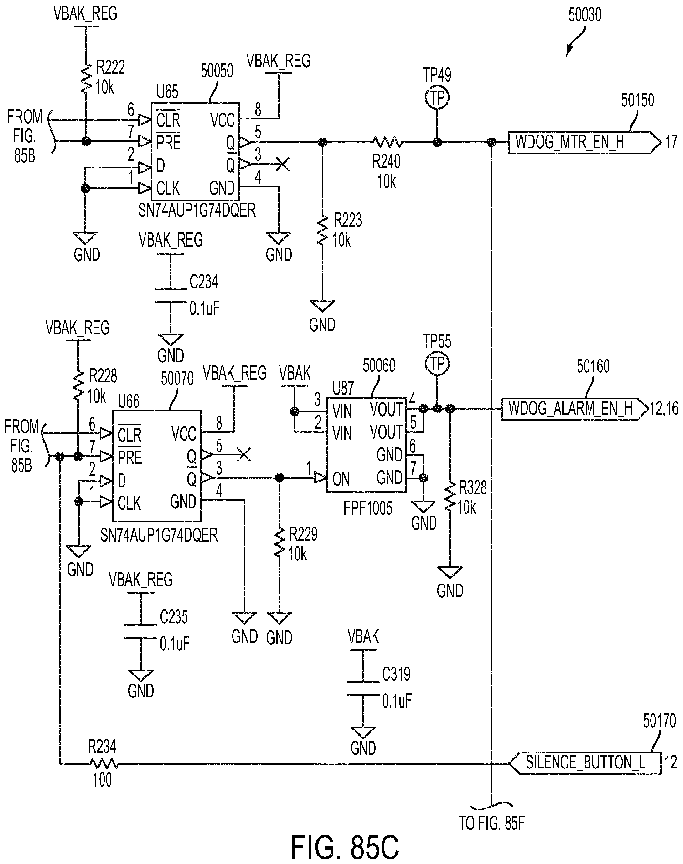

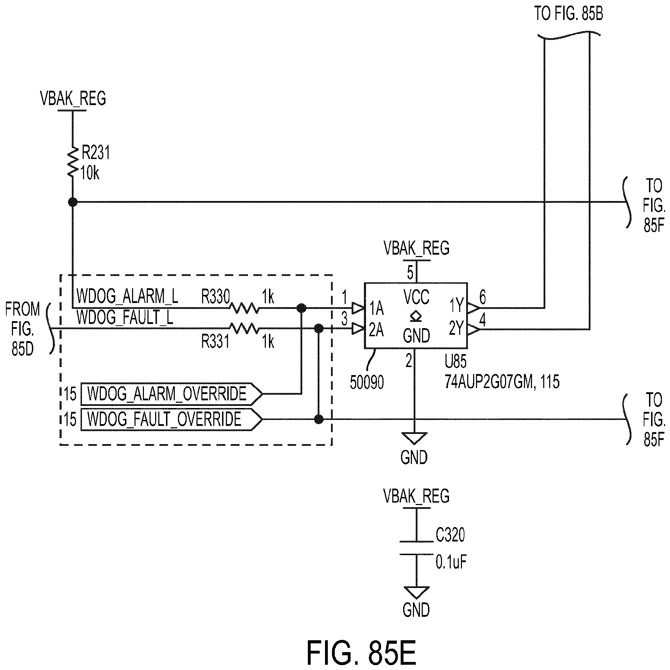

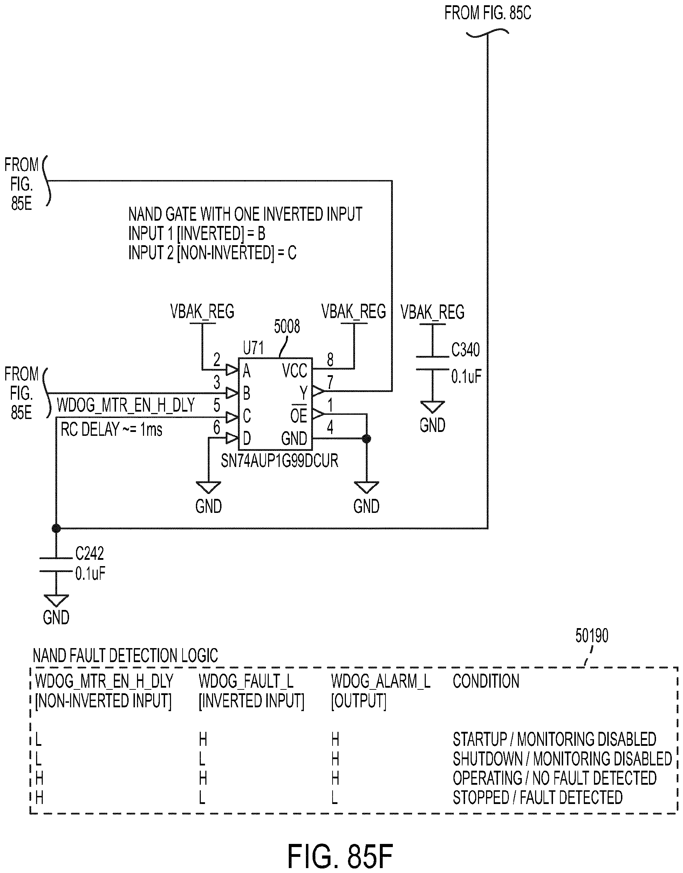

The method 50650 may be implemented by software, hardware, software in execution, or some combination thereof (e.g., as a hardware watchdog system). The method 5065 may be implemented by the watchdog 3460 of Fig. 59J such that it provides a motor enable signal to the motor controller 3431. Figs. 85A-85F show one specific embodiment of a system that implements the method 50650 of Fig. 84.

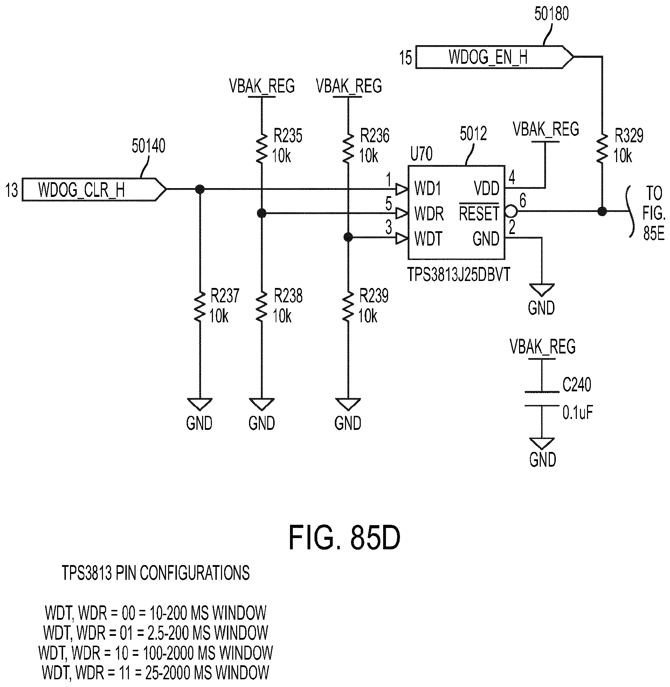

Refer now to Figs. 84, and 85A-85F. When the power is supplied to the watchdog system (e.g., system 50030), the method 50650 transitions 50660 to the watchdog system off state 50670 where the motor enable signal is off (e.g., line 50150), the alarm is off (e.g., line 50160), and the timer is in an unknown state. The timer may be part of the watchdog IC 50120. The watchdog IC 50120 is a window watchdog. The system 50030 also includes an I2C control lines 50130 that interface with an I/O expander 50040 (or other hardware latches). The I2C control lines 50130 may be part of the connections from the RTP 35000 to the watchdog 3460 of Fig. 59J. Additionally, a watchdog clear signal (line 50140 of Fig. 85D) may also be received from the RTP 35000 to the watchdog 34600. That is, the watchdog clear line 50140 "pets" the watchdog IC 50120.

In transition 50680, the RTP 3500 (see Fig. 59J) clears the watchdog IC's 50120 timer via the watchdog clear line 50140 and the RTP 35000 enables the watchdog IC's 50120 output via the I2C control lines 50130 by instructing the I/O expander 50040 to enable a watchdog enable line 50180. This causes the method 50650 to enter into the state 50690. In state 50690, the timer is initialized (set to zero), the motor enable line 50150 is set to off and the alarm line 50160 is set to off.

The RTP 3500 enables the motor power via the I2C control lines 50130 by setting the D-flip-flop to true (using the preset pin of a D-flip-flop 50050) and pauses for 1ms in transition 50700. The method 50650 transitions to state 50990 where the watchdog IC's 5012 timer is running, the motor enable line 50150 is enabled, and the timer is less than 200 milliseconds. If the RTP 3500 sets the watchdog clear line 50140 when the watchdog is greater than 10 milliseconds and less than 200 milliseconds, the transition 50710 transitions the method 50650 to state 50720 wherein the timer is reset. The method 50650 will transition back to state 50990.

If the timer reaches 200 milliseconds or the timer is less than or equal to 10 milliseconds and the RTP 3500 sets the watchdog clear line 50140, transition 50740 transitions the method to state 50750. In state 50750, the watchdog IC 50120 sends out a fault signal that is buffered by a buffer 50090 which clears the D-flip-flop 50050 thereby turning the motor line 50150 off. In state 50750, the watchdog IC 50120 also sends out the fault signal which is received by a NAND gate 50080 via an inverted input, which outputs a signal to a logic buffer 50090 which clears a D-flip-flip 50070 and thereby turns on the a alarm line 50160. The output of the D-flip-flop 50070 is amplified by a load switch 50060.

When the motor enable signal line 50150 is set to turn the motor off, the off signal propagates through the non-inverting input of the NAND gate 50080 after about 1 millisecond, which causes the transition 50760 to transition to state 50770 thereby allowing the alarm to be disabled. An I2C command may cause transition 50800 to reset the system 50030 back to state 50670.

Otherwise, the alarm line 50160 will continue to alarm until a silence button 50170 is pressed which is coupled to the preset of the D-flip-flop 50070 to set the alarm line 50160 to off. That is, the button will cause the transition 50780 to transition the method 50650 to state 50790. An I2C signal via the I2C control lines 50140 to the IO expander 50040 may cause the method 50650 to transition to state 50670.

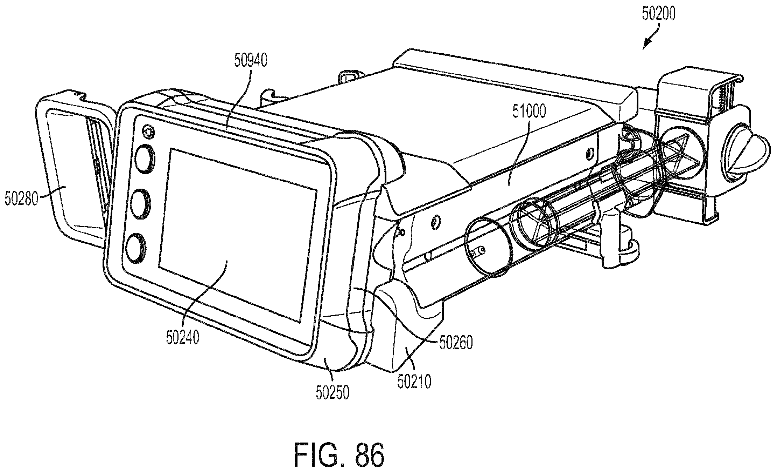

Fig. 86 shows another embodiment of syringe pump 50200 having a bumper 50210 in accordance with an embodiment of the present disclosure. The pump 50200 may couple to a pole via the clamp 50280. The pump 50200 includes a syringe seat 51000 that accommodates a bumper 50210.

The pump 50200 also includes a touchscreen 50240 coupled to the pump 50200 via an outer periphery 50250. The outer periphery 50250 includes an indicator light 50260. The indicator light 50260 may wholly wrap around the touchscreen 50240. The indicator light 50260 may include a diffuser wrapped around the touchscreen 50240 with a plurality of LED lights embedded therein (or optically coupled thereto). The indicator light 50260 may blink when the pump 50200 is running and/or it may be a specific color when the pump is running (e.g., red, blue, green, yellow, etc.). The indicator light 50260 may be continuously on when the pump 50200 is not running or is in a standby state. Additionally, alternatively, or optionally, the indicator light 50260 may be a specific color when the pump is not running or is in a standby state (e.g., red, blue, green, yellow, etc.).

The pump 50200 may also include a gesture-recognition apparatus 50940, which may be a camera. A processor of the pump 50200 may be coupled to the gesture-recognition apparatus 50940 to receive user input from a gesture by a user. That is, the processor may be configured to present a user with at least one option via the user interface 50240 and receive a selected one of the at least one option via the gesture-recognition apparatus 50940. The processor coupled to the user interface 50240 may be configured provide a plurality of pump parameter inputs where each of the plurality of pump parameter inputs is configured to receive a user inputted parameter. The processor may be configured to determine whether all of the user inputted parameters of all of the plurality of pump parameters meets at least one predetermined safety criterion. Each of the plurality of pump parameter inputs may be present without another one of the plurality of pump parameters inputs.

The processor may be configured to provide a plurality of pump parameter inputs where each of the plurality of pump parameter inputs is configured to receive a user inputted parameter. The processor may be configured to require that all of the plurality of pump parameter inputs are inputted within a predetermined amount of time. The processor may be configured to receive a corresponding user inputted parameter for the plurality of pump parameter inputs in any order.

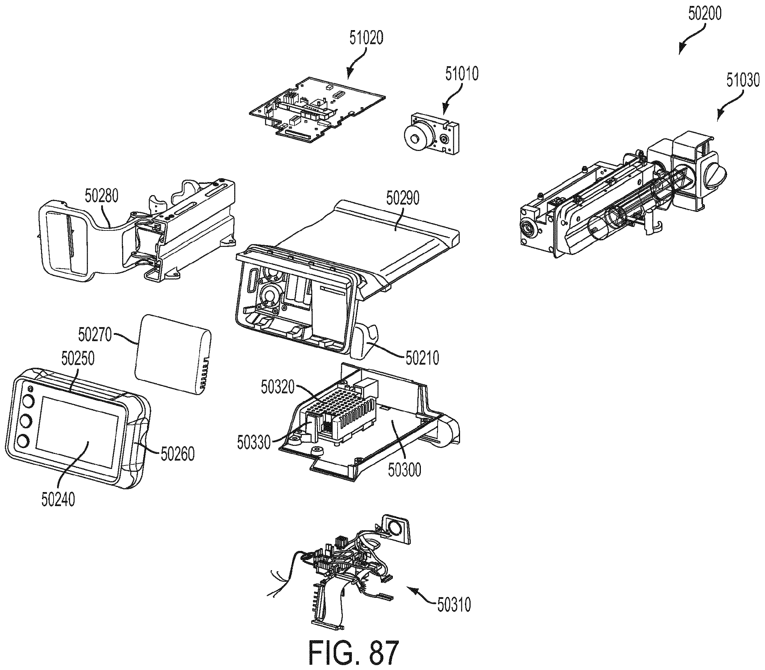

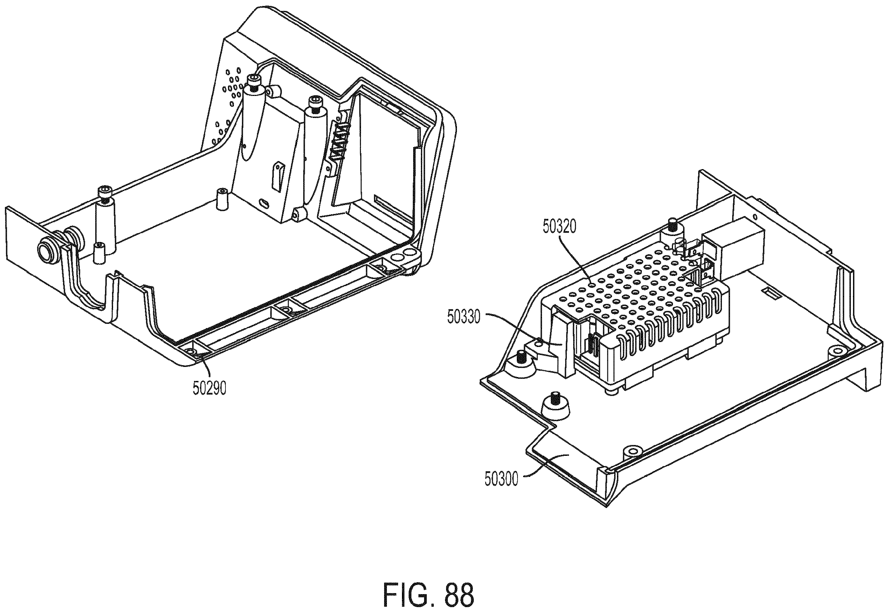

Fig. 87 shows an exploded view of the syringe pump 50200 of Fig. 86 in accordance with an embodiment of the present disclosure. The pump 50200 includes an upper housing portion 50290 and a lower portion housing 50300. Additionally or alternatively, the upper portion 50290 and the lower portion 50300 of the housing 50290, 50300 may be unitarily formed in some specific embodiments. A modular syringe pumping mechanism 51030 may be coupled to the housing 50290, 50300. A motor 51010 actuates the modular syringe pumping mechanism 51030. The motor 51010 may be controlled via a circuit board 51020 that is coupled to the motor 51010 and to various sensors, actuators, the touchscreen 50240, etc. The pump 50200 also includes cabling 50310 and a battery 50270 disposed behind the touchscreen 50240 (when assembled). Fig. 88 shows a close-up view of the upper housing 50290, the lower housing 50300, and the power supply 50320. Note how the power supply 50320 is thermally coupled to the lower housing portion 50600 via the conductive path 50330.

The pump 50200 includes a power supply 50320. The power supply 50320 is coupled to a conductive path 50330 to the housing 50300, 50290 (when assembled). The conductive path 50330 may be a piece of metal and may be unitarily formed with the housing 50300 (or 50290). The power supply 50320 may use the housing 50290, 50300 as a heat sink. The power supply 50320 may use any surface of the housing 50290, 50300 so that it is thermally coupled thereto and/or may be thermally coupled to the housing 50290, 50300 via the thermally conductive path 50330.

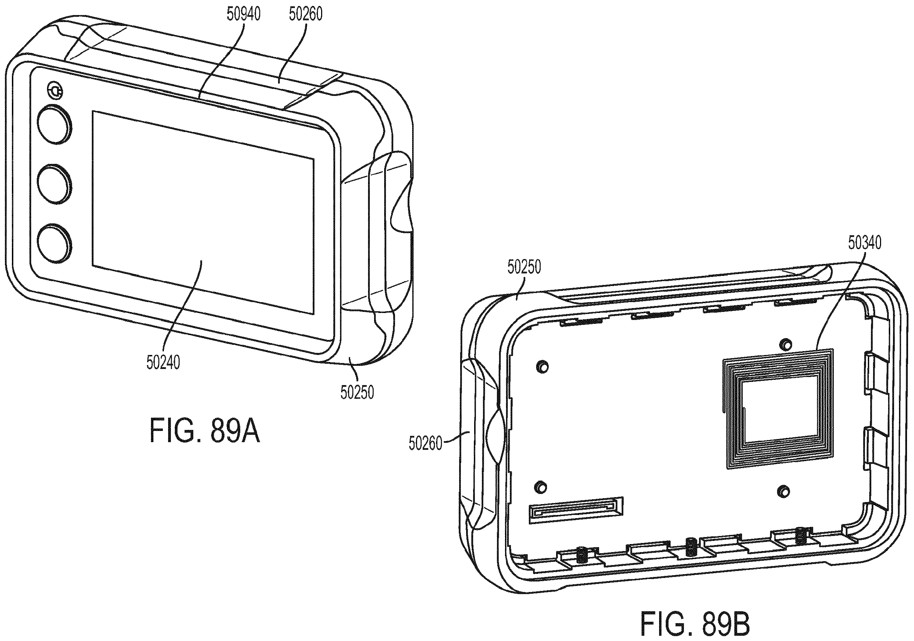

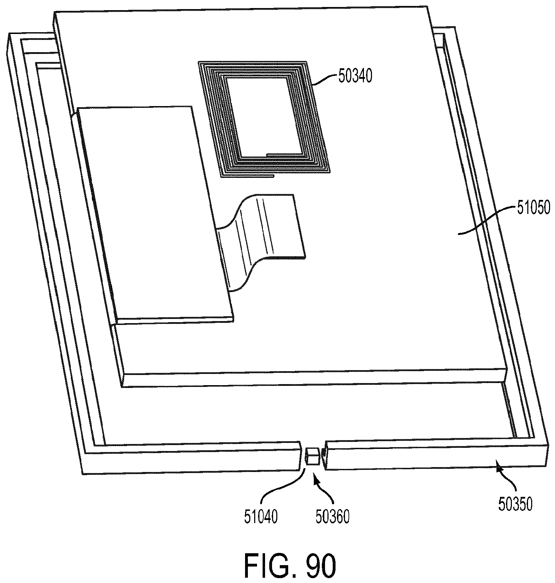

Fig. 89A shows a front view of the display of the pump 50200 and Fig. 89B shows a back view of the display of the pump 50200 in accordance with an embodiment of the present disclosure. On the back of the touchscreen 50240 (seen easily in Fig. 89B) a near-field antenna 50340 is disposed. Fig. 90 shows the sensor portion 51050 of the touchscreen with the near-filed antenna 50340 disposed adjacent to the backside of the sensor portion 51050 of the touchscreen 50240 (see Figs. 89A-89B). A frame 50350 is shown that forms a loop of metal with a gap 51040 having a dielectric 50360 disposed within the gap 51040. The frame 50350 may be a frame of the sensor 51050 and/or the touchscreen 50240. The antenna 50340 may operate at 13.56 Megahertz and/or may be an NFC antenna. The metal frame 50350 in conjunction with the gap 51040 and the dielectric 50260 disposed within the gap may form a split-ring resonator. The metal frame 50350 forms an inductive element of the split-ring resonator, and the gap 50140 with the dielectric 50360 disposed therein form a capacitive element of the split-ring resonator.

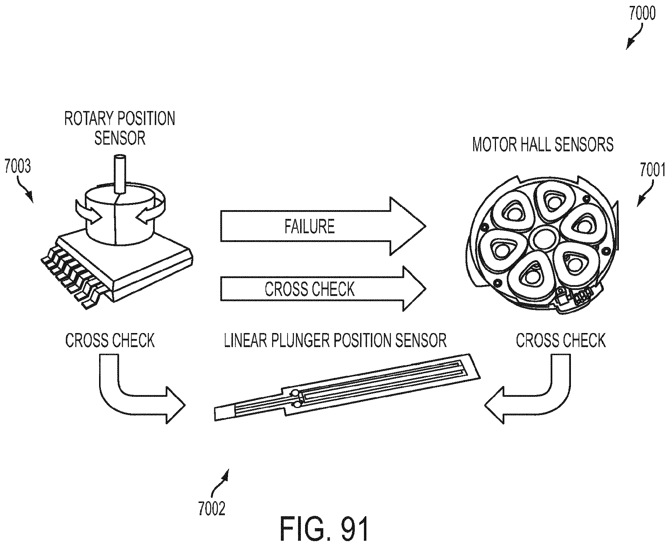

Fig. 91 shows a chart diagram illustrating the use of the sensors of the pump of Fig. 86 when one or more of the sensors are unavailable in accordance with an embodiment of the present disclosure. Fig. 91 shows sensors 7001, 7002, and 7003. The rotary position sensor 7003 may be the rotary sensor 1202 of Figs. 59J and 60 (e.g., an encoder). The motor hall sensors 7001 may be the Hall Sensors 3436 on the motor 1200 of Fig. 59J and 60. The linear plunger position sensor 7002 may, for example, be the linear sensor 3950 of Fig. 59B or the linear position sensor 1100 as shown in Fig. 57B.

Fig. 91 may be implemented as a method of using feedback sensors of a syringe pump 50206. The RTP 3500 of Fig. 59J may receive signals from the sensors 7001, 7002, 7003.

The RTP 3500 may cross-check the position of the sliding bock assembly 800 using all three sensors 7001, 7002, and 7003 relative to each other. The RTP 3500 may cross check the rotary position sensor 7003 with the motor hall sensors 7001, and if they are out of agreement by a predetermined amount, the RTP 3500 will compare them to the linear plunger position sensor 7002 to determine which one of the sensors 7001 and 7003 is operating properly. Thereafter, the RTP 3500 will use the properly operating one of the sensors 7001 and 7003. If the rotary position sensor 7003 is unavailable, the RTP 3500 will use the motor hall sensors 7001. The RTP 3500 may also cross check the rotary position sensor 5042 with the motor hall sensors 5043.

If it is determined that both of the motor hall sensors 7001 and the rotary position sensor 7003 are inoperative, the RTP 3500 may use only the linear plunger position sensor 7002.

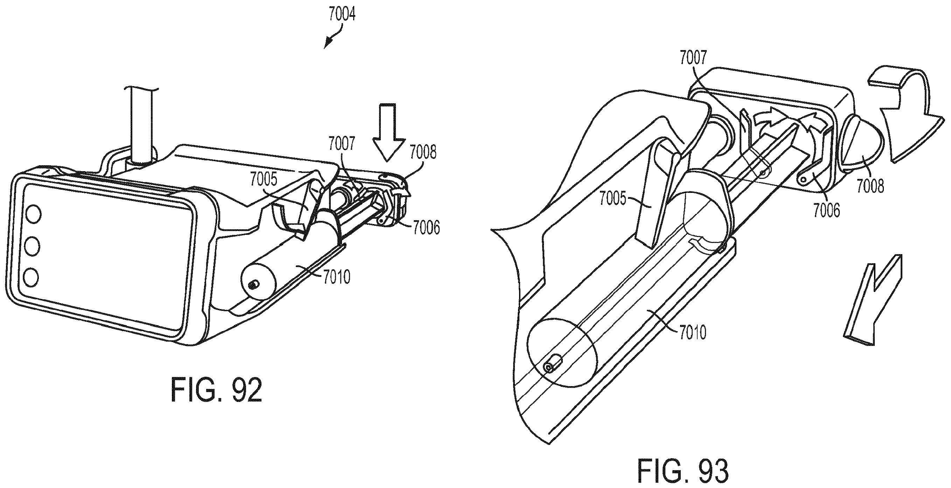

Fig. 92 shows a side view of a syringe pump 7004 having a retaining finger 7005 to retain a syringe and Fig. 93 shows a close-up, partial view of the syringe pump 7004 of Fig. 92 in accordance with an embodiment of the present disclosure. The end of the syringe 7010 may be retained by pivotal jaw members 7006, and 7007. The pivotal jaw members 7006 and 7007 may include bends as shown. The dial 7008 may be operatively coupled to the pivotal jaw members 7006 and 7007 to cause them to pivot. The dial 7008 may be biased to rotate the dial 7008 to cause the pivotal jaw members 7006 and 7007 to rotate toward each other or to rotate away from each other.

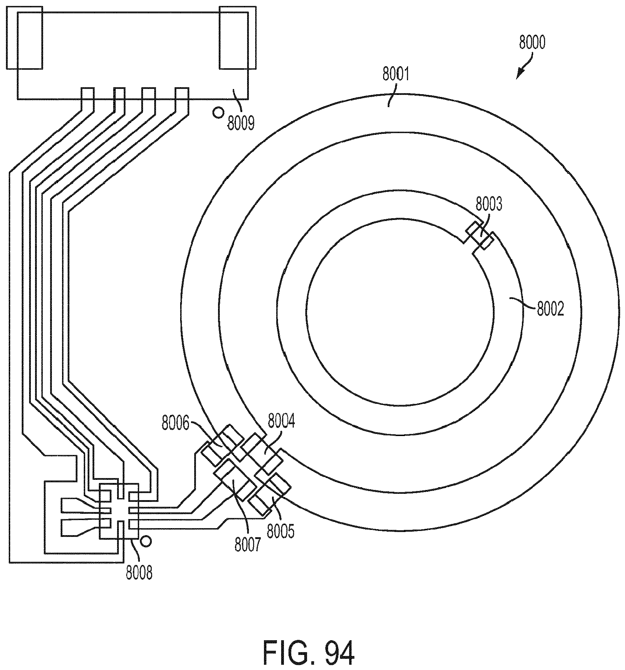

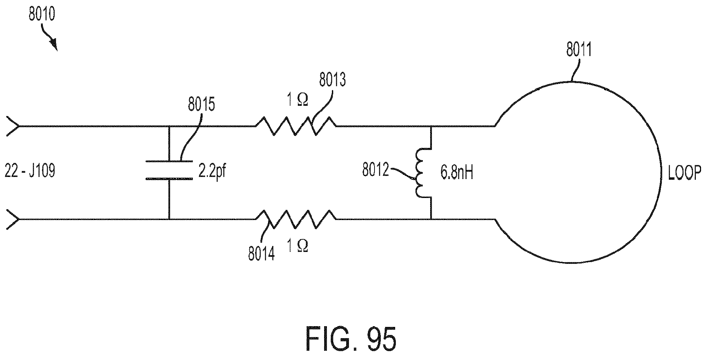

Fig. 94 shows a circuit 8000 for storing data within an RFID tag 8008 associated with an syringe pump (e.g., the syringe pump 500 of Fig. 29, the syringe pump 50200 of Fig. 86, or any other syringe pump) in accordance with an embodiment of the present disclosure. The RFID tag 8009 of Fig. 94 may be the RFID tag 3670 of Fig. 95E. The antenna 8001 of Fig. 94 may be the antenna 3955 of Fig. 59E.

The antenna 8001 is coupled to an RFID tag 8008 such that an RFID reader (i.e., RFID interrogator) can communicate with the RFID tag 8008. The circuit 8000 may be placed on a 1x1 PCB inch board with a solid-metal ground plane of the back side.

An inner loop 8002 with a capacitor 8003 may form a split-ring resonator to enhance the read range capability of the circuit 8000. The RFID tag 8008 may be coupled to the antenna 8001 via an impedance matching network 8004, 8005, 8006, 8007. The circuit 8000 may be configured for use with a 900 Megahertz RFID reader.

A reader chip 8009 may interface with the RFID tag 8008 to write data (e.g., log data) thereto. The reader chip 8009 may communicate with the RFID tag 8008 using I2C, a CAN bus, or other communications link. Alternatively, 8009 may be an electrical connector, in some embodiments.

Fig. 95 shows an equivalent circuit 8010 for impedance as seen from the RFID tag 8008 of Fig. 94 in accordance with an embodiment of the present disclosure. A loop 8011 shows the antenna 8001 of Fig. 94. The inductor 8012 shows the inductor 8004 of Fig. 94. The resistors 8013 and 8014 are schematic representations of the resistors 8006 and 8005, respectively. The capacitor 8015 shows the capacitor 8007 of Fig. 94. The circuit elements 8012-8015 are used for impedance matching so that the RFID tag 8008 is efficiently coupled to the loop antenna 8001 such as in the circuit 8000 of Fig. 94.

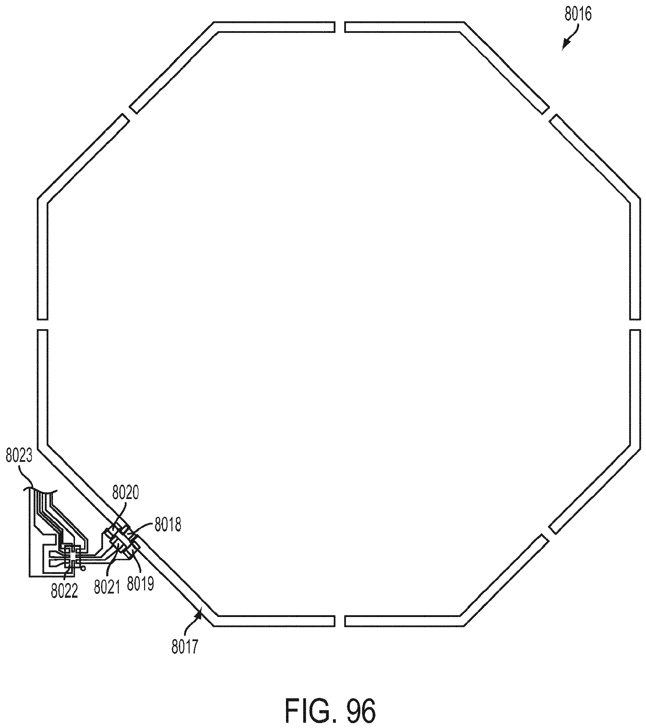

Fig. 96 shows another circuit 8016 for storing data within an RFID tag 8022 associated with an infusion pump (e.g., the syringe pump 500 of Fig. 29, the syringe pump 50200 of Fig. 86, or any other syringe pump) in accordance with an embodiment of the present disclosure. The antenna 8017 is shown. The RFID tag 8022 of Fig. 96 may be the RFID tag 3670 of Fig. 95E. The antenna 8017 of Fig. 96 may be the antenna 3955 of Fig. 59E.

The antenna 8017 may have capacitors coupled to the gaps in the antenna 8017, in some embodiments. An impedance matching network 8018, 8020, 8021 may be used to efficiently couple the RFID tag 8022 to the antenna 8017. An interface 8023 may be used to communicate with the RFID tag 8022 (e.g., an I2C interface, a CAN interface, etc.).

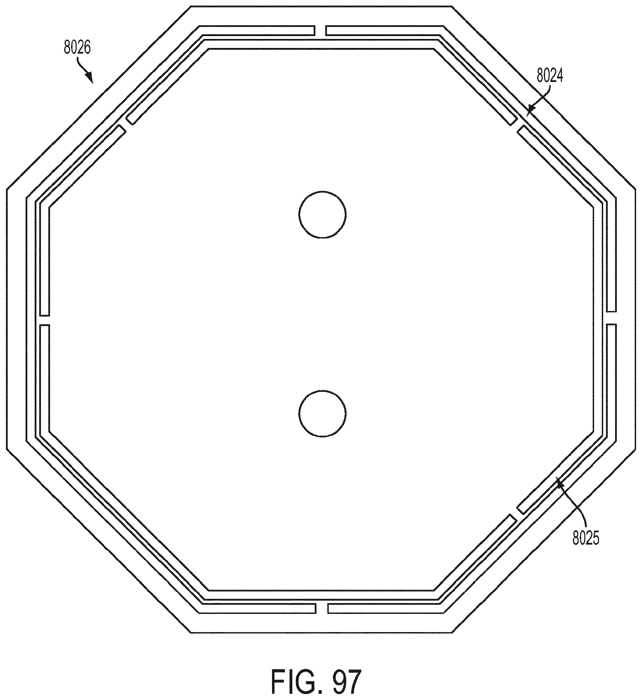

Fig. 97 shows a split-ring resonator 8026 used with the circuit 8016 of Fig. 96 in accordance with an embodiment of the present disclosure. The split-ring resonator 8026 may be printed on a PCB board with an inner loop 8025 and an outer loop 8024. The split-ring resonator 8026 may be placed adjacent to the circuit 8016 of Fig. 96 to enhance its read range (e.g., the two planes defined by the two circuit's PCB boards may be parallel to each other).

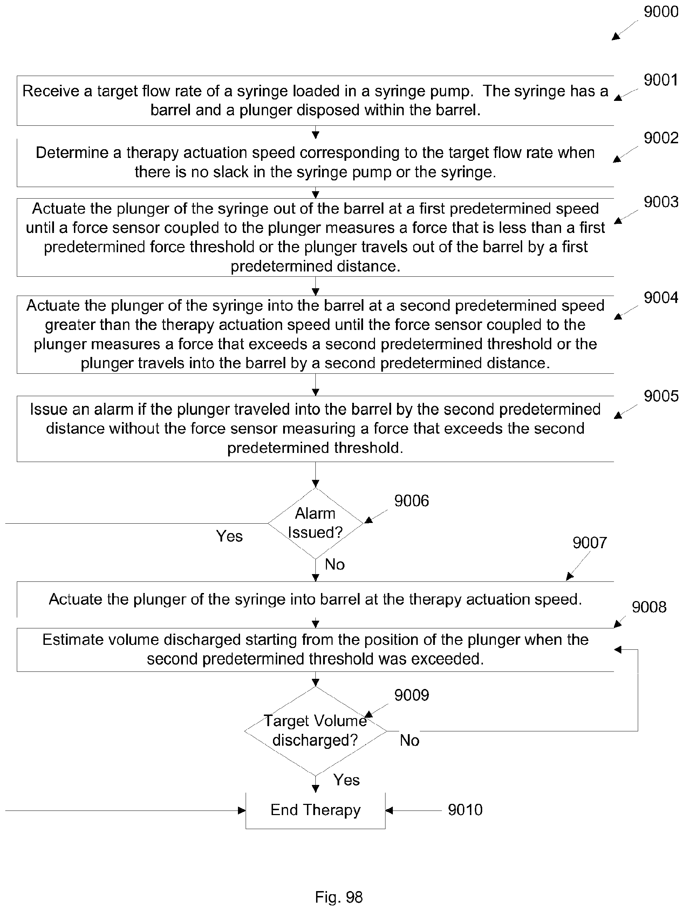

Fig. 98 shows a flow chart diagram illustrating a method 9000 for removing the effects of slack in a syringe pump (e.g., the syringe pump 500 of Fig. 29, the syringe pump 50200 of Fig. 86, or any other syringe pump) having a syringe loaded on the syringe pump in accordance with an embodiment of the present disclosure. The Method 9000 includes acts 9001-9010 including two decision acts 9006 and 9009.

Act 9001 receives a target flow rate of a syringe loaded in a syringe pump. The syringe has a barrel and a plunger disposed within the barrel. Act 9002 determines a therapy actuation speed corresponding to the target flow rate when there is no slack in the syringe pump or the syringe. Act 9003 actuates the plunger of the syringe out of the barrel at a first predetermined speed until a force sensor coupled to the plunger measures a force that is less than a first predetermined force threshold or the plunger travels out of the barrel by a first predetermined distance. Act 9004 actuates the plunger of the syringe into the barrel at a second predetermined speed greater than the therapy actuation speed until the force sensor coupled to the plunger measures a force that exceeds a second predetermined threshold or the plunger travels into the barrel by a second predetermined distance. Act 9005 issues an alarm if the plunger traveled into the barrel by the second predetermined distance without the force sensor measuring a force that exceeds the second predetermined threshold. If an alarm is issued in act 9005, act 9006 branches the method 9000 to end the therapy 9010. Act 9007 actuates the plunger of the syringe into the barrel at the therapy actuation speed. Act 9008 estimates volume discharged starting from the position of the plunger when the second predetermined threshold was exceeded. Act 9009 will repeat act 9008 until the target volume is discharged, after which case, act 9009 will end the therapy 9010.

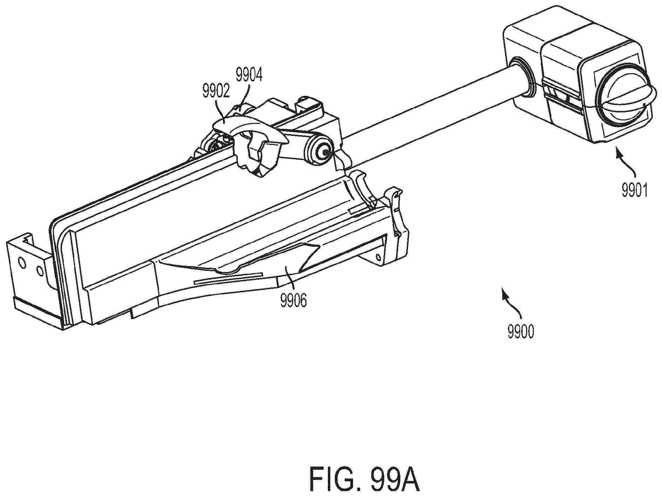

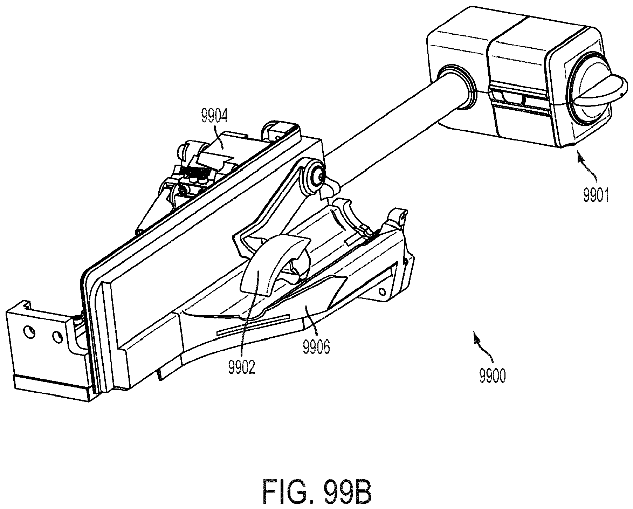

Figs. 99A-99B show an apparatus 9900 for side loading a syringe on an infusion pump in accordance with an embodiment of the present disclosure. Fig. 99A shows the apparatus 9900 with a securing arm 9902 in a loading position while Fig. 99B shows the apparatus 9900 with the securing arm 9902 in a securing position. The apparatus 9900 as shown in Figs. 99A-99B, in addition to the securing arm 9902, includes a platform (also referred to as a syringe seat) 9906 and a force mechanism 9904 to securely hold a syringe. A plunger head assembly 9901 may be coupled to a syringe to discharge the fluid within the syringe (the syringe is not shown in Figs. 99A-99B) into a patient.

The force mechanism 9904 imparts a rotational force on the securing arm 9902 driving it towards the platform 9906. When a syringe is placed on the platform 9906, the securing arm 9902 engages the syringe with enough force to securely hold it in place during operation of the pump. Syringe pumps using smaller syringes may need about one pound of force applied to the syringe to secure it, while larger syringes may need about three pounds of force applied thereto. The force mechanism 9904 may be capable of locking in an up position as shown in Fig 99A allowing a pump operator to easily position the syringe on the platform 9906 before securing the syringe with the securing arm 9902. The up position may be referred to as the loading position because moving the securing arm 9902 away from the platform 9906 facilitates loading of the syringe onto the platform 9906.

The securing arm 9902 may be designed to allow sufficient viewing of the syringe. In some embodiments of the present disclosure, the securing arm 9902 may be configured to be substantially contiguous with the pump casing and only cover the syringe at the point of contact between the securing arm 9902 and the syringe. A wire structure may also be added to the engaging portion of the securing arm 9902 holding the bulk of the securing arm 9902 arm away from the syringe leaving only a relatively thin wire contacting the syringe. Other arrangements in which the securing arm 9902 is fashioned to minimally obscure a syringe may also be used.

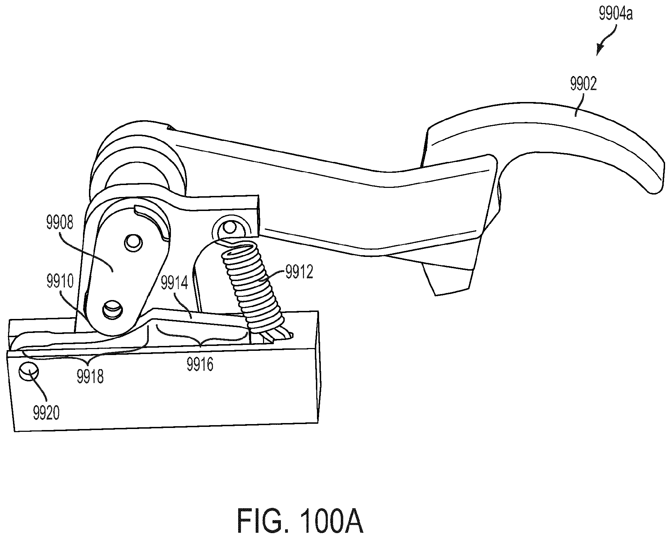

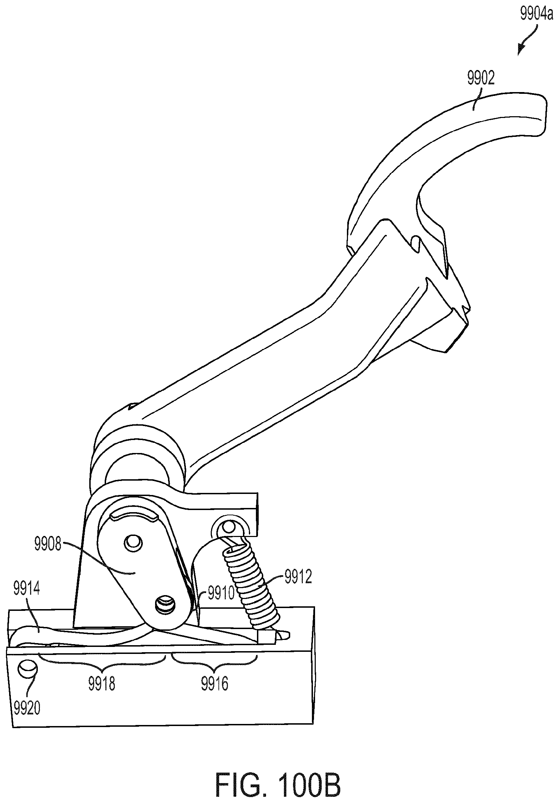

Figs. 100A-100B show an embodiment of a force mechanism used with the apparatus described in Figs. 99A-99B or similar apparatuses. The embodiment shown in Figs. 100A-100B includes a secondary arm (hereinafter also referred to as a second arm) 9908, a roller 9910, an engaging plate 9914, and a bias member or spring 9912. The second arm 9908 is connected to the securing arm's 9902 axis of rotation and is laterally removed from the securing arm 9902 in order to position it over the engaging plate 9914. A roller 9910 is attached to the secondary arm 9908 on the end opposite the axis of rotations and extends past the end of the secondary arm 9908, so only the roller 9910 engages the engaging plate 9914. The engaging plate 9914 is positioned to be engaged by the roller 9910. One end of the plate 9914 is secured by a pivot 9920 and the other end is connected to a spring 9912 that pulls the plate 9914 towards the roller 9910 on the secondary arm 9908. The engaging face of the engaging plate 9914 is angled with respect to the secondary arm 9908 which creates a rotational force in the secondary arm 9908 when the plate 9914 is urged towards the secondary arm 9908. The rotational force from the second arm 9908 is transferred to the securing arm 9902 which results in the force securing the syringe. The engaging face of the engaging plate 9914 may also define a peak having a first side 9918 oriented to cause a rotational force in the engaged secondary arm 9908 and a second side 9916 which locks the secondary arm 9908 in a position where the securing arm 9902 is removed from the platform 9906 and a syringe that may be on the platform 9906 (see Figs. 99A-99B) thereby keeping the securing arm 9902 in a loading position to load the syringe (shown in Fig. 100B).

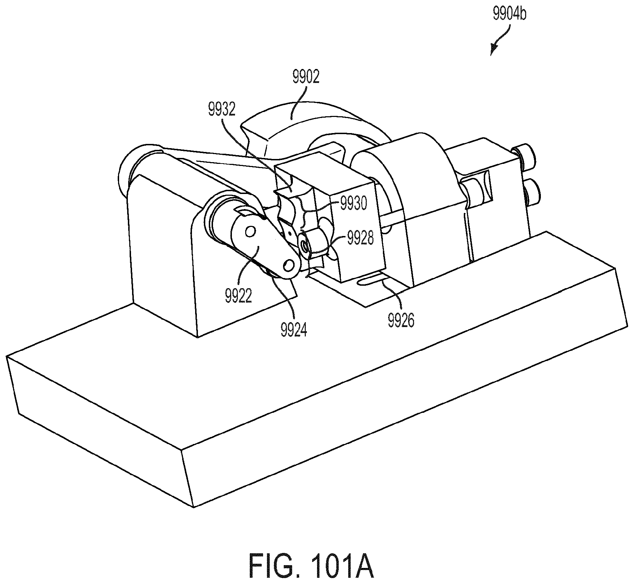

Figs. 101A-101B show another embodiment of a force mechanism used with the apparatus described in Figs. 99A-99B or similar apparatuses. The engaging plate 9932 is not hinged at one end, it is on a track 9926. The engaging plate 9932 may be spring biased toward a secondary arm 9922. The track 9926 guides the engaging plate 9932 toward the secondary arm 9922 and allows for linear movement instead of rotational movement. Having the engaging plate 9932 on the track 9926 does not result in a drop in the moment arm. The decreasing moment arm means that a stiffer spring may be used to create the force output at the securing arm 9902.

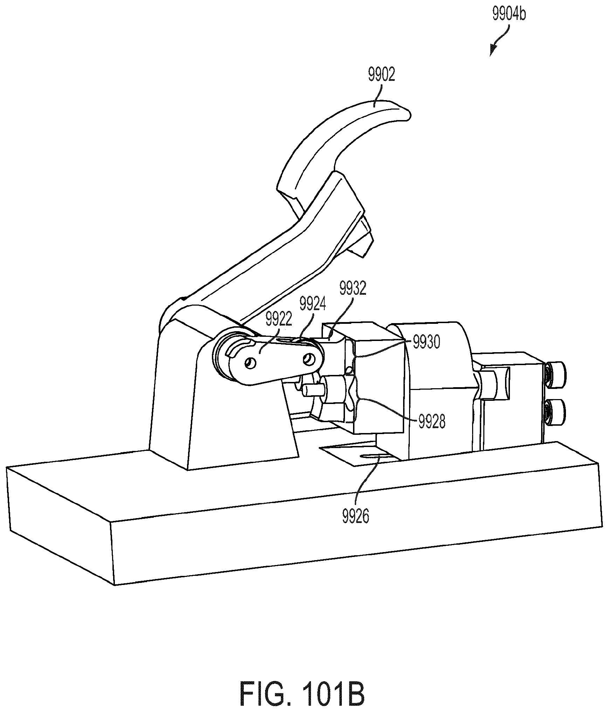

A spring urges the engaging plate 9932 towards a roller 9924 on the secondary arm 9922. The engaging face of the engaging plate 9932 is shaped to impart a rotational force on the secondary arm 9922 which transfers the rotational force to the connected securing arm 9902. A peak on the engaging surface of the plate 9932 may define a parked section 9930 and a section causing the rotational force 9928. The securing arm 9902 is shown in a securing position in Fig. 101A and in the loading position in Fig 101B.

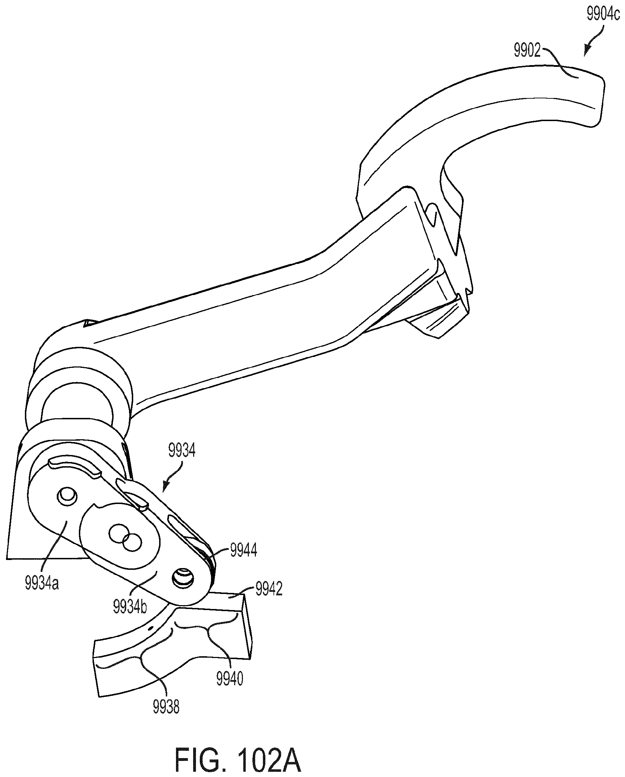

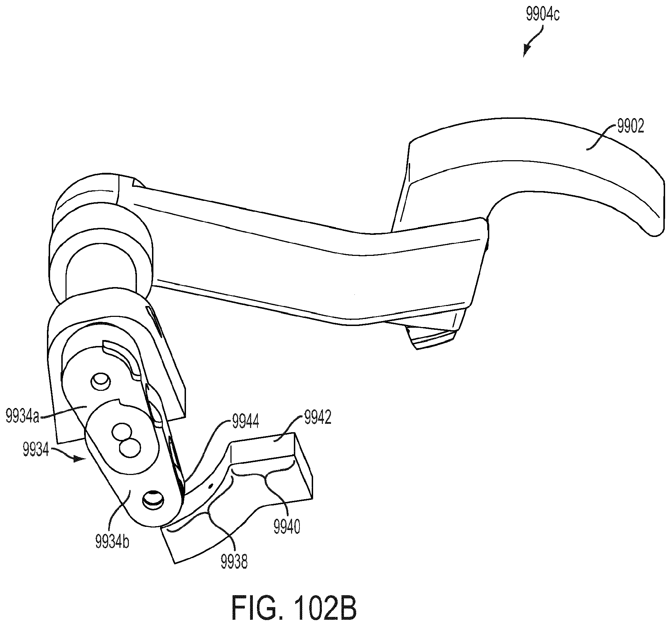

Figs. 102A-102B show yet another embodiment of a force mechanism that may be used with the apparatus described in Figs. 99A-B or similar apparatuses. In the embodiment 9904c shown in Figs. 102A-102B, an engaging plate 9942 is fixed and a secondary arm 9934 telescopes when rotated because of the variable surface of the plate 9942. The secondary arm 9934 is made up of two components, including: a first component 9934a connected to the securing arm 9902 at its axis of rotation; and a second component 9934b that telescopes on the first component 9934a. A spring positioned between the components 9934a, 9934b forces the two away from each other. A roller 9944 is attached to the end of the second component 9934b to engage the engaging plate 9942. The engaging plate 9942 is positioned to be engaged by the secondary arm 9934 and compress the spring located between the two secondary arm components 9934a, 9934b as the secondary arm 9934 is rotated. A section 9940 of the plate 9942 locks the mechanism in a position where the securing arm 9902 is removed from the syringe (i.e., a loading position) and rotation of the securing arm moves the secondary arm 9934 to the section 9938 of the plate that imparts a rotational force on the arm (i.e., to rotate the securing arm 9902 to a securing position). The loading position of the securing arm 9902 is illustrated in Fig. 102A and the securing position of the securing arm 9902 is illustrated in Fig. 102B.

In yet additional embodiments, the secondary arm can be laterally located anywhere as long as it is connected to the securing arm. It may also be attached to the securing arm at a point other than the axis of rotation. In the embodiments described herein, the location of the engaging plates and angles of the securing arm in the figures are just examples and may be oriented in any configuration to thereby provide the same or substantially the same function, result, configuration or aspect.

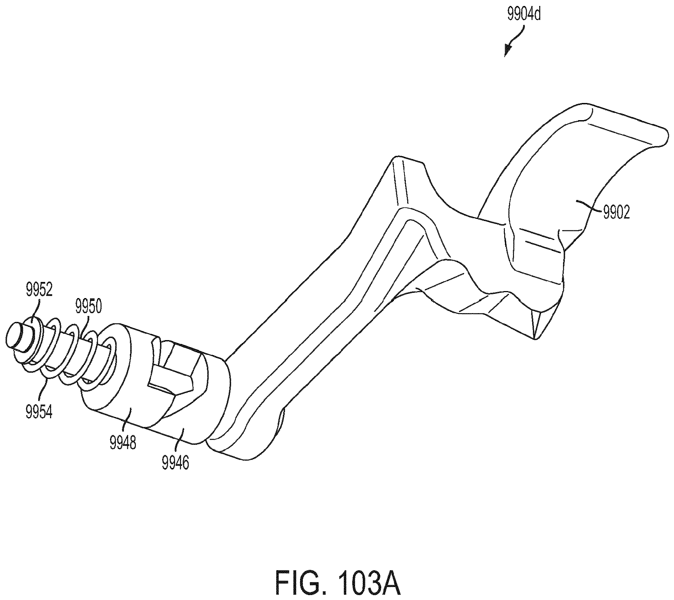

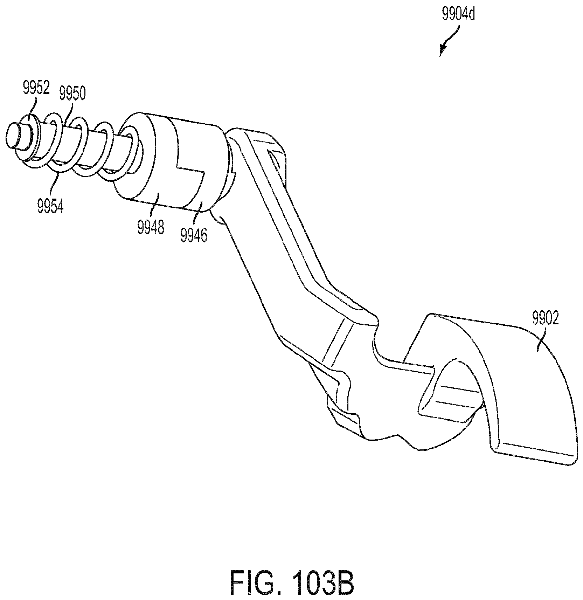

Figs. 103A-103B show yet another embodiment of a force mechanism 9904d for use with the apparatus described in Figs. 99A-B or similar apparatuses. The mechanism 9904d includes a shaft 9950, a first cam component 9946, a second cam component 9948, a spring 9954, and a backstop 9952. The shaft 9950 is pivotally connected to the securing arm 9902 and shares its axis of rotation. The first cam component 9946 is connected to the securing arm 9902 and is disposed around the shaft 9950 while having the capability to pivot with the securing arm 9902. The side of the first cam component 9946 facing the second cam component 9948 has a major planar portion, a portion set back from the planar portion, and a portion connecting the two with a taper. The second cam component 9948 is positioned immediately next to the first cam component 9946, and mirrors the shape of the first 9946 component allowing them to uniformly interlock to create a cylinder shape as shown in Fig. 103B. The second cam component 9948 is held at a constant rotational alignment, but has the ability to translate back and forth on the shaft 9950. The spring 9954 configured to urge the second cam component 9948 towards the first 9946 is disposed around the shaft 9950 between the second component 9948 and the backstop 9952. The parked position is shown in Fig 103A and the engaged position is shown in Fig 103B.

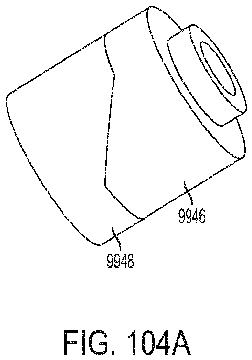

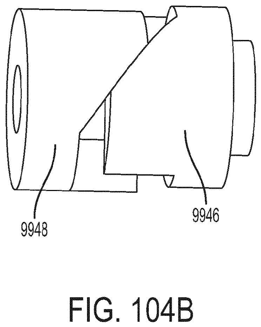

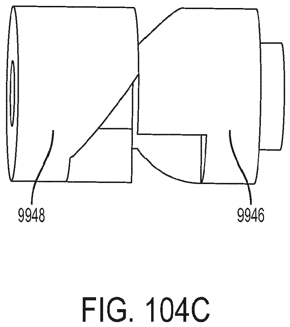

Figs. 104A-104C show the different positions of the cam components 9946, 9948. Fig. 104A is a depiction of the cam when the securing arm 9902 (see Fig. 103B) is in a down position. In this position the second cam component 9948 is at its furthest point away from the backstop 9952 (see Fig. 103B). Fig. 104B shows the cams 9946, 9948 when the securing arm 9902 is rotated. The tapered portions of both cams 9946, 9948 slide along each other, forcing the second cam component 9948 away from the first cam portion 9946 as the cams 9946, 9948 are rotated along the shaft 9950 (see Fig. 103B). The spring 9954 urges the second cam component 9948 towards the first 9946 which makes them want to slide back to the initial down position. This feature creates the rotational force causing the securing arm 9902 to push down on the syringe. Fig. 104C shows the cams 9946, 9948 when the securing arm 9902 is in the parked position. Once the securing arm 9902 is rotated to the point where the tapered parts are no longer in contact, the planar surfaces will contact which results in no rotational force caused by the spring 9954, therefore the securing arm 9902 will stay in place.

A sensor may be used to track the position or angle of the securing arm 9902. The sensor data can be used for multiple applications. The position of the sensor can be used to determine if the syringe is properly secured. This would be used in situations where the sensor already knew what type or at least what size diameter syringe is being used and what angle the securing arm 9902 or secondary arm should be at when secured. The sensor may also be used to determine one or more characteristic of a syringe, for example, what size or even what specific model of syringe is being used. By determining what syringe is being used the pump can calculate flow rate relative to plunger displacement. Data from a sensor on the mechanism that drives the plunger of the syringe may be used in conjunction with the securing arm sensor data to determine the model of syringe being used. The sensor to determine position of the securing arm 9902 may be a Hall-Effect sensor.

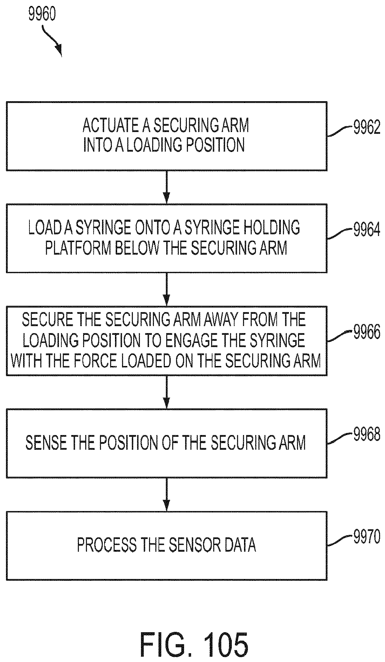

Fig. 105 shows a method 9960 for side loading a syringe on an infusion pump in accordance with an embodiment of the present disclosure. The method 9960 includes an actuating act 9962, a loading act 9964, a securing act 9966, a sensing act 9968, and a processing act 9970. The actuating act 9962 involves actuating a securing arm into a loading position. Act 9962 may be performed by an operator of the pump. Once the securing arm has been lifted into the loading position, the method 9960 moves to act 9964.

Act 9964 loads a syringe onto a syringe holding platform (also referred to herein as a syringe holding ledge) located below a securing arm. For example, the flange on the syringe is inserted into a slot or the barrel of the syringe is inserted into a barrel groove. Once the syringe has been placed on the platform below the securing arm, the method 9960 moves to act 9966.

The securing act 9966 secures the securing arm away from the loading position to engage the syringe with the force loaded on the securing arm, causing the securing arm to engage the syringe with the force loaded on it. Once the syringe has been secured, the method 9960 can continue to act 9968. The sensing act 9968 senses the position of the securing arm. This may be accomplished using a Hall-Effect sensor or a rotational potentiometer. After the sensing act 9968 the method 9960 may implement the processing act 9970.

The processing act 9970 processes the data from the position of the arm. A processor can use this data to determine what size syringe is being used. Knowing the size of the syringe allows the pump to control fluid flow with respect to plunger position. If the type of syringe is preset, the sensor can alert the operator if the securing arm is not in the right position. If the securing arm is not in the right position, the syringe is not properly secured.

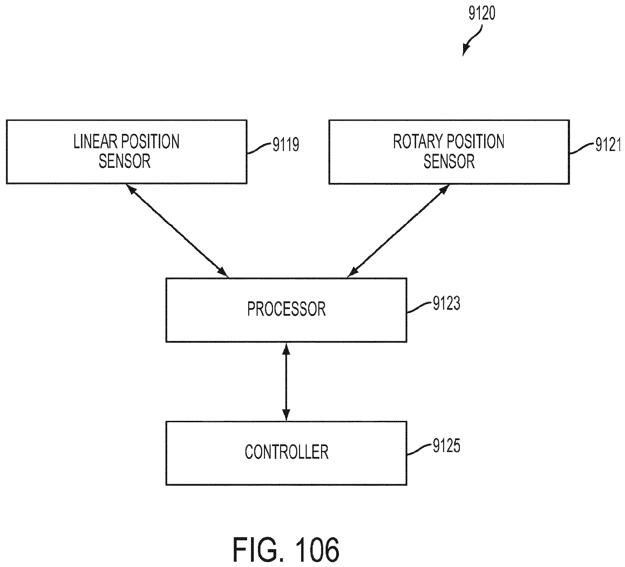

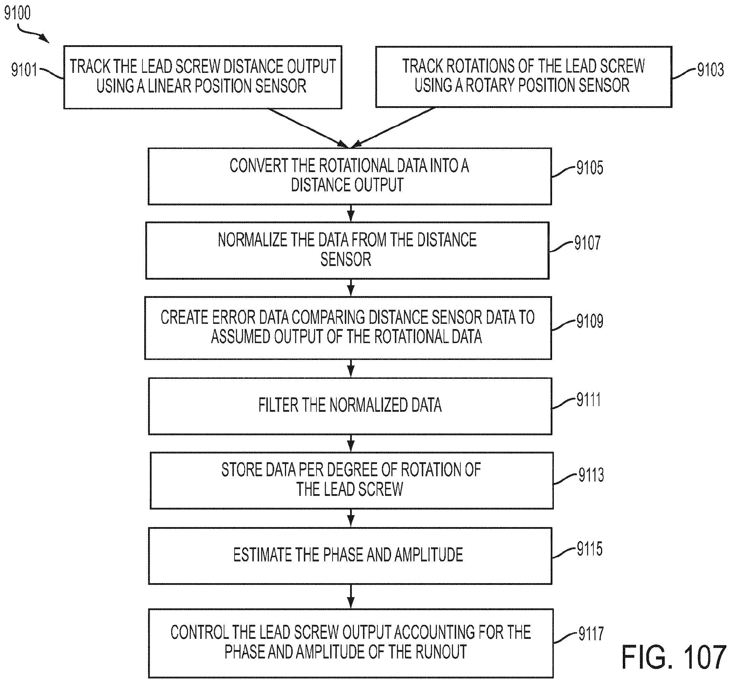

Fig. 106 shows an embodiment of a system for mitigating lead screw runout error, and Fig. 107 shows a flow chart diagram of a method for mitigating lead screw runout error in accordance with an embodiment of the present disclosure. Lead screw runout is a cyclic deviation from the assumed direct relation between a lead screw's rotations and the change in distance of a device being moved by the threads (e.g., a half-nut assembly or a nut on the threads, etc.) This may be caused by the half nut changing orientation with respect to the threads through a rotation due to forces acting on the mechanism. The lead screw error can be minimized by milling drive shafts and half nuts with high precision.

The system 9210 of Fig. 106 can implement the method 9100 of Fig. 107. The lead screw runout may be mitigated by estimating the cyclic deviations caused by the runout and compensating for the deviations when controlling for the distance output of the lead screw.

Fig. 106 shows an embodiment of a system 9120 for mitigating lead screw runout error. This system 9120 includes a linear position sensor 9119, a rotary position sensor 9121, a processor 9123 and a controller 9125. The rotary position sensor 9121 tracks the rotations of the lead screw. An equation for determining distance output in centimeters ("CM") based on rotational data is shown as follows:
Δ*θ* = *lead screw rotational change in degrees*
*β = lead screw Threads Per CM* $Distance output = \frac{360 °}{Δ θ} ∗ \frac{1}{B} .$
This equation for determining the distance actuated assumes that there is a direct relationship between the lead screw's rotations and distance output. Runout error is a cyclic deviation from the assumed linear distance output.

The linear position sensor 9119 is used to detect the runout deviations through sensing the distance output of the lead screw. In some embodiments of the present disclosure, an optical sensor, such as an optical mouse sensor, is coupled to the half-nut described herein which is used to measure the displacement of the half-nut by examining movement as detected against a surface of the housing of the syringe pump. In some embodiments, the optical sensor outputs change in position data in counts per inch (CPI). In some embodiments, the receiver is recalibrated by the processor 9123 to the current CPI, which is also referred to as normalizing. Normalization is accomplished using the equation below:
*θ = Current Lead Screw Angle in degrees*
*M = Optical Mouse Counts*
*R = Rotary Distance in millimeters (mm)*
*f* = *filter discovered emperically* $Inst {CPI}_{i} = \frac{{M}_{θ} - {M}_{θ - 10 °}}{{R}_{θ} - {R}_{θ - 10 °}}$ ${CPI}_{i} = f ∗ \left(Inst {CPI}_{i}-{CPI}_{i - 1}\right) .$
This equation recalibrates the CPI every 10 degrees; however, other recalibration rates may be used as well.

The magnitude and derivation of the signal may shift the phase of the signal by 180° resulting in the normalization data needing to be multiplied by -1. The magnitude may also be affected and the correction for this can be discovered empirically through a comparison of the deviations using a second more precise distance measuring device.

The processor 9123 uses the normalized distance data to estimate a phase and amplitude of the runout deviations. The oscillations of the runout deviation may occur in sync with each rotation of the lead screw. A low pass filter may be applied to filter the sensor data and then store the data for a given lead screw angle into one value. An example of the algorithm used is:
*θ = Lead Screw Angle*
*x* = *sensor data*
*ω*(*θ*) = *Sinusoidal sensor data* $ω {\left(θ\right)}_{i} = 0.3 \left({x}_{i}-ω{\left(θ\right)}_{i - 1}\right) + ω {\left(θ\right)}_{i - 1} .$
An array of data is created using this algorithm which may be used for cross correlating. Cross correlating with an array of data that consists of one or more rotations may be used to produce phase and/or amplitude results. The array size may be the previous 4 rotations, in some embodiments, which may consist of 1440 elements (360 degrees/rotation * 4 rotations).

Once the processor 9123 has created an array it will cross correlate the data with a sine and a cosine wave to determine the phase and amplitude of the data. The equation for cross correlating two discrete functions is defined as follows: $\left(f*g\right) \left[n\right] = {\sum }_{m = - ∞}^{∞} {f}^{∗} \left[m\right] g \left[n+m\right] .$ The equation used for this application is as follows:
*I = length of input array* $\left(f*g\right) = \frac{2}{l} {\sum }_{m = 0}^{l} f \left[l-m\right] g \left[m\right]$
* *sin* = *signal cross correlated with sine wave*
* *cos* = *signal cross correlated with cosine wave*
***α*** = ***Signal Amplitude***
*ϕ = Phase Offset* ${α}_{inst} = \sqrt{* {sin}^{2} + * {cos}^{2}}$ ${ϕ}_{inst} = atan 2 \left(*cos,*sin\right) .$

In some embodiments, the phase offset may be constant throughout the travel, while the amplitude may rise and fall as the half nut assembly travels away from or near the end of the lead screw. The phase and amplitude estimates can be filtered by the processor 9123 to integrate this amplitude shift using the following algorithm: ${α}_{i} = {α}_{i - 1} - 0.0005 \left({α}_{i - 1}-{α}_{inst}\right)$ *Cᵢₙᵢₜ* = 1 ${C}_{near} = 5 E - 4$ ${C}_{mid} = 5 E - 5$ ${C}_{far} = 5 E - 6$ ${ϕ}_{error} = {ϕ}_{i - 1} - {ϕ}_{inst}$ ${ϕ}_{i} = {ϕ}_{i - 1} - {Cϕ}_{error}$
*If* |*ϕₑᵣᵣₒᵣ*| > 3, *C* = *C_{far}*
*Else If* |*ϕₑᵣᵣₒᵣ*| > 1, C = *C_{mid}*
*Else, C = Cₙₑₐᵣ.*

Upon completing the filtering, the processor 9123 uses the amplitude and phase estimations to estimate the current error between the rotary position estimate and the current position of the lead screw mechanism. This is accomplished using the following equation:
*θᵢ* = *Current Lead Screw Angle*
*Δᵢ*= *Current Position Correction* ${Δ}_{i} = {α}_{i} cos \left({ϕ}_{i}+{θ}_{i}\right)$
*r₁* = *Current Rotary* - *Based Position*
*xᵢ* = *Adjusted Target Position* ${x}_{i} = {r}_{i} + {Δ}_{i} .$

Once the error between the rotary position estimate and the true output of the lead screw mechanism has been determined, this data is sent to the controller 9125. The controller 9125 incorporates this data with the assumed direct relation between lead screw rotations and distance output of the lead screw to thereby increase the accuracy of the output. This algorithm used to detect phase and amplitude of the error may be used with any sufficient sensor input to detect, estimate, and/or compensate for the lead screw runout.

Fig. 107 shows a flow chart diagram of a method 9100 for mitigating lead screw runout error in accordance with an embodiment of the present disclosure. The method 9100 includes a rotation tracking act 9103, a distance tracking act 9101, a conversion act 9105, a normalizing act 9107, an error creation act 9109, a filtering act 9111, a storing act 9113, an estimating act 9115, and a controlling act 9117.

The rotation tracking act 9103 involves tracking the rotations of the threaded driveshaft of a lead screw mechanism using a rotary position sensor. A Hall-Effect sensor may be used as the rotary position sensor as described herein. The distance tracking act 9101 tracks the distance output of the lead screw mechanism using a linear position sensor. An optical mouse sensor may be used for the linear position sensor; however, in some embodiments, any sensor capable of tracking linear position may be used. In some embodiments, acts 9101 and 9103 may occur simultaneously, step-wise, or in any order or variation.

The converting act 9105 converts the rotational data into estimated distance output data of the lead screw mechanism. The method 9100 may proceed to act 9107 when or after the rotational data has been converted.

The normalizing act 9107 normalizes the distance sensor data to create a data set with reduced sensor drift. The sensor may be recalibrated every ten degrees of lead screw rotation when normalizing the data, in some specific embodiments. The method 9100 may move on to act 9109 when or after the data has been normalized, in some embodiments.

The error creation act 9109 creates error data comparing the distance sensor data to the output of the rotational data. The filtering act 9111 filters the normalized data. The storing act 9113 stores the data as a value for each degree of rotation of the lead screw. The estimating act 9115 uses the data stored as the value for each degree of rotation of the lead screw to determine amplitude and a phase of the error. Estimating the phase and amplitude may be accomplished by cross-correlating a sine and cosine wave with the data. The estimation act 9115 may also account for the position of the half nut on the lead screw and account for a decrease in amplitude when the half nut nears an end of the lead screw. Once the amplitude and phase of the error have been determined, the method 9100 moves to act 9117.

The controlling act 9117 controls the rotations of the lead screw with the estimated phase and amplitude deviations incorporated into the assumed direct relation between lead screw rotations and output.

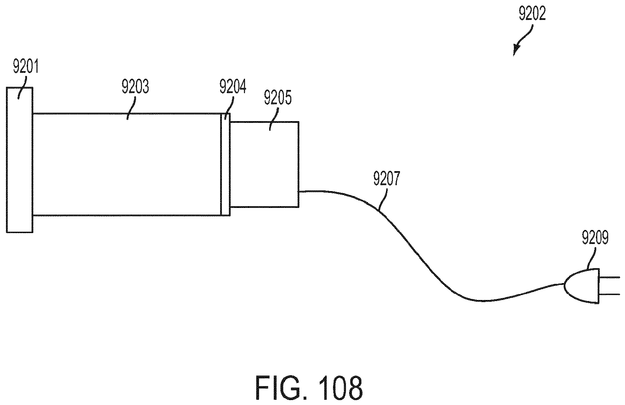

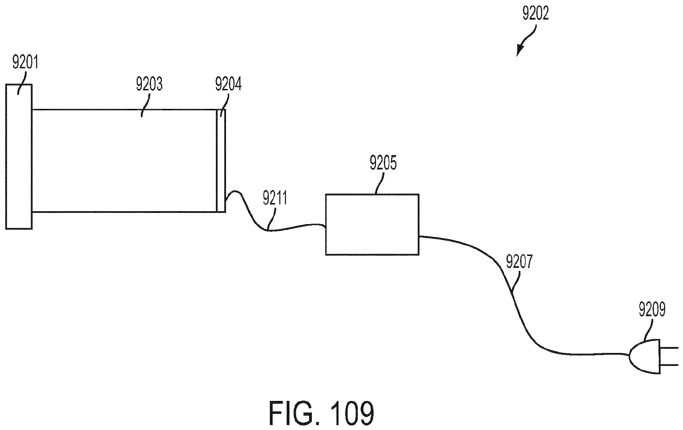

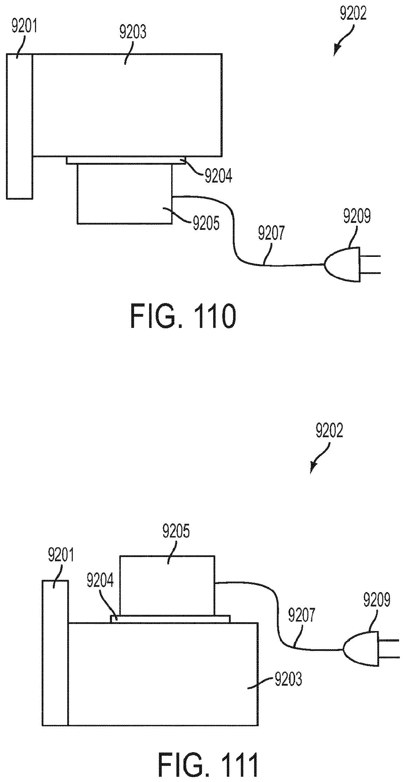

Figs. 108-111 shows several views of an infusion pump with a modular power supply coupled thereto in accordance with an embodiment of the present disclosure. Fig. 108 shows a side view of a pump with a modular power supply attached to the back of the pump. Fig. 109 shows a side view of a pump with an external power supply. Fig. 110 shows a side view of a pump with a power supply attached to the bottom of the pump. Fig. 111 shows a side view of a pump with a power supply attached to the top of the pump.

As shown in Figs. 108-111, the various embodiments show an infusion pump 9202 with a power entry module 9204, a power supply 9205, and an outlet adapter 9209. In some embodiments, the power entry module 9204 is attached to a housing 9203 of an infusion pump 9202 and has a port configured to receive DC current to supply the pump 9202 with power. The power supply 9205 has the capability to be removeably attachable to the power entry module 9204. The power entry module 9204 may be an electrical connector having conductive contacts. The power supply 9205 may be coupled to an AC plug 9209 configured to receive an AC signal. The power supply 9205 may include an AC-to-DC conversion module within the power supply 9205 to convert the AC signal received via a power cord 9207 to a DC current. A DC out connection 9211 provides DC current to the power entry module 9204.

Fig. 108 shows an embodiment having the power supply 9205 secured to the back of the pump 9202 by the power entry module 9204. The power entry module 9204 may secure the power supply 9205 in place. The power supply 9205 receives AC power through a power cord 9207 connected to the AC plug 9209.

Fig. 109 depicts an embodiment of the power supply 9205 in which a power cord 9211 connects the DC out jack of the power supply 9205 to the power entry module 9204. The pump 9202 may be configured to secure the power supply 9205 to the outside of its housing 9203.

Fig. 110 shows an embodiment of the pump 9202 that shows the power supply 9205 attached to the bottom of the pump 9202. Fig. 111 shows an embodiment in which the power supply 9205 is attached to the top side of the pump 9202.

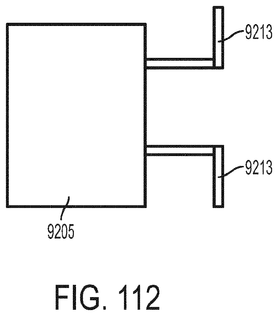

Fig. 112 shows an embodiment in which a power supply (hereinafter also referred to as a power source) 9205 having a structure 9213 for winding up the power cord 9207 of Figs. 108-111. In some embodiments, a mechanism which automatically wraps up the cord 9207 may be used.

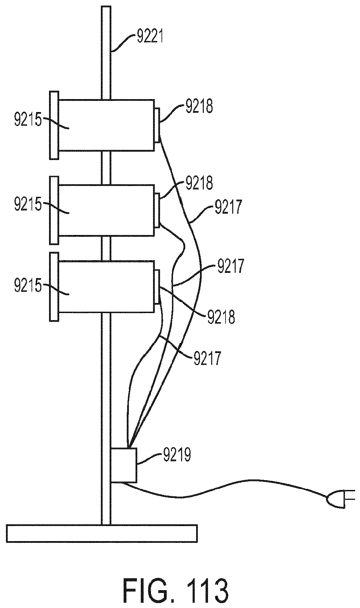

Fig. 113 shows an embodiment in which a power supply 9219 supplies power to multiple pumps 9215 in accordance with another embodiment of the present disclosure. That is, a single power supply 9219 may be configured to provide power (e.g., DC power) to multiple pumps 9215. In Fig. 113, the power supply 9219 is attached to a pole 9221 on which pumps 9215 are mounted. The power supply 9219 may have multiple power cords 9217 in electrical communication with the power out jack of the power supply 9219 which is connected to the power entry modules 9218 of the pumps 9215 attached to the pole 9221.

The power supply 9205 may also include a battery that is charged by the power supply and has the capability to power the pump when the power supply isn't receiving AC power. In most cases this battery will supplement a battery within the pump housing 9203. This could be used to extend the operating time of the pump 9202 when no AC current is available, for example when the patient is being moved to a different location. It may also allow the pump 9202 to have a smaller battery within.

A pump 9202 may be attached to a rack which powers the pump 9202 and allows the pump 9202 to communicate with other pumps on the rack. When attached to the rack the pump 9202 will not need the power source 9205. The power entry module 9204 may be designed so the rack and power supply 9205 connect the same way, making the two interchangeable.

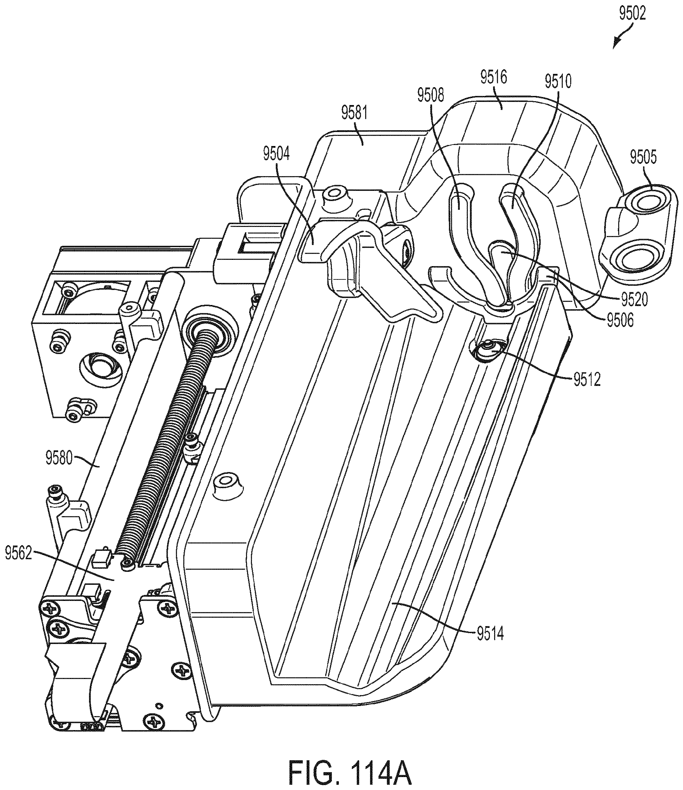

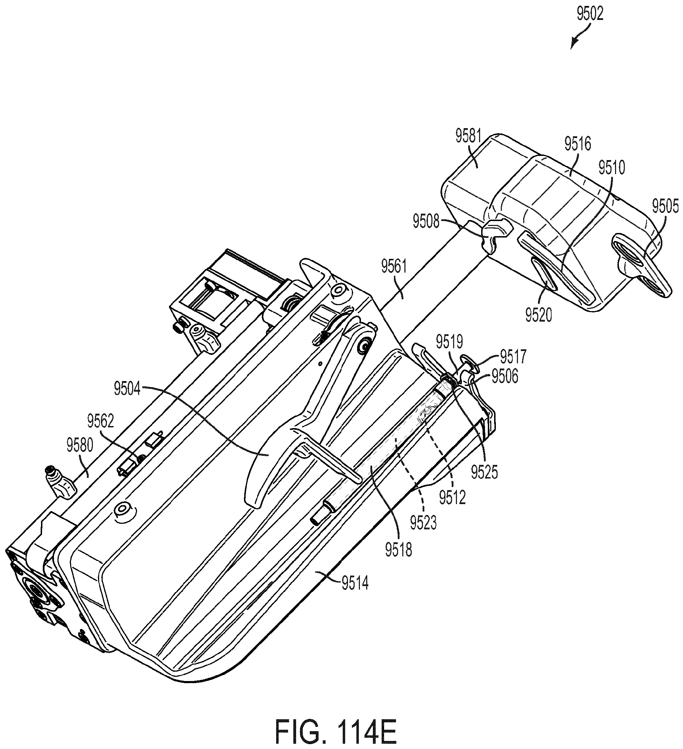

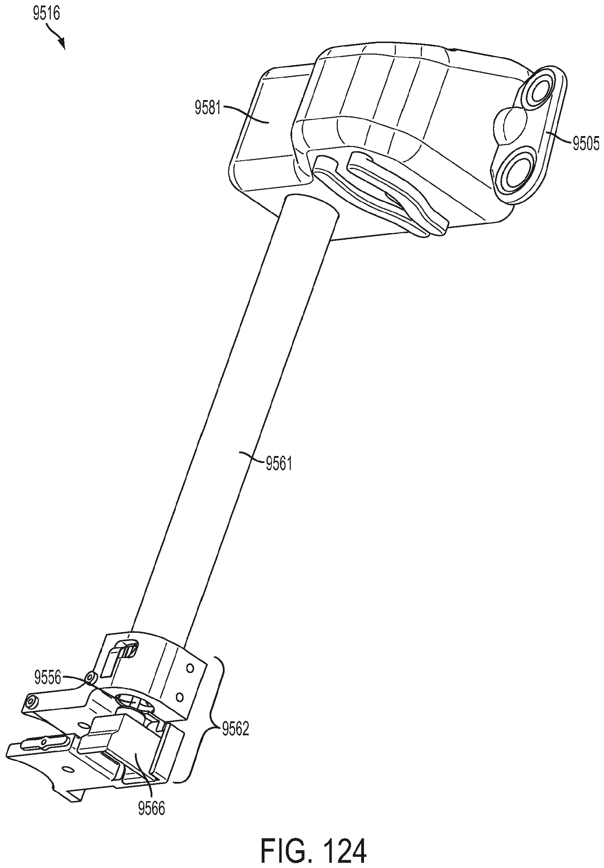

Figs. 114A-114J show several views of a syringe pump assembly 9502 in accordance with an embodiment of the present disclosure. Referring to Fig. 114A, the syringe pump assembly 9502 is shown and includes a body 9580, a syringe seat 9514, and a plunger head assembly 9516. The plunger head assembly 9516 includes a plunger head 9581, a half-nut assembly 9562, and a plunger tube 9561 (refer to Fig. 124). A syringe (e.g., see Fig. 114E for the syringe 9518) may be placed into the syringe seat 9514, which is secured by the retaining member 9504 and a retaining clip 9506 (described below). A dial 9505 opens the pivotal jaw members 9508, 9510 and allows the plunger head assembly 9516 to move away from or toward the syringe seat 9514.

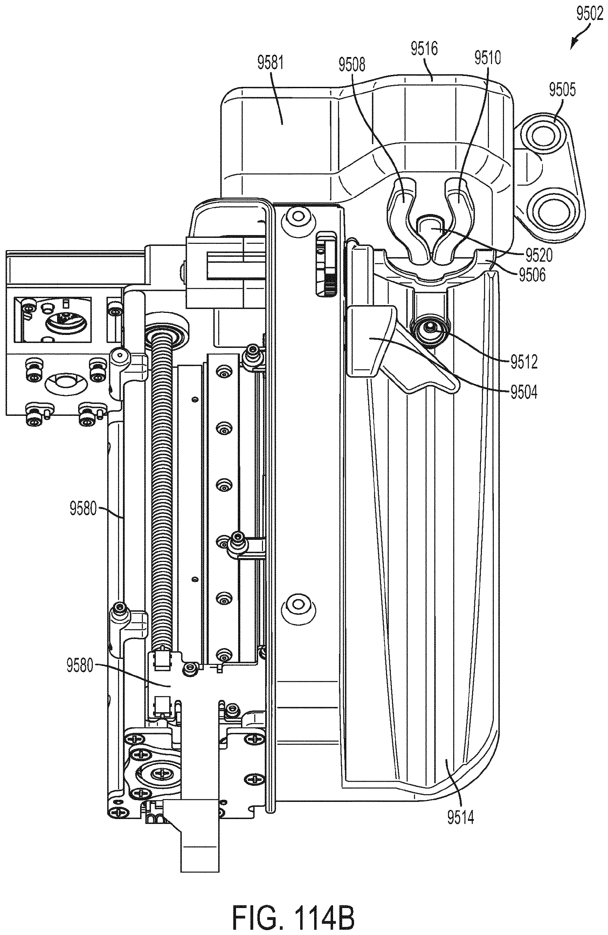

Referring now to Fig. 114B, a top view of the syringe pump assembly 9502 is shown which provides a clear view of a sensor 9512. The sensor 9512 may detect the presence or absence of a syringe seated within the syringe seat 9514. The sensor 9512 is coupled to one of the processor of the syringe pump that the syringe pump assembly 9502 is coupled to such that the processor can detect the presence or absence of a syringe loaded into the syringe seat 9514.

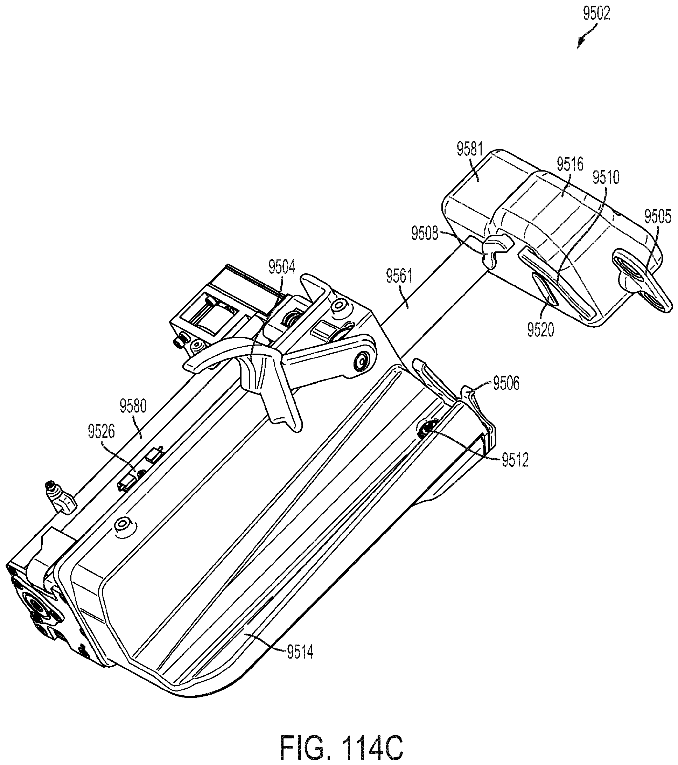

Fig. 114C shows the syringe pump assembly 9502 in a configuration ready to receive a syringe within the syringe seat 9514. That is, the retaining member 9504 is in an up position and the dial 9505 is turned in an open position that is clockwise 90 degrees from the closed position. The rotation of the dial 9505 also rotates the pivotal jaw members 9508, 9510 away from each other. The dial 9505 may be held in the open position as shown in Fig. 114C by an internal mechanism (described below) allowing the user to stop applying a torque on the dial 9505 and take their hand off of the dial 9505, all while the dial 9505 remains in the open position. This allows a user to easily load a syringe, optionally using both hands, and to slide the plunger head assembly 9516 such that the pivotal jaw members 9508, 9510 can operatively couple to the flange of the syringe. The retaining member 9504 is spring-biased toward the syringe seat 9514; however, when the retaining member 9504 is in a fully open position, an internal mechanism may hold the retaining member 9514 in an open position without any required torque applied by a user.

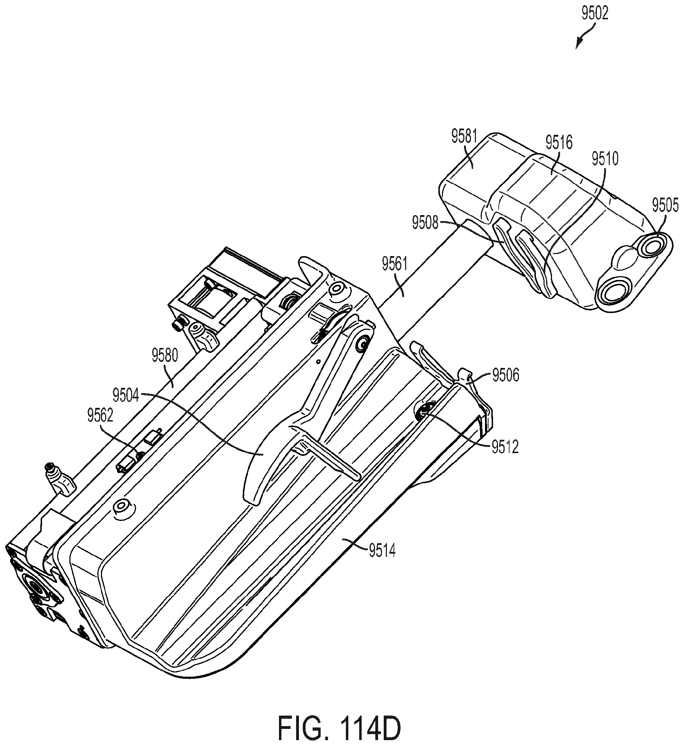

Fig. 114D shows the syringe pump assembly 9502 in a configuration where the retaining member 9504 is in a down position and the dial 9505 is turned in a closed position. The rotation of the dial 9505 also biases the pivotal jaw members 9508, 9510 toward each other. The dial 9505 may be held in the closed position as shown in Fig. 114D by an internal bias mechanism (described below) allowing the user to stop applying a torque on the dial 9505 and take their hand off of the dial 9505 all while the dial 9505 remains in the closed position. When the dial 9505 is rotated away from the open position (see Fig. 114C) by a predetermined amount toward the closed position, the plunger head assembly 9516 is locked into position and cannot freely move into or out of the rest of the syringe pump assembly 9502 (described more below).

With reference to Figs. 114E-115B, an overview of the operation of loading a syringe 9518 into the syringe pump assembly 9502 is illustrated. After the retaining member 9504 is in the open position (as shown in Fig. 114C), the syringe 9518 may be placed into the syringe seat 9514 and the retaining member 9504 rotated onto the syringe 9518 as is shown in Fig. 114E. The syringe 9518 may be retained by a retaining clip 9506 that secures a flange 9525 of a barrel 9523 of the syringe 9518 between the syringe seat 9514 and the retaining clip 9506.

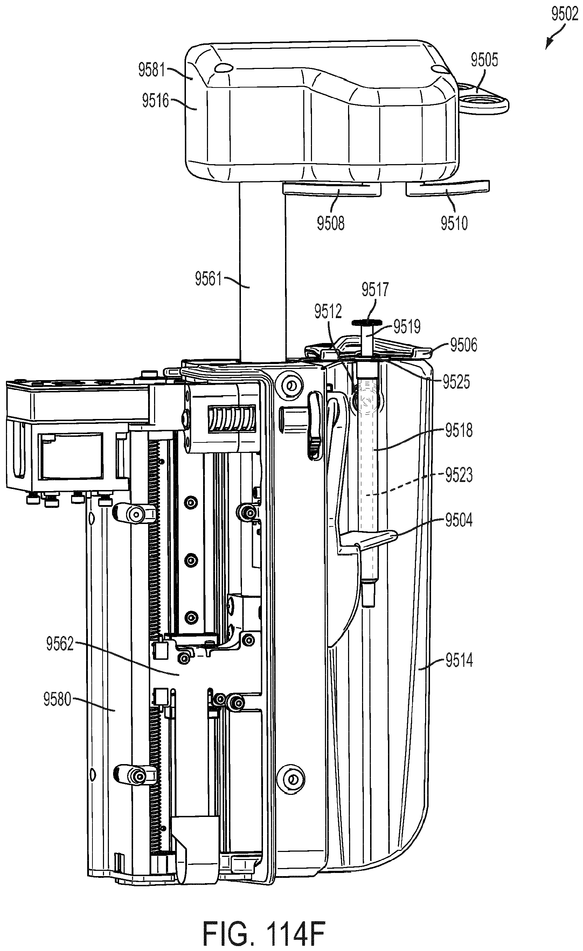

When the syringe 9518 is sufficiently placed into the syringe seat 9514, the syringe 9519 may trigger the sensor 9512 when the syringe 9518 is loaded into the syringe seat 9514. The sensor 9512 is more easily seen in Fig. 114F. A processor may be coupled to the sensor 9512 and is configured to receive this notification. Additionally, a radial angle sensor (described below) may be coupled to the processor to measure the radial angle of the retaining member 9504 (refer again to Fig. 114E) to estimate the size of the syringe 9518.

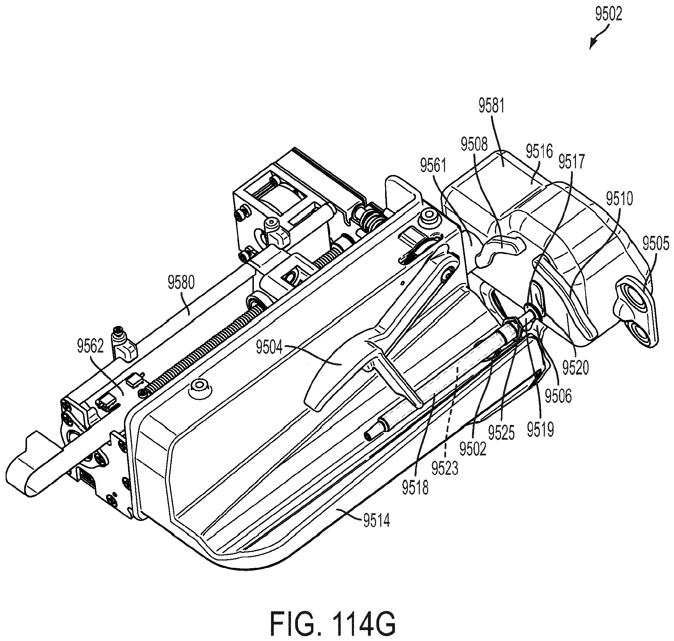

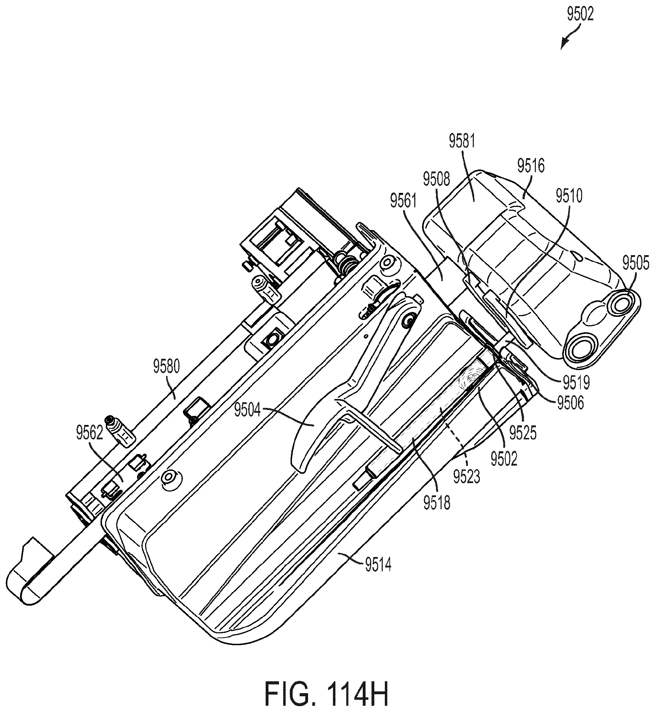

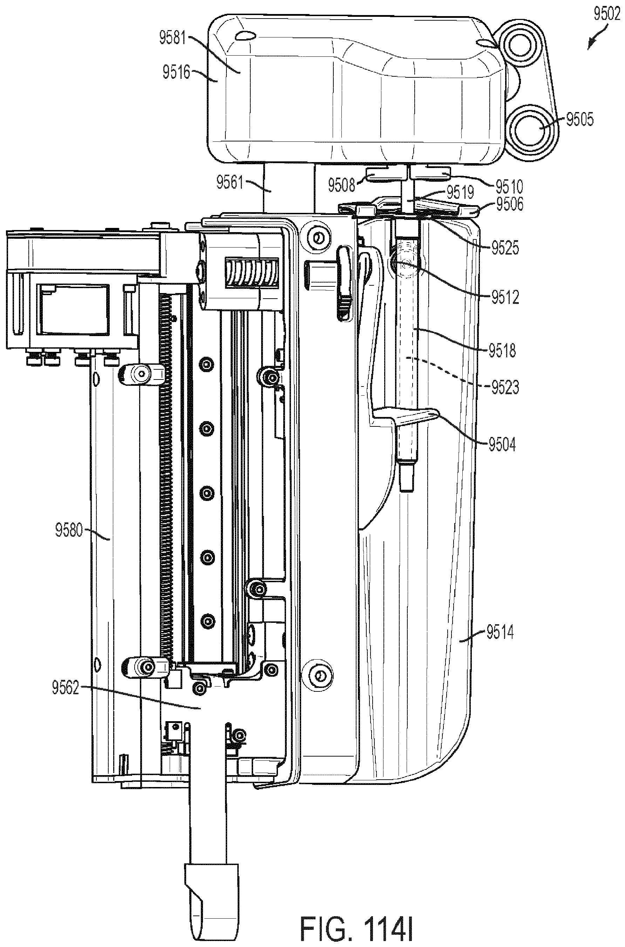

As shown in Fig. 114G, after the syringe 9518 is placed within the syringe seat 9514, the retaining member 9504 may be rotated toward the syringe and the plunger head assembly 9516 may be moved toward the syringe 9518 until a force sensor 9520 contacts an end 9517 (which may be a flange) of a plunger 9519 of the syringe 9518. The dial 9505 may be rotated causing the pivotable jaw members 9508, 9510 to rotate toward the flange 9517 of the plunger 9519 of the syringe 9518 and grasp onto the flange 9517 of the plunger 9519 of the syringe 9518, as shown in Fig. 114H. Fig. 114I shows this configuration from an overhead view.

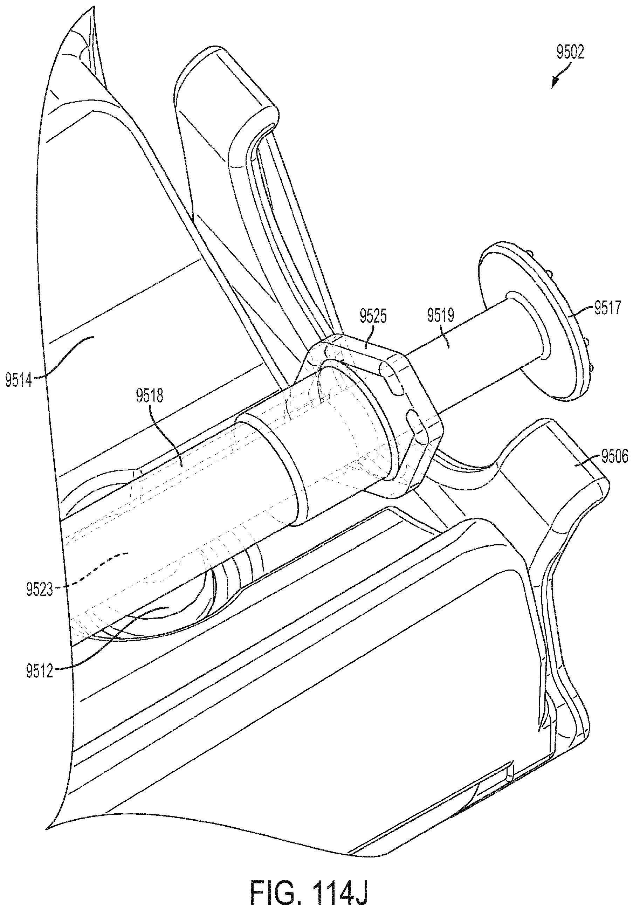

Fig. 114J shows a close up view of the operation of the retaining clip 9506 and the sensor 9512 of the syringe pump assembly of Figs. 114A-114J. As is easily seen in Fig. 114J, the flange 9525 of the barrel 9523 of the syringe 9518 is disposed between the syringe seat 9514 and the retaining clip 9506. The resiliency of the retaining clip 9506 may frictionally lock the barrel 9523 of the syringe 9518 into place. Also shown is the sensor 9512, which may be a button type sensor that is actuatable into the syringe seat 9514 when the syringe 9518 is placed within the syringe seat 9514.

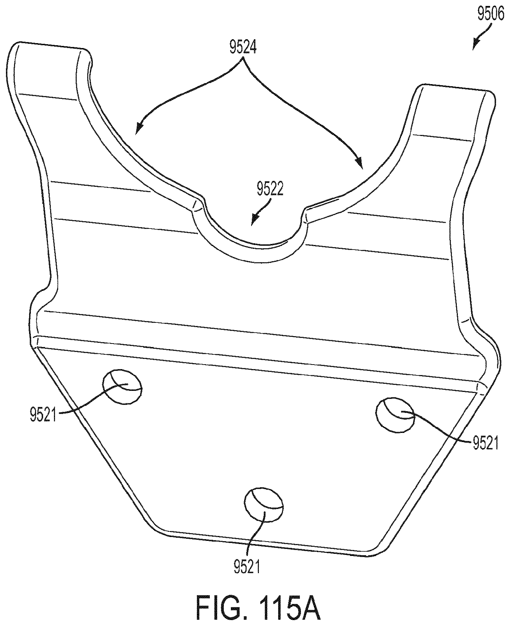

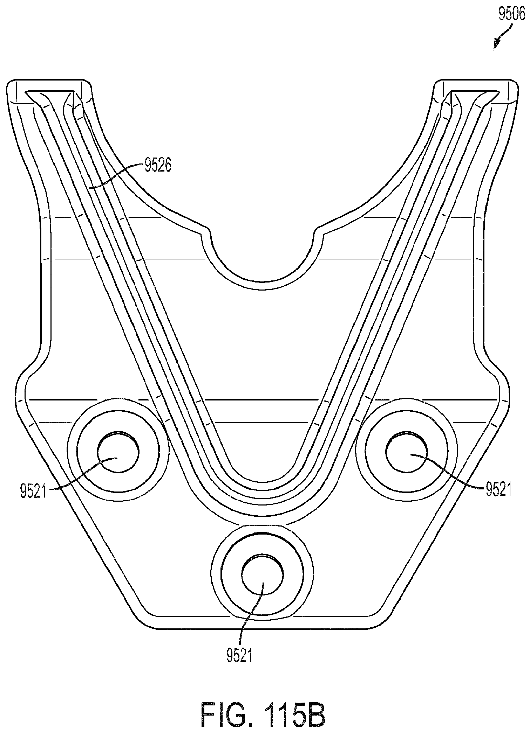

Figs. 115A and 115B show two sides of the retaining clip 9506. The retaining clip 9506 includes three holes 9521 so that the retaining clip 9506 can be fastened to the syringe seat 9514. The retaining clip 9506 includes an inner recess 9522, to receive smaller syringes, and an outer recess 9524, to receive larger syringes. Note in Fig. 115B that the retaining clip 9506 includes a support structure 9526 to provide further resiliency to apply greater forces on the flange 9525 of the barrel 9523 of the syringe 9518 (see Fig. 114J).

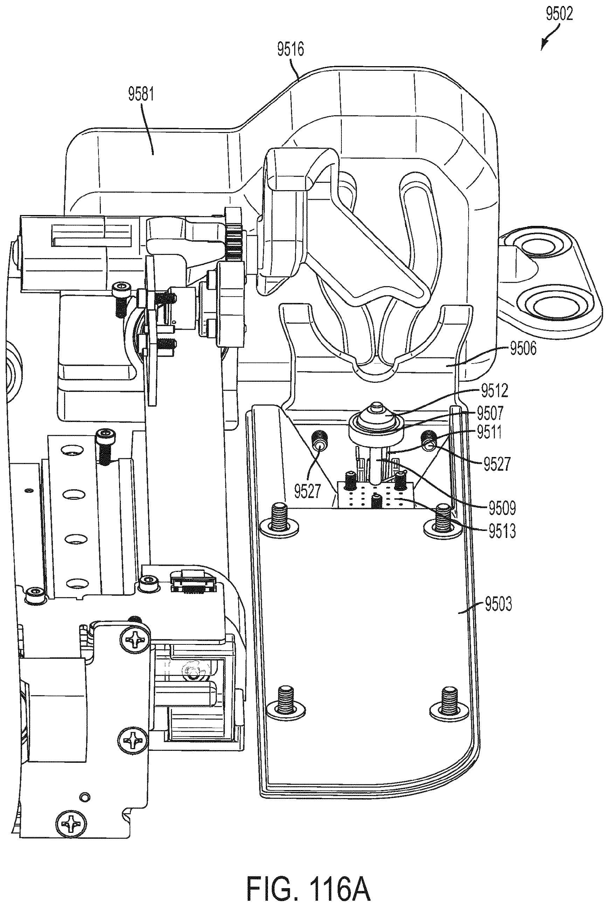

As shown in Fig. 116A, the sensor 9512 is easily viewable because the syringe seat 9514 has been removed. Also shown in Fig. 116A, is a bottom cover 9503 that is attached to the bottom of the syringe seat 9514 to cover the sensor 9512 and optionally allow the retaining clip 9506 a place to be secured to. That is, the retaining clip 9506 may be optionally secured to the bottom cover 9503 via fasteners 9527 (e.g., screws), in some embodiments.

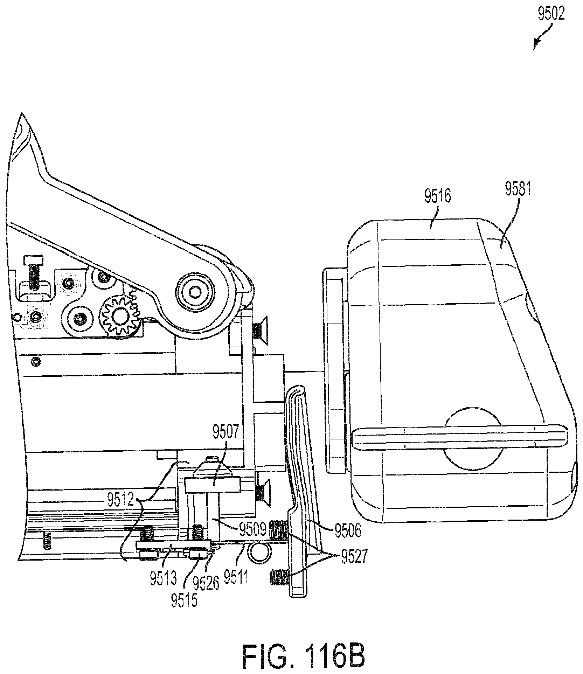

Fig. 116B shows a side view of the syringe pump assembly 9502 with the syringe seat 9514 and bottom cover 9503 removed. As is easily seen in Fig. 116B, the sensor 9512 includes a plunger head 9507, a plunger shaft 9509, a spring 9511, and a sensor board 9513. The sensor board 9513 includes a switch 9515 having a paddle 9526. The spring 9511 is coupled to the plunger shaft 9509 to bias the plunger shaft 9509 and the plunger head 9507 toward the location in the syringe seat 9514 in which a syringe 9518 may be placed (refer again to Fig. 114E).

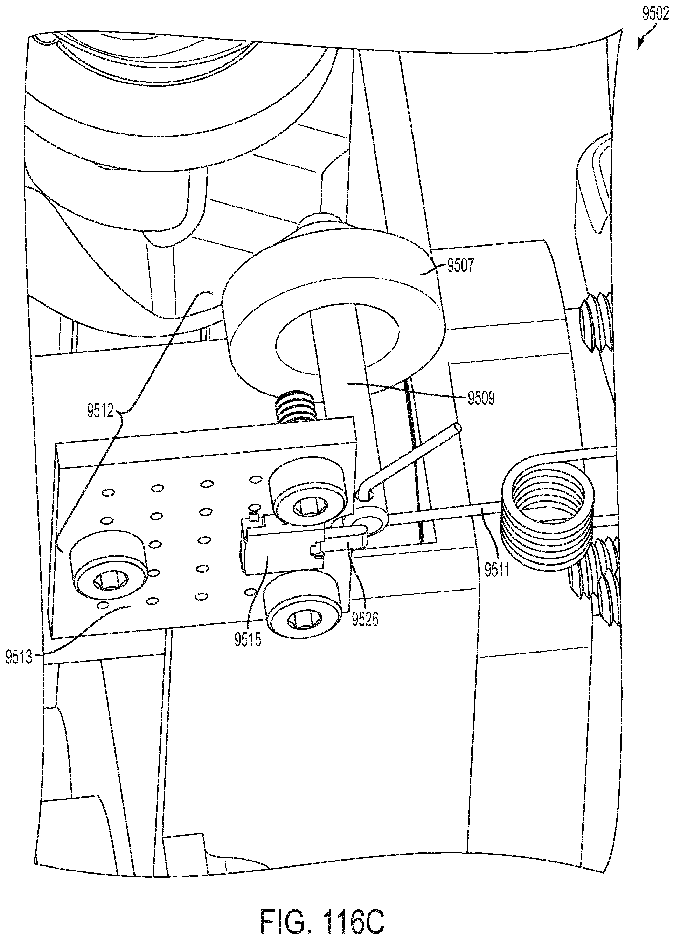

When a syringe (e.g., syringe 9518 of Fig. 114J) presses against the plunger head 9507 of the sensor 9512, the plunger head 9507 retracts into the syringe seat 9514 (see Fig. 114E for a view of the syringe seat 9514). Referring again to Fig. 116B, when a syringe presses against the plunger head 9507 of the sensor, the plunger head 9507 moves the plunger shaft 9509. The plunger shaft 9509 is coupled to a spring 9511 such that the plunger shaft 9509 may overcome the bias of the spring 9511 to engage the switch 9515 of the sensor board 9513. That is, when the plunger shaft 9509 is sufficiently actuated against the bias of the spring 9511, the plunger shaft 9509 presses against a paddle 9526 of a switch 9515 on the sensor board 9513(refer to Fig. 116C). Fig. 116C shows a close-up view of the interaction of the plunger shaft 9509 and the paddle 9526 of the switch 9515. When the switch 9515 detects movement by a predetermined amount, the sensor board 9513 provides a signal of the sensor 9512 to the processor to notify it that a syringe 9518 has been loaded into the syringe seat 9514 (as shown in Fig. 114E).

Referring again to Fig. 116C, although the switch 9515 may be a discrete switch (e.g., only two discrete states), in some embodiments, the switch 9515 provides an analog position of the paddle 9526 to the sensor board 9513, which is provided to the processor as the sensor's 9512 signal.

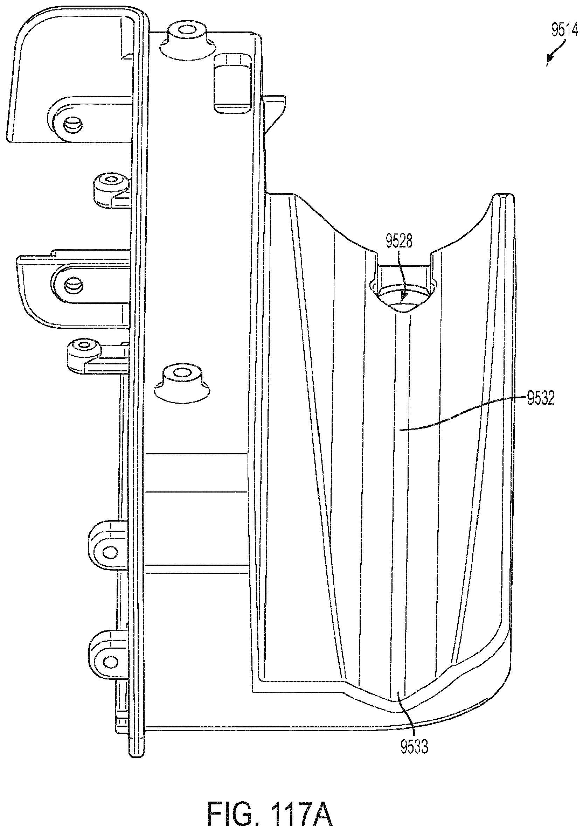

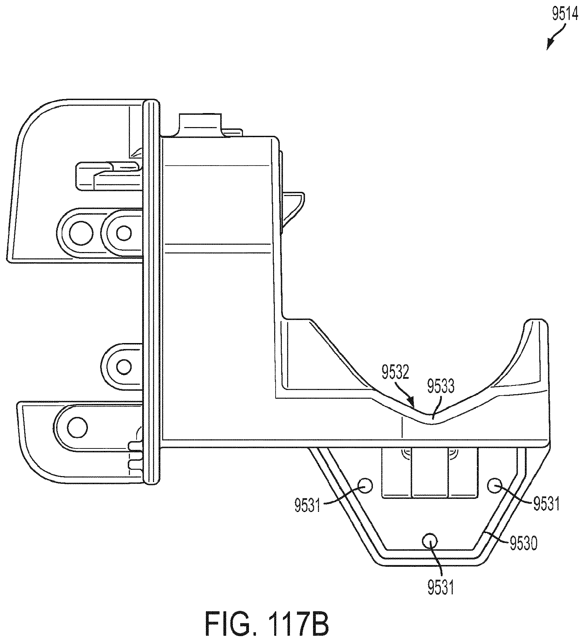

Figs. 117A-117C show several views of the syringe seat 9514 of the syringe pump assembly 9502 shown in Figs. 114A-114J in accordance with an embodiment of the present disclosure. As is easily seen in Fig. 117A, the syringe seat 9514 includes a hole 9528 for the sensor 9512 (e.g.s., see Fig. 114A). The syringe seat 9514 also includes a surface 9532 having series of wedge-shaped surfaces approaching an end 9533 of the surface 9532. The surface 9532 slopes downward as it approaches the end 9533. Fig. 117B shows the end positioned head on with the sloped surface 9532.

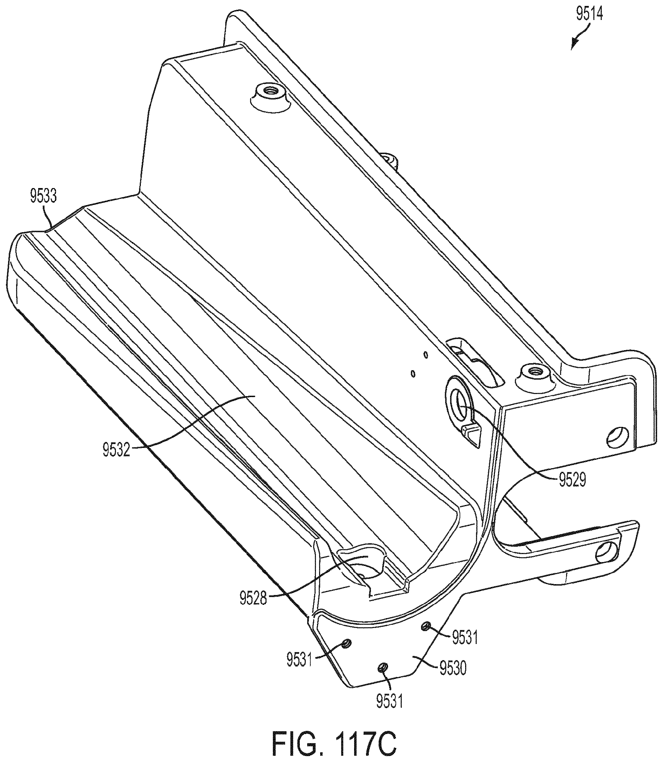

Referring to Fig. 117C, the syringe seat 9514 also includes a surface 9530 having holes 9531 in which the screws 9527 of the retaining clip 9506 may use to secure the retaining clip 9506 thereto. Also viewable in Fig. 117C, is a hole 9529 in which the retaining member 9504 (see Fig. 114A) may be partially positioned therein.

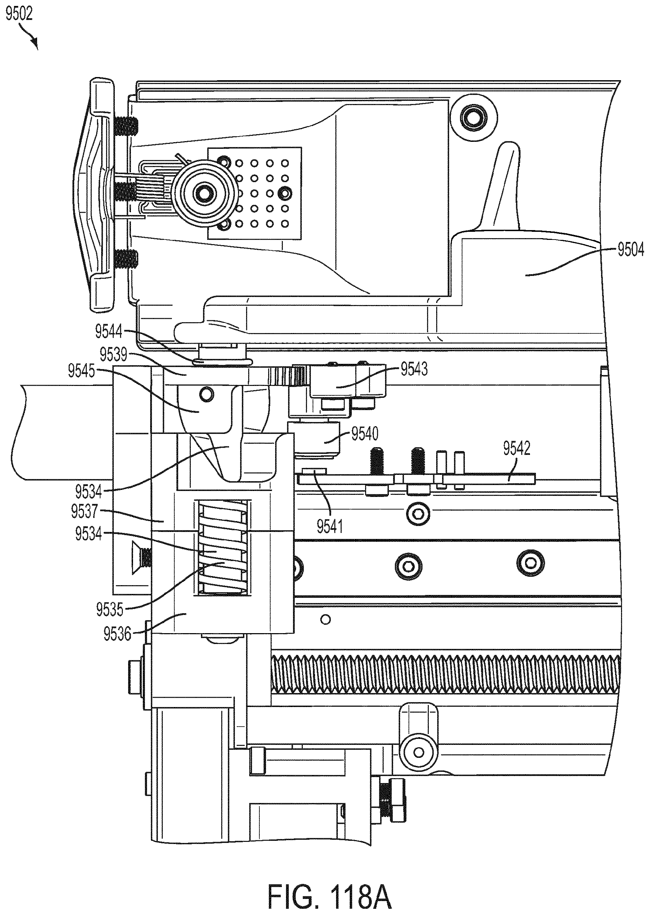

Fig. 118A-118B show several views of the syringe pump assembly 9502 shown in Figs. 114A-114J with the syringe seat 9514 removed in accordance with an embodiment of the present disclosure. Figs. 118A-118B will now be described in relationship to the syringe's 9518 diameter estimation.

As shown in Fig. 118A, the retaining member 9504 is in a fully open position. The retaining member 9504 is coupled to a shaft 9535. An O-ring helps seal the internals of the syringe pump assembly 9502 preventing contamination through the hole 9529 (see Fig. 117a). As shown in Fig. 118A, a fixed cam 9536 is positioned at the distal end of the shaft 9534 while a moveable cam 9537 is positioned at proximal end of the shaft 9534. A spring 9535 biases the moveable cam 9537 away from the fixed cam 9536.

The retaining member 9504 is coupled to the shaft 9534 such that rotating the retaining member 9504 also rotates the shaft 9534. Also coupled to the shaft 9534 is a rotating cam 9545. The rotating cam 9545 rotates as the retaining member 9504 is actuated (e.g., rotated between open and closed positions). When the retaining member 9504 is in the fully open position, the rotating cam 9545 and the moveable cam 9537 may engage each other such that the retaining member 9504 remains in the fully open position even when a user's hand is removed from the retaining member 9504 (i.e., the retaining member 9504 is in a dwelling position). That is, the rotating cam 9545 and the moveable cam 9537 may engage each other with opposing surfaces that are perpendicular to an axis defined by the shaft 9534.

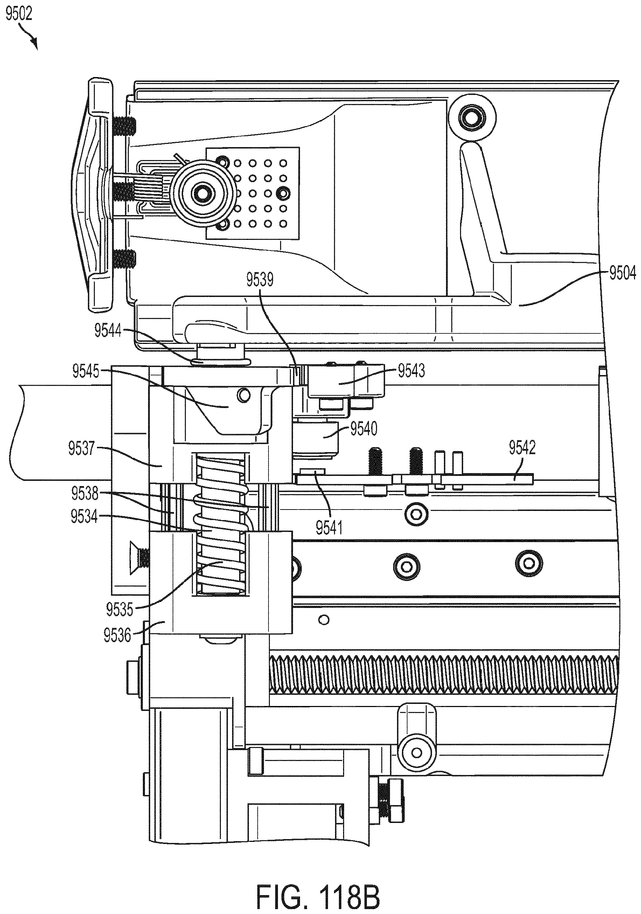

As the retaining member 9504 is rotated, the rotating cam rotates 9545 such that the movable cam 9537 and the rotating cam 9545 engage each other via opposing surfaces that are not perpendicular to an axis defined by the shaft 9534. This causes the force of the spring 9535 to translate from the moveable cam 9537 to the rotating cam 9545 such that the rotating cam 9545 rotates thereby rotating the retaining member 9504 toward its closed position. That is, the spring 9535 ultimately can cause a rotational bias force on the retaining member as long at the retaining member 9504 is not in a dwelling position. Fig. 118B shows the retaining member 9504 in the retaining position, e.g., when the retaining member is rotated toward any loaded syringe. Guiding rods 9538 prevent the moveable cam 9537 from rotating with the shaft 9534 or because of the spring 9535 and guide the moveable cam 9537 away from and toward the fixed cam 9536. A syringe loaded 9518 into the syringe seat 9514 may stop the retaining member 9504 from fully rotating to the closed position (see Fig. 114E). Fig. 118B shows the retaining member 9504 fully rotated to the closed position.

A gear 9539 is also coupled to the shaft 9534 and rotates therewith. The gear 9539 engages a gear assembly 9543. The gear assembly 9543 may increase or decrease the gearing to rotate a magnet 9540. A sensor board 9542 includes a hall-effect sensor 9541 (e.g., a rotating encoder) that can determine the rotational angle of the magnet 9540 and therefore determine the position of the retaining member 9504. The sensor board 9542 may transmit a signal encoding the retaining member's 9504 position to the processor where the processor correlates the position of the retaining member's 9504 position with a diameter of the barrel 9523 of the syringe (refer to Fig. 114E).

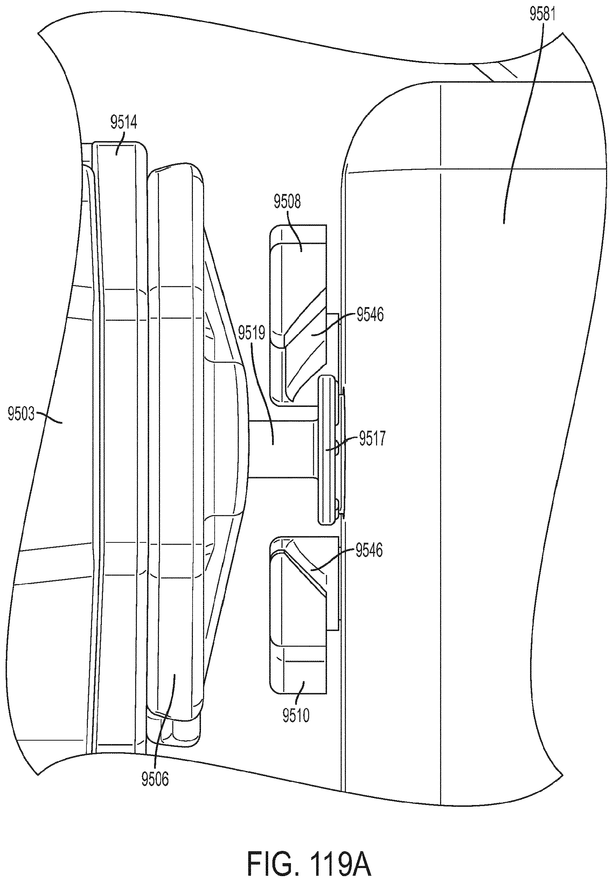

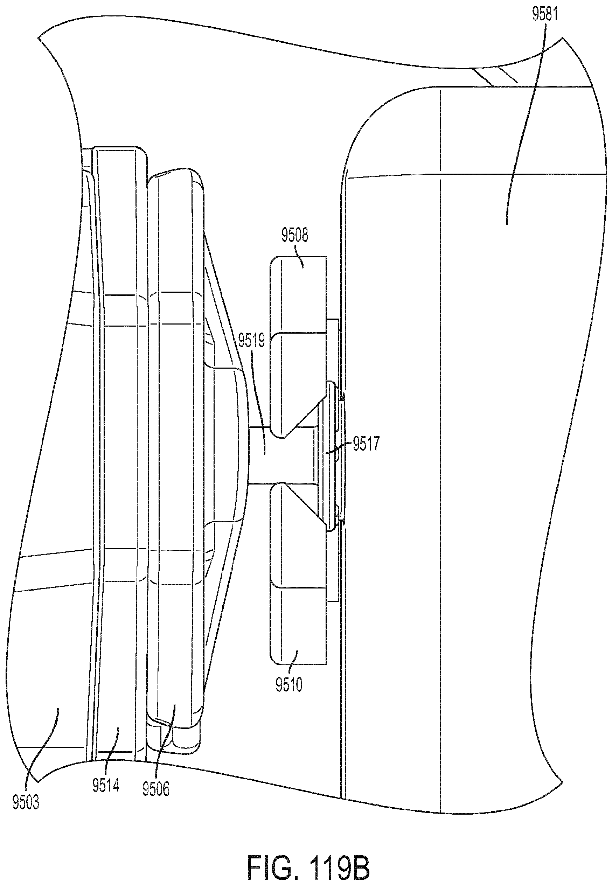

Figs. 119A-119B shows several views of the syringe pump assembly shown in Figs. 114A-114J to illustrate the jaw member's 9508, 9510 action of grasping onto a flange 9517 of a plunger 9519 of a syringe (e.g., syringe 9518 as shown in Fig. 114E) in accordance with an embodiment of the present disclosure. Fig. 119A shows the pivotal jaw members 9508, 9510 in an open position and Fig. 119B shows the pivotal jaw members 9508, 9510 grasping on the flange 9517 of the plunger 9519. As is easily seen in Fig. 119A, ramps 9546 are used so that as the pivotal jaw members 9508, 9510 grasp onto the flange 9517 of the plunger 9519 (as in Fig. 119B), the flange 9517 is held against the plunger head assembly 9516 (see Fig. 114A) more securely.

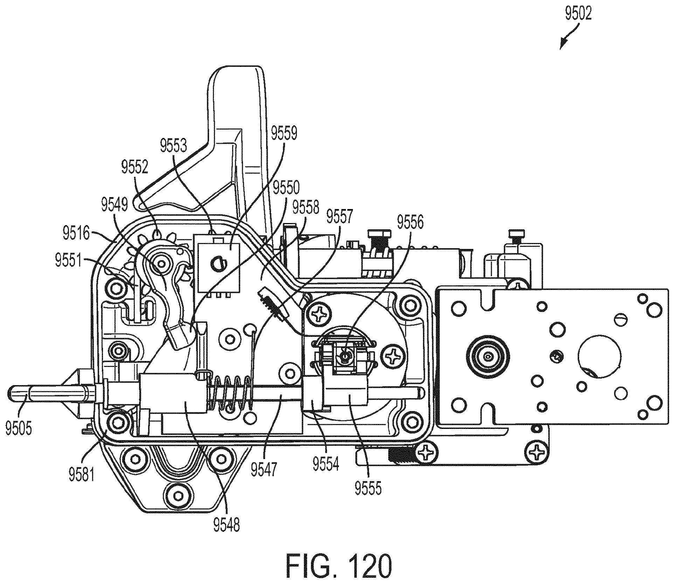

Fig. 120 shows the plunger head of the plunger head assembly 9516 (of the syringe pump assembly shown in Figs. 114A-114J) with the cover removed to illustrate the mechanical effects of rotation of the dial 9505 in accordance with an embodiment of the present disclosure. As shown in Fig. 120, the dial 9505 is coupled to a shaft 9547, a cam 9548, and a rod actuator 9554. A spring 9557 is operatively coupled to the shaft 9547 to bias the dial 9505 and the shaft to rotate toward a closed position (as shown in Fig. 120).

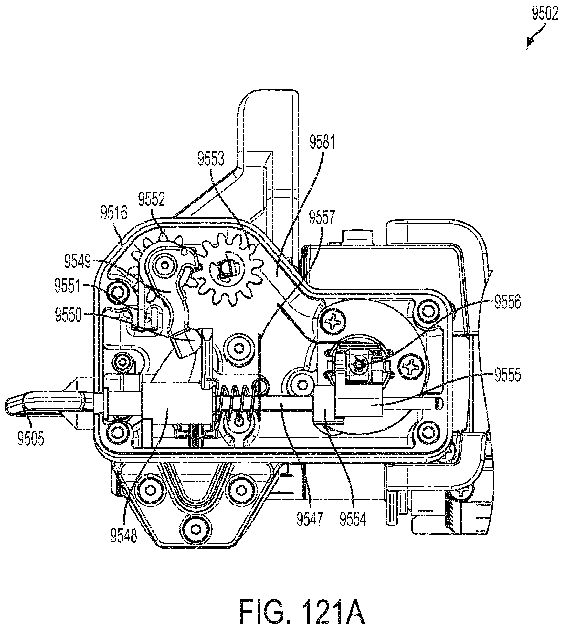

A gear 9553 is operatively coupled to a potentiometer 9559. The potentiometer 9559 is coupled to a circuit board 9558 which is configured to provide the processor with the rotational position of the gear 9553 (described below). Refer now to Figs. 121A-121C where the circuit board 9558 and the potentiometer 9559 have been removed to aid in viewing the internal parts of the plunger head assembly 9516. That is, Figs. 121A-121C show several views of the plunger head with the cover and a circuit board removed to illustrate the mechanical effects of rotation of the dial in accordance with an embodiment of the present disclosure;

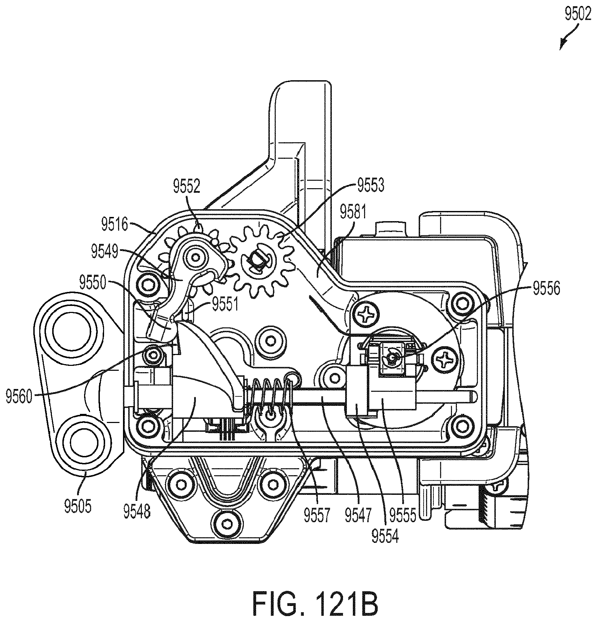

As shown in Fig. 121A, the dial 9505 is coupled to the cam 9548 such that rotation of the dial 9505 into an open position causes the cam 9548 to rotate such that the rocker arm 9549 rotates as a cam follower 9550 of the rocker arm 9549 engages with the cam 9548. The rocker arm 9549 is coupled to a gear 9552. A gear 9553 is coupled to the gear 9552 that is coupled to the rocker arm 9549. The gear 9552 and rocker arm 9549 are coupled to a spring 9551 such that the rocker arm 9549 is biased such that the cam follower 9550 is biased toward the cam 9548. Fig. 121B shows the configuration in which the dial 9505 is in the fully open position. Note that the rocker arm 9549 has rotated from its position in Fig. 121A, and note also that the gear 9553 has rotated by a corresponding amount . Referring now to Figs. 114C and 121B, the gear 9552 is coupled to the pivotable jaw member 9510 and the gear 9553 is coupled to the pivotable jaw member 9508. Fig. 121B and Fig. 114C shows the configuration in which the dial 9505 has been turned to the open position.

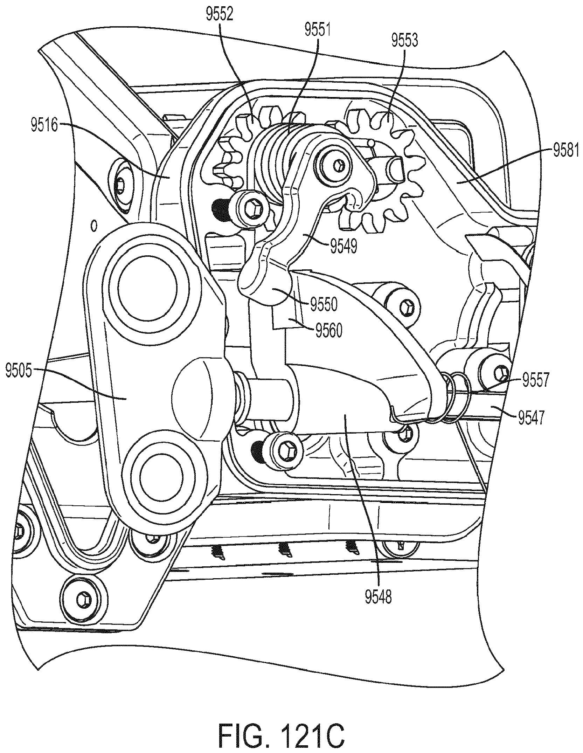

When the dial 9505 has been turned to a fully open position, the cam 9548 engages into a detent 9560 of the cam 9548. Fig. 121C shows a close-up view to illustrate the detent 9560. As is easily seen in Fig. 121C, the cam follower 9550 may fit into the detent 9560, which holds the dial 9505 in a "dwell" position. That is, although a user may remove their hand from the dial 9505, the dial 9505 remains in the fully open position as shown in Fig. 121C. In some embodiments, the spring 9557 does not provide enough torque on the shaft 9547 to overcome the detent 9560 without user assistance.

When the dial 9505 is turned from the open position as in Fig. 121B back to the closed position, the pivotable jaw members 9508, 9510 will rotate toward a flange 9517 of a plunger 9519 of a syringe 9518 (see Figs. 114G and 114H). However, the pivotable jaw members 9508, 9510 will stop rotating toward each other when they contact the flange 9517 of the plunger 9519 as shown in Fig. 114H). Referring again to Figs. 121A-121B, this will cause the cam follower 9550 to leave the cam 9548 because the surface of the cam 9548 will continue to move away from the cam follower 9550. The rocker arm 9549 is unable to rotate further because it is coupled to the jaw members 9510 (see Fig. 114H) whose movement is constrained by the flange 9517 of the plunger 9519 of the syringe 9518.The position of the pivotable jaw members 9508, 9510 may be determined by one or more potentiometer(s) 9559 and communicated to a processor. The processor may use this position to estimate a size characteristic of the syringe 9518. That is, the position of the pivotable jaw members 9508 when grasped around the plunger 9515 of the syringe 9518 and/or the position of the retaining member 9504 may be input parameters into a syringe database to determine which syringe model number is loaded to determine the internal diameter of the syringe. The syringe database may be stored internally (e.g., within a DAL file) and is downloaded via an enterprise system.

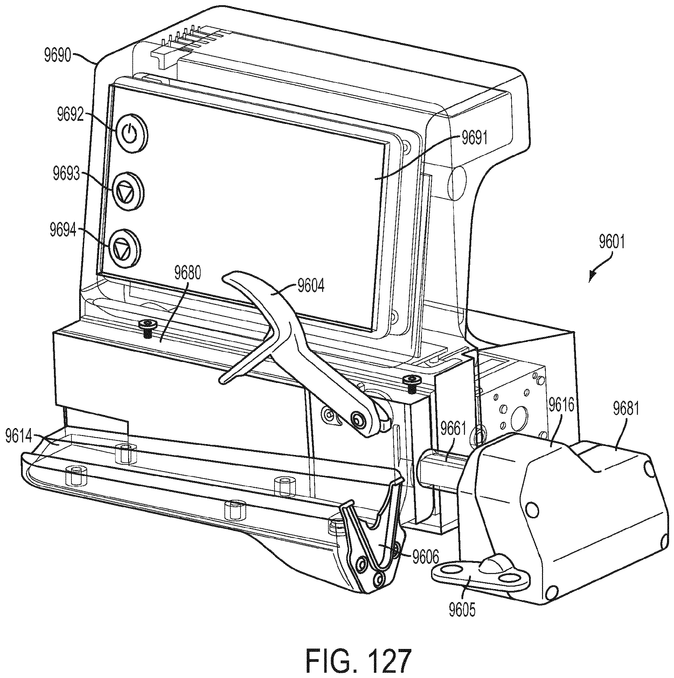

If the database identifies which syringe is loaded using the position of the pivotable jaw members 9508 when grasped around the plunger 9515 of the syringe 9518 and/or the position of the retaining member 9504, the internal diameter is used in the flow control algorithm as indicated in the database. However, there may be a collision in the database in which one or more syringes meet the criteria from the two sensors (in some specific embodiments). A touch screen (e.g., the touch screen 9691 of Fig. 127) may then request information from the user when the syringe 9515 is loaded. The user may be prompted by a touch screen that requests the user to enter into the touch screen 304 the manufacturer of the syringe 305, the model number of the syringe and/or other parameters to further narrow the list of possible syringes as found in the database. If a collision still exists, the user may be prompted by the display on the touch screen 304 to select the syringe model from a list or enter the model of the syringe that will deliver the medication. The user may be guided through a selection process on the touchscreen 304 to identify the syringe loaded into the machine using one or more of the following aspects: syringe barrel size, plunger head size, manufacturer names, images of syringes, and model numbers. The selection process may access a database of syringes including manufacturer, model, internal diameter and image. The syringe pump may use the identified syringe to set the internal diameter value for volume calculations (e.g., for the fluid delivery control algorithm).

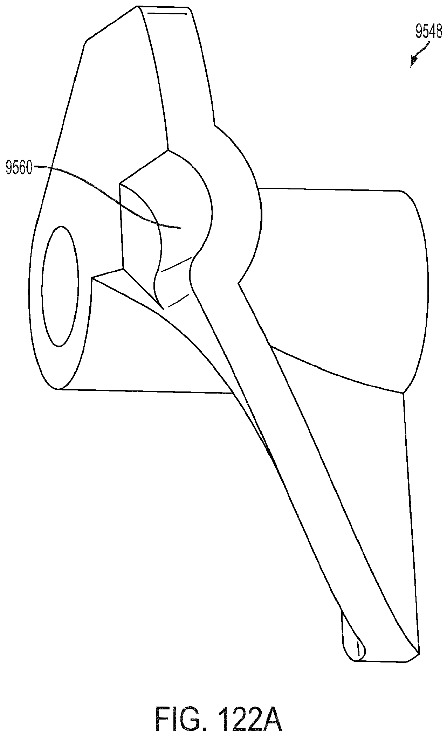

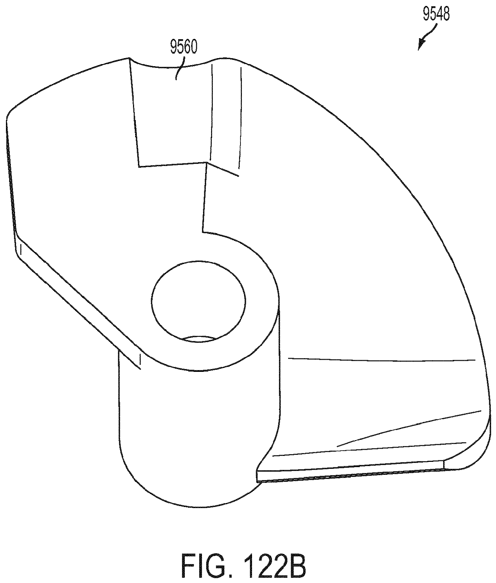

Figs. 122A-122B show two views of a cam 9548 (e.g., a dial shaft cam) which may, for example, be used within the plunger head assembly 9516 of the syringe pump assembly 9502 shown in Figs. 114A-114J in accordance with an embodiment of the present disclosure. The detent 9560 is easily seen in Figs. 121A-121B.

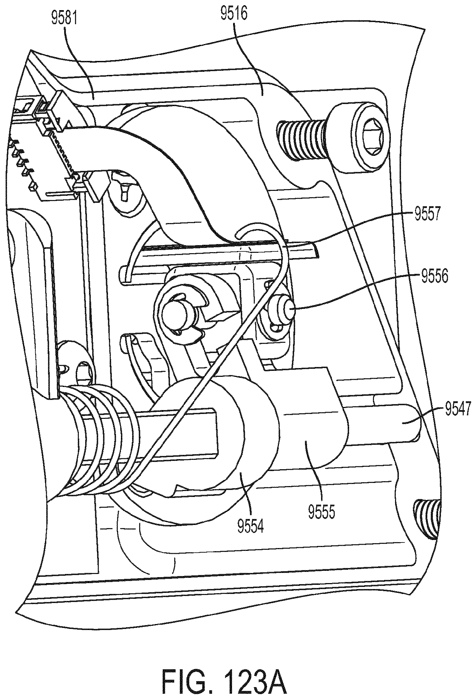

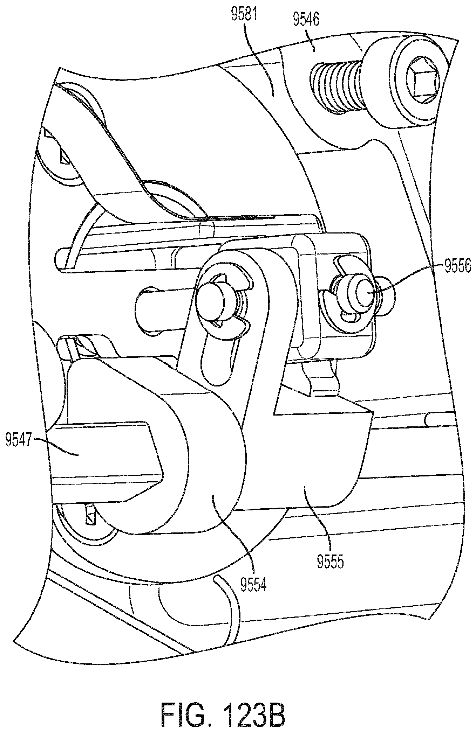

Figs. 123A-123B show two close-up views of the inner cavity of the plunger head assembly of the syringe pump assembly shown in Figs. 114A-114J in accordance with an embodiment of the present disclosure. As the shaft 9547 is rotated, the rod actuator 9554 rotates. When the dial 9505 (see Fig. 120) is near the fully open position, the rod actuator 9554 engages the link 9555 to pull the rod 9556 out as shown in Fig. 123B. The rod 9556 is spring biased into the plunger head assembly 9516.

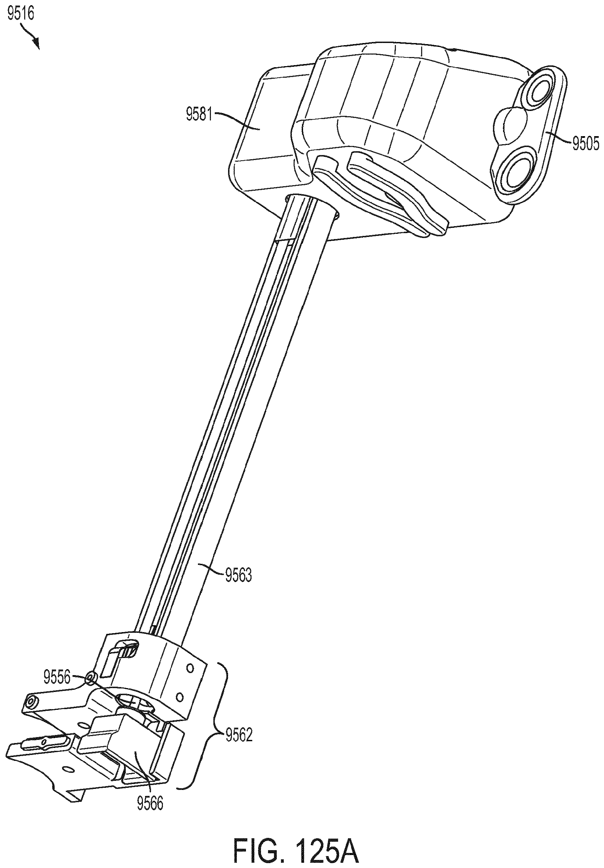

Fig. 124 shows the plunger head assembly 9516 of the syringe pump assembly shown in Figs. 114A-114J in accordance with an embodiment of the present disclosure. As is seen in Fig. 124, the plunger head assembly 9516 includes a half-nut assembly 9562 having a linear cam 9566 coupled to the rod 9556. A plunger tube 9561 connects the half nut assembly 9562 with the rest of the plunger head assembly 9516. The plunger tube 9561 shown in Fig. 124 is removed in Figs. 125A-125B showing a rod guide 9563. As is easily seen in Figs. 125A-125B, the rod guide 9563 guides the rod 9556. Note that a spring 9564 is coupled to a collar 9565 to bias the rod 9556 toward the half-nut assembly 9562.

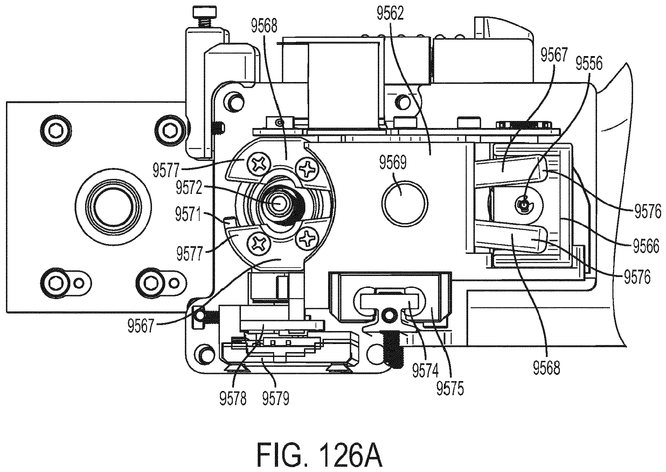

Figs. 126A-126I show several additional views of the syringe pump assembly 9502 of Figs. 114A-114J in accordance with an embodiment of the present disclosure. Referring to Fig. 126A, the half-nut assembly 9562 is easily viewable because the syringe seat 9514 (see Fig. 114A) is removed and a cover of the syringe pump assembly 9502 is also removed.

The half-nut assembly 9562 can be coupled to a lead screw 9572 such that rotation of the lead screw 9572, linearly actuates the half-nut assembly 9562. The half nut assembly 9562 includes a linear bearing 9575 that can travel on a track 9574. As the half nut assembly 9562 travels, a sensor 9578 engages with a linear resistance 9579 to form a linear potentiometer, which is used to estimate the linear position of the half nut assembly 9562 which is communicated to the processor to estimate the discharge of fluid from a syringe (e.g., syringe 9518 of Fig. 114E).

The half nut assembly 9562 also includes a linear cam 9566 coupled to the rod 9556 (also see Fig. 124), first and second half-nut arms 9567, 9568, and a pivot pin 9569. When the linear cam 9566 moves toward the first ends 9576 of the first and second half-nut arms 9567, 9568, the first and second half-nut arms 9567, 9568 pivot along the pivot pin 9569 such that the second ends 9577 of the first and second half-nut arms 9567, 9568 engage with the leadscrew. Each of the second ends 9577 of the first and second half-nut arms 9567, 9568 includes threads to engage with the lead screw 9572. A spacer 9571 ensures the distance between the first and second ends 9577 of the first and second half-nut arms 9567, 9568 are sufficiently distanced so that the half-nut assembly 9562 fully engages the lead screw 9572.

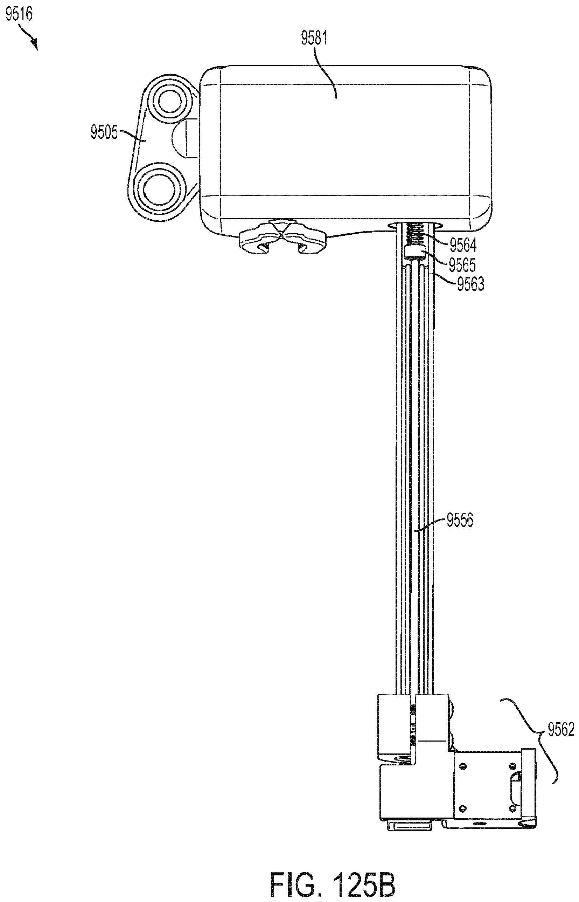

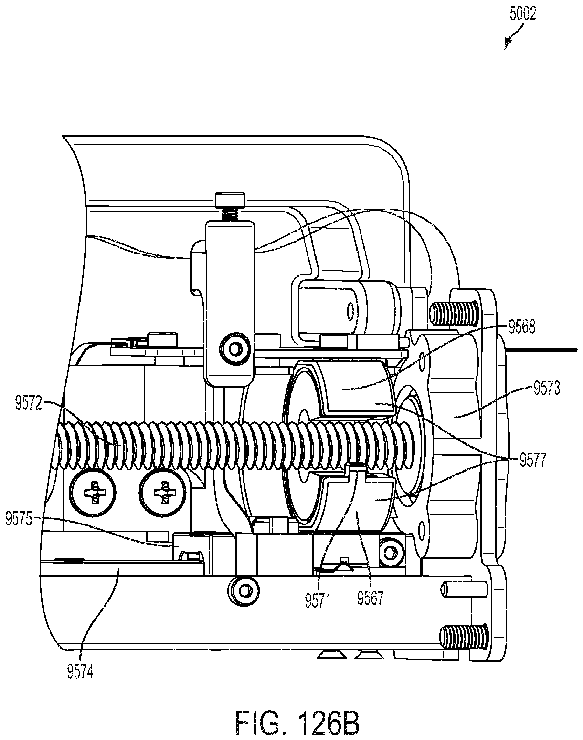

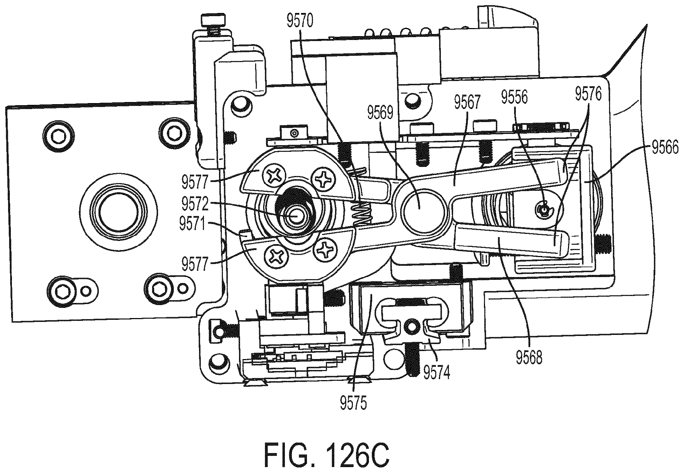

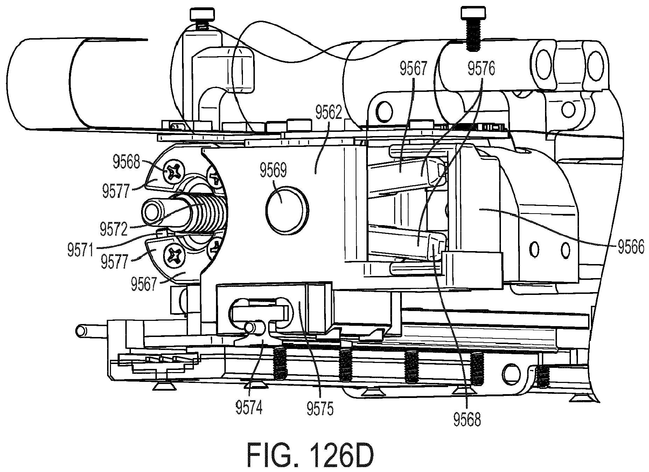

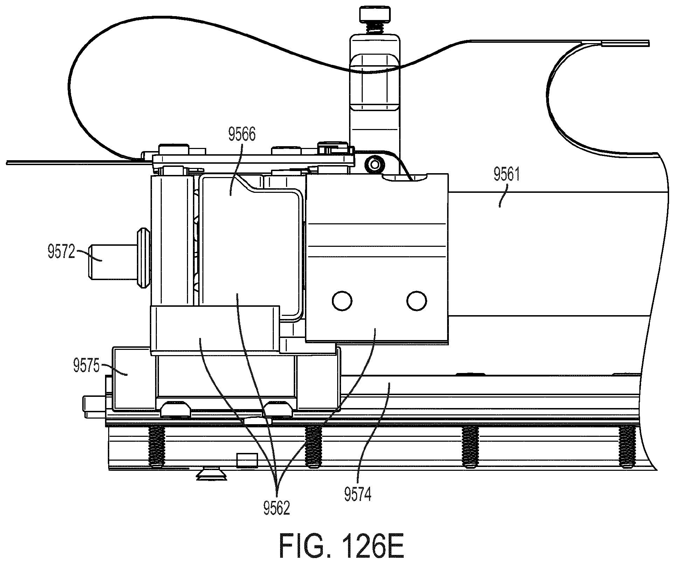

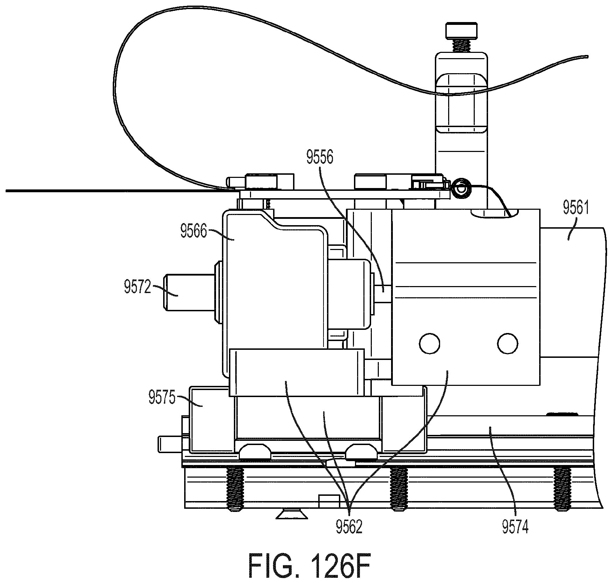

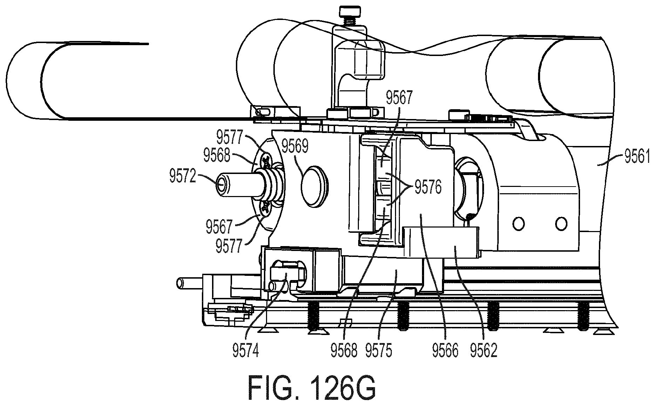

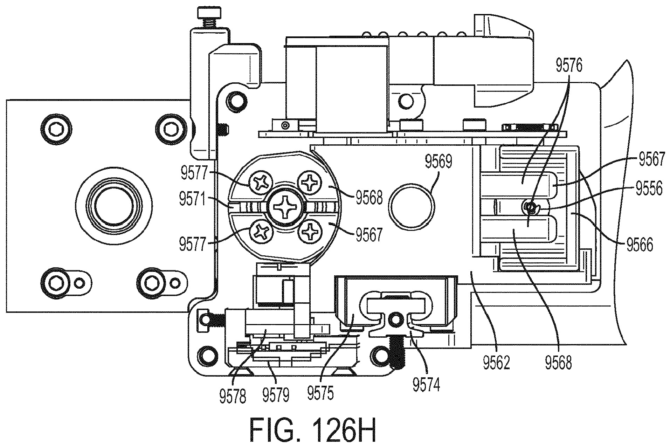

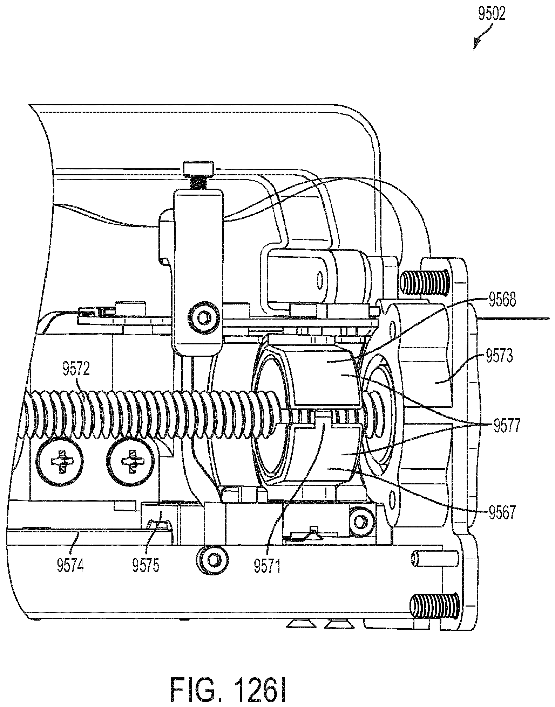

Fig. 126B shows a perspective, side-view of the syringe pump assembly 9502. Note the first and second half-nut arms 9567, 9568, include internal threads to engage with the lead screw 9572. A bearing 9573 is coupled to the lead screw 9572 to allow it to rotate. Fig. 126C shows the plunger head assembly 9516 with the cover of the half-nut assembly 9562 off. Note that a spring 9570 opens the first ends 9577 of the first and half-nut arms 9567, 9568, away from the lead screw 9572. Fig. 126D shows a perspective angled-view to illustrate how the first ends 9576 of the first and second half-nut arms 9567, 9568, engage with the linear cam 9566. Fig. 126E shows a side view of the half-nut assembly 9562. The linear cam 9566 is in a retracted position which occurs when the dial 9505 is in a fully-open position. Note that the rod 9556 is retracted by a spring 9564 (see Fig. 125B). Fig. 126F shows the linear cam 9566 is an engagement position. As is viewable in Fig. 126G, the linear cam's 9566 surface has actuated the first ends 9576 of half-nut arms 9567, 9568. When in this position, the linear cam's 9566 surface engages with the first ends 9576 of half-nut arms 9567, 9568 such if a force was applied to open the first ends 9576 of half-nut arms 9567, 9568 away from each other, no translation of force will be experienced by the rod 9556. That is, the linear cam's 9566 surface engages with the first ends 9576 of half-nut arms 9567, 9568 such that the contacting surfaces are pallalel with each other and parallel with an axis of the rod 9556. Figs. 126H and 126I shows two views where the half-nut assembly 9562 is fully engaged with the lead screw 9572 wherein rotation of the lead screw 9572 linearly actuates the half-nut assembly 9562 (and hence the entire plunger head assembly 9516 relative to the syringe pump assembly 9502.

Fig. 127 shows a perspective, side-view of the syringe pump assembly 9601 coupled to a display 9690. Note the syringe pump assembly 9601 is shown and includes a body 9680, a syringe seat 9614, and a plunger head assembly 9616. The plunger head assembly 9616 includes a plunger head 9681, a half-nut assembly 9562 (refer to Fig. 114A), and a plunger tube 9661. A syringe (e.g., see Fig. 114E for the syringe 9518) may be placed into the syringe seat 9614, which is secured by the retaining member 9604 and a retaining clip 9606. A dial 9605 opens the pivotal jaw members 9508, 9510 (refer to Fig. 114A) and allows the plunger head assembly 9616 to move away from or toward the syringe seat 9614. The display 9690 includes a screen 9691, a power button 9692, an alarm silence button 9693, and a menu button 9694. The pump assembly 9601 is configured to show a plurality of displays on the screen 9691 relating to pump operation and patient data.

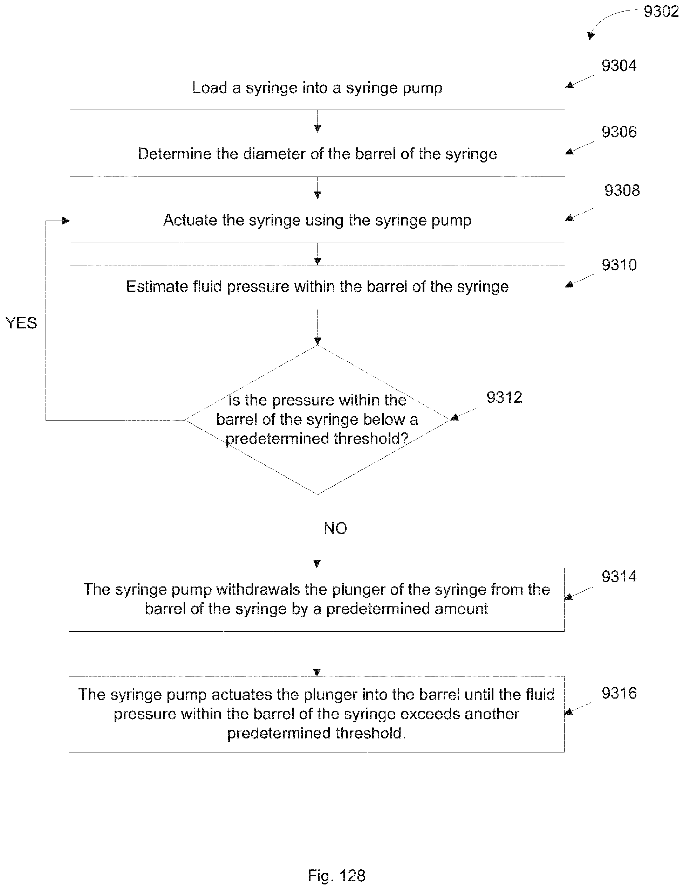

Fig. 128 shows a flow chart diagram of a method 9302 for discharging fluid from a syringe and for providing mitigation for an occlusion condition in accordance with an embodiment of the present disclosure. The method 9302 may be implemented by a syringe pump, such as the syringe pump shown in Fig. 127. The acts may be implemented by or using one or more processors on a syringe pump.

The method 9302 will be described as being implemented by the syringe pump shown in Fig. 127; however, such description should not be construed as limiting. The method 9302 may be implemented on any pump that discharges fluid, e.g., any syringe pump described herein. The method 9302 includes acts 9304-9316. Act 9304 loads a syringe into a syringe pump. For example, a syringe may be loaded into the syringe seat 9614. Act 9306 determines the diameter of a barrel of the syringe. The syringe's barrel diameter may be determined by the position of the retaining finger 9604. Act 9308 actuates the syringe using the syringe pump. The plunger head assembly 9616 may actuate a plunger of the syringe. Act 9310 estimates fluid pressure within the barrel of the syringe. Act 9312 makes a decision based upon whether the pressure within the barrel of the syringe is below a predetermined threshold? If the decision is yes, then acts 9308-9312 may continue to achieve a target flow rate until a target fluid discharged dose is achieved.

If the decision is no in Act 9312, in Act 9314: the syringe pump withdrawals the plunger of the syringe from the barrel of the syringe by a predetermined amount (which may be a distance of actuation or a volume of actuation of the syringe. In Act 9316, the syringe pump actuates the plunger into the barrel until the fluid pressure within the barrel of the syringe exceeds another predetermined threshold. The one or more processors may sound an alarm or alert notifying a caregiver of the occlusion.

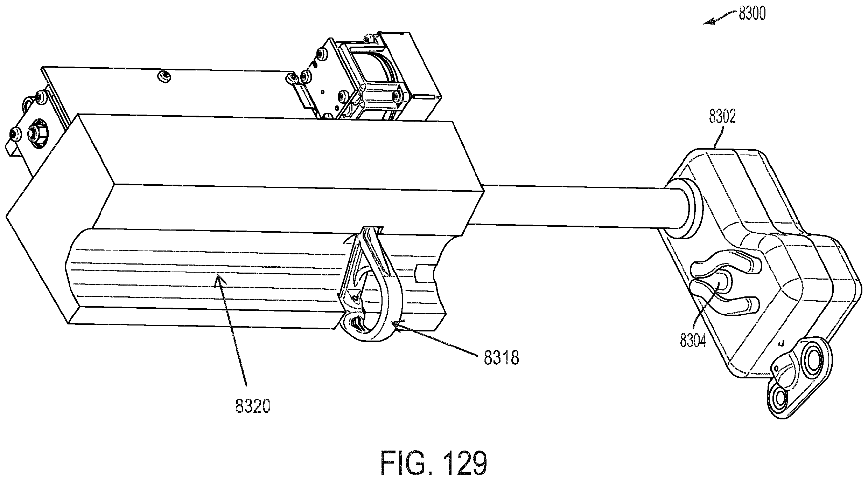

Fig. 129 shows a syringe pump assembly 8300 in accordance with another embodiment of the present disclosure. The syringe pump assembly 8300 includes a plunger head 8302. The plunger head 8203 includes a pressure sensor assembly 8304 to sense the force that a loaded syringe has on the plunger head 8302. Note that the retaining members are approximately co-aligned with the length of the pressure sensor assembly 8304 and allow for a variety of sizes and shapes of syringes to be retained by the syringe pump assembly 8300. When a syringe is loaded into the syringe bed 8320, a retaining member 8318 may retain the syringe and the plunger head 8302 may securely be coupled to the flange of the plunger of the syringe such that the barrel of the syringe is firmly coupled to the pressure sensor assembly 8304.

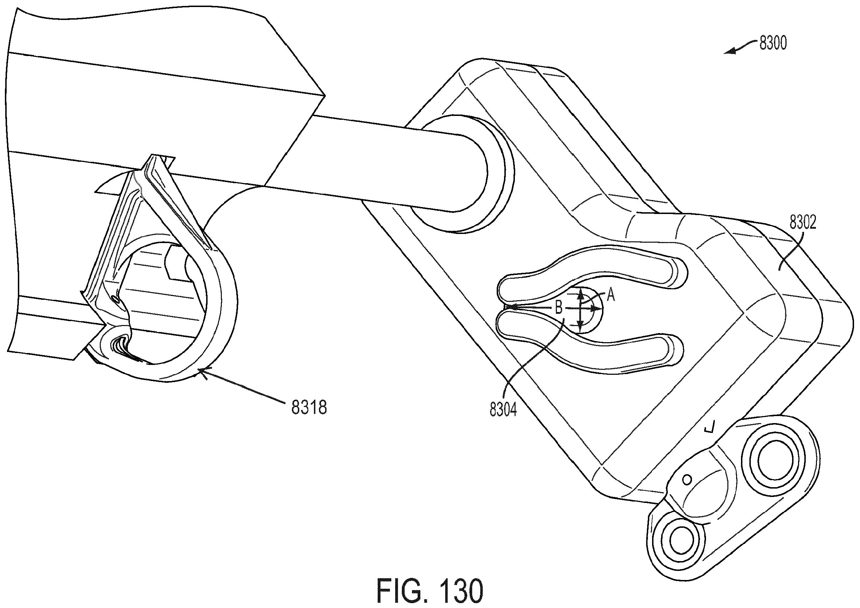

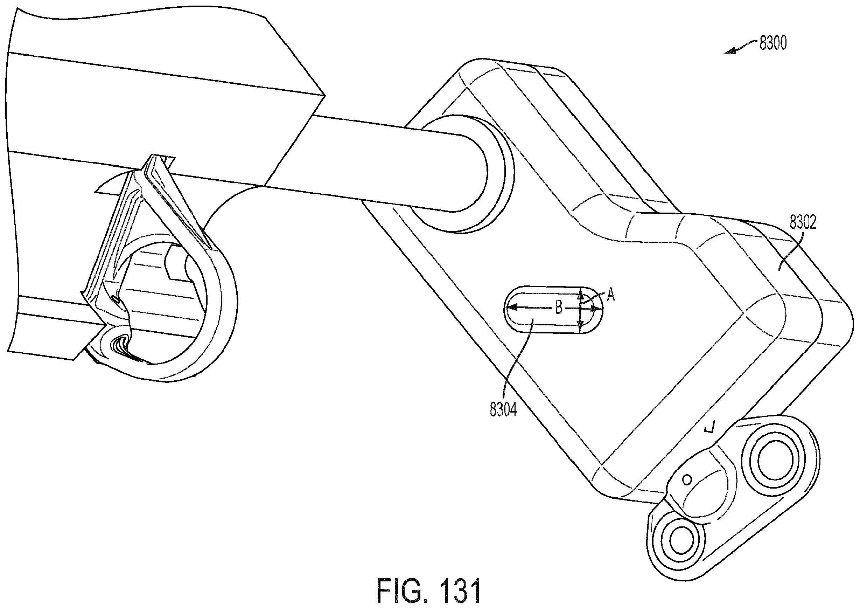

Fig. 130 shows a close-up view of the plunger head 8302 of the syringe pump assembly 8300 of Fig. 129 in accordance with an embodiment of the present disclosure. As is easily seen in Fig. 130, the pressure sensor assembly 8304 includes an elongated portion, designated by a line B and another portion designated by a line A that is orthogonal to line A. Fig. 131 shows the same view of Fig. 130 with the retaining members removed to facilitate viewing of the pressure sensor assembly 8304.

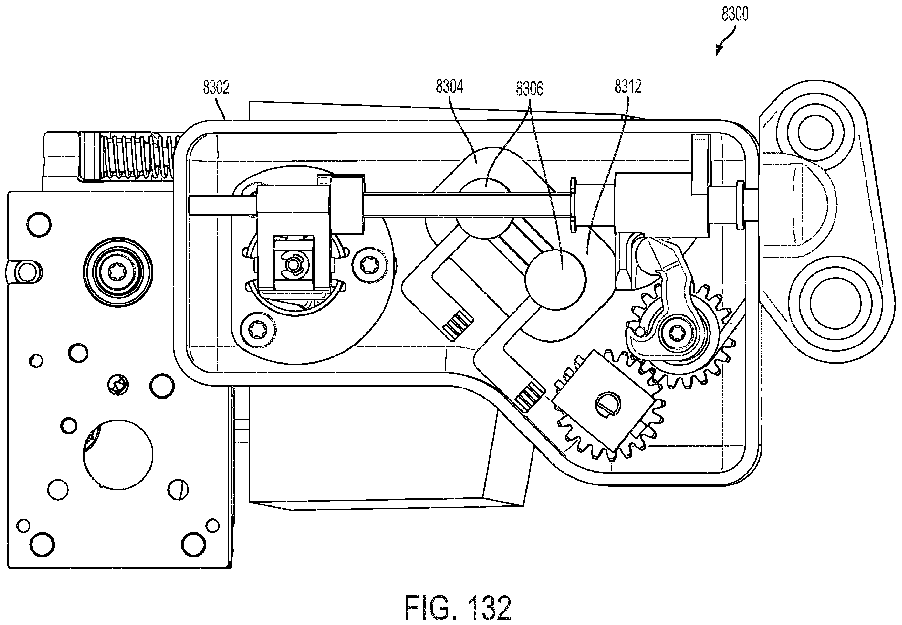

Fig. 132 shows the back of the plunger head assembly 8302 with the back cover removed to facilitate viewing of the two pressure sensors 8306 of the pressure sensor assembly 8304 in accordance with an embodiment of the present disclosure. The two pressure sensor 8306 can estimate the total force applied to the pressure sensor assembly 8304, estimate the location the force is applied, monitor the force, etc. A processor (e.g., processor 3500 of Fig. 59C and/or processor 3600 of Fig. 59D) coupled to the two pressure sensors 8306 can use the information to determine the pressure within the syringe, the location the syringe engages the pressure sensor assembly 8304 along line B (see Fig. 131). It is well known in the art how to use vector analysis to calculate the force vector applied to the pressure sensor assembly 8304 using the two pressure sensors 8306. The position in which the force is applied to the pressure sensor assembly 8304 may be used to correlated which syringe is loaded into the syringe pump as described above. This may be an additional parameter used by the syringe database to determine which syringe model is loaded into the syringe pump 8300 and/or may be a safety check in which if the force vector is applied in an unexpected location in accordance with the syringe database, the syringe pump may alarm and stop infusion.

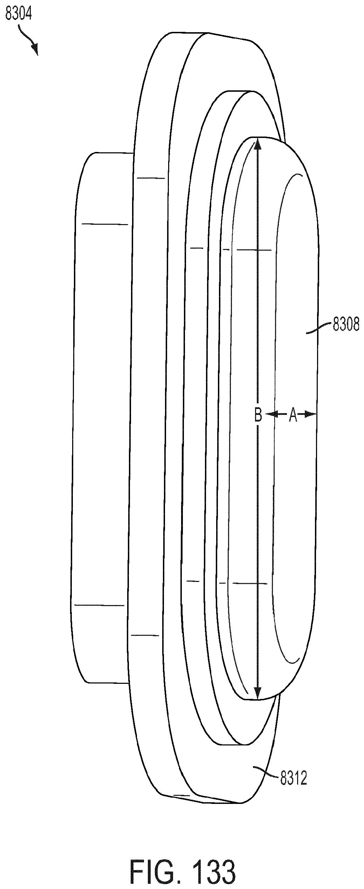

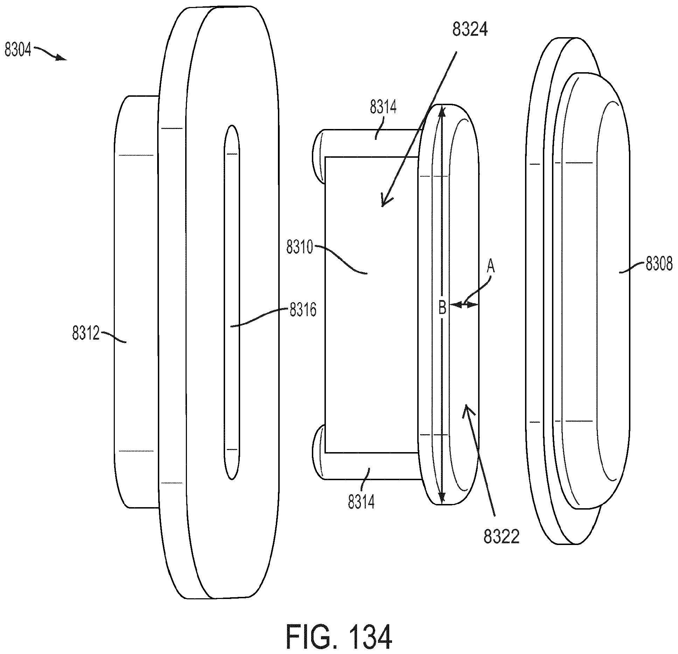

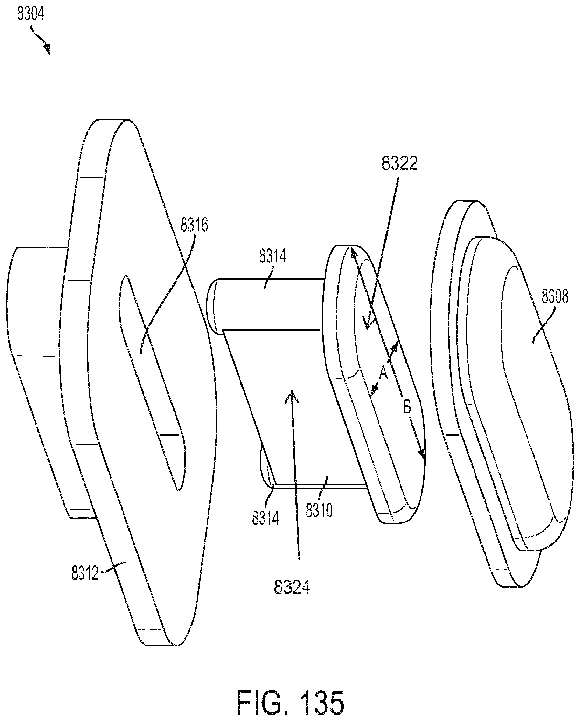

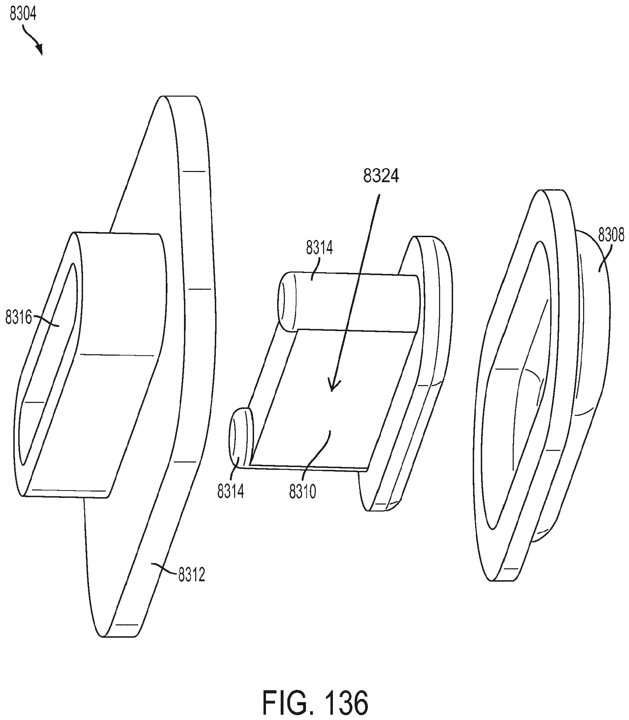

Fig. 133 shows the pressure sensor assembly 8304 without the pressure sensors 8306 (see Fig. 132, for example) in accordance with an embodiment of the present disclosure. Figs. 134-136 show exploded views of the pressure sensor assembly 8304. The pressure sensor assembly 8304 includes a cover 8308, a guide 8312, and a plunger 8314. The guide 8312 guides the plunger 8310 such that pressure applied to the cover 8308 is translated to the pressure sensors 8306. Extensions 8314 extend from the sensing surface 8322. The extensions 8314 may be secured together via a brace 8324 to couple together the extensions 8314.

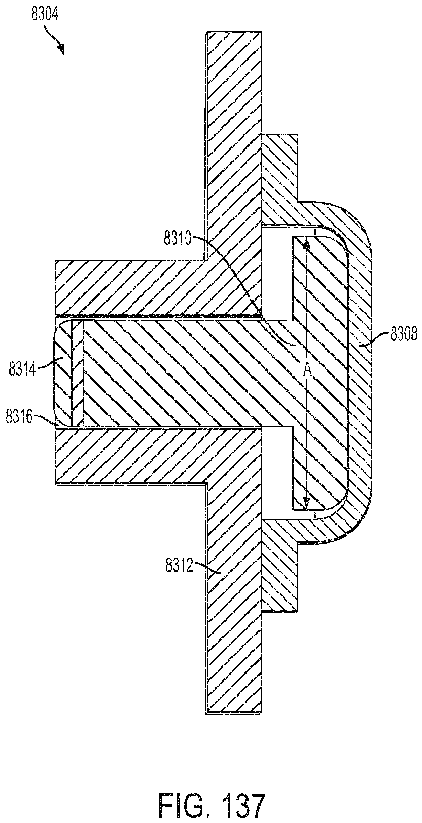

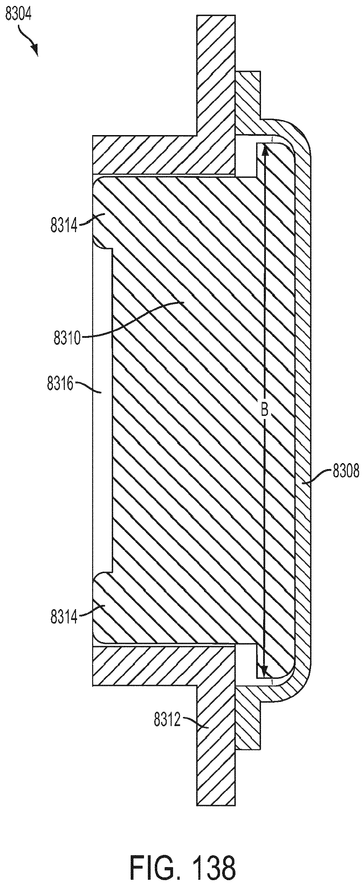

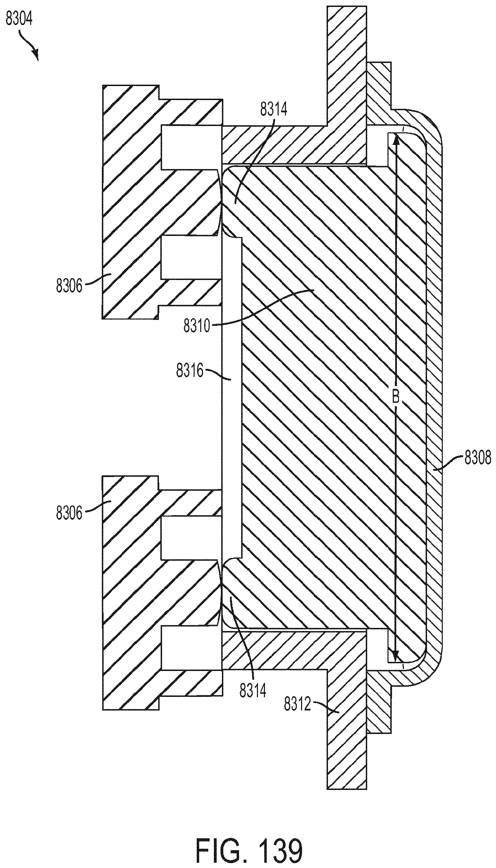

Fig. 137 shows a cross-sectional view of the pressure sensor assembly 8304 without the pressure sensors of Fig. 133 in accordance with an embodiment of the present disclosure. The cross-sectional view of Fig. 137 cuts a plane parallel with line A of Figs. 134-136. Fig. 138 shows another cross-sectional view of the pressure sensor assembly 8304 without the pressure sensors of Fig. 133 cut across a plane parallel with line B of Figs. 134-136. Fig. 139 the same cross-sectional view as Fig. 138 with the pressure sensors 8306 added. As is seen in Fig. 139, the extensions 8314 are shown as in contact with the pressure sensors 8306.

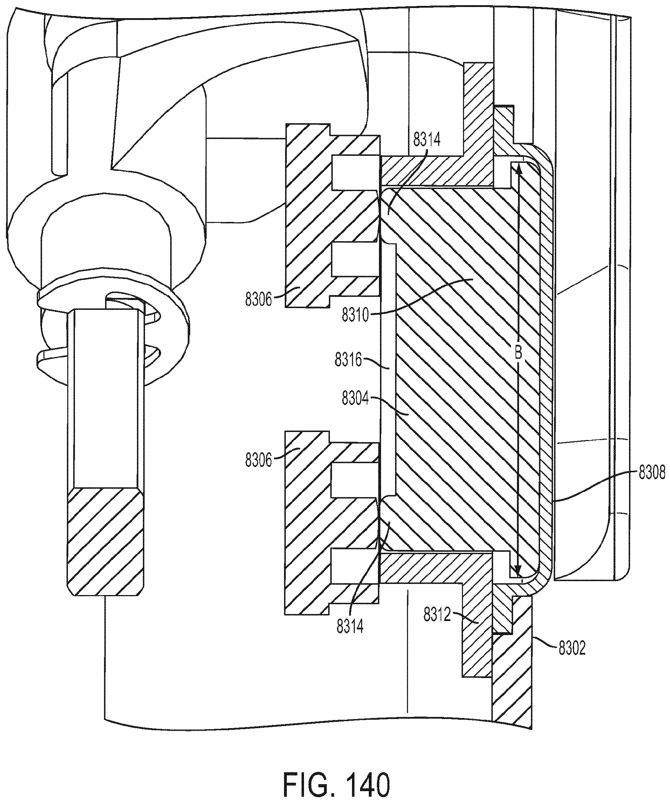

Fig. 140 shows the cross-sectional view of Fig. 139 with the pressure sensor assembly in the plunger head assembly in accordance with an embodiment of the present disclosure. Note the positioning of the membrane 8308 relative to the pressure sensors 8306.

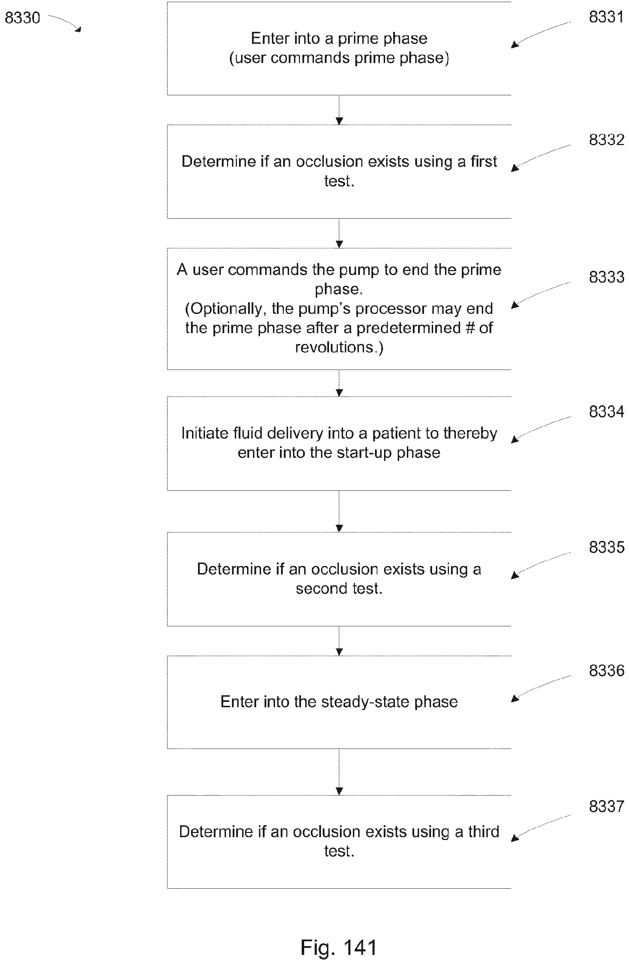

Fig. 141 shows a method 8330 for occlusion detection in accordance with an embodiment of the present disclosure. The method 8330 includes acts 8331-8337. The method may be for used for detecting an occlusion that occurs within a syringe pump and will described. However, the method may be used with any suitable pump.

The method 8330 may be implemented by one or more processors on a syringe pump. For example, the method 8330 may be implemented by the processor 3500 of Fig. 59C and/or the processor 3600 of Fig. 59D. The method may be performed by the one or more processors by executing a set of instructions stored in memory (e.g., an internal memory or memory coupled to the processor).

Act 8331 enters the syringe pump into a prime phase. The prime phase may be initiated by a user by pressing a prime button on a touchscreen display. The prime phase may be to clear out any air in the line connected to the syringe pump. The prime phase may end when the user presses a stop button, or, in some embodiments, the prime phase may last as long as the user continues to press down on the prime button. The button may be on the touchscreen or may be a pushbutton.

While in the prime phase, act 8332 determines if an occlusion exists using a first test. A user may command the pump to end the prime phase in act 8333. The prime phase may be ended in act 8333 by the user pressing a user interface (e.g., a touchscreen) in operative communication with the syringe pump (e.g., physically attached to the syringe pump, or may be in communication with the syringe pump). Optionally, in some embodiments of the present disclosure, the pump's processor may end the prime phase after a predetermined number of revolutions or after a predetermined distance of actuation. After act 8333, the syringe pump may pause until act 8334 occurs.

Act 8334 initiates fluid delivery into a patient which also starts the start-up phase. Fluid delivery may be initiated by user input into a user interface in operative communication with the syringe pump. Act 8335 determines if an occlusion exists using a second test. That is, act 8335 uses the second test to determine if an occlusion in the fluid line occurs during the start-up phase.

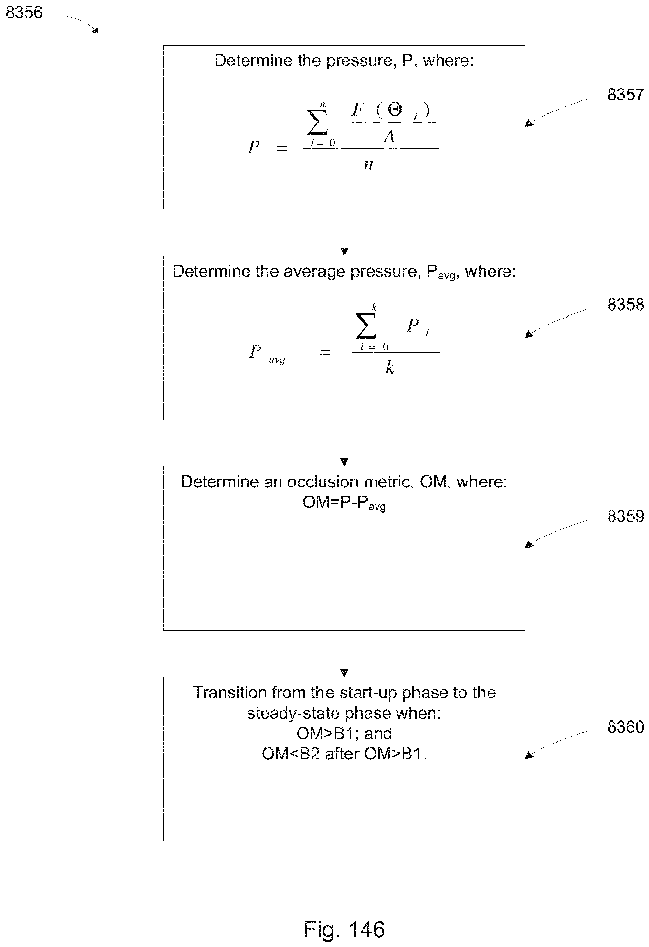

In act 8336, the method 8330 enters into the stead-state phase. The syringe pump may transition into the steady-state phase from the start-up phase using one or more criteria. An example is provided below in regard to Fig. 146. While in the steady-state phase, the method 8330 determines in act 8337 if an occlusion exists using a third test.

Although the acts 8331-8337 are shown in flow chart form, and combination or ordering may be used. For example, some acts may occur simultaneously, in a step-wise fashion, or in another order.

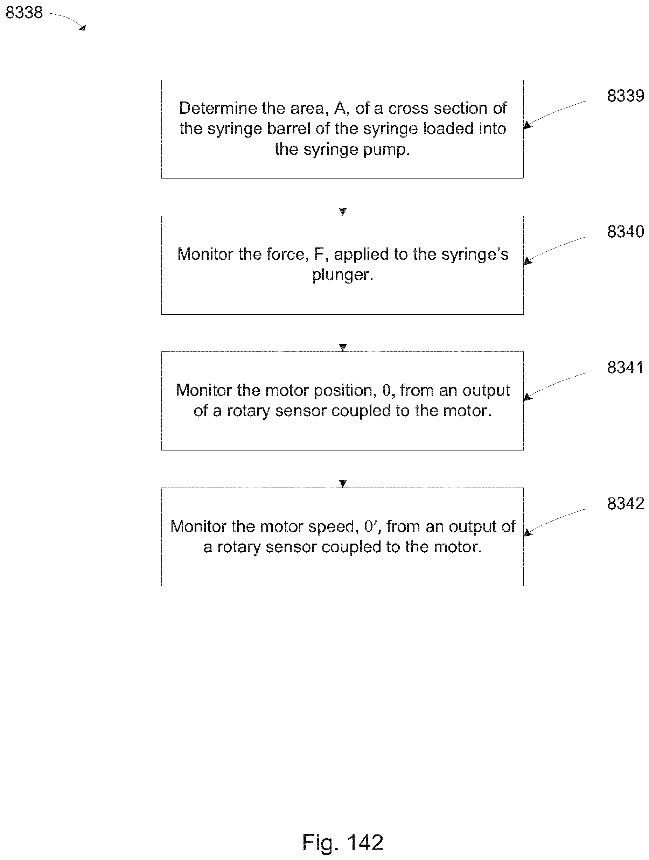

Fig. 142 shows a method 8338 of monitoring a syringe pump's sensors for occlusion detection in accordance with an embodiment of the present disclosure. The method 8338 may occur at anytime, for example, during power-up, during a power-on-self-test, or when the syringe pump enters into the prime phase. The method 8338 may be used by the first, second and/or third tests of the method 8330 of Fig. 141. Act 8339 determines the area, A, of a cross section of the syringe barrel of the syringe loaded into the syringe pump. This may be done by using sensors as described above to determine the syringe's make and/or model numbers. The user may be asked to select from a list of possible syringes if the measured parameters correspond to more than one syringe. In some embodiments, the area, A, is determined by directly entering the value into a user interface of the syringe pump. In yet additional embodiments, the area A, is determined by reference to a look-up table using the model number as entered by the user into a user interface of the syringe pump or as indicated by a bar code scanned by the syringe pump or as scanned by a bar code scanner in operative communication with the syringe pump.

Act 8340 monitors the force, F, applied to the syringe's plunger 8340. Pressure within the syringe may be determined using the force measurement, F, and the area, A, determined in act 8339.

Act 8341 monitors the motor position, θ, from an output of a rotary sensor coupled to the motor. Act 8342 monitors the motor speed, θ', from an output of a rotary sensor coupled to the motor. The rotary sensor of acts 8341 and 8342 may be the same rotary sensor or may be different rotary sensors. The area, A, the force, F, the motor position, θ, and the motor speed θ' may be used by the first, second and/or third tests.

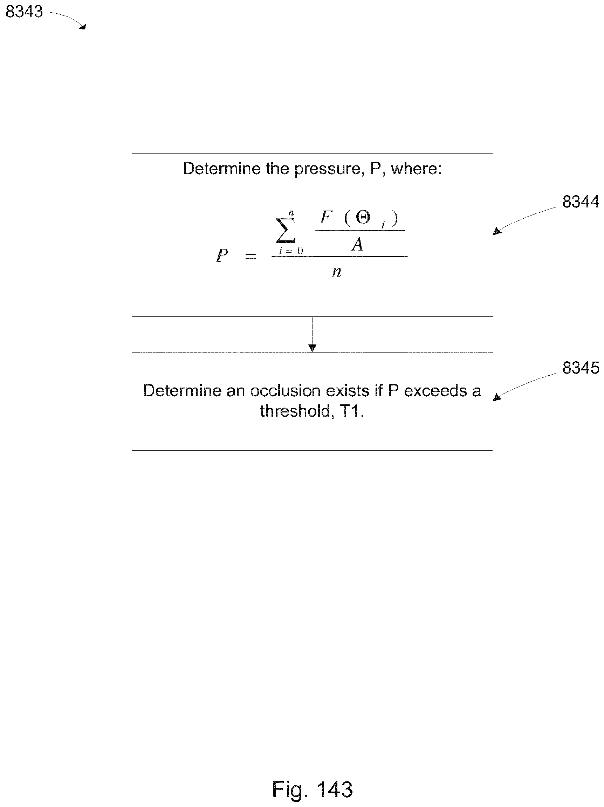

Fig. 143 shows a methodology 8343 for performing a test used by the method illustrated in Fig. 141 in accordance with an embodiment of the present disclosure. That is, the methodology 8343 may be the first test of act 8332 of Fig. 141.

As shown in Fig. 143, the methodology 8343 includes acts 8344-8345. Act 8344 determines the pressure, P, in accordance with the following formula:
$P = \frac{{\sum }_{i = 0}^{n} \frac{F \left({Θ}_{i}\right)}{A}}{n} ,$ where F(θi) is the force sample taken at a particular θi where the 'i' denotes a series of samples from 0 to n. The A is the area a cross section of the syringe barrel of the syringe loaded into the syringe pump. A summation of forces, F(θi) where i=0...n, are divided by the area A; the result of which is divided by n (the number of samples). The samples may be over a half-motor revolution (e.g., the sample n may be set to the most recent sample and the sample 0 is the sample taken at the beginning of the half-motor revolution). That is, the samples from i=0...n may be the trailing samples previously taken in accordance with the half-revolution of the motor. Act 8345 determines that an occlusion exists when P exceeds a threshold, T1. The threshold, T1 may be a predetermined threshold and may be determined empirically.

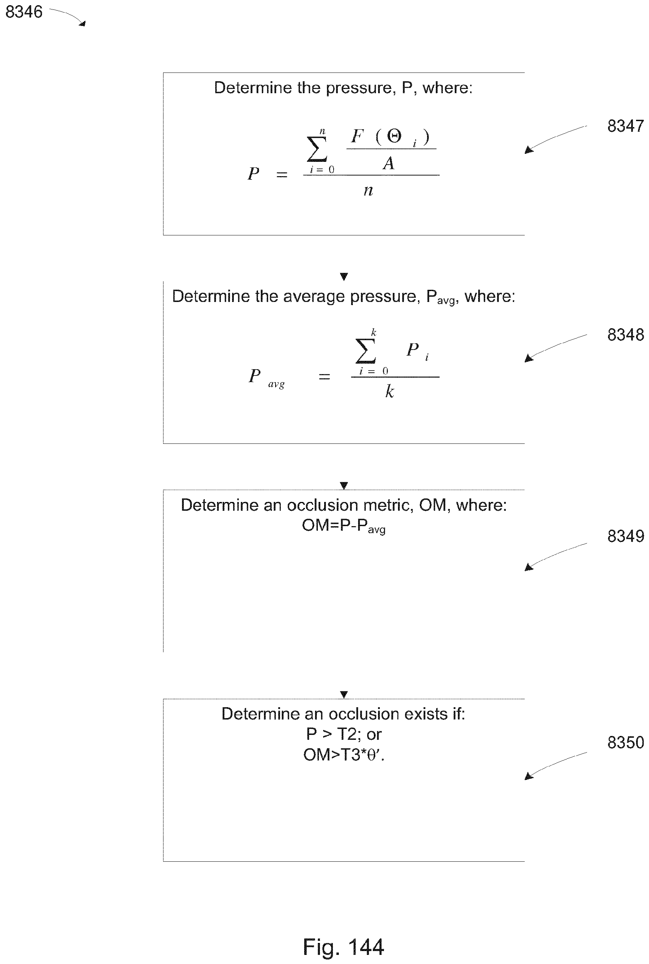

Fig. 144 shows another methodology 8346 for performing a test used by the method 8330 (in some embodiments) illustrated in Fig. 141 in accordance with an embodiment of the present disclosure. The methodology 8346 may be the second test of act 8335 of Fig. 141.

As shown in Fig. 144, the methodology 8346 includes acts 8347-8350. Act 8347 determines the pressure, P. Act 8348 determines the average pressure, P_{avg}. P_{avg} may be calculated as follows:
${P}_{avg} = \frac{{\sum }_{i = 0}^{k} {P}_{i}}{k} .$ The Pi are pressure values (e.g., taken in accordance with P of act 8347). The i=0...k denotes that a plurality of samples are taken and summed together, the result of which is divided by k; for example, the samples may be the samples taken of P during the last 5 revolutions of the motor thereby if P is taken every half-motor revolution, k will be equal to 10. Act 8349 determines an occlusion metric, OM, where OM=P- P_{avg}. Act 8350 determines an occlusion exists if: P>T2, or OM>T3* θ'. T2 and T3 are second and third thresholds. T2 and T3 may be predetermined and/or may be determined empirically.

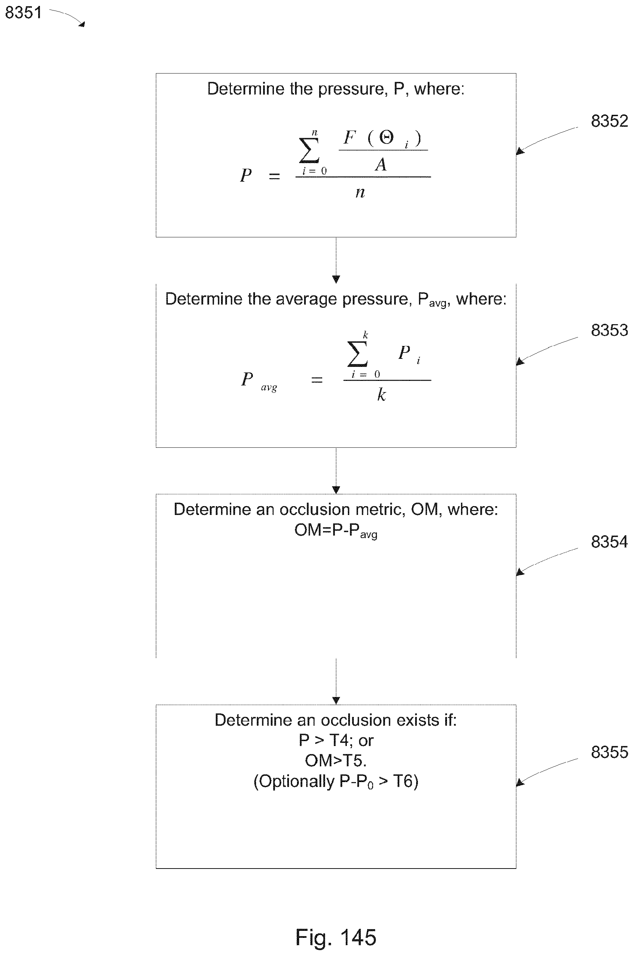

Fig. 145 shows a yet another methodology 8351 for performing a test used by the method 8330 illustrated in Fig. 141 in accordance with an embodiment of the present disclosure. The test of the methodology 8351 may be the third test performed in act 8337 of Fig. 141. Acts 8352-8354 may be the same acts as acts 8347-8349 of Fig. 144. That is, the values P, P_{avg}, and OM may be determined during acts 8352-8354.

Act 8355 determines an occlusion exists if: P>T4; or OM>T5. Optionally, in some additional embodiments, act also determines if P-P₀>T6 to determine if an occlusion exists. T4, T5, and T6 may be predetermined thresholds and/or may be determined empirically. P₀ may be the first pressure sample taken when or after the syringe pump transitions from a start-up phase to a steady-state phase.

Fig. 146 shows a methodology 8356 for transitioning from a start-up phase to a steady-state phase of a syringe pump within the method of Fig. 141 in accordance with an embodiment of the present disclosure. That is, the methodology 8356 may be used by the method 8330 of Fig. 141 to determine when to perform act 8336.

Fig. 146 shows acts 8357-5360 of the methodology 8356. Acts 8357-5359 may be the same acts as acts 8347-8349 of Fig. 144. That is, the values P, P_{avg}, and OM may be determined during acts 8357-5359. During act 8360, the syringe pump may transition from the start-up phase to the steady-state phase when OM>B1, and OM<B2 after OM>B1. B1 and B2 may be predetermined values that are determined empirically.

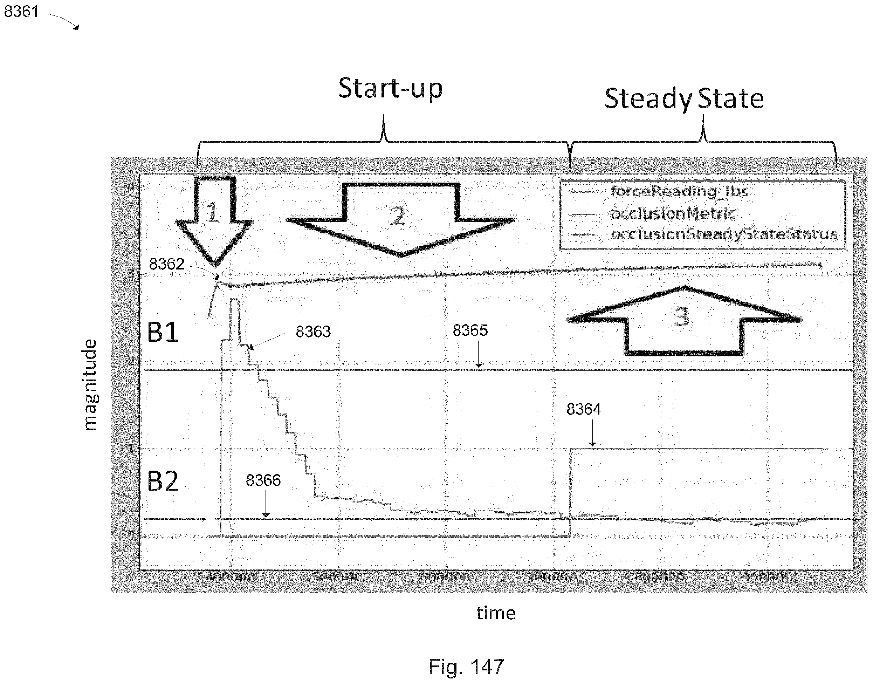

Fig. 147 show a graphic illustration 8361 of a syringe pump transitioning from a start-up phase to a steady-state phase in accordance with an embodiment of the present disclosure. Fig. 147 is an illustration to show the operation of an embodiment of the methods of Fig. 146.

Line 8362 is a representation of the force applied to a syringe. Line 8363 is a calculated occlusion metric ("OM"). Line 8364 illustrates the transition from the start-up phase to the steady-state phase. A value B1 is labeled as 8365, and a value B2 is labeled 8366. As shown in Fig. 147, the OM 8363 must exceed a value B1 8365, and then, thereafter, must be less than a second value B2 8366, which causes the syringe pump to transition from the start-up phase to the steady-state phase as indicated by the line 8364 (line 8363 is at zero when the syringe pump is in the start-up phase and is at one when the syringe pump is in the stead-state phase.

Note that at the start of a delivery, the force (line 8362) spikes as the mechanical backlash in the syringe is taken up by the movement of the plunger arm. This causes the OM (line 8362) to spike and then drop. This quick increase and decrease is determined using the thresholds 8365 and 8366 to determine when the syringe has reached the steady state.

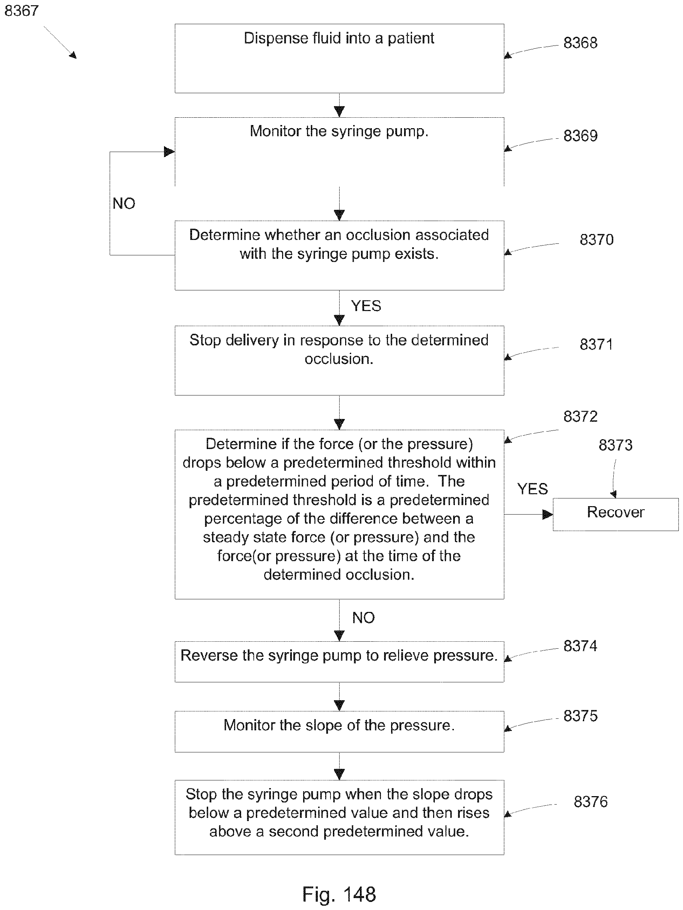

Fig. 148 shows a flow chart diagram used to illustrate a method 8367 for recovering from an occlusion in accordance with an embodiment of the present disclosure. The method 8367 includes acts 8368-8376.

Act 8368 dispenses fluid into a patient. Act 8369 monitors the syringe pump. Act 8370 determines whether an occlusion associated with the syringe pump exists. Act 8371 stops delivery in response to the determined occlusion. Act 8372 determines if the force (or the pressure) drops below a predetermined threshold within a predetermined period of time. The predetermined threshold is a predetermined percentage of the difference between a steady state force (or pressure) and the force (or pressure) at the time of the determined occlusion.

Act 8373 recovers when the force (or the pressure) drops below a predetermined threshold within a predetermined period of time Act 8374. Act 8373 may cause the syringe pump to alarm, alert and/or reattempt to dispense fluid into the patient.

Act 8375 reverse the syringe pump to relive the pressure. Act 8376 stops the syringe pump when the slope drops below a predetermined value and then rises above a second predetermined value.

Various alternatives and modifications can be devised by those skilled in the art without departing from the disclosure. Accordingly, the present disclosure is intended to embrace all such alternatives, modifications and variances. Additionally, while several embodiments of the present disclosure have been shown in the drawings and/or discussed herein, it is not intended that the disclosure be limited thereto, as it is intended that the disclosure be as broad in scope as the art will allow and that the specification be read likewise. Therefore, the above description should not be construed as limiting, but merely as exemplifications of particular embodiments. And, those skilled in the art will envision other modifications within the scope and spirit of the claims appended hereto. Other elements, steps, methods and techniques that are insubstantially different from those described above and/or in the appended claims are also intended to be within the scope of the disclosure.

The embodiments shown in the drawings are presented only to demonstrate certain examples of the disclosure. And, the drawings described are only illustrative and are non-limiting. In the drawings, for illustrative purposes, the size of some of the elements may be exaggerated and not drawn to a particular scale. Additionally, elements shown within the drawings that have the same numbers may be identical elements or may be similar elements, depending on the context.

Where the term "comprising" is used in the present description and claims, it does not exclude other elements or steps. Where an indefinite or definite article is used when referring to a singular noun, e.g., "a," "an," or "the," this includes a plural of that noun unless something otherwise is specifically stated. Hence, the term "comprising" should not be interpreted as being restricted to the items listed thereafter; it does not exclude other elements or steps, and so the scope of the expression "a device comprising items A and B" should not be limited to devices consisting only of components A and B. This expression signifies that, with respect to the present disclosure, the only relevant components of the device are A and B.

Furthermore, the terms "first," "second," "third," and the like, whether used in the description or in the claims, are provided for distinguishing between similar elements and not necessarily for describing a sequential or chronological order. It is to be understood that the terms so used are interchangeable under appropriate circumstances (unless clearly disclosed otherwise) and that the embodiments of the disclosure described herein are capable of operation in other sequences and/or arrangements than are described or illustrated herein.

The present application is a divisional of EP15707510.2. EP15707510.2 is a European regional phase application originating from PCT/US2015/016796. The original claims of PCT/US2015/016796 are presented as statements below so that the subject-matter of those claims is included in its entirety in the present application.
1. A syringe pump comprising:
   a body;
   a syringe seat coupled to the body;
   a syringe actuator configured to actuate a syringe secured within the syringe seat;
   a memory configured to store a plurality of instructions stored thereon; and
   at least one processor, in accordance with the plurality of instructions, configured to:
      prime the syringe pump in a prime phase;
      determine if an occlusion exists during the prime phase using a first test;
      stop the prime phase;
      initiate fluid delivery into a patient;
      enter into a start-up phase;
      determine if an occlusion exists using a second test during the start-up phase;
      transition from the start-up phase into a steady-state phase; and
      determine if an occlusion exists during the steady-state phase using a third test.
2. The syringe pump according to Para 1, wherein the at least one processor is configured to start the prime phase in response to a user input.
3. The syringe pump according to Para 2, further comprising a touch screen configured such that the user input is entered into the touch screen, the touch screen is in operative communication with the at least one processor, wherein the at least one processor and the touch screen are configured such that the at least one processor receives confirmation of the user input.
4. The syringe pump according to Para 1, further comprising:
   a force sensor configured to determine a force applied to the syringe by actuation of the syringe actuator; and
   a motor sensor operatively coupled to a motor of the syringe pump.
5. The syringe pump according to Para 4, wherein the at least one processor is in operative communication with the force sensor and the motor sensor.
6. The syringe pump according to Para 5, wherein the at least one processor is configured to use the motor sensor to determine a motor position and a motor rotation speed.
7. The syringe pump according to Para 6, wherein the at least one processor is configured to use the motor position, the motor rotation speed, and the force applied to the syringe to perform at least one of the first, second, and third tests.
8. The syringe pump according to Para 1, wherein the at least one processor is configured to perform the first test such that the at least one processor determines if the pressure, P, exceeds a first threshold to trigger an occlusion alarm.
9. The syringe pump according to Para 8, wherein the pressure, P, is determined by the at least one processor in accordance with: $P = \frac{{\sum }_{i = 0}^{n} \frac{F \left({Θ}_{i}\right)}{A}}{n} .$
10. The syringe pump according to Para 1, wherein the at least one processor is configured to perform the second test such that the at least one processor determines if the pressure, P, exceeds a second threshold or an occlusion metric exceeds a third threshold multiplied by a motor speed to trigger an occlusion alarm.
11. The syringe pump according to Para 10, wherein the at least one processor is configured to determine the pressure, P, in accordance with: $P = \frac{{\sum }_{i = 0}^{n} \frac{F \left({Θ}_{i}\right)}{A}}{n} .$
12. The syringe pump according to Para 10, wherein the occlusion metric, OM, is the pressure, P, minus an average pressure, Pavg.
13. The syringe pump according to Para 12, wherein the at least one processor is configured to determine the pressure, P, in accordance with: $P = \frac{{\sum }_{i = 0}^{n} \frac{F \left({Θ}_{i}\right)}{A}}{n} ,$ and the average pressure, Pavg, in accordance with: ${P}_{avg} = \frac{{\sum }_{i = 0}^{k} {P}_{i}}{k} .$
14. The syringe pump according to Para 1, wherein the at least one processor is configured to perform the third test such that the at least one processor determines if: a pressure, P, exceeds a fourth threshold; or an occlusion metric, OM, exceeds a fifth threshold to trigger an occlusion alarm.
15. The syringe pump according to Para 1, wherein the at least one processor is configured to perform the third test such that the at least one processor determines if: a pressure, P, exceeds a fourth threshold; an occlusion metric, OM, exceeds a fifth threshold; or the pressure, P, minus a first, P₀, of a series of the pressure, P, exceeds a sixth threshold to trigger an occlusion alarm.
16. The syringe pump according to Para 15, wherein the first, P₀, of a series of the pressure, P, is a first measurement of the pressure when the syringe pump enters into the steady-state phase.
17. The syringe pump according to Para 14, wherein the at least one processor is configured to determine the pressure, P, in accordance with: $P = \frac{{\sum }_{i = 0}^{n} \frac{F \left({Θ}_{i}\right)}{A}}{n} ,$ and the average pressure, Pavg, in accordance with: ${P}_{avg} = \frac{{\sum }_{i = 0}^{k} {P}_{i}}{k} .$
18. The syringe pump according to Para 1, wherein the at least one processor is configured to transition from the start-up phase into the steady-state phase when:
   an occlusion metric, OM, exceeds a first value; and
   the occlusion metric, OM, is less than a second value.
19. The syringe pump according to Para 18, wherein the at least one processor is configured to transition from the start-up phase into the stead-state phase when the occlusion metric, OM, exceeds the first value before the occlusion metric, OM, is less than the second value.
20. The syringe pump according to Para 19, wherein the occlusion metric, OM, is the pressure, P, minus an average pressure, Pavg.
21. The syringe pump according to Para 20, wherein the at least one processor is configured to determine, P, in accordance with: $P = \frac{{\sum }_{i = 0}^{n} \frac{F \left({Θ}_{i}\right)}{A}}{n} ,$ and the average pressure, Pavg, in accordance with: ${P}_{avg} = \frac{{\sum }_{i = 0}^{k} {P}_{i}}{k} .$
22. A method for operating a syringe pump, at least partially implemented by at least one processor executing a plurality of instructions configured for execution by the at least one processor, the method comprising:
   priming the syringe pump in a prime phase;
   determining if an occlusion exists during the prime phase using a first test;
   stopping the prime phase;
   initiating fluid delivery into a patient;
   entering into a start-up phase;
   determining if an occlusion exists using a second test during the start-up phase;
   transitioning from the start-up phase into a steady-state phase; and
   determining if an occlusion exists during the steady-state phase using a third test.
23. The method according to Para 22, further comprising receiving user initiation of the prime phase.
24. The method according to Para 22, further comprising determining a force applied to a syringe by actuation of a syringe actuator.
25. The method according to Para 22, further comprising:
   determining a motor position; and
   determining a motor rotation speed.
26. The method according to Para 22, further comprising performing the first test by determining if a pressure, P, exceeds a first threshold to trigger an occlusion alarm.
27. The method according to Para 26, wherein the pressure, P, is determined in accordance with: $P = \frac{{\sum }_{i = 0}^{n} \frac{F \left({Θ}_{i}\right)}{A}}{n} .$
28. The method according to Para 22, further comprising performing the second test by determining if the pressure, P, exceeds a second threshold or an occlusion metric exceeds a third threshold multiplied by a motor speed to trigger an occlusion alarm.
29. The method according to Para 28, wherein the pressure, P, is determined in accordance with: $P = \frac{{\sum }_{i = 0}^{n} \frac{F \left({Θ}_{i}\right)}{A}}{n} .$
30. The method according to Para 28, wherein the occlusion metric, OM, is the pressure, P, minus an average pressure, Pavg.
31. The method according to Para 30, the pressure, P, is determined in accordance with: $P = \frac{{\sum }_{i = 0}^{n} \frac{F \left({Θ}_{i}\right)}{A}}{n} ,$ and the average pressure, Pavg, is determined in accordance with: ${P}_{avg} = \frac{{\sum }_{i = 0}^{k} {P}_{i}}{k} .$
32. The method according to Para 22, further comprising performing the third test by determining if: a pressure, P, exceeds a fourth threshold; an occlusion metric, OM, exceeds a fifth threshold; or the pressure, P, minus a first, P₀, of a series of the pressure, P, exceeds a sixth threshold to trigger an occlusion alarm.
33. The method according to Para 22, further comprising performing the third test by determining if: a pressure, P, exceeds a fourth threshold; an occlusion metric, OM, exceeds a fifth threshold; or the pressure, P, minus a first, P₀, of a series of the pressure, P, exceeds a sixth threshold to trigger an occlusion alarm.
34. The method according to Para 33, wherein the first, P₀, of a series of the pressure, P, is a first measurement of the pressure when the syringe pump enters into the steady-state phase.
35. The method according to Para 33, wherein the pressure, P, is determined in accordance with: $P = \frac{{\sum }_{i = 0}^{n} \frac{F \left({Θ}_{i}\right)}{A}}{n} ,$ and the average pressure, Pavg, is determined in accordance with: ${P}_{avg} = \frac{{\sum }_{i = 0}^{k} {P}_{i}}{k} .$
36. The method according to Para 22, wherein the act of transitioning from the start-up phase into the steady-state phase occurs when:
   an occlusion metric, OM, exceeds a first value; and
   the occlusion metric, OM, is less than a second value.
37. The method according to Para 36, wherein act of transitioning from the start-up phase into the stead-state phase occurs only if the occlusion metric, OM, exceeds the first value before the occlusion metric, OM, is less than the second value.
38. The method according to Para 37, wherein the occlusion metric, OM, is the pressure, P, minus an average pressure, Pavg.
39. The method according to Para 38, wherein the pressure, P, is determined in accordance with: $P = \frac{{\sum }_{i = 0}^{n} \frac{F \left({Θ}_{i}\right)}{A}}{n} ,$ and the average pressure, Pavg, is determined in accordance with: ${P}_{avg} = \frac{{\sum }_{i = 0}^{k} {P}_{i}}{k} .$
40. A method for recovering from an occlusion, the method comprising:
   dispensing fluid into a patient using a syringe pump;
   monitoring the syringe pump;
   determining whether an occlusion associated with the syringe pump exists;
   stopping delivery in response to the determined occlusion;
   determining if a force drops below a predetermined threshold within a predetermined period of time, wherein the predetermined threshold is a predetermined percentage of the difference between a steady state force and the force at the time of the determined occlusion;
   reversing the syringe pump to relieve a pressure within the syringe pump is the syringe pump does not drop below the predetermined threshold within the predetermined period of time; and
   stopping the syringe pump when the slope drops below a predetermined value and then rises above a second predetermined value.
41. A pressure sensor assembly, comprising:
   a plunger having a sensing surface;
   a first pressure sensor operatively coupled to the plunger and configured to estimate a force applied to the sensing surface; and
   a second pressure sensor operatively coupled to the plunger and configured to estimate the force applied to the sensing surface.
42. The assembly according to Para 41, wherein a processor is coupled to the first and second pressure sensors.
43. The assembly according to Para 42, wherein the processor is configured to estimate a magnitude of the force applied to the sensing surface using the first and second pressure sensors.
44. The assembly according to Para 42, wherein the processor is configured to estimate a position on the sensing surface where the force is applied thereto.
45. The assembly according to Para 41, further comprising a guide configured to guide the plunger such that the sensing surface moves one of away from and toward the first and second pressure sensors.
46. The assembly according to Para 45, further comprising a seal disposed over the sensing surface.
47. A syringe pump, comprising:
   a plunger head assembly configured to receive an actuatable portion of a syringe;
   the pressure sensor assembly according to Para 41, the pressure sensor assembly is coupled to the plunger head such that the sensing surface receives the actuatable portion of the syringe.
48. A peristaltic pump, comprising:
   a body configured receive an infusion tube;
   the pressure sensor assembly according to Para 41, the pressure sensor assembly is coupled to the infusion tube such that the sensing surface responds to pressure within the infusion tube.
49. A syringe pump, comprising:
   a plunger head assembly configured to receive an actuatable portion of a syringe;
   a pressure sensor assembly coupled to the plunger head assembly and configured to sense a force applied to the plunger head assembly, the pressure sensor assembly comprising:
      a plunger having a sensing surface configured to receive the force;
      a first pressure sensor operatively coupled to the plunger and configured to estimate the force applied to the sensing surface; and
      a second pressure sensor operatively coupled to the plunger and configured to estimate the force applied to the sensing surface; and
   a processor coupled to the first and second pressure sensors, wherein the processor is configured to estimate a magnitude of the force.
50. The syringe pump according to Para 49, wherein the magnitude of the force is correlated with a pressure within the syringe.
51. The syringe pump according to Para 49, wherein the pressure sensor assembly further includes a seal disposed over the sensing surface of the plunger, wherein the seal is configured to seal the plunger head assembly from fluid ingress.
52. The syringe pump according to Para 49, wherein the plunger head assembly is configured to actuate the syringe and the processor is configured to determine if an occlusion exists using the first and second pressure sensors.
53. The syringe pump according to Para 49, wherein the processor is configured to determine where on the sensing surface the force is applied.
54. The syringe pump according to Para 49, wherein the processor is configured to estimate a magnitude of the force applied to the sensing surface using the first and second pressure sensors.
55. The syringe pump according to Para 49, wherein the processor is configured to estimate a position on the sensing surface where the force is applied thereto.
56. The syringe pump according to Para 49, further comprising a guide configured to guide the plunger such that the sensing surface moves one of away from and toward the first and second pressure sensors.
57. The syringe pump according to Para 49, wherein the sensing surface is elongated along a first direction.
58. The syringe pump according to Para 57, wherein the elongated sensing surface is configured to receive a plurality of syringe sizes loaded into the syringe pump.
59. The syringe pump according to Para 49, wherein the sensing surface is configured to receive a plurality of syringe sizes loaded into the syringe pump
60. A pressure sensor assembly, comprising:
   a plunger comprising:
      a sensing surface, the sensing surface having an elongated portion along a first axis and a width along a second axis, the first and second axes are orthogonal relative to each other, wherein the width is about constant along a substantial portion of elongated portion, wherein the elongated portion of the sensing surface defines first and second ends, wherein the first and second ends are curved;
      a first extension disposed adjacent to or on the first end of the sensing surface and extending along a third axis orthogonal to the first and second axes away from sensing surface; and
      a second extension disposed adjacent to or on the second end of the sensing surface and extending along a fourth axis orthogonal to the first and second axes away from sensing surface, wherein the third and fourth axes are parallel to each other;
   a guide configured to guide the movement of the plunger along the third and fourth axes;
   a first pressure sensor disposed adjacent to an end of the first extension opposite to the sensing surface, the first pressure sensor is configured to sense movement of the plunger within the guide; and
   a second pressure sensor disposed adjacent to an end of the second extension opposite to the sensing surface, the second pressure sensor is configured to sense movement of the plunger within the guide.
61. The pressure sensor assembly according to Para 60, further comprising a seal at least partially disposed over at least a portion of the sensing surface.
62. The pressure sensor assembly according to Para 60, further comprising a brace operatively coupled to the first and second extensions.

## Claims

1. A pressure sensor assembly, comprising:
a plunger having a sensing surface;
a first pressure sensor operatively coupled to the plunger and configured to estimate a force applied to the sensing surface; and
a second pressure sensor operatively coupled to the plunger and configured to estimate the force applied to the sensing surface

2. The pressure sensor assembly according to claim 1, wherein a processor is coupled to the first and second pressure sensors.

3. The pressure sensor assembly according to claim 2, wherein the processor is configured to estimate a magnitude of the force applied to the sensing surface using the first and second pressure sensors.

4. The pressure sensor assembly according to claim 2 or 3, wherein the processor is configured to estimate a position on the sensing surface where the force is applied thereto.

5. The pressure sensor assembly according to any preceding claim, further comprising a guide configured to guide the plunger such that the sensing surface moves one of away from and toward the first and second pressure sensors.

6. The pressure sensor assembly according to any preceding claim, further comprising a seal disposed over the sensing surface

7. The pressure sensor assembly according to any preceding claim, wherein the sensing surface has an elongated portion along a first axis and a width along a second axis, wherein the first and second axes are orthogonal relative to each other, wherein the elongated portion of the sensing surface defines first and second ends, wherein the plunger further comprises a first extension disposed adjacent to or on the first end of the sensing surface and extending along a third axis orthogonal to the first and second axes away from sensing surface, wherein the third axis is a longitudinal axis of the first extension, wherein the plunger further comprises a second extension disposed adjacent to or on the second end of the sensing surface and extending along a fourth axis orthogonal to the first and second axes away from sensing surface, wherein the fourth axis is a longitudinal axis of the second extension, wherein the third and fourth axes are parallel to each other.

8. The pressure sensor assembly according to claim 7, further comprising a brace operatively coupled to the first and second extensions.

9. The pressure sensor assembly according to claim 8 when appended to claim 5, wherein the brace is planar, wherein the plunger is received by a slot in the guide, wherein a portion of the slot receiving the brace is planar.

10. The pressure sensor assembly according to claim 8 or 9, wherein the brace extends away from the sensing surface, wherein the first extension extends along the third axis beyond the brace, wherein the second extension extends along the fourth axis beyond the brace, wherein the first extension extends along the third axis by the same extent to which the second extension extends along the fourth axis.

11. The pressure sensor assembly according to any of claims 7 to 10, wherein ends of the first and second extensions are rounded.

12. The pressure sensor assembly according to any of claims 7 to 11 when appended to claim 5, wherein the first extension and the second extension have cylindrical outer surfaces, wherein a first internal surface of the guide that receives the first extension is cylindrical, wherein a second internal surface of the guide that receives the second extension is cylindrical.

13. The pressure sensor assembly according to any of claims 7 to 12, wherein the first pressure sensor is disposed adjacent to an end of the first extension opposite to the sensing surface, wherein the second pressure sensor is disposed adjacent to an end of the second extension opposite to the sensing surface

14. A syringe pump, comprising:
a plunger head assembly configured to receive an actuatable portion of a syringe;
the pressure sensor assembly according to any preceding claim, wherein the pressure sensor assembly is coupled to the plunger head such that the sensing surface receives the actuatable portion of the syringe

15. A peristaltic pump, comprising:
a body configured receive an infusion tube;
the pressure sensor assembly according to any preceding claim, wherein the pressure sensor assembly is coupled to the infusion tube such that the sensing surface responds to pressure within the infusion tube.
